# EUROPEAN PATENT SPECIFICATION

(11) **EP 1 763 509 B1**
(45) Date of publication and mention of the grant of the patent: **21.02.2018**
(21) Application number: 05756678.8
(22) Date of filing: 01.07.2005
(51) Int. Cl.: C07C 275/50, C07C 275/54, A61K 31/17, A61P 35/00, A61P 35/02, A61P 29/00, C07D 333/60, C07C 311/51, C07C 317/50, C07C 323/25, C07C 323/44, C07D 209/20, C07D 295/185, C07D 213/56

(54) **ALPHA-HELICAL MIMETICS**
ALPHA-HELIX-MIMETIKA
MIMÉTIQUES D'HÉLICE ALPHA

(30) Priority: 02.07.2004 US 584473 P
(43) Date of publication of application: 21.03.2007
(73) Proprietor: THE WALTER AND ELIZA HALL INSTITUTE OF MEDICAL RESEARCH, Parkville, VIC 3052 (AU)
(72) Inventor: LESSENE, Guillaume, Laurent, Coburg, Victoria 3058 (AU); BAELL, Jonathan, Ivanhoe, Victoria 3079 (AU)
(74) Representative: Matthews, Derek Peter
(86) International application number: PCT/AU2005/000968
(87) International publication number: WO 2006/002474

(56) References cited:
- US-A- 4 016 282
- US-A1- 2003 195 209
- DRIVER M J ET AL: 'further studies on bisnorpenicillins' JOURNAL OF ANTIBIOTICS vol. 38, no. 4, 1985, pages 550 - 553, XP008116185
- NOBUHIRO O ET AL: 'Smisynthetic beta lactam antibiotics IV. Synthesis and antibacteria activity of new ureidocepalosporin and ureidcephamycin derivatives containing a catechol moiety or its acetate' CHEMICAL & PHARMACEUTICAL BULLETIN vol. 35, no. 5, 1987, pages 1903 - 1909, XP008115997
- NOBUHIRO O ET AL: 'Semi synthetic beta-lactam antibiotics I.synthesis and antibacterial activity of new ureidopenicillin derivatives having catechol moieties' JOURNAL OF ANTIBIOTICS vol. 39, no. 2, 1986, pages 230 - 241, XP008116186
- DATABASE WPI Week 198045, 20 September 1980 Derwent Publications Ltd., London, GB; Class B05, AN 1980-79635C, XP008138544 & JP 55 122754 (CHUGAI PHARMACEUTICAL)
- LAURENT DUCRY ET AL: "Antibody-Drug Conjugates: Linking Cytotoxic Payloads to Monoclonal Antibodies", BIOCONJUGATE CHEMISTRY, vol. 21, no. 1, 20 January 2010 (2010-01-20), pages 5-13, XP055000588, ISSN: 1043-1802, DOI: 10.1021/bc9002019
- HIROSHI MAEDA ET AL: "CONJUGATES OF ANTICANCER AGENTS AND POLYMERS: ADVANTAGES OF MACROMOLECULAR THERAPEUTICS IN VIVO", BIOCONJUGATE CHEMISTRY,, vol. 3, no. 5, 1 September 1992 (1992-09-01), pages 351-362, XP000300789, ISSN: 1043-1802, DOI: 10.1021/BC00017A001
- PATANI G A ET AL: "BIOISOSTERISM: A RATIONAL APPROACH IN DRUG DESIGN", CHEMICAL REVIEWS, AMERICAN CHEMICAL SOCIETY, US, vol. 96, no. 8, 1 January 1996 (1996-01-01), pages 3147-3176, XP000652176, ISSN: 0009-2665, DOI: 10.1021/CR950066Q

## Description

### Field of the Invention

The invention relates generally to compounds that mimic alpha-helical sequences of peptides and proteins, to compositions containing them and to their use. In particular, the invention relates to compounds that mimic the alpha-helical sequences of BH3-only proteins and are capable of binding to and neutralising pro-survival Bcl-2 proteins. The disclosure also relates to processes of preparing the compounds that mimic alpha-helical portions of peptides and proteins, and to the use of such compounds in the regulation of cell death and the treatment and/or prophylaxis of diseases or conditions associated with the deregulation of cell death.

### Background of the Invention

Bibliographical details of various publications referred to in this specification are collected at the end of the description.

The reference to any prior art in this specification is not, and should not be taken as, an acknowledgment or any form of suggestion that that prior art forms part of the common general knowledge in Australia.

Apoptosis is now recognized as an essential biological process in the tissue homeostasis of all living species [Kaufmann and Hengartner, 2001]. In mammals in particular, it has been shown to regulate embryonic development. Later in life, cell death is a default mechanism that removes potentially dangerous cells (e.g. cells carrying cancerous defects). Several apoptotic pathways have been uncovered and one of the most important involves the Bcl-2 family of proteins [Cory and Adams, 2002]. The structural homology domains BH1 to BH4 are characteristic of this family. Further classification into of three subfamilies depends on how many of these homology domains a protein contains and on its biological activity (pro- or anti-apoptotic).

The first subgroup contains proteins having all 4 homology domains BH1 to BH4. Their general effect is anti-apoptotic thus preserving the cell from starting a cell death process. Proteins such as Bcl-2, Bcl-w and Bcl-x_{L} are members of this first subgroup. Proteins belonging to the second subgroup have a pro-apoptotic effect and contain the three homology domains BH1 to BH3. The two main representative proteins of this second subgroup are Bax and Bak. Finally, the third subgroup is composed of protein containing only the BH3 domain and members of this subgroup are usually referred to as "BH3-only proteins". Their biological effect on the cell is pro-apoptotic. Bim, Bad, Bmf, and Bid are examples of this third subfamily of proteins.

The delicate balance between the three subgroups is the key to homeostasis of the cells. Recent studies have tried to elucidate the mechanisms involving the Bcl-2 family of proteins that allow a cell to undergo programmed cell death upon receiving intra- or extracellular signal. Such a signal induces the activation (post translational or transcriptional) of BH3 only proteins. These proteins are the primary inducers of the cascade that leads to cell death. The BH3-only proteins mainly interact with the Bcl-2 subgroup and stop proteins such as Bcl-2, Bcl-x_{L} or Bcl-w from inhibiting the Bax/Bak subgroup. These later proteins are either already anchored to the mitochondrial membrane or migrate to this membrane. Their activation leads to membrane swelling, release of cytochrome C and downstream activation of effector caspases.

As already mentioned the balance between these proteins is essential to the correct cellular response to various stimuli. Any perturbation of this balance will instigate or worsen major diseases. Thus apoptosis perturbations have been shown to be at the origin of important diseases such as neurodegenerative conditions (up-regulated apoptosis [Bouillet *et*. *al*., 2001]) for example, Alzheimer's disease, or proliferative diseases (down-regulated apoptosis [Cory and Adams, 2002]) for example, cancer and autoimmune diseases.

The discovery that several proteins of the Bcl-2 family are involved in the onset of cancerous malignancy has unveiled a completely novel way of targeting this still elusive disease [Baell and Huang, 2002]. It has been shown in particular that pro-survival proteins such as Bcl-2 are over-expressed in many cancer types (see Table 1) [Zhang, 2002]. The effect of this deregulation is the survival of altered cells which would have undergone apoptosis in normal conditions. The repetition of these defects associated with unregulated proliferation is thought to be the starting point of cancerous evolution [Green and Evan, 2002]. In other experiments, results have shown that BH3-only proteins can act as tumor suppressors when expressed in diseased animals [Egle *et. al*., 2003].

**TABLE 1: Bcl-2 over-expression in cancer**

| **Cancer type** | **Bcl-2 over-expression** |
|---|---|
| Hormone-refractory prostate cancer | 90-100% |
| Malignant melanoma | 90% |
| Oestrogen-receptor-positive breast cancer | 80-90% |
| Non-Hodgkin's lymphoma | 50% |
| Colon Cancer | 30-50% |
| Chronic lymphocytic leukaemia | 25-50% |

These findings as well as numerous others have made possible the emergence of new concept in anti-cancer strategies and drug discovery. Indeed, if an entity mimicking the effect of BH3-only proteins were able to enter the cell and overcome the pro-survival protein over-expression, it could be possible to reset the apoptotic process [Baell and Huang, 2002]. This strategy presents several advantages, it does not involve the use of DNA damaging agents that are prescribed in classical chemotherapies therefore avoiding undesirable side effects, and it would also alleviate the problem of drug resistance which is usually a consequence of apoptotic deregulation (abnormal survival).

A considerable effort has been made to understand the structural details of the key interactions between BH3-only proteins and the pro-survival subgroup. Fesik and co-workers have demonstrated in the case of the dimer Bad/ Bcl-x_{L} the importance of some structural elements [Muchmore *et. al*., 1996; Sattler *et. al*., 1997 and Petros *et. al*., 2000]:
- Binding occurs between a hydrophobic groove located on Bcl-x_{L} and the BH3 domain of Bad.
- The BH3-only protein Bad adopts a helix structure upon binding to the hydrophobic groove of Bcl-x_{L}.
- Four hydrophobic amino-acids of the BH3 domain located at i, i+3, i+7 and i+11 intervals are essential to the binding of Bad to Bcl-x_{L} and interact in four hydrophobic pockets situated in the Bcl-x_{L} binding groove. Moreover, studies of members of the BH3-only subgroups have shown that these four hydrophobic amino-acids are conserved through the subgroup.

Recently the structure of the pro-survival protein Bcl-w [Hinds *et. al*., 2003] and the structure of BH3-only protein Bim in interaction with Bcl-x_{L} [Liu *et. al*., 2003] have been published. This latter structure confirms the findings of the Bad/Bcl-x_{L} interaction.

A potential target for new drug therapy is small molecules that mimic the interaction between a BH3-only protein and the Bcl-2 family of proteins.

The alpha-helix is a common recognition motif displayed in peptides and proteins. Alpha-helical sequences are often involved in protein-protein interactions, such as enzyme-receptor and antibody-receptor interactions. Targeting these protein-protein interactions is now recognised as one of the major challenges in drug discovery.

One of the difficulties with the development of drug candidates is that short peptide sequences, that are alpha-helical when part of a protein structure, do not necessarily maintain their alpha-helical conformation when isolated from the protein. Furthermore, peptide sequences are often not suitable drug candidates as they readily undergo hydrolysis under biological conditions and upon exposure to proteolytic enzymes making it difficult to deliver them to the desired site of action.

Small molecules that mimic alpha-helical peptide sequences and act as scaffolds for placing substituents in positions that simulate the side chains of amino acids in alpha-helical sequences in proteins are potential drug candidates.

One such small molecule alpha-helical peptidomimetic scaffold is the terephthalamide scaffold developed by Hamilton and co-workers (Yin and Hamilton, 2004). The terephthalamide scaffold was able to provide substituents that mimic the i, i+3 and i+7 side chains of an alpha-helical sequence. However, the terephthalamide scaffolds require a complex multistep synthesis and are not readily adapted to the easy preparation of analogues.

US 2003/0195209 describes imidazopyridine compounds having immunomodulatory activity effective for treating viral and neoplastic diseases. They are not mentioned as an alpha-helical peptidomimetic scaffold.

There is a need for small molecule scaffolds which may be easily synthesised with a versatile array of substituents and which mimic the alpha-helical sequences of proteins.

### Summary of the Invention

The present invention is predicated in part on the discovery that benzoylurea derivatives provide an alpha-helical peptidomimetic scaffold which is able to interact with a Bcl-2 protein. Benzoylurea derivatives are well known in the art and are disclosed in US 4016282, for example. However, the present discovery has been reduced to practice in novel compounds, compositions containing them and in methods for their preparation and use, as described hereafter.

### Detailed Description of the Invention

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. The invention is solely defined by the appended claims. In a first aspect of the invention, there is provided a compound of formula (I): 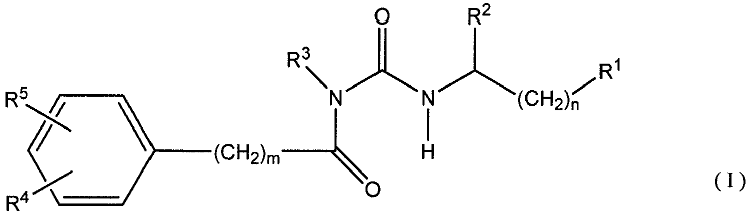 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 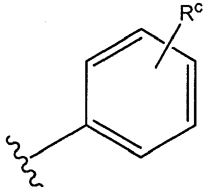 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂-₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)pCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ, heteroarylSO₂(CH₂)ₚ-, heterocyclylSO₂(CH₂)ₚ, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen;
and pharmaceutically acceptable salts and prodrugs selected from N-α-acyloxy amides, N-(acyloxyalkoxycarbonyl)amines and α-acyloxyalkyl esters of alcohols and phenols; with the proviso that when (i) R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.
In a second aspect of the invention, there is provided a compound of formula (Ia): 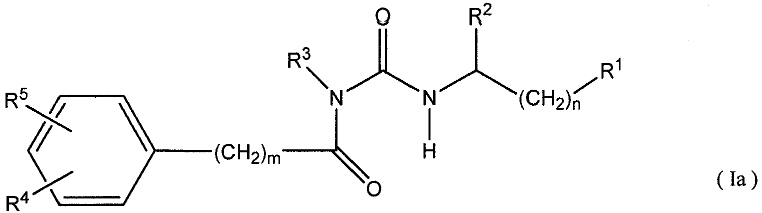 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, -(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 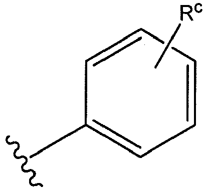 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, cycloalkenyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)pCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁-₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is 2 4 5 3 COOH, R is C₆H₅-CH₂S-CH₂-, R is 3-C₆H₅ and R is H, R is not
CH₃CH₂- and (ii) when R is CH₃CH(CH₃)CH₂SCH₂, R is 3-phenethynyl, R is COOH, m and n are 0 and R⁵ is H, R³ is not n-propyl.
In another aspect of the invention, there is provided a compound of formula (Ib): 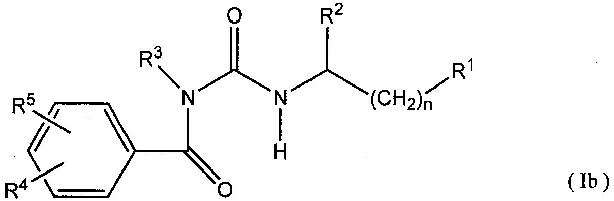 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 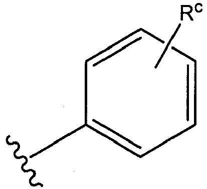 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ, C₂₋₆alkenyloxy(CH₂)ₚ-, C₂₋₆alkynyloxy(CH₂)ₚ, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ- , -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R₁ is COOH, n is 0 and R⁵ is H, R³ is not n-propyl.
In yet another aspect of the invention, there is provided a compound of formula (Ic): 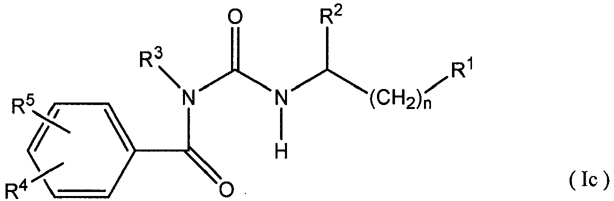 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 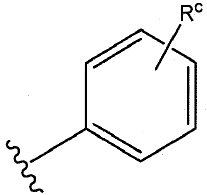 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, cycloalkenyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂,-(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R is C₆H₅-CH₂S-CH₂-, R is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
In a further aspect of the invention, there is provided a compound of formula (Id): 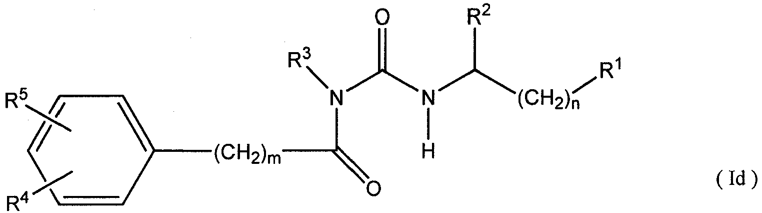 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 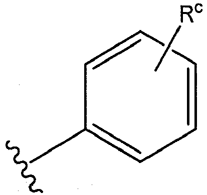 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, _(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is CO₂H, m and n are 0 and R⁵ is H, R³ is not n-propyl.

As used herein, the term "alkyl" refers to a straight-chain or branched saturated hydrocarbon group and may have a specified number of carbon atoms. For example, C₁-C₆ as in "C₁-C₆alkyl" includes groups having 1, 2, 3, 4, 5 or 6 carbons in a linear or branched arrangement. Examples of suitable alkyl groups include, but are not limited to, methyl, ethyl, *n*-propyl, *i*-propyl, *n*-butyl, *i*-butyl, *t*-butyl, *n*-pentyl, 2-methylbutyl, 3-methylbutyl, 4-methylbutyl, *n*-hexyl, 2-methylpentyl, 3-methylpentyl, 4-methylpentyl, 5-methylpentyl, 2-ethylbutyl and 3-ethylbutyl.

As used herein, the term "alkenyl" refers to a straight-chain or branched hydrocarbon group having one or more double bonds between carbon atoms and may have a specified number of carbon atoms. For example, C₂-C₆ as in "C₂-C₆alkenyl" includes groups having 2, 3, 4, 5 or 6 carbon atoms in a linear or branched arrangement. Examples of suitable alkenyl groups include, but are not limited to, ethenyl, propenyl, isopropenyl, butenyl, pentenyl and hexenyl.

As used herein, the term "alkynyl" refers to a straight-chain or branched hydrocarbon group having one or more triple bonds between carbon atoms, and may have a specified number of carbon atoms. For example, C₂-C₆ as in "C₂-C₆alkynyl" includes groups having 2, 3, 4, 5 or 6 carbon atoms in a linear or branched arrangement. Examples of suitable alkynyl groups include, but are not limited to, ethynyl, propynyl, butynyl, pentynyl and hexynyl.

The term "cycloalkyl" as used herein, refers to cyclic hydrocarbon groups and may have a specified number of carbon atoms. For example, C₃-C₁₀ as in "C₃-C₁₀cycloalkyl" includes groups having 3, 4, 5, 6, 7, 8, 9 or 10 carbon atoms in the hydrocarbon ring. Examples of suitable cycloalkyl groups include, but are not limited to, cyclopropyl, cyclobutyl, cyclopentyl and cyclohexyl.

The term "cycloalkenyl" as used herein, refers to cyclic hydrocarbon groups having one or more double bonds. Suitable cycloalkenyl groups include, but are not limited to, cyclopentenyl, cyclohexenyl, cyclohexa-1,3-dienyl and cyclohexa-1,4-dienyl.

As used herein the term "halo" or "halogen" refers to fluorine (fluoro), chlorine (chloro), bromine (bromo) and iodine (iodo).

The terms "alkyloxy", "alkenyloxy", "alkynyloxy" and "cycloalkoxy" as used herein represent an alkyl, alkenyl, alkynyl or cycloalkyl group as defined above attached through an oxygen bridge. Examples of suitable alkyloxy, alkenyloxy, alkynyloxy and cycloalkoxy groups include, but are not limited to, methoxy, ethoxy, *n*-propyloxy, *n-*butyloxy, *n*-pentyloxy, *n*-hexyloxy, ethenyloxy, propenyloxy, butenyloxy, pentenyloxy, hexenyloxy, ethynyloxy, propynyloxy, butynyloxy, pentynyloxy, hexynyloxy, cyclopropyloxy, cyclobutyloxy, cyclopentyloxy and cyclohexyloxy.

The terms "alkylthio", "alkenylthio", "alkynylthio" and "cycloalkylthio" as used herein represent an alkyl, alkenyl, alkynyl or cycloalkyl group as defined above attached through a sulfur bridge. Examples of suitable alkylthio, alkenylthio, alkynylthio and cycloalkylthio include, but are not limited to, methylthio, ethylthio, propylthio, butylthio, pentylthio, hexylthio, ethenylthio, propenylthio, butenylthio, pentenylthio, hexenylthio, ethynylthio, propynylthio, butynylthio, pentynylthio, hexynylthio, cyclopropylthio, cyclobutylthio, cyclopentylthio and cyclohexylthio.

As used herein, the term "aryl" is intended to mean any stable, monocyclic or bicyclic carbon ring of up to 7 atoms in each ring, wherein at least one ring is aromatic. Examples of such aryl groups include, but are not limited to, phenyl, naphthyl, tetrahydronaphthyl, indanyl, biphenyl and binaphthyl.

The term "heterocyclyl" as used herein is intended to mean a 3- to 10-membered nonaromatic heterocycle containing from 1 to 4 heteroatoms selected from the group consisting of O, N and S, and includes bicyclic groups.

The term "heteroaryl" as used herein, represents a stable monocyclic or bicyclic ring of up to 7 atoms in each ring, wherein at least one ring is aromatic and at least one ring contains from 1 to 4 heteroatoms selected from the group consisting of O, N and S. Heteroaryl groups within the scope of this definition include, but are not limited to, acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline.

Further examples of "heterocyclyl" and "heteroaryl" include, but are not limited to, the following: benzoimidazolyl, benzofuranyl, benzofurazanyl, benzopyrazolyl, benzotriazolyl, benzothiophenyl, benzoxazolyl, benzopyrrolyl, carbazolyl, carbolinyl, cinnolinyl, furanyl, imidazoyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrazolyl, tetrazolopyridyl, thiadiazolyl, thiazolyl, thienyl, triazolyl, azetidinyl, aziridinyl, 1,4-dioxanyl, hexahydroazepinyl, piperazinyl, piperidinyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof. Attachment of a heterocyclyl substituent can occur via a carbon atom or via a heteroatom.

The term "amino acid side chain" as used herein includes the α-R group of a naturally occurring α-amino acid and is selected from -CH₃, -(CH₂)₃NHC(=NH)NH₂, -CH₂CONH₂, -CH₂CO₂H, -CH₂SH, -(CH₂)₂CO₂NH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl) and -CH(CH₃)₂. Disclosed are the α-R groups of non-naturally occurring α-amino acid such as those found in homoarginine, homoserine, homocysteine, norvaline, norleucine or amidino derivatives. Further the α-side chains are selected from -(CH₂)₄NHC(=NH)NH₂, -(CH₂)₂OH, -(CH₂)₂SH, -CH₂CH₂CH₃, -(CH₂)₃CH₃, or (CH₂)ᵥC(=NH)NH₂ where v is an integer from 1 to 4. Other derivatives may include α-side chains in which carboxy, hydroxy, thiol or amino groups are protected with suitable carboxy, hydroxy, thiol or amino protecting groups (see "Protective Groups in Organic Synthesis" Theodora Greene and Peter Wuts, third edition, Wiley Interscience, 1999).

Each alkyl, alkenyl, alkynyl, cycloalkyl, cycloalkenyl, aryl, heterocyclyl and heteroaryl may be optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen. Examples of suitable substituents include, but are not limited to, methyl, ethyl, propyl, isopropyl, butyl, *sec*-butyl, *tert*-butyl, vinyl, methoxy, ethoxy, propoxy, isopropoxy, butoxy, methylthio, ethylthio, propylthio, isopropylthio, butylthio, hydroxy, hydroxymethyl, hydroxyethyl, hydroxypropyl, hydroxybutyl, fluoro, chloro, bromo, iodo, cyano, nitro, CO₂H, CO₂CH₃, CH₂CO₂CH₃, trifluoromethyl, trifluoromethoxy, trifluoromethylthio, acetyl, amino, methylamino, dimethylamino, phenyl, phenylcarbonyl, -N=NHC(O)NH₂, -CH=C(CN)₂ and phenoxy. Preferred substituents include fluoro, chloro, methyl, ethyl, propyl, isopropyl, butyl, *tert*-butyl, methoxy, ethoxy, propoxy, isopropoxy, trifluoromethyl, trifluoromethoxy, cyano, acetyl, amino, methylamino and dimethylamino.

The compounds of the invention may be in the form of pharmaceutically acceptable salts. Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

It will also be recognised that many compounds of the invention possess asymmetric centres and are therefore capable of existing in more than one stereoisomeric form. The invention thus also relates to compounds in substantially pure isomeric form at one or more asymmetric centres eg., greater than about 90% ee, such as about 95% or 97% ee or greater than 99% ee, as well as mixtures, including racemic mixtures, thereof. Such isomers may be prepared by asymmetric synthesis, for example using chiral intermediates, or by chiral resolution.

The term "carboxylic acid or carboxylate bioisostere" refers to a group which is physiochemically or topologically similar to carboxylic acid. Carboxylic acid or carboxylate bioisosteres are selected from tetrazole, tetrazolate, oxidiazole, acylsulfonamides such as optionally substituted alkyl or optionally substituted aryl acylsulfonamides, especially optionally substituted benzene acylsulfonamides, phosphate (PO₃H₂) and sulfonic acid (SO₃H) [See Patani and LaVoie, 1996].

The term "prodrug" indicates a group which is selected from N-α-acyloxy amides, N-(acyloxyalkoxy carbonyl) amine derivatives and α-acyloxyalkyl esters of phenols and alcohols. A prodrug may include modifications to one or more of the functional groups of a compound of the invention. Disclosed is the use of fusion proteins or peptides comprising cell-permeant proteins or peptides and the compounds of the invention. Such fusion proteins or peptides allow the translocation of the compounds of the invention across a cellular membrane and into a cell cytoplasm or nucleus. Examples of such cell-permeant proteins and peptides include membrane permeable sequences, cationic peptides such as protein transduction domains (PTD), eg: antennapedia (penetratin), tat peptide, R7, R8 and R9 and other drug delivery systems. (see Dunican and Doherty, 2001, Shangary and Johnson, 2002; Letai *et. al*., 2002; Wang *et. al*., 2000, Schimmer *et. al*., 2001; Brewis *et. al*., 2003; Snyder *et. al*., 2004). Disclosed is also the combination of lipids with the compounds of the invention. The presence of lipids may assist in the translocation of the compounds across a cellular membrane and into a cell cytoplasm or nucleus. Suitable lipids include fatty acids which may be linked to the compound by formation of a fatty acid ester. Preferred fatty acids include, but are not limited to, lauric acid, caproic acid, palmitic acid and myristic acid.

The phrase "a derivative which is capable of being converted *in vivo"* as used in relation to another functional group includes all those functional groups or derivatives which upon administration into a mammal may be converted into the stated functional group. Those skilled in the art may readily determine whether a group may be capable of being converted *in vivo* to another functional group using routine enzymatic or animal studies.

In preferred embodiments at least one of the following applies:
R¹ is CO₂H, tetrazole, tetrazolate or an optionally substituted benzene acylsulfonamide, especially CO₂H;
R² is R^{a}-(CHR')ₓ-A-(CH₂)_{y}-, wherein R^{a} is H, optionally substituted cycloalkyl or optionally substituted aryl, x is 0 or 1 to 4, R' is H or C₁₋₃alkyl, A is O, S or SO and y is 1 to 3, or R^{a} is optionally substituted aryl or optionally substituted heteroaryl, R' is H, the sum of x and y is 1 to 4 and A is a covalent bond. In some embodiments R² is R^{a}-(CH₂)ₓ-A-(CH₂)_{y}- wherein R^{a} is aryl, x is 1 to 3, y is 1 to 3 and A is selected from O, S and SO; or R^{a} is aryl, A is a covalent bond, and the sum of x and y is 1 to 4;
R³ is C₁₋₆alkyl, optionally substituted cycloalkyl or a group R^{d}-(CH₂)ₚ-W-(CH₂)_{q}- in which R^{d} is optionally substituted cycloalkyl, optionally substituted aryl, optionally substituted heterocyclyl or optionally substituted heteroaryl, W is a covalent bond and the sum of p and q is 1 to 3; or R^{d} is H, W is O or S and the sum of p and q is 2 to 4, especially preferred is C₃₋₆alkyl, benzyl and cyclohexylmethyl; and when R^{d} is aryl, the aryl is preferably optionally substituted with one or more halogen, C₁₋₆alkyl or C₁₋₆alkoxy groups.
R⁴ is 3- or 4-aryl, aryl(C₁₋₃alkyl)-, aryl(C₂₋₃alkenyl) or aryl(C₂₋₃alkynyl) wherein aryl is optionally substituted with one or more halogen, C₁₋₆alkyl, C₁₋₆alkoxy groups, trifluoromethyl groups, hydroxy(C₁₋₆alkyl), CN or C₁₋₆acyl, especially optionally substituted 3- or 4-phenyl, naphthyl or phenyl(ethynyl), more especially optionally substituted 3-phenyl, naphthyl or phenyl(ethynyl); or R⁴ is C₁₋₆alkyl, C₂₋₆alkenyl or C₂₋₆alkynyl.
R⁵ is hydrogen, halogen, methyl or methoxy, especially hydrogen;
m is 0; and n is 0.

Especially preferred compounds are those of formula (II): 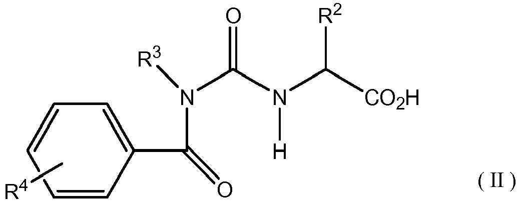 wherein R² and R³ are defined as for formulae (I) or (Ib) above and R⁴ is a 3-phenyl, 3-(2-naphthyl), 3-(1-naphthyl), 3-benzyl, 4-phenyl, 4-benzyl group, 3-(phenylethynyl) or 4-(phenylethynyl), wherein each phenyl, naphthyl or benzyl group is optionally substituted with one or more substituents selected from C₁₋₆alkyl, C₂₋₆alkenyl, C₂₋₆alkynyl, C₁₋₆alkoxy, C₂₋₆alkenyloxy and halogen; or R⁴ is C₁₋₆alkyl, C₂₋₆alkenyl or C₂₋₆alkynyl; and pharmaceutically acceptable salts or prodrugs selected from N-α-acyloxy amides, N-(acyloxyalkoxycarbonyl)amines and α-acyloxyalkyl esters of alcohols and phenols, with the proviso than (i) when R² is C₆H₅-CH₂S-CH₂- and R⁴ is 3-phenyl, R³ is not ethyl and (ii) when R² is CH₃CH(CH₃)CH₂SCH₂ and R⁴ is 3-phenethynyl, R³ is not n-propyl or a compound of formula (IIa): 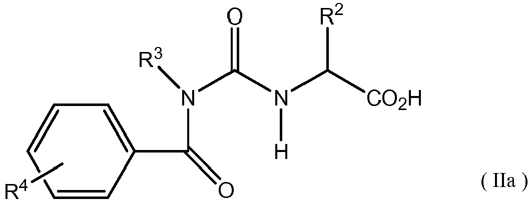
wherein R² is defined as for formulae (I) or (Ib) above;
R³ is selected from C₃₋₆alkyl, C₂₋₆alkenyl, C₂₋₆alkynyl, cycloalkyl, aryl, heterocyclyl, heteroaryl and a group

   R^{d}-(CH₂)ₚ-W-(CH₂)_{q}-

   wherein W is selected from a covalent bond, O, S or NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is a 3-phenyl, 3-(2-naphthyl), 3-(1-naphthyl), 3-benzyl, 4-phenyl, 4-benzyl group, 3-(phenylethynyl) or 4-(phenylethynyl), wherein each phenyl, naphthyl or benzyl group is optionally substituted with one or more substituents selected from C₁₋₆alkyl, C₂₋₆alkenyl, C₂₋₆alkynyl, C₁₋₆alkoxy, C₂₋₆alkenyloxy and halogen; or R⁴ is C₁₋₆alkyl, C₂₋₆alkenyl or C₂₋₆alkynyl; and pharmaceutically acceptable salts or prodrugs selected from N-α-acyloxy amides, N-(acyloxyalkoxycarbonyl)amines and α-acyloxyalkyl esters of alcohols and phenols, with the proviso that (i) when R² is C₆H₅-CH₂S-CH₂- and R⁴ is 3-C₆H₅, R³ is not CH₃CH₂- and (ii) when R² is CH₃CH(CH₃)CH₂SCH₂ and R⁴ is 3-phenethynyl, R³ is not n-propyl. Preferred compounds of the disclosure include:

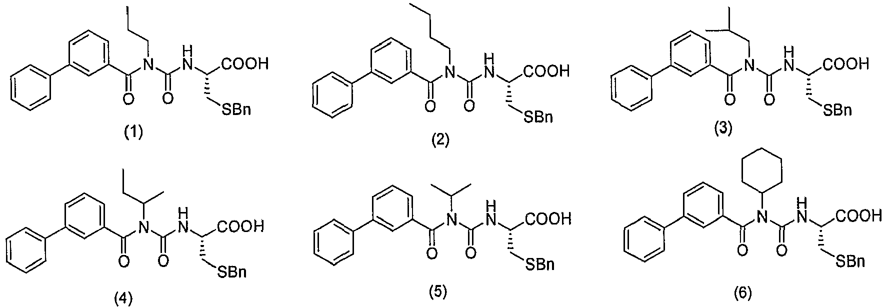

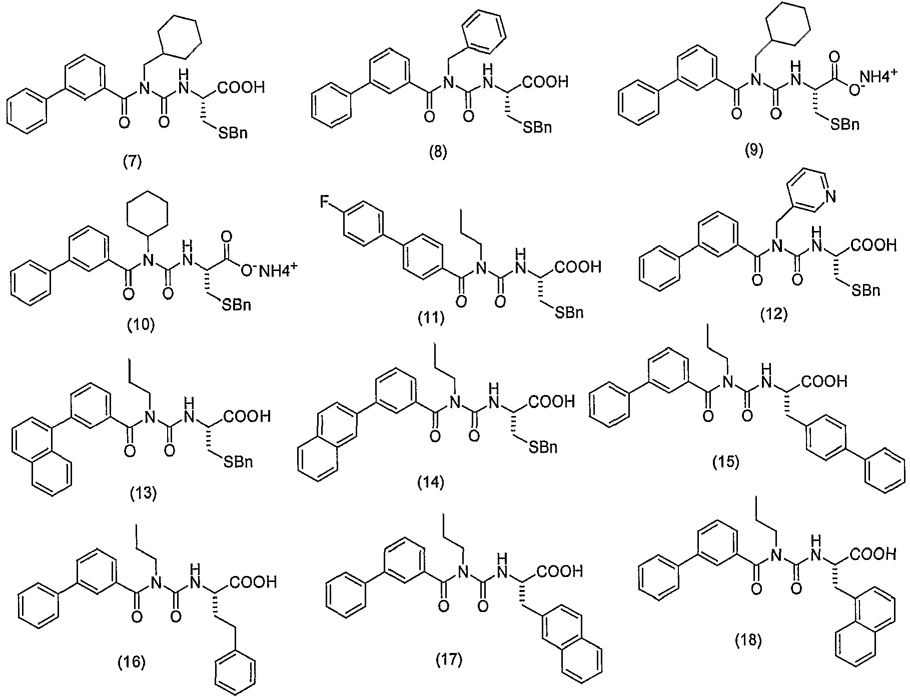

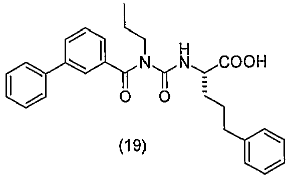

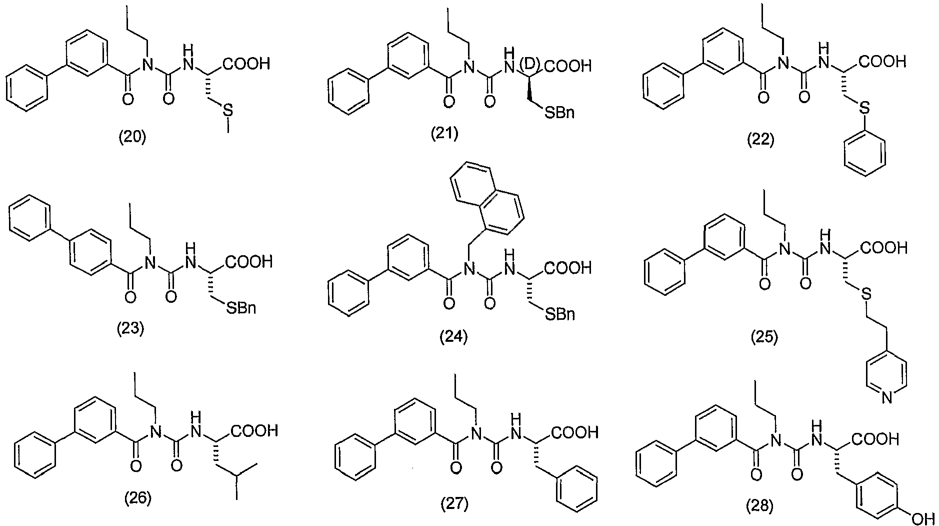

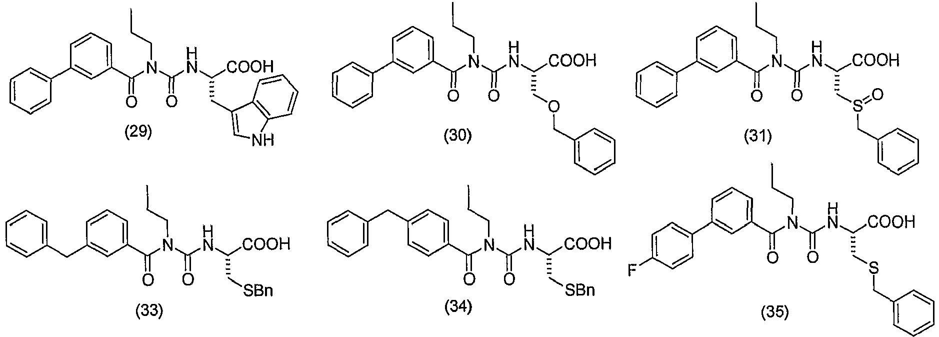

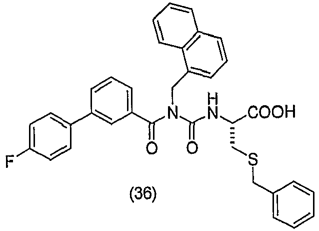

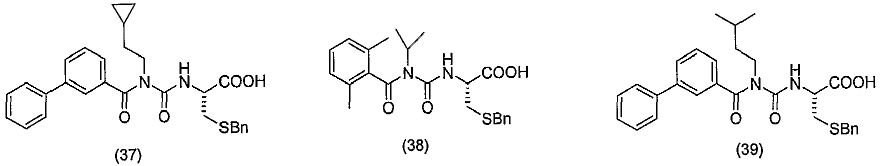

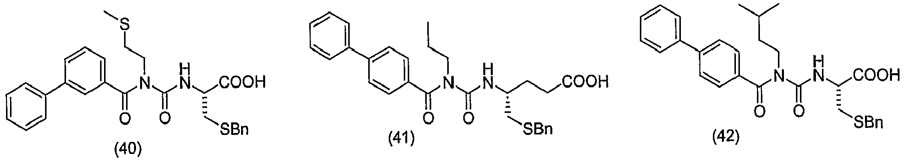

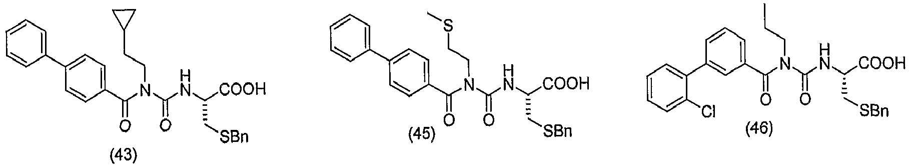

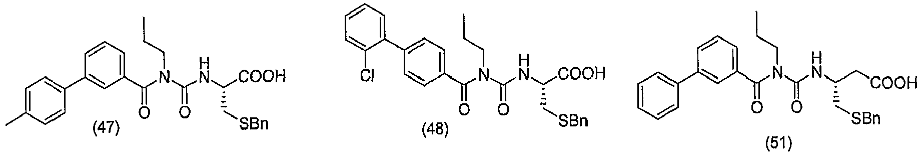

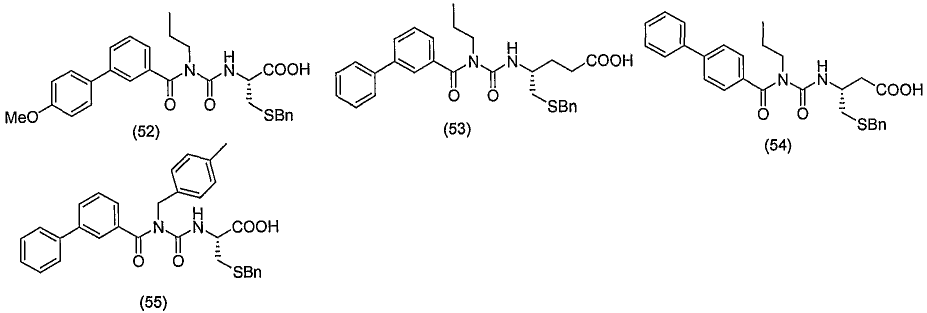

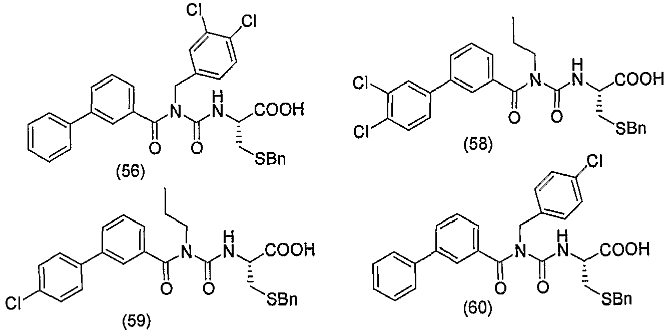

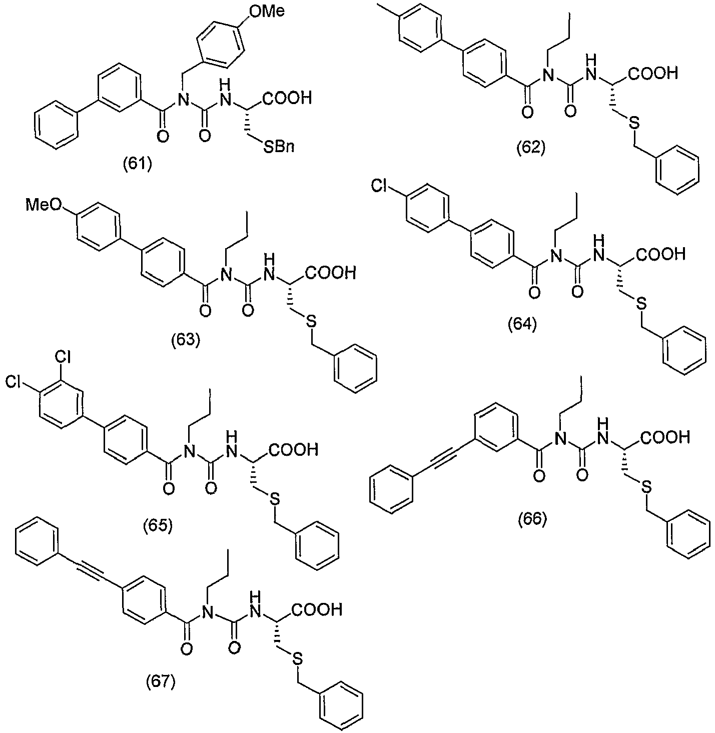

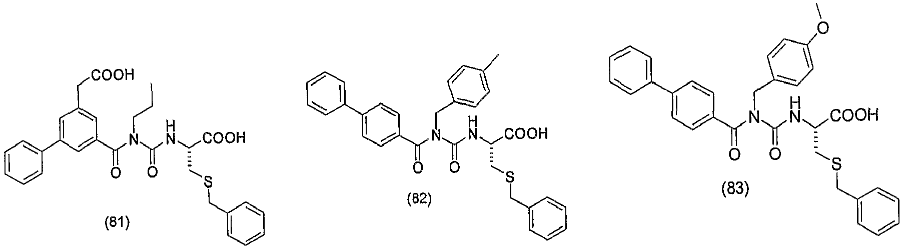

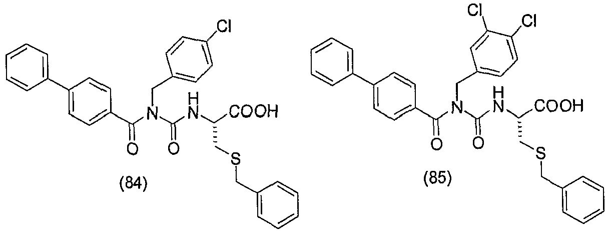

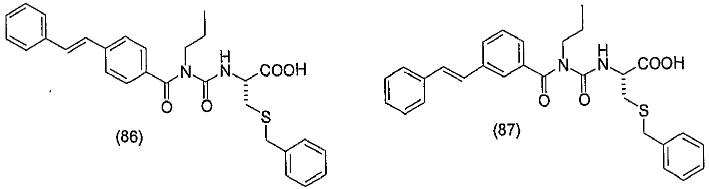

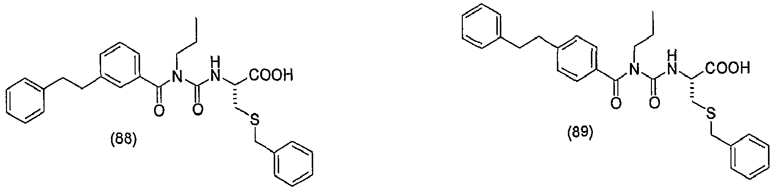

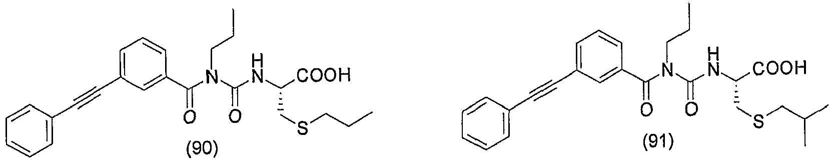

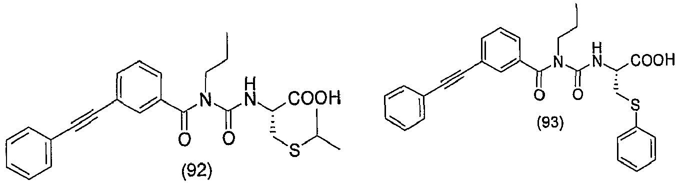

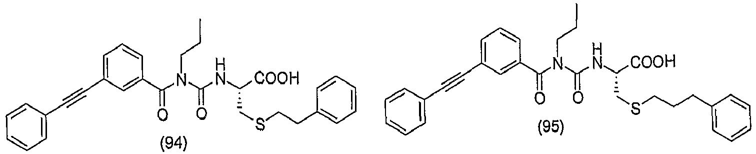

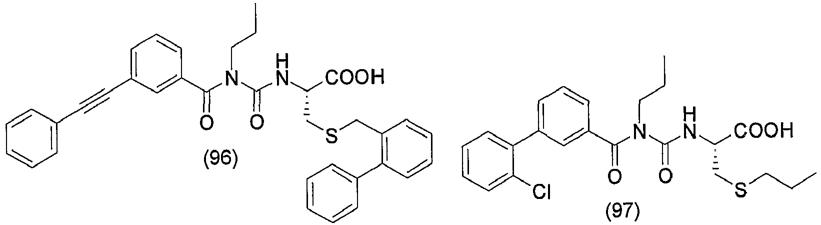

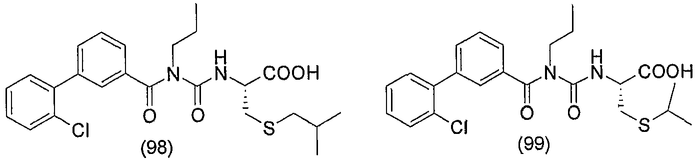

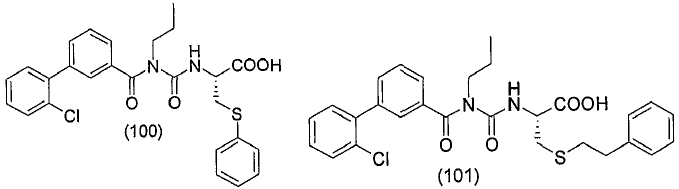

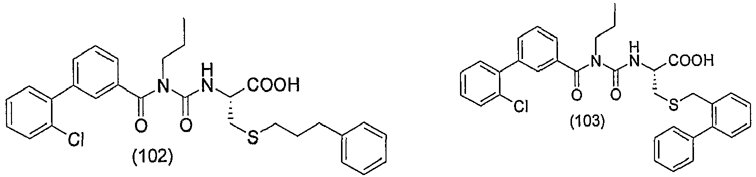

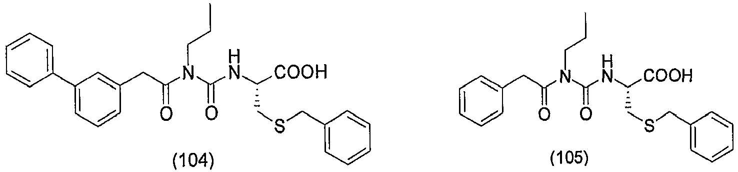

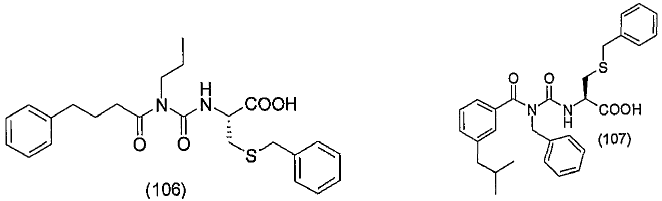

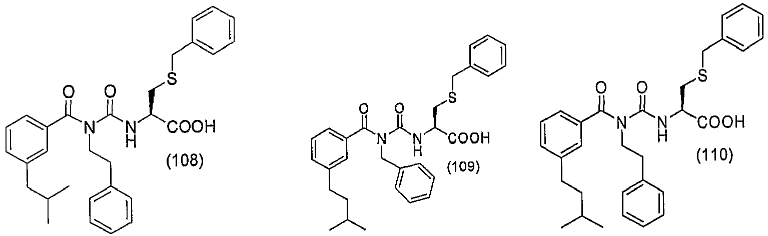

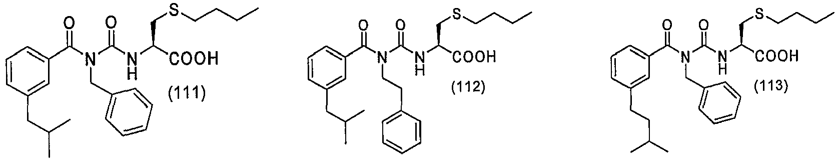

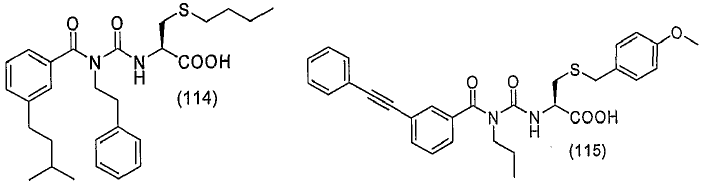

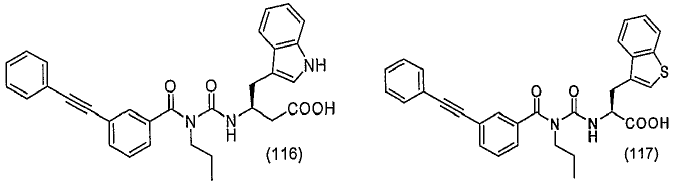

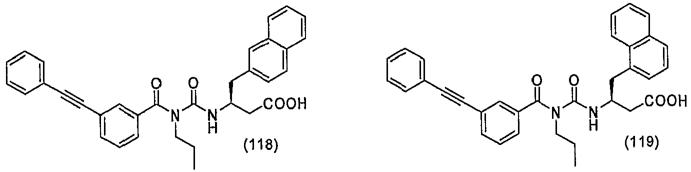

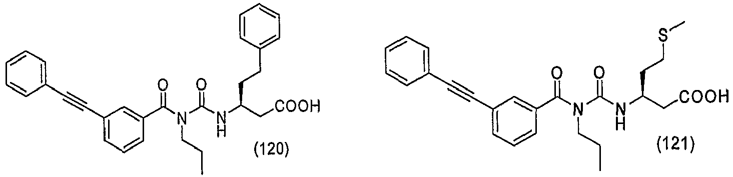

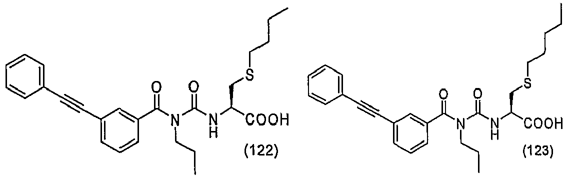

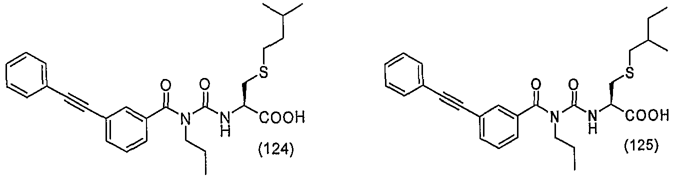

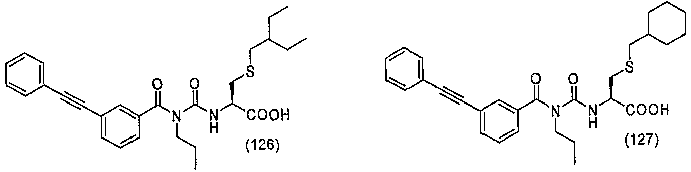

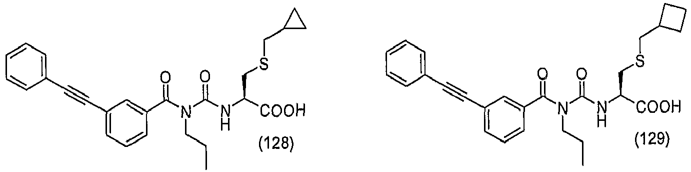

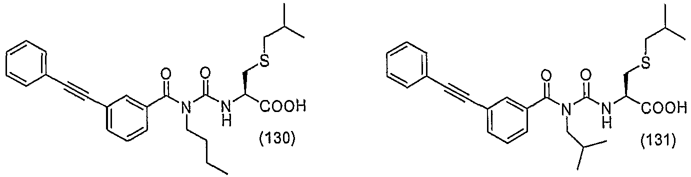

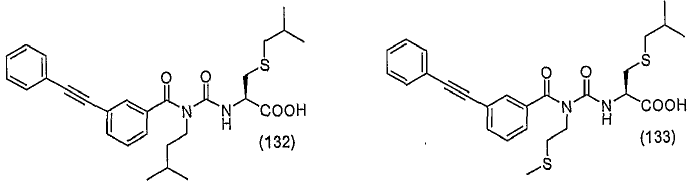

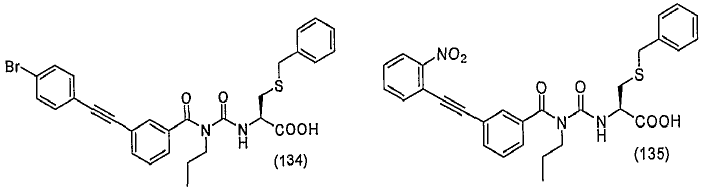

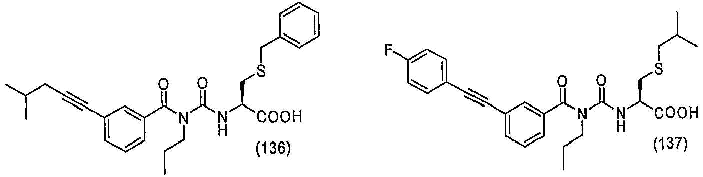

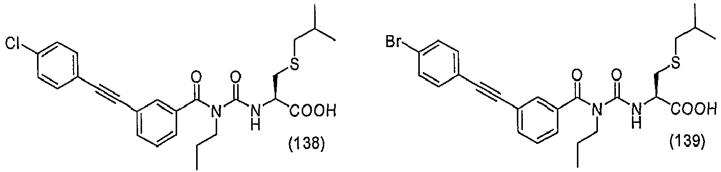

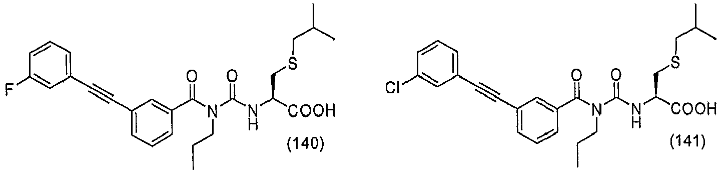

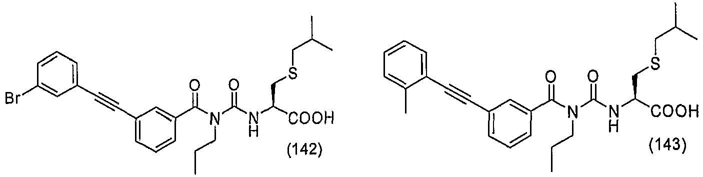

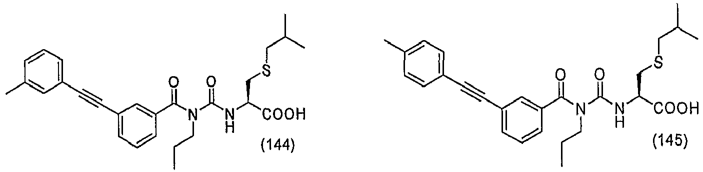

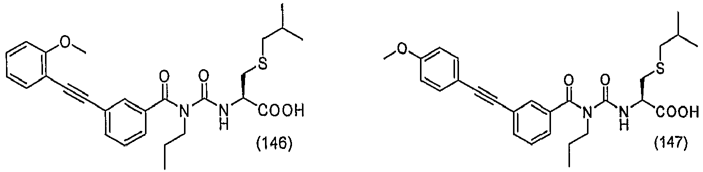

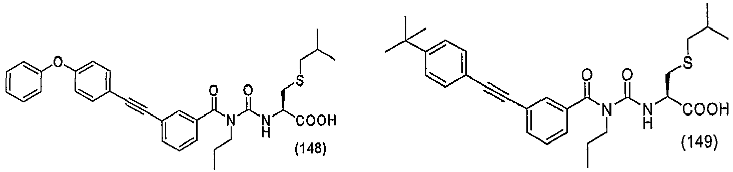

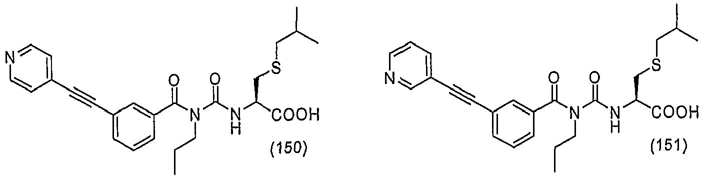

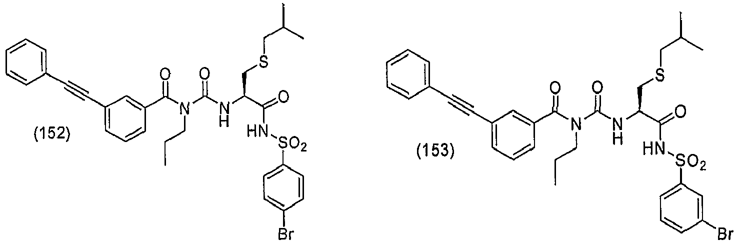

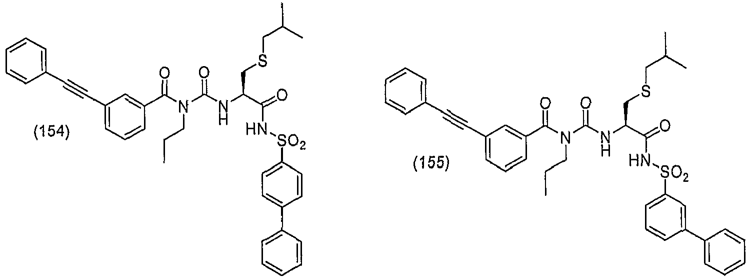

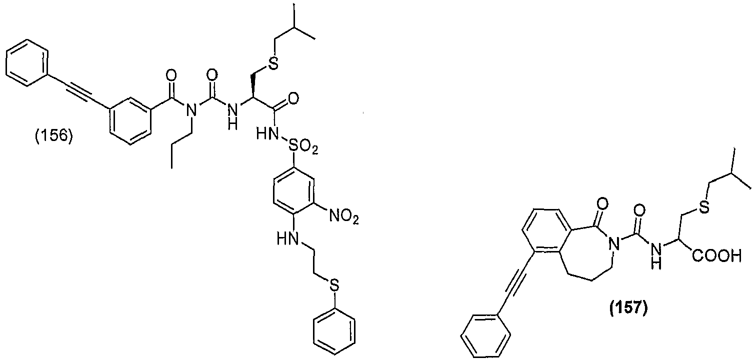

Especially preferred compounds include compounds (1), (2), (3), (6), (7), (8), (9), (10), (11), (12), (13), (14), (15), (17), (18), (21), (22), (23), (24), (29), (33), (34), (35), (36), (37), (39), (40), (42), (43), (45), (46), (47), (48), (52), (58), (59), (62), (64), (65), (66), (67), (82), (83), (84), (85), (86), (87), (88), (89), (90), (91), (92), (93), (94), (95), (96), (97), (98), (99), (100), (101), (102), (103), (105), (107), (108), (109), (110), (111), (112), (113), (114), (115), (116), (117), (118), (119), (120), (122), (123), (124), (125), (126), (127), (128), (129), (130), (131), (132), (133), (134), (135), (136), (137), (138), (139), (140), (141), (142), (143), (144), (145), (146), (147), (148), (149), (152), (153), (154), (155) and (156). Especially compounds (1), (7), (8), (9), (10), (12), (13), (14), (15), (24), (36), (39), (42), (46), (58), (62), (82), (83), (84), (85), (88), (89), (90), (91), (92), (93), (94), (95), (98), (100), (101), (102), (103), (107), (108), (109), (110), (111), (112), (113), (114), (115), (116), (117), (118), (120), (121), (122), (123), (124), (125), (127), (129), (130), (131), (132), (133), (135), (136), (137), (138), (139), (140), (141), (142), (143), (144), (145), (146), (147), (152), (153), (154), (155) and (156). More especially preferred compounds include (9), (83), (84), (91), (94), (100), (101), (102), (103), (116), (131), (137) and (156).

The benzoylurea derivatives of the invention may be prepared by a method adapted from DE 2514020, Eli Lilly & Co., 1975 as shown in Scheme 1 wherein P is a protecting group.

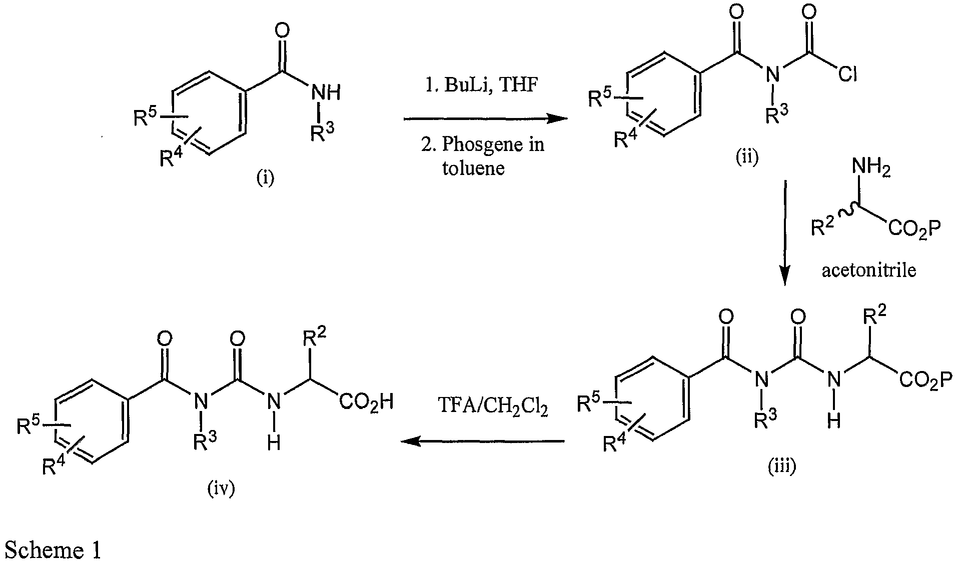

However, the synthetic procedure shown in Scheme 1 is not suitable when R³ is a bulky group which sterically hinders the benzoylamide (i) nitrogen.

A second synthetic strategy which allows the preparation of the carbamoylchloride (ii), even in the presence of a bulky substituent as R³ is shown in Scheme 2. Treatment of an amide with trimethylsilyltriflate (TMSOTf) in ether in the presence of triethylamine provides a silylated intermediate that readily reacts with phosgene to form a carbamoylchloride (ii).

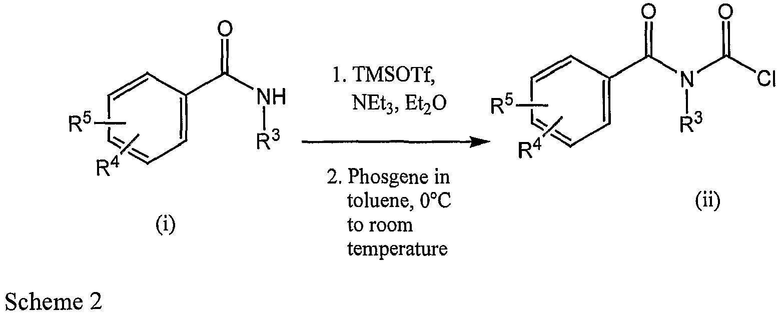

The carbamoylchloride (ii) was readily reacted with a trimethylsilyl (TMS) O-protected amino acid (v) which is prepared and added to the carbamoylchloride (ii) in acetonitrile without purification [Horner *et. al.,* 1998], as shown in Scheme 3.

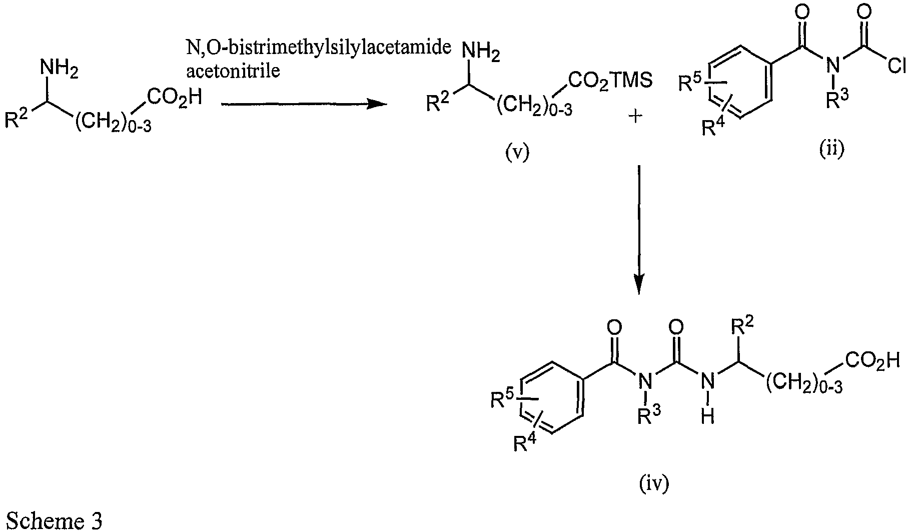

This process allows for a wide diversity of substituents represented by R², R³, R⁴ and R⁵ and provides the benzoylurea derivatives in high yields. Also this process is applicable to both amino acids and amino hydrochlorides.

Although Scheme 3 shows reaction of the carbamoylchloride with a suitably protected amino acid, this may be adapted to allow reaction between any primary amine and the carbamoylchloride. For example, the carboxylic acid may be replaced by a cyano group, which may be further reacted with sodium azide to give a tetrazole. The reaction shown in Scheme 3 may be adapted to allow reaction with a cyano substituted primary amine or a tetrazole substituted primary amine thereby providing means of introducing a carboxylic acid or carboxylate bioisostere as R¹.

Another means of preparing the compounds of the invention is by condensation of a nitrosulphonamide with an isocyanate as shown in Scheme 4.

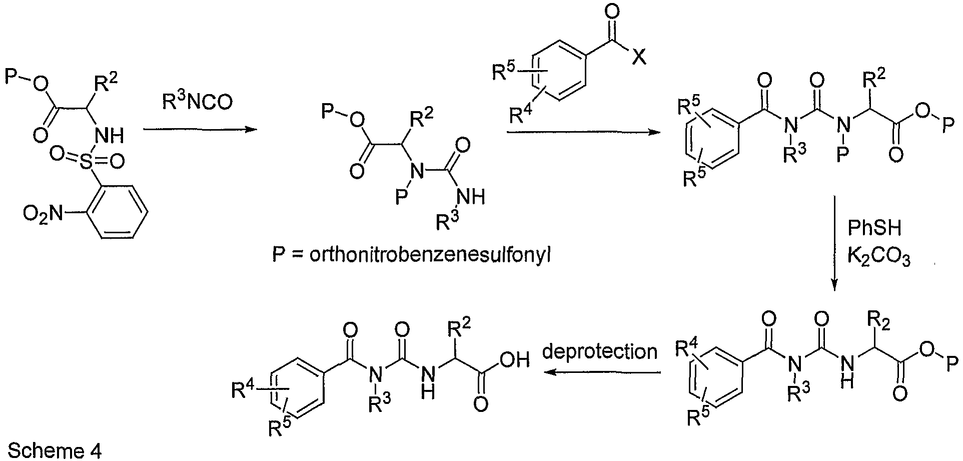

The starting benzoylamide (i) in Scheme 2 may be readily prepared from commercially available starting materials. For example, a range of benzylamides may be prepared from a suitably substituted bromophenylcarboxylic acid by amidation followed by Suzuki coupling as shown in Scheme 5.

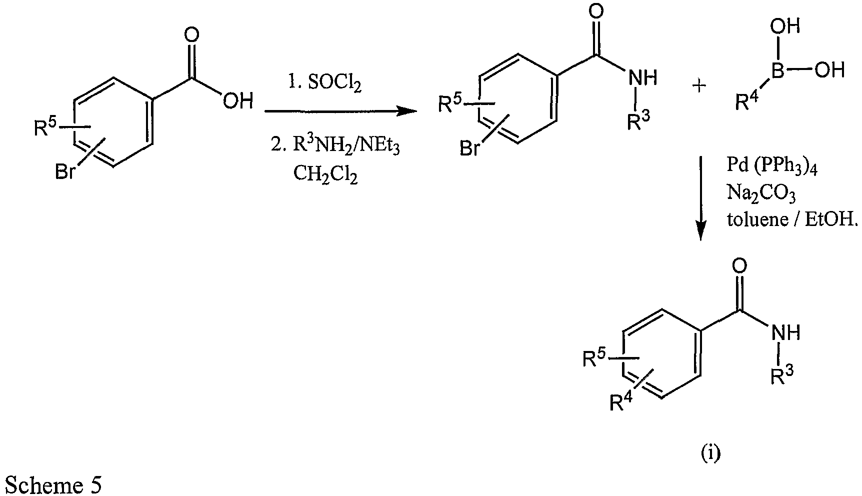

Arylethynyl substituents may be introduced as R⁴ using the Sonogashira coupling [Negishi and Anastasia, 2003] as shown in Scheme 6.

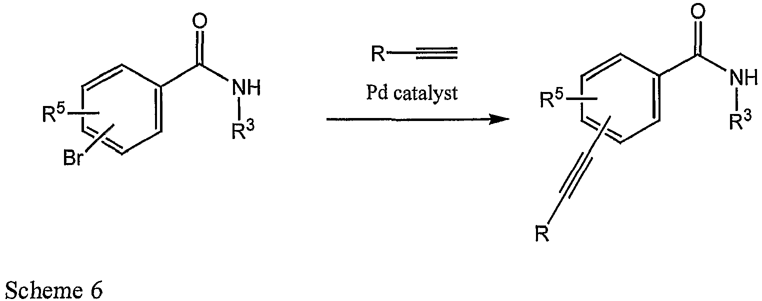

The carbamoylchloride (ii) and compound of the invention (iv) may then be prepared as shown in Schemes 2 and 3. Alternatively, when R is aryl or heteroaryl, it may be introduced using the Sonogashira coupling after preparation of compounds (i) and (iii). For example, the Sonogashira coupling may also be performed as shown in Scheme 7.

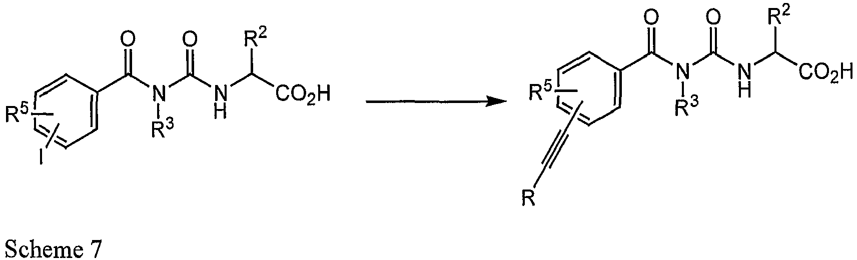

The benzoylurea compounds of the invention in which R² is CH₂SR may also be prepared from S-derivatised cysteine residues. The carbamoylchloride (ii) prepared as in Scheme 1 or Scheme 2 may be reacted with a S-derivatised cysteine residue as shown in Scheme 8.

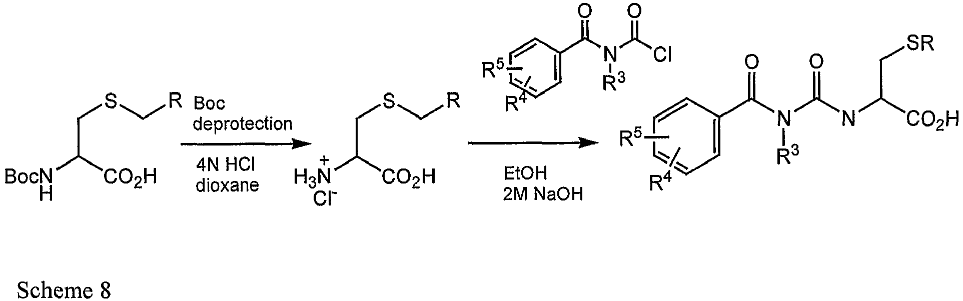

Advantageously, Boc protected unnatural amino acids may be used in this synthesis directly from their preparation. Another advantage is that Boc protected amino acids, both natural or unnatural, used as shown in Scheme 8 provide a very simple and clean method for obtaining an amino acid hydrochloride salt. Suitably derivatised cysteine residues for use in Scheme 8 may be prepared by the method of Seko *et. al.* (2003) as shown in Scheme 9.

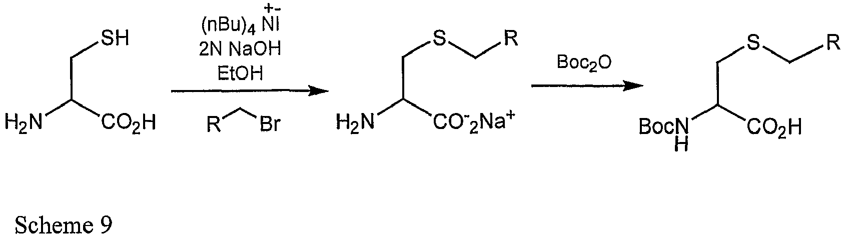

Alternatively, the benzoylurea compounds may be prepared directly from unprotected amino acids as shown in Scheme 10.

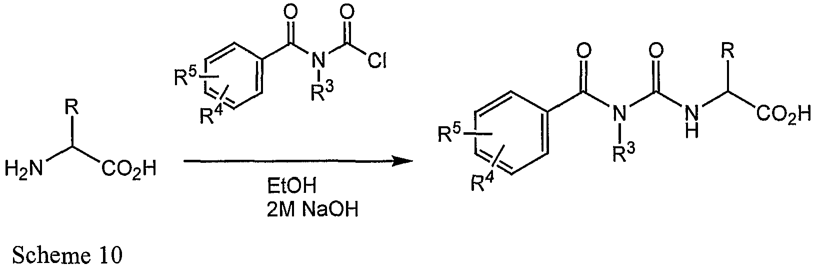

The substituent R⁵ may be present in the starting benzoic acid or may be introduced later in the synthetic process by procedures known in the art. For example, alkyl groups may be introduced by Friedel-Crafts alkylation, halogens can be introduced by treatment with dihalogen in the presence of a catalyst, alkylthio groups may be introduced by sulfonylation followed by reduction. Suitable methodology may be found in, for example, March J., "Advanced Organic Chemistry", Wiley & Sons, 1985.

Compounds in which R¹ is a tetrazole or tetrazolate can be prepared by known methods from a cyano group and sodium azide by cycloaddition [Davies D.T. "Aromatic Heterocyclic Chemistry", Oxford University Press, 1992]. The cyano group may be present in the amine reacted with the carbamoylchloride, or may be reacted with sodium azide before reaction with the carbamoylchloride.

The substituents R₁ to R₅ may be present during the synthesis in protected or unprotected form. Suitable protecting and deprotecting methods for reactive functional groups such as carboxylic acids, ketones, amines and hydroxy groups are known in the art, for example, in Protective Groups in Organic Synthesis, T.W. Green & P. Wutz, John Wiley & Son, 3rd Ed., 1999.

The substituents R₁ to R₅ may also undergo further manipulation to provide different substituents during or after the synthetic process described above.

In order to prepare large numbers of analogues, the strategy is also compatible with a solid phase synthetic approach. The synthetic process would then be reversed as the amino acid is immobilized onto a solid phase polymer support. Examples of this approach are provided in Schemes 11-17.

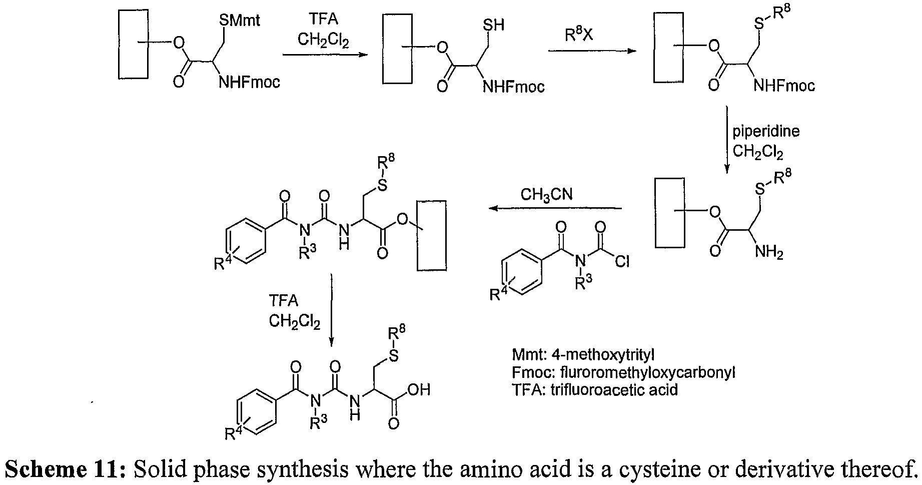

Diversity in the substitution at the cysteine sulfur atom can be introduced by reactions known in the art. For example, the deprotected thiol of the cysteine residue may be alkylated with an alkyl halide or alkyl halide derivative (R⁸-X). Examples of suitable alkyl halides include, but are not limited to CH₃Cl, CH₃Br, CH₃I, CH₃CH₂Cl, CH₃CH₂Br, CH₃CH₂I, CH₃(CH₂)₂Cl, CH₃(CH₂)₂Br, CH₃(CH₂)₂I, (CH₃)₂CHCl, (CH₃)₂CHBr, (CH₃)₂CHI, CH₃(CH₂)₃Cl, CH₃(CH₂)₃Br, CH₃(CH₂)₃I, (CH₃)₂CHCH₂Cl, (CH₃)₂CHCH₂Br, (CH₃)₂CHCH₂I, CH₃(CH₂)₄Cl, CH₃(CH₂)₄Br, CH₃(CH₂)₄I, (CH₃)₂CH(CH₂)₂Cl, (CH₃)₂CH(CH₂)₂Br, (CH₃)₂CH(CH₂)₂I, CH₃CH₂CH(CH₃)CH₂Cl, CH₃CH₂CH(CH₃)CH₂Br, CH₃CH₂CH(CH₃)CH₂I, CH₃CH₂CH(CH₃CH₂)CH₂Cl, CH₃CH₂CH(CH₃CH₂)CH₂Br, CH₃CH₂CH(CH₃CH₂)CH₂I, 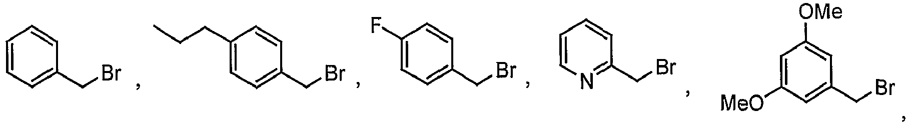 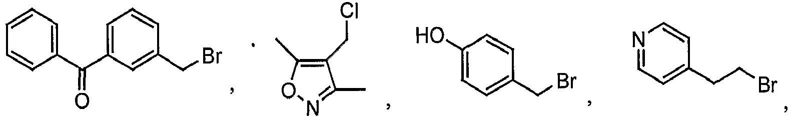 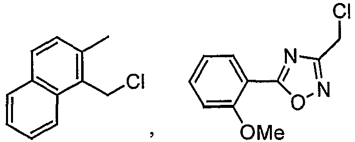 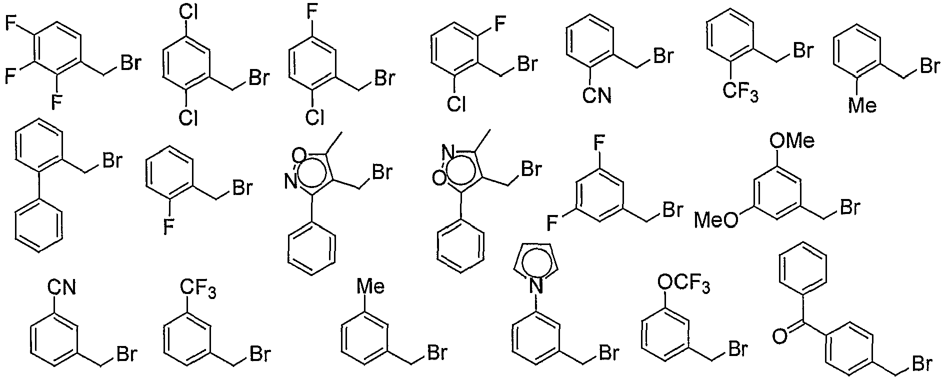 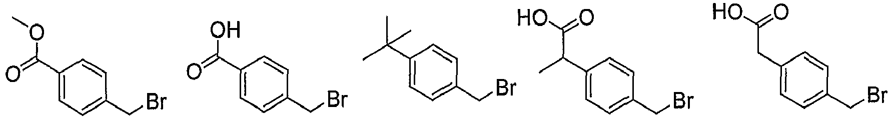 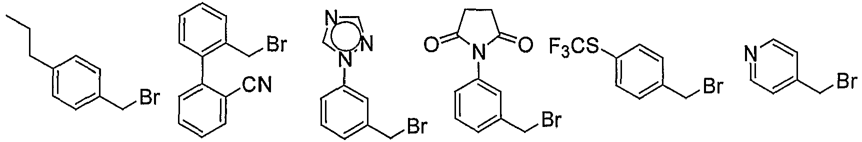 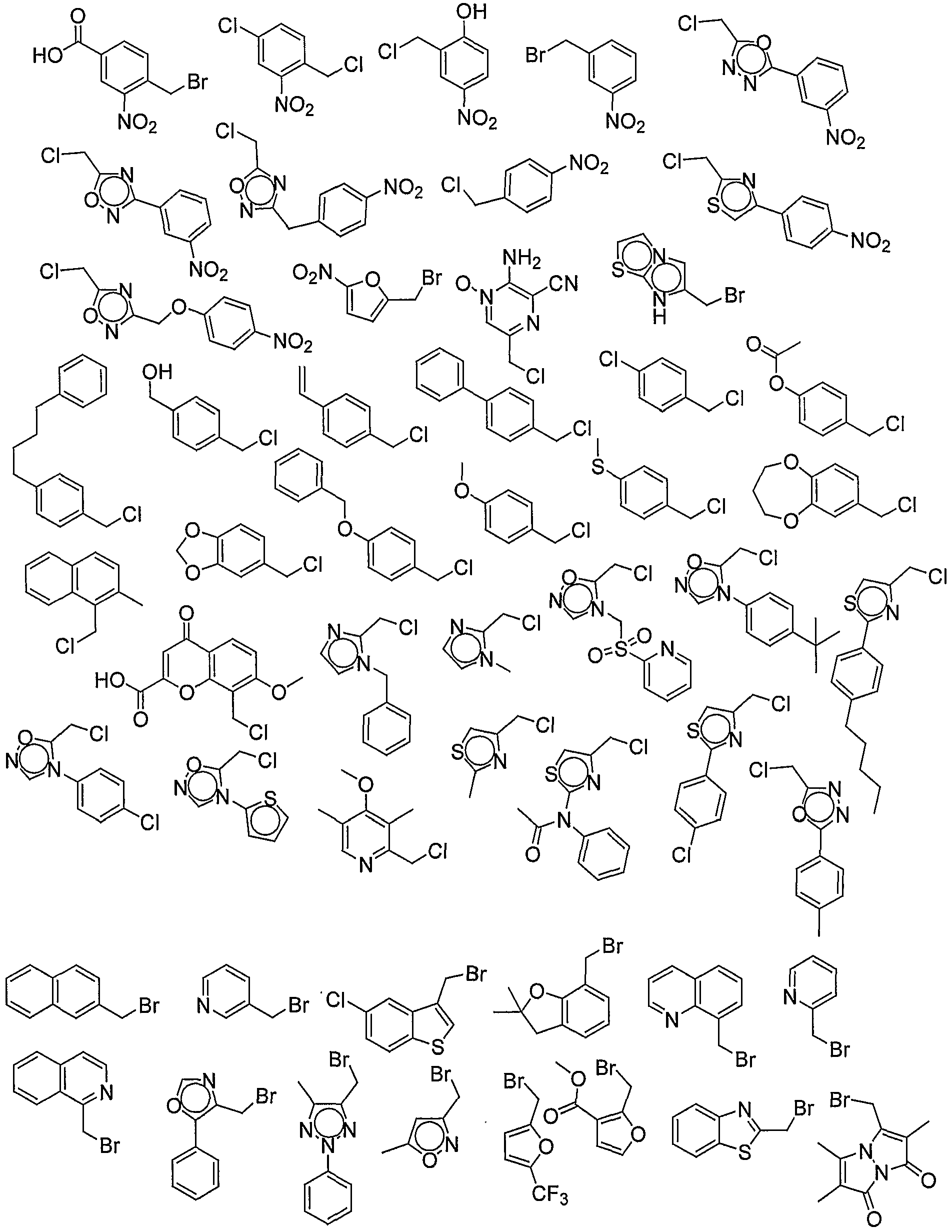 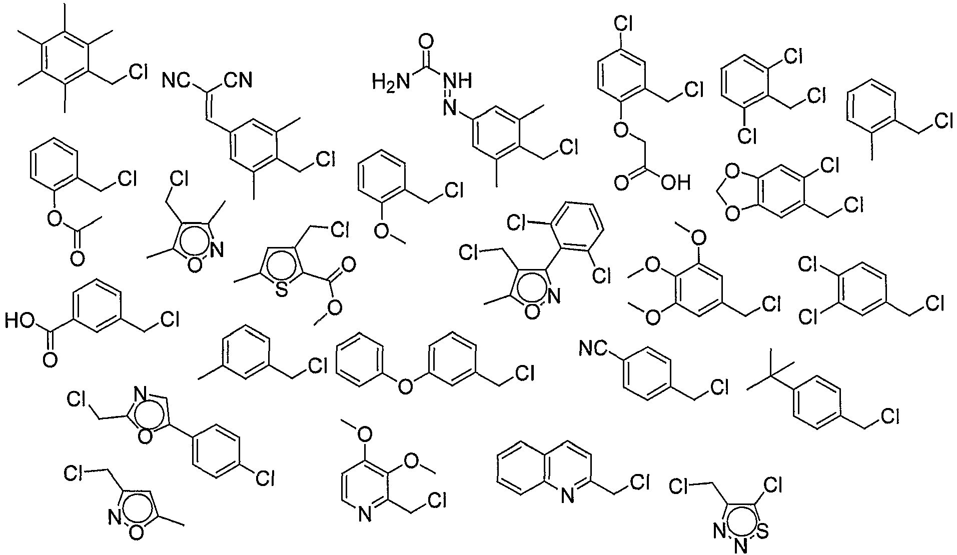 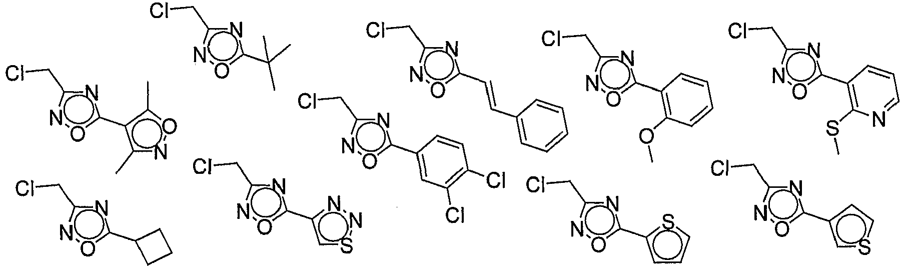 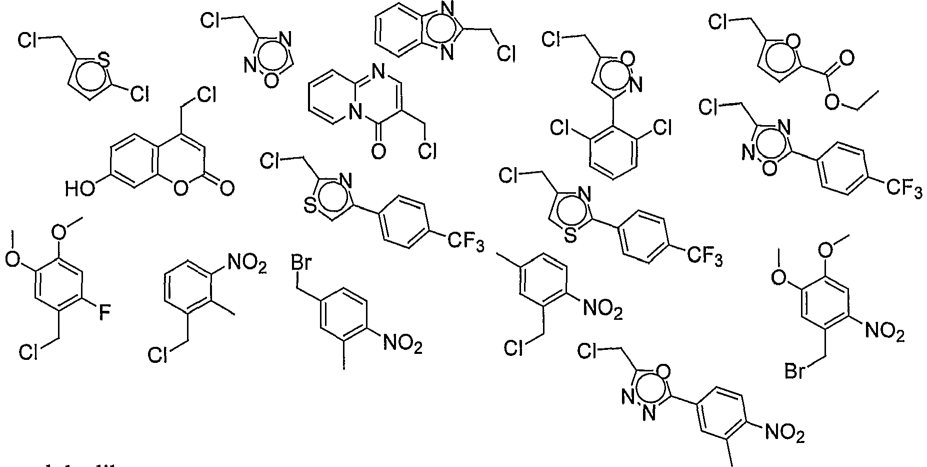 and the like.

Alternatively, thiophenyl substituents at R² may be prepared from serine derivatives as shown in Scheme 12. 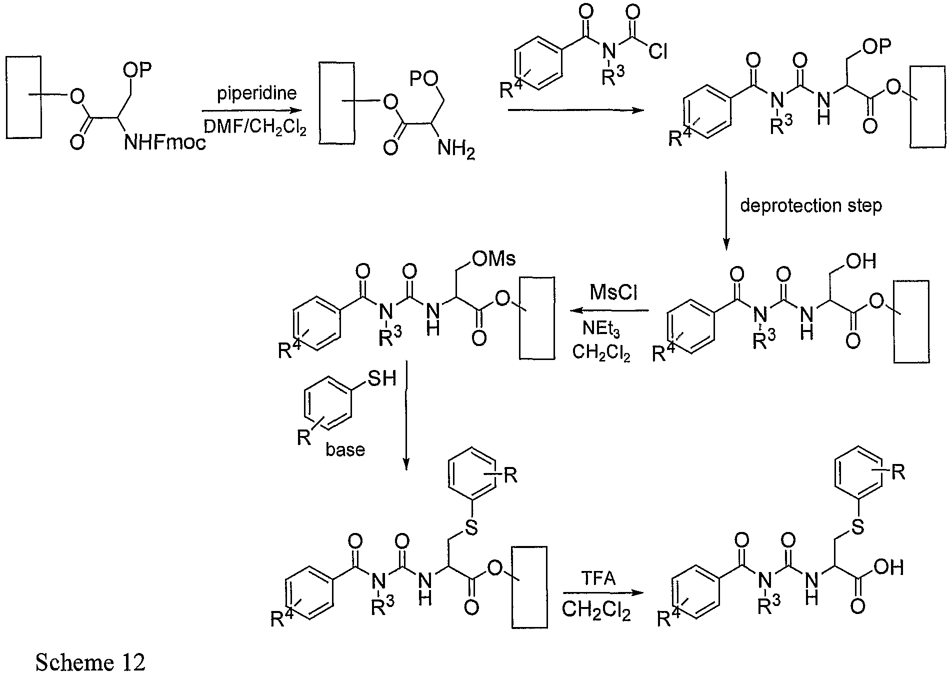 where P is a protecting group (see Green & Wutz, 1999) and R is at least one optional substituent.

Diversity may also be introduced into R² by use of a halo-substituted phenylamine residue and Suzuki coupling as shown in Scheme 13.

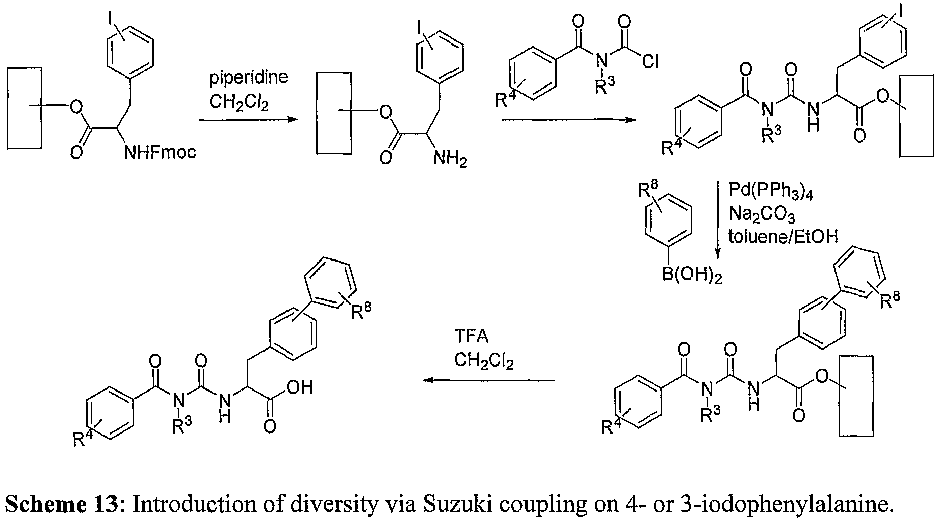

Suzuki coupling may also be used to introduce R⁴ as shown in Scheme 14. 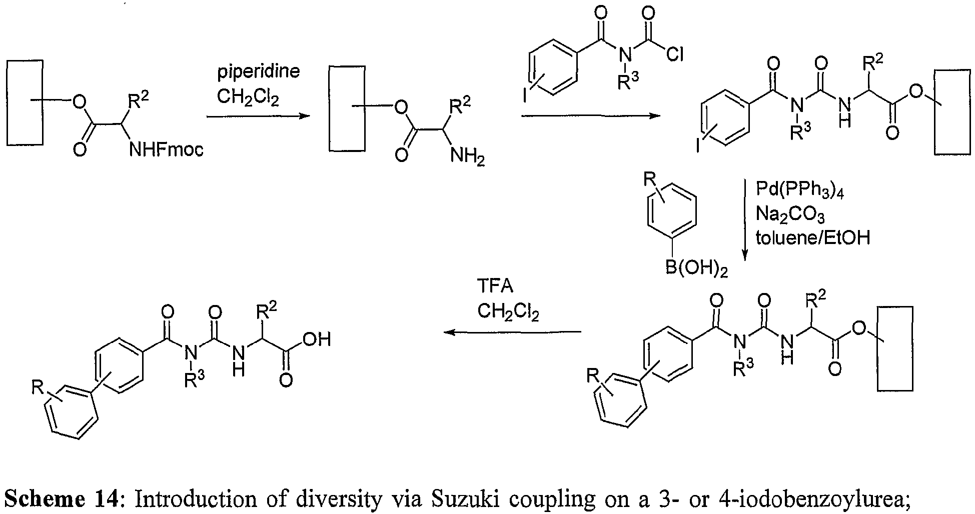 where R is one or more optional substituents

Phenylethynyl substituents may also be introduced at R⁴ using Sonogashira coupling [Negishi and Anastasia, 2003] and a solid phase synthesis approach as shown in Scheme 15.

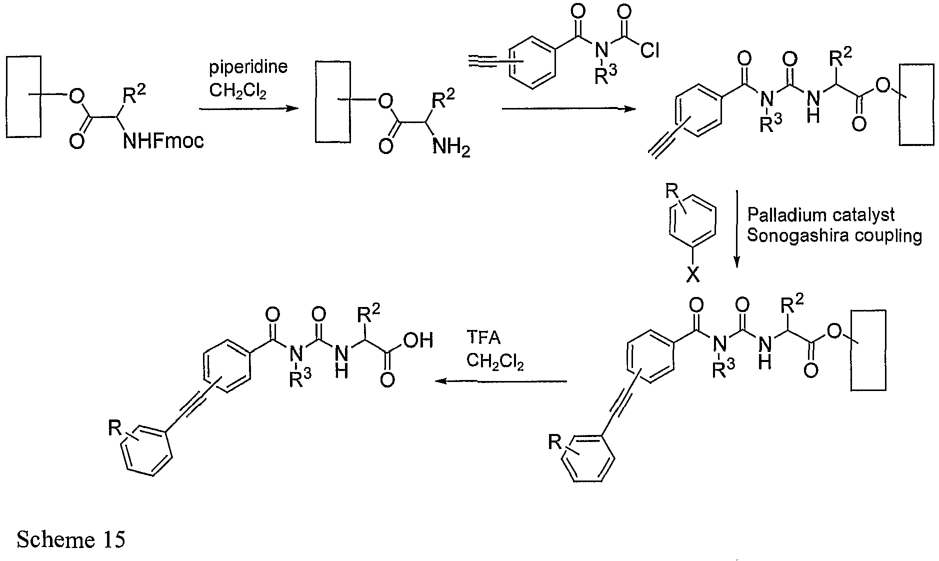

In Scheme 15, R represents one or more optional substituents.

Variation of R³ may also be achieved by substitution at the N¹ nitrogen. For example, by using the Mitsunobu reaction, as shown in Scheme 16. 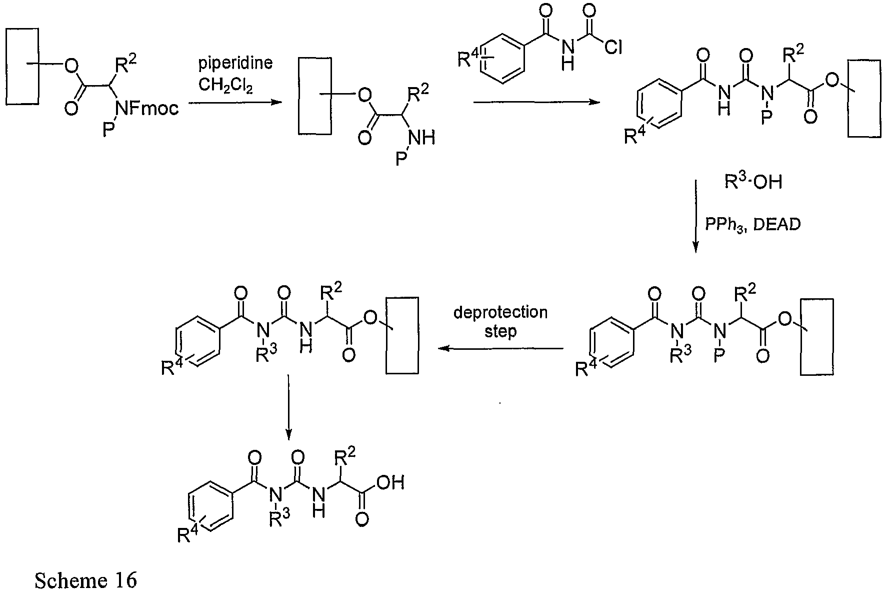 where P is a nitrogen protecting group such as t-butyl, p-methoxybenzyl, benzyl and methylbenzyl.

Another method for use in solid phase synthesis is condensation of a nitrosulphonamide with an isocyanate as shown in Scheme 17.

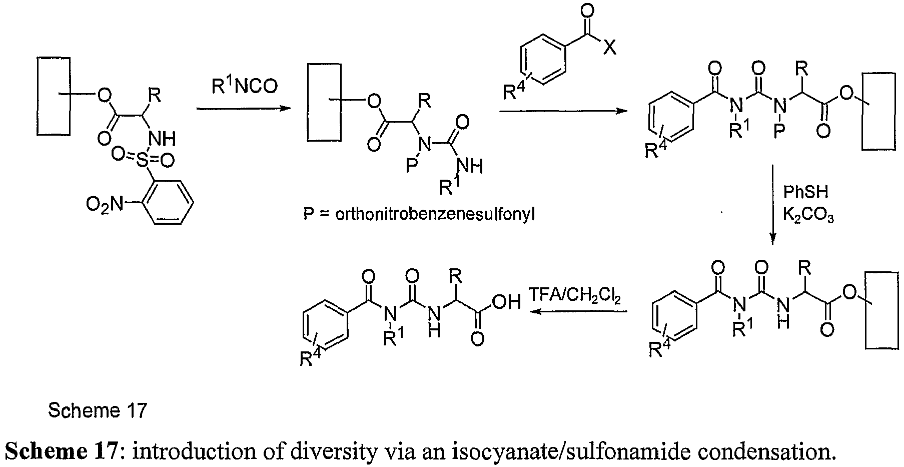

Suitable boronic acids for use in Suzuki couplings, whether in solution phase or solid phase synthesis, include, but are not limited to:

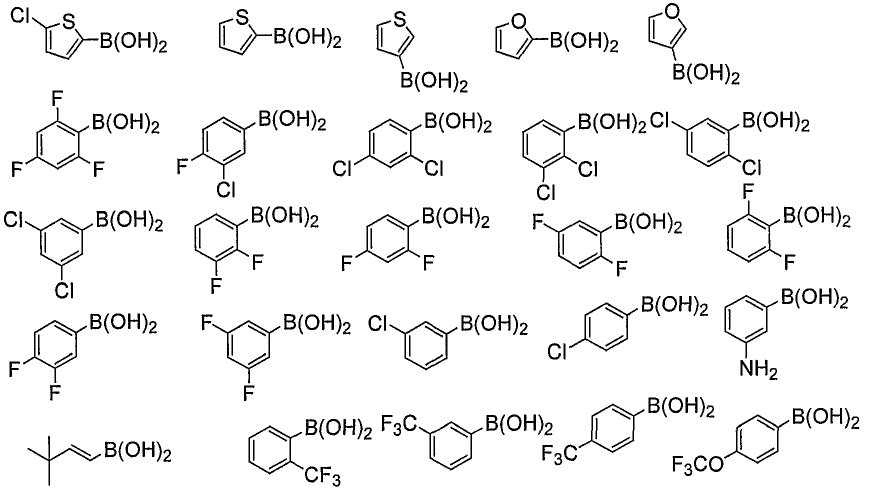

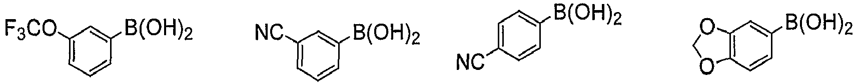

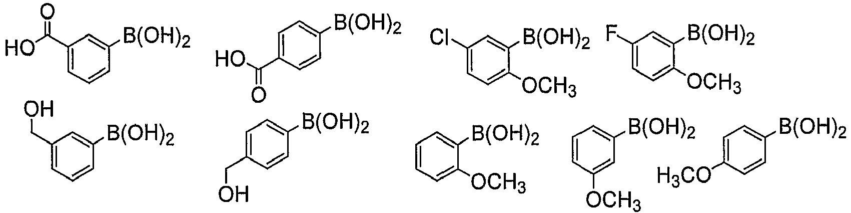

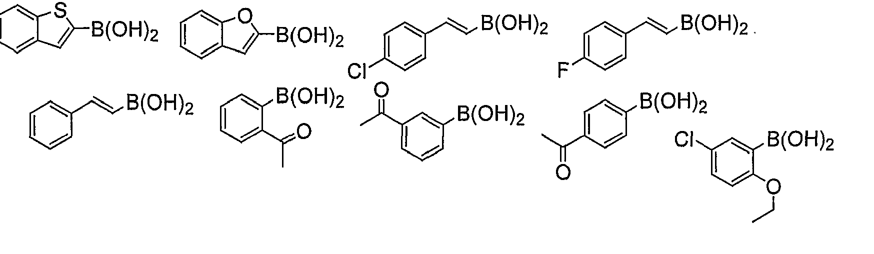

When introducing aryl rings into the molecule, for example as shown in Schemes 11-14, suitable substituents that may be present on the aryl rings are depicted above in relation to the boronic acids and aryl halides used in Suzuki couplings and substitution at the cysteine thiol (Scheme 10) respectively.

Suitable acetylenic derivatives for use in the Sonogashira coupling reactions, whether in solution phase or solid phase synthesis, include but are not limited to:

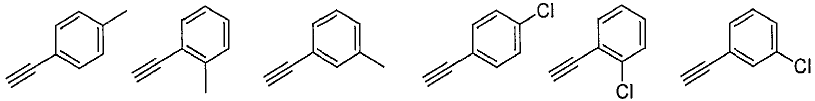

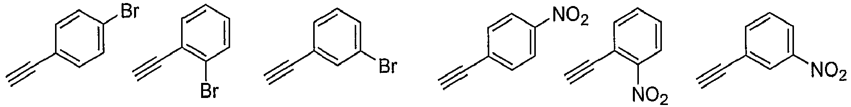

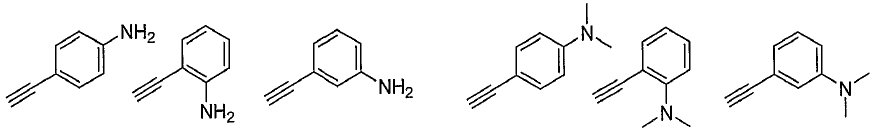

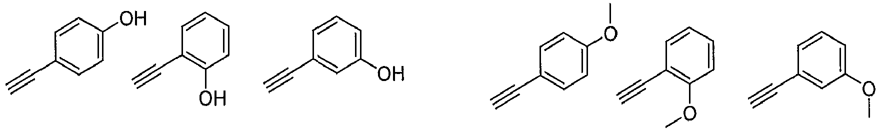

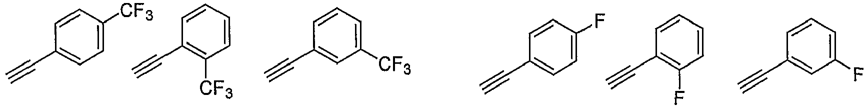

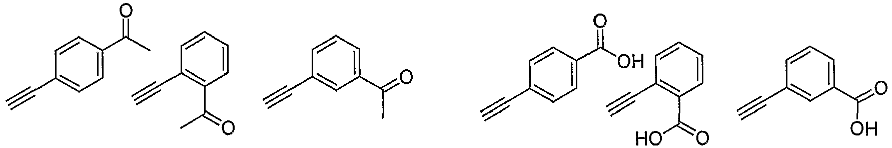

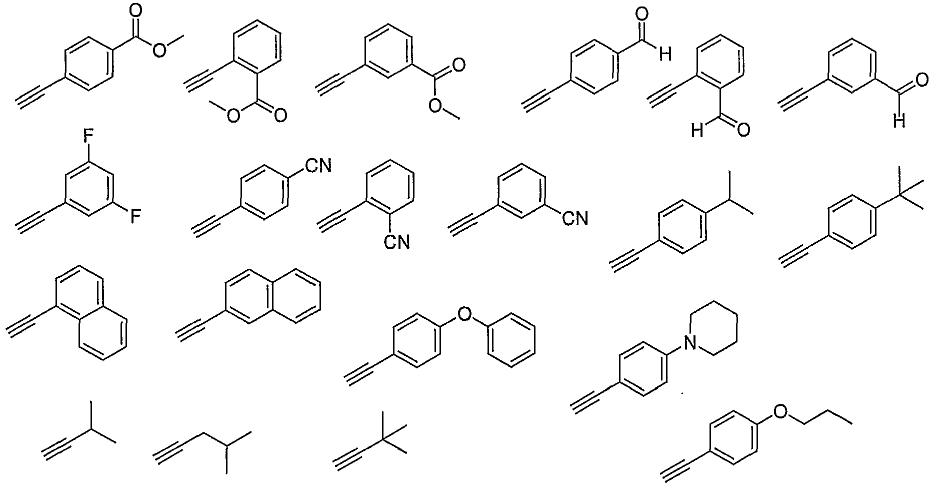

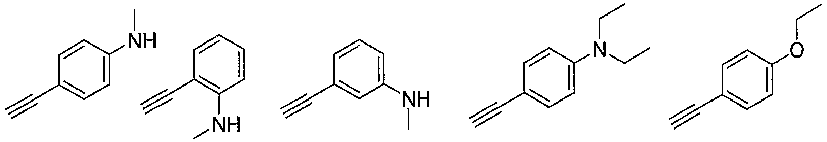

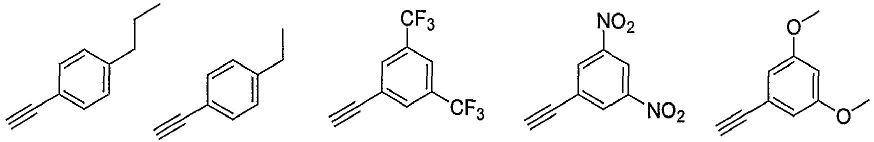

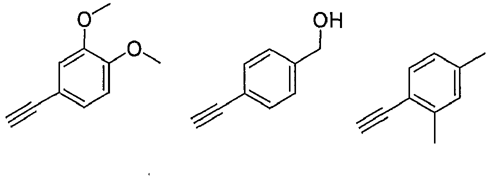

In preferred synthetic procedures, solution phase synthesis is used.

While not wishing to be bound by theory, it appears that the benzoylurea compounds of the invention are able to form an intramolecular hydrogen bond which stabilizes a conformation in which the substituents R², R³ and R⁴ simulate the spatial arrangement of the side chains of an alpha helical peptide. The conformation having an intramolecular hydrogen bond is an equilibrium with an open linear conformation as shown in Scheme 18.

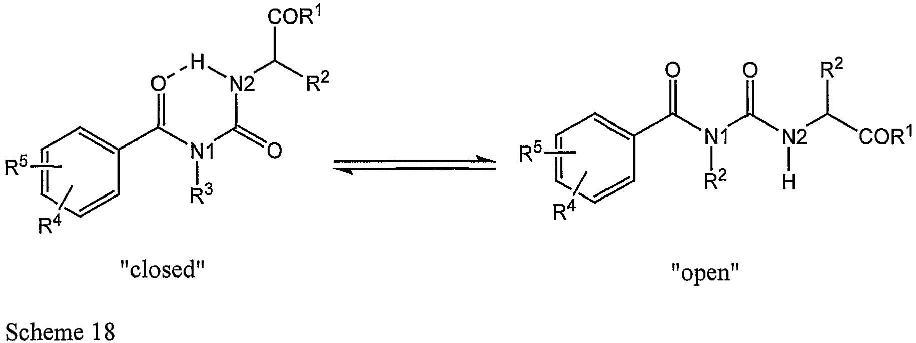

The NMR chemical shift for the N²H proton showed in general a downfield shifted signal which can be considered as a sign of hydrogen bonding. It was observed that compounds bearing a sterically-hindered substituent at the N¹ position had a reduced downfield shift (see Table 2). This difference may be due to the possibility of a change of conformation for these structures where the downfield chemical shift is representative of the closed conformation (consequence of the intramolecular hydrogen bonding) and the more upfield chemical shifts are indicative of the open conformation (hydrogen atom solvent exposed).

The importance of steric hindrance at the R³ position may be observed by ¹HMR chemical shift of the N² hydrogen atom. Those compounds in which R³ was linear or in which branching was not directly attached to the N¹ nitrogen atom had downfield shifted signals compared to the N² hydrogen atom signals of compounds in which R³ was a branched or cyclic group directly attached to the N¹ nitrogen atom as shown in Table 2.

**Table 2:**

| Compound | (62) | (1) | (2) | (5) | (3) | (4) | (6) | (7) | (8) |
|---|---|---|---|---|---|---|---|---|---|
| R³ | Et | *n*-Pr | *n*-Bu | *i*-Pr | *i*-Bu | *sec*-Bu | cyclohexyl | cyclohexylmethyl | benzyl |
| δ (N²H) | 9.72 | 9.68 | 9.68 | 8.52 | 9.57 | 8.58 | 7.91 | 9.6 | 9.8 |

It is postulated that the substitution pattern of the benzoylurea compounds affects the position of the equilibrium between the closed and open conformations. However, upon binding in the hydrophobic pocket of the Bcl-2 hydrophobic groove, the closed conformation will be favoured because of the general hydrophobicity of the interaction area (dielectric constant effect) and the specific interactions in the hydrophobic pockets bringing the molecules into the preferred closed conformation and adopting the spatial arrangement required to mimic an alpha helical peptide.

The decrease in binding of compounds bearing a hindered group on the N¹ nitrogen (like compound 5) could therefore be explained by the surplus of energy required during the binding event to bring the molecule into the closed conformation. However it is possible that compounds binding with high affinity having the appropriate N¹ substituent would adopt an open conformation in the cell medium and the closed conformation in the binding groove and as such constitute a "conformational prodrug". This could be important in terms of selectivity and toxicity. Helical mimetics are known to be able to interact with hydrophobic membranes therefore causing homeostatic disruption via non-selective pathways (e.g. swelling of mitochondrial membrane). The compounds of the present invention in the open conformation do not appear to be a helical mimetic and therefore in such a conformation they are unlikely to interfere with other non-selective pathways.

In another aspect of the disclosure there is provided a method of regulating the death of a cell, comprising contacting the cell with an effective amount of a compound of formula (I): 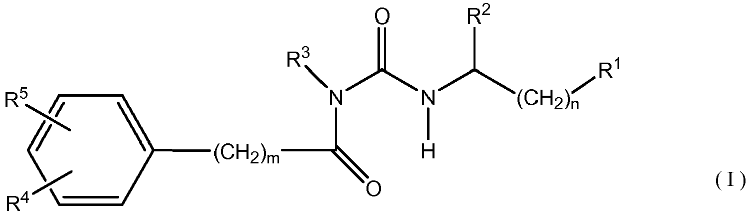 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)y-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 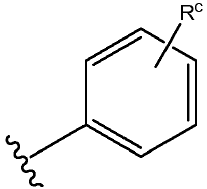 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso than (i) when R¹ is COOH, R² is C₆H₅-CH₂S-CH₂-, R⁴ is 3-C₆H₅ and R⁵ is H, R³ is not CH₃CH₂ and (ii) when R² is CH₃CH(CH₃)CH₂SCH₂, R⁴ is 3-phenethynyl, R¹ is COOH, m and n are 0 and R⁵ is H, R³ is not n-propyl.
In another aspect of the disclosure there is provided a method of regulating the death of a cell, comprising contacting the cell with an effective amount of a compound of formula (Ia): 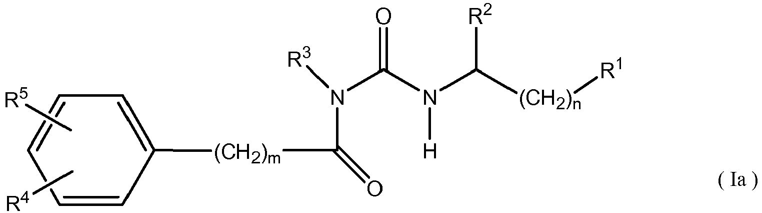 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 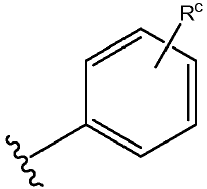 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.

In another aspect of the disclosure there is provided a method of regulating the death of a cell, comprising contacting the cell with an effective amount of a compound of formula (Ib): 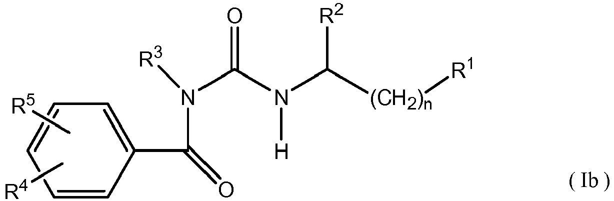 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 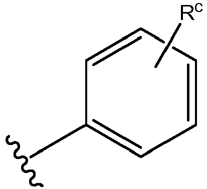 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
In another aspect of the disclosure there is provided a method of regulating the death of a cell, comprising contacting the cell with an effective amount of a compound of formula (Ic): 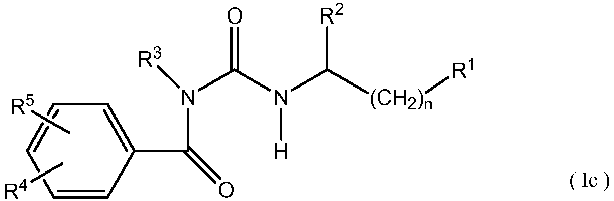 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 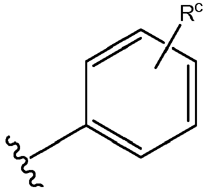 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
In another aspect of the disclosure there is provided a method of inducing apoptosis in unwanted or damaged cells comprising contacting said damaged or unwanted cells with an effective amount of a compound of formula (I): 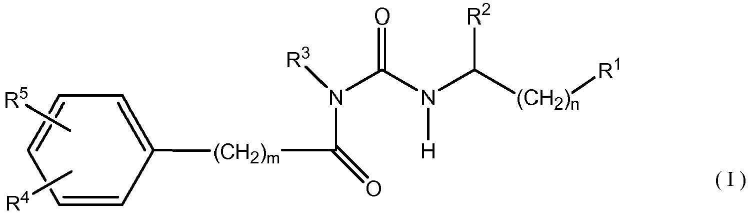 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 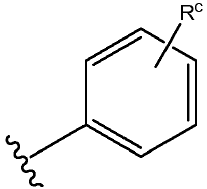 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.

In another aspect of the disclosure there is provided a method of inducing apoptosis in unwanted or damaged cells comprising contacting said damaged or unwanted cells with an effective amount of a compound of formula (Ia): 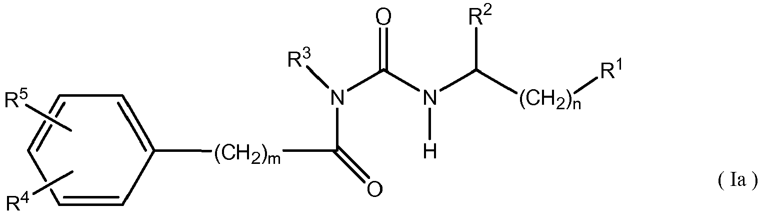 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 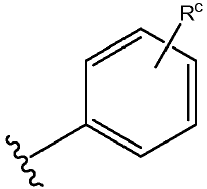 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.
In yet another aspect of the disclosure there is provided a method of inducing apoptosis in unwanted or damaged cells comprising contacting said damaged or unwanted cells with an effective amount of a compound of formula (Ib): 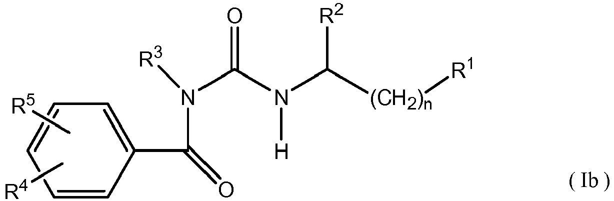 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 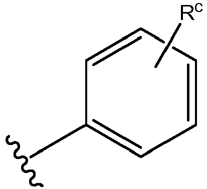 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
In another aspect of the disclosure there is provided a method of inducing apoptosis in unwanted or damaged cells comprising contacting said damaged or unwanted cells with an effective amount of a compound of formula (Ic): 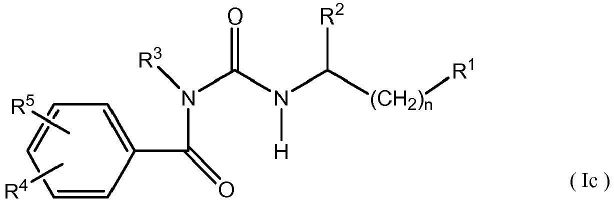 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 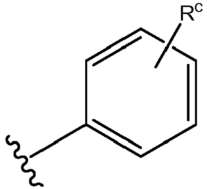 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
It should be understood that the cell which is treated according to a method of the present invention is located *ex vivo.* By *"ex vivo"* is meant that the cell has been removed from the body of a subject wherein the modulation of its activity will be initiated *in vitro.* For example, the cell may be a cell which is to be used as a model for studying any one or more aspects of the pathogenesis of conditions which are characterised by aberrant cell death signalling. In yet another aspect of the present invention there is provided a use of a compound of formula (I): 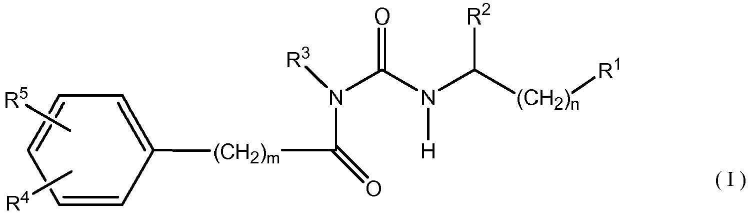 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 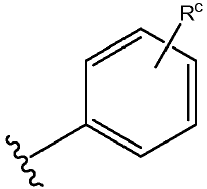 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl, in the manufacture of a medicament for regulating the death of a cell, or for inducing apoptosis in unwanted or damaged cells, or for the treatment and/or prophylaxis of a pro-survival Bcl-2 family member-mediated disease or condition, or for the treatment and/or prophylaxis of a disease or condition characterised by the inappropriate persistence or proliferation of unwanted or damaged cells.

In yet another aspect of the present invention there is provided a use of a compound of formula (Ia): 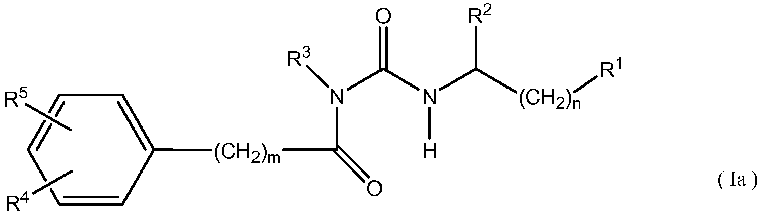 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 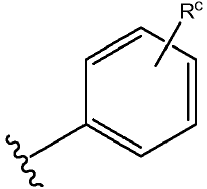 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl in the manufacture of a medicament for regulating the death of a cell, or for inducing apoptosis in unwanted or damaged cells, or for the treatment and/or prophylaxis of a pro-survival Bcl-2 family member-mediated disease or condition, or for the treatment and/or prophylaxis of a disease or condition characterised by the inappropriate persistence or proliferation of unwanted or damaged cells.

In yet another aspect of the present invention there is provided a use of a compound of formula (Ib): 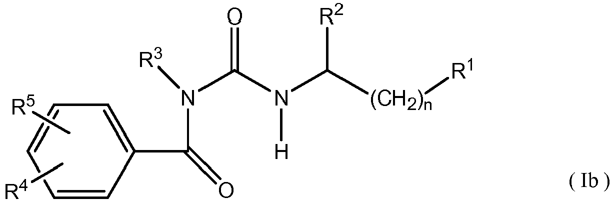 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 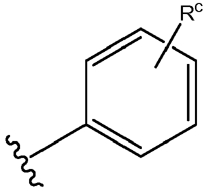 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)pNH(C₁₋₆alkyl), -(CH₂)ₚN(C₁₋₆alkyl)2, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl in the manufacture of a medicament for regulating the death of a cell, or for inducing apoptosis in unwanted or damaged cells, or for the treatment and/or prophylaxis of a pro-survival Bcl-2 family member-mediated disease or condition, or for the treatment and/or prophylaxis of a disease or condition characterised by the inappropriate persistence or proliferation of unwanted or damaged cells.

In yet another aspect of the present invention there is provided a use of a compound of formula (Ic): 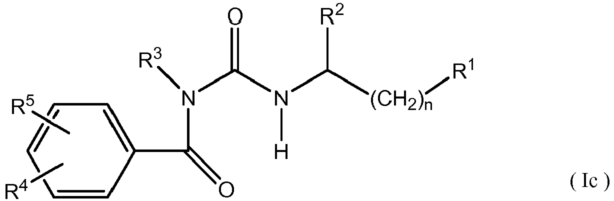 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃,-(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 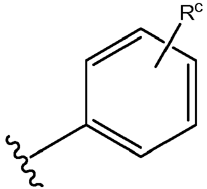 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R3 is not n-propyl in the manufacture of a medicament for regulating the death of a cell, or for inducing apoptosis in unwanted or damaged cells, or for the treatment and/or prophylaxis of a pro-survival Bcl-2 family member-mediated disease or condition, or for the treatment and/or prophylaxis of a disease or condition characterised by the inappropriate persistence or proliferation of unwanted or damaged cells.

A compound of formula (Id) may also be used in the above methods and uses.

The term "mammal" as used herein includes humans, primates, livestock animals (eg. sheep, pigs, cattle, horses, donkeys), laboratory test animals (eg. mice, rabbits, rats, guinea pigs), companion animals (eg. dogs, cats) and captive wild animals (eg. foxes, kangaroos, deer). Preferably, the mammal is human or a laboratory test animal. Even more preferably, the mammal is a human.

As used herein, the term "pro-survival Bcl-2 family member-mediated disease or condition" refers to diseases or conditions where unwanted or damaged cells are not removed by normal cellular process, or diseases or conditions in which cells undergo aberrant, unwanted or inappropriate proliferation. Such diseases include those related to inactivation of apoptosis (cell death), including disorders characterised by inappropriate cell proliferation. Disorders characterised by inappropriate cell proliferation include, for example, inflammatory conditions such as inflammation arising from acute tissue injury including, for example, acute lung injury, cancer including lymphomas, such as prostate hyperplasia, genotypic tumours, autoimmune disorders, tissue hypertrophy etc. For example, diseases or conditions associated with or characterised by inappropriate persistence or proliferation of unwanted or damaged cells include those relating to unwanted or damaged B cells, for example B cell non-Hodgkin's lymphoma, B cell acute lymphoblastic leukemia, rheumatoid arthritis, systemic Lupus erythematosis and related arthropathies. Diseases and conditions associated with or characterised by the inappropriate persistence of unwanted or damaged T cells include T cell acute lymphoblastic leukemia, T cell non-Hodgkin's lymphoma and graft vs Host disease. Diseases and conditions associated with or characterised by the inappropriate persistence of unwanted or damaged myeloid cells include acute myelogenous leukemia, chronic myelogenous leukemia and chronic myelomonocytic leukemia. Diseases and conditions associated with or characterised by the inappropriate persistence of unwanted or damaged plasma cells include multiple myeloma. Diseases and conditions associated with or characterised by the inappropriate persistence of unwanted or damaged cancer cells, include cancers, especially ovarian cancer, breast cancer and prostate cancer cells.

An "effective amount" means an amount necessary at least partly to attain the desired response, or to delay the onset or inhibit progression or halt altogether, the onset or progression of a particular condition being treated. The amount varies depending upon the health and physical condition of the individual to be treated, the taxonomic group of individual to be treated, the degree of protection desired, the formulation of the composition, the assessment of the medical situation, and other relevant factors. It is expected that the amount will fall in a relatively broad range that can be determined through routine trials. An effective amount in relation to a human patient, for example, may lie in the range of about 0.1 ng per kg of body weight to 1 g per kg of body weight per dosage. The dosage is preferably in the range of 1µg to 1 g per kg of body weight per dosage, such as is in the range of 1mg to 1g per kg of body weight per dosage. In one embodiment, the dosage is in the range of 1 mg to 500mg per kg of body weight per dosage. In another embodiment, the dosage is in the range of 1 mg to 250 mg per kg of body weight per dosage. In yet another embodiment, the dosage is in the range of 1 mg to 100 mg per kg of body weight per dosage, such as up to 50 mg per kg of body weight per dosage. In yet another embodiment, the dosage is in the range of 1 µg to 1 mg per kg of body weight per dosage. Dosage regimes may be adjusted to provide the optimum therapeutic response. For example, several divided doses may be administered daily, weekly, monthly or other suitable time intervals, or the dose may be proportionally reduced as indicated by the exigencies of the situation.

Reference herein to "treatment" and "prophylaxis" is to be considered in its broadest context. The term "treatment" does not necessarily imply that a subject is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. The term "prophylaxis" may be considered as reducing the severity or onset of a particular condition. "Treatment" may also reduce the severity of an existing condition.

The present disclosure further contemplates a combination of therapies, such as the administration of the compounds of the invention or pharmaceutically acceptable salts or prodrugs thereof together with the subjection of the mammal to other agents or procedures which are useful in the treatment of diseases and conditions characterised by the inappropriate persistence or proliferation of unwanted or damaged cells. For example, the compounds of the present invention may be administered in combination with other chemotherapeutic drugs, or with other treatments such as radiotherapy. Suitable chemotherapeutic drugs include, but are not limited to, cyclophosphamide, doxorubicine, etoposide phosphate, paclitaxel and vincristine.

While it is possible that, for use in therapy, a compound of the invention may be administered as a neat chemical, it is preferable to present the active ingredient as a pharmaceutical composition.

Thus, in a further aspect of the invention, there is provided a pharmaceutical composition comprising a compound of formula (I): 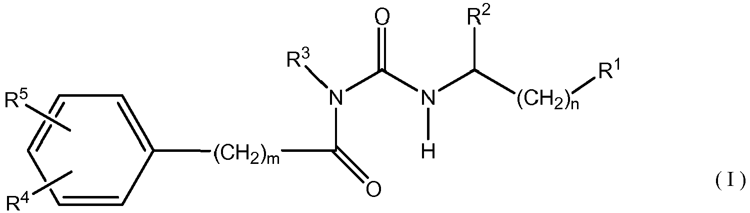 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 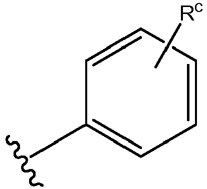 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)pNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl, together with one or more pharmaceutically acceptable carriers and optionally, other therapeutic and/or prophylactic ingredients.

In a further aspect of the invention, there is provided a pharmaceutical composition comprising a compound of formula (Ia): 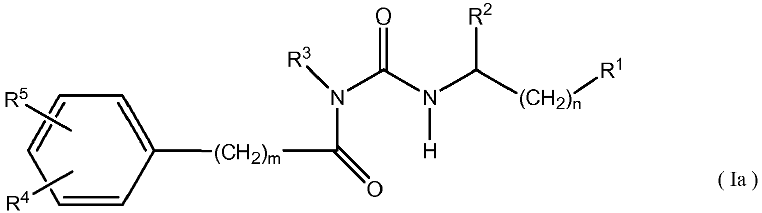 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 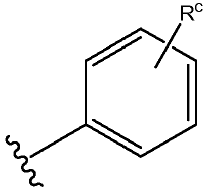 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)pOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl, together with one or more pharmaceutically acceptable carriers and optionally, other therapeutic and/or prophylactic ingredients.

In a further aspect of the invention, there is provided a pharmaceutical composition comprising a compound of formula (Ib): 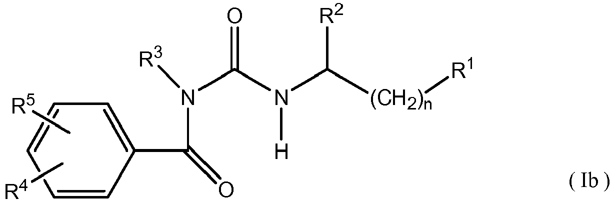 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 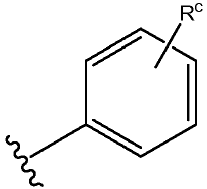 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl, together with one or more pharmaceutically acceptable carriers and optionally, other therapeutic and/or prophylactic ingredients.

In a further aspect of the invention, there is provided a pharmaceutical composition comprising a compound of formula (Ic): 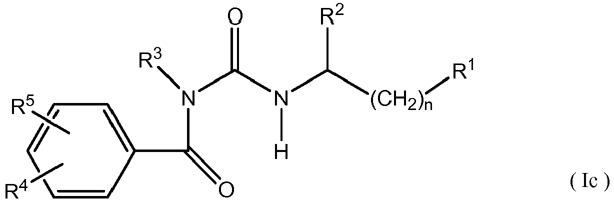 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 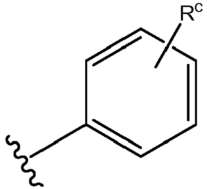 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl, together with one or more pharmaceutically acceptable carriers and optionally, other therapeutic and/or prophylactic ingredients.

The pharmaceutical compositions may also comprise a compound of formula (Id).

The carrier(s) must be "acceptable" in the sense of being compatible with the other ingredients of the composition and not deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation or insufflation. The compounds of the invention, together with a conventional adjuvant, carrier, or diluent, may thus be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

Formulations containing ten (10) milligrams of active ingredient or, more broadly, 0.1 to two hundred (200) milligrams, per tablet, are accordingly suitable representative unit dosage forms. The compounds of the present invention can be administered in a wide variety of oral and parenteral dosage forms. It will be obvious to those skilled in the art that the following dosage forms may comprise, as the active component, either a compound of the invention or a pharmaceutically acceptable salt or derivative of the compound of the invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilising and/or dispersing agents. Alternatively, the active ingredient may be in powder form, obtained by aseptic isolation of sterile solid or by lyophilisation from solution, for constitution with a suitable vehicle, e.g. sterile, pyrogen-free water, before use.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Formulations suitable for topical administration in the mouth include lozenges comprising active agent in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multidose form. In the latter case of a dropper or pipette, this may be achieved by the patient administering an appropriate, predetermined volume of the solution or suspension. In the case of a spray, this may be achieved for example by means of a metering atomising spray pump. To improve nasal delivery and retention the compounds according to the invention may be encapsulated with cyclodextrins, or formulated with their agents expected to enhance delivery and retention in the nasal mucosa.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas.

The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP).

Conveniently the powder carrier will form a gel in the nasal cavity. The powder composition may be presented in unit dose form for example in capsules or cartridges of, e.g., gelatin, or blister packs from which the powder may be administered by means of an inhaler.

In formulations intended for administration to the respiratory tract, including intranasal formulations, the compound will generally have a small particle size for example of the order of 1 to 10 microns or less. Such a particle size may be obtained by means known in the art, for example by micronization.

When desired, formulations adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Liquids or powders for intranasal administration, tablets or capsules for oral administration and liquids for intravenous administration are preferred compositions.

Bcl-2 proteins are not only present in persistent damaged or unwanted cells related to disease states such as malignant disease and autoimmunity. In order to minimise the risk of apoptosis of healthy cells caused by compounds that bind to Bcl-2 proteins, it is desirable to target delivery of the compounds to specific unwanted cells.

The use of certain antibodies to target particular cell types is an active area of research, particularly where the antibody is conjugated to the cell active agent (Wang *et. al.,* 1997; Goulet *et. al.,* 1997; Sapra and Allen, 2002; Marks *et. al.,* 2003; Deardon, 2002; Ludwig *et. al.,* 2003; Uckun *et. al.,* 1995). For example, CD19, as a pan B-cell antigen, is an ideal target for immunotoxin therapy of B-lineage leukemia and lymphomas (Wang *et. al.,* 1997; Goulet *et. al.,* 1997; Sapra and Allen, 2002; Marks *et. al.,* 2003; Deardon, 2002). Various cytotoxic agents, such as genistein, ricin analogues, doxorubicin, and cytotoxic peptides have been conjugated to anti-CD19 antibodies (Wang *et. al.,* 1997; Goulet *et. al.,* 1997; Sapra and Allen, 2002; Marks *et. al.,* 2003; Deardon, 2002; Uckun *et. al.,* 1995), in order to target and kill B-cells and treat B-cell associated cancer.

A BH₃ peptide has been conjugated to leutinising hormone releasing hormone (LHRH) to target LHRH receptors, which are overexpressed in several cancer cell lines but are not expressed in healthy human visceral organs (Dharap and Minko, 2003).

In a further aspect of the invention there is provided a conjugate solely defined by the appended claims, comprising at least one cell targeting moiety and at least one compound of formula (I): 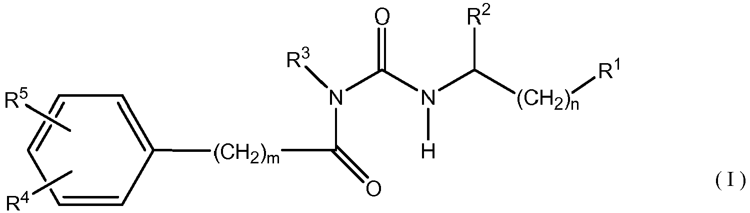 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 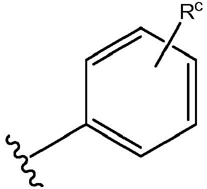 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.

In yet a further aspect of the invention there is provided a conjugate solely defined by the appended claims, comprising at least one cell targeting moiety and at least one compound of formula (Ia): 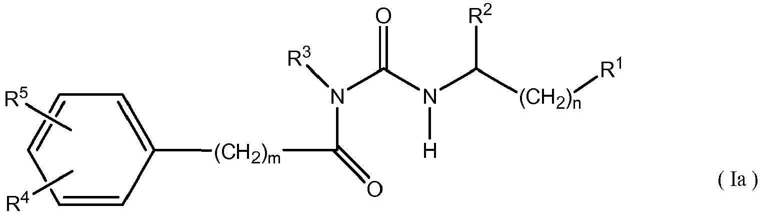 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CHR')ₓ-A-(CH₂)_{y}-

   wherein A is a covalent bond or is selected from O, S, SO, SO₂ or NR⁶, R^{a} is H, cycloalkyl, cycloalkenyl, aryl, heterocyclyl, heteroaryl or R^{b} where R^{b} is 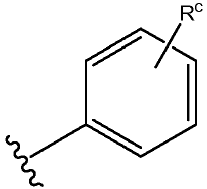 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, m and n are 0 and R⁵ is H, R³ is not n-propyl.

In a further aspect of the invention there is provided a conjugate solely defined by the appended claims, comprising at least one cell targeting moiety and at least one compound of formula (Ib): 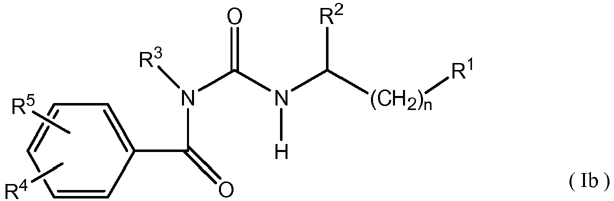 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)x-A-(CH₂)_{y}-

   wherein A is a covalent bond or is selected from O, S, SO, SO₂ and NR⁶, R^{a} is cycloalkyl, cycloalkenyl, aryl, heterocyclyl, heteroaryl or R^{b} where R^{b} is 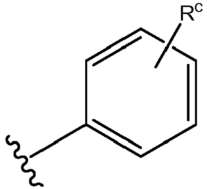 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}-

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)pNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)phalogen, -(CH₂)pheterocyclyl, heterocyclyloxy(CH₂)ₚ-, -(CH₂)pheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols; with the proviso than (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.
In yet a further aspect of the invention there is provided a conjugate solely defined by the appended claims, comprising at least one cell targeting moiety and at least one compound of formula (Ic): 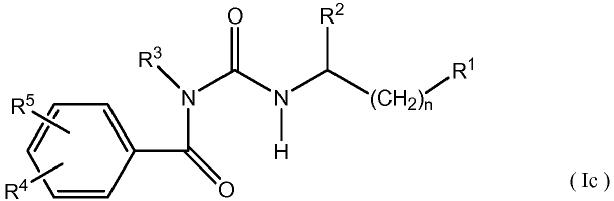 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, - (CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group

   R^{a}-(CH₂)ₓ-A-(CH₂)_{y}-

   wherein A is a covalent bond or is selected from O, S, SO, SO₂ or NR⁶, R^{a} is cycloalkyl, cycloalkenyl, aryl, heterocyclyl, heteroaryl or R^{b} where R^{b} is 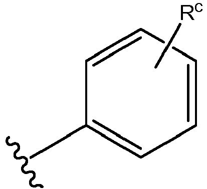 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), x and y are independently 0 or an integer from 1 to 6 provided that the sum of x and y is 1 to 6;
R³ is selected from C₃₋₆alkyl, C₂₋₆alkenyl, C₂₋₆alkynyl, cycloalkyl, cycloalkenyl, aryl, heterocyclyl, heteroaryl and a group

   R^{d}-(CH₂)ₚ-W-(CH₂)_{q}

   wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃;
R⁶ is selected from H, C₁₋₆alkyl, C₂₋₆alkenyl and C₂₋₆alkynyl;
Each R⁷ is independently selected from H and halogen; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)pOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen; and pharmaceutically acceptable salts and prodrugs selected from N-α-acyloxy amides, N-(acyloxyalkoxycarbonyl)amines and α-acyloxyalkyl esters of alcohols and phenols, with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is COOH, n is 0 and R⁵ is H, R³ is not n-propyl.

The conjugates of the invention may also comprise compounds of formula (Id).

As used herein, the term "conjugate" refers to a molecule composed of at least two moieties, at least one cell targeting moiety coupled to at least one compound of formula (I) or formula (Ia). The at least two moieties are releasably coupled, preferably by a covalent bond, more preferably a covalent bond that is able to be hydrolysed under specific cellular conditions to release the compound of formula (I) or formula (Ia) within a damaged or unwanted cell at its site of action. Examples of suitable covalent bonds able to be hydrolysed intracellularly include disulfide bonds, ester bonds and amide bonds. The conformationally constrained peptide moiety or a spacer, which may be present between the compound of the invention and the cell targeting moiety, may include an enzyme, for example, a protease, recognition sequence to provide hydrolysis of a bond under specific conditions thereby releasing the compound of formula (I) or formula (Ia).

As used herein, the term "cell targeting moiety" refers to a moiety which is able to interact with a target molecule expressed by an unwanted or damaged cell, preferably on the cell surface. Preferably, the target molecule is overexpressed in the unwanted or damaged cell and is not expressed in healthy cells. Suitable cell targeting moieties include proteins and antigen-binding molecules, which interact with target molecules in the damaged or unwanted cells. Suitable cell targeting moieties include, but are not limited to, hormones such as leutinising hormone receptor hormone and cytokines such as VEGF and EGF, and antibodies such as CD19, CD20, CD22, CD79a, CD2, CD3, CD7, CD5, CD13, CD33 and CD138, or antibodies targeting receptors such as Erb1 (also called EGFR), Erb2 (also called HER2 and NEU), Erb3 and Erb4. In a preferred embodiment the cell targeting moiety is an antibody that targets B-cells, for example, CD 19, CD20, CD22 and CD79a.

The conjugate may include one cell targeting moiety and one compound of formula (I) or formula (Ia), one cell targeting moiety and multiple compounds of formula (I) or formula (Ia), more than one cell targeting moiety and one compound of formula (I) or formula (Ia) or more than one cell targeting moiety and multiple compounds of formula (I) or formula (Ia). In some embodiments, the conjugate comprises one cell targeting moiety and between one and 100 compounds of formula (I) or formula (Ia), preferably one and 50, more preferably one and 20, most preferably 3 and 15. In other embodiments the conjugate may have more than one cell targeting moiety. The two or more cell targeting moieties may be the same or different. If the two or more cell targeting moieties are different, the conjugate may be used to target cells which express target molecules for each cell targeting moiety, thereby increasing cell specificity.

As disclosed herein, the term "antigen-binding molecule" refers to a molecule that has binding affinity for a target antigen, and extends to immunoglobulins, immunoglobulin fragments and non-immunoglobulin derived protein frameworks that exhibit antigen-binding activity.

In some embodiments, the cell-targeting moiety is an antigen-binding molecule that is immuno-interactive with a target molecule, typically a cell surface protein (e.g., a receptor), expressed by a cell that is the subject of targeting. Reference herein to "immuno-interactive" includes reference to any interaction, reaction, or other form of association between molecules and in particular where one of the molecules is, or mimics, a component of the immune system.

The antigen-binding molecule may be selected from immunoglobulin molecules such as whole polyclonal antibodies and monoclonal antibodies as well as sub-immunoglobulin-sized antigen-binding molecules. Polyclonal antibodies may be prepared, for example, by injecting a target molecule of the invention into a production species, which may include mice or rabbits, to obtain polyclonal antisera. Methods of producing polyclonal antibodies are well known to those skilled in the art. Exemplary protocols which may be used are described for example in Coligan et al., "Current Protocols In Immunology", (John Wiley & Sons, Inc, 1991), and Ausubel et al., "Current Protocols In Molecular Biology" (1994-1998), in particular Section III of Chapter 11.

In lieu of the polyclonal antisera obtained in the production species, monoclonal antibodies may be produced using the standard method as described, for example, by Köhler and Milstein, 1975, or by more recent modifications thereof as described, for example, in Coligan *et al.,* 1991, by immortalising spleen or other antibody-producing cells derived from a production species which has been inoculated with target molecule of the invention. Suitable sub-immunoglobulin-sized antigen binding molecules include, but are not restricted to, Fv, Fab, Fab' and F(ab')₂ immunoglobulin fragments. In some embodiments, the sub-immunoglobulin-sized antigen-binding molecule does not comprise the Fc portion of an immunoglobulin molecule.

In some embodiments, the sub-immunoglobulin-sized antigen-binding molecule comprises a synthetic Fv fragment. Suitably, the synthetic Fv fragment is stabilised. Exemplary synthetic stabilised Fv fragments include single chain Fv fragments (sFv, frequently termed scFv) in which a peptide linker is used to bridge the N terminus or C terminus of a V*_{H}* domain with the C terminus or N-terminus, respectively, of a V*_{L}* domain. ScFv lack all constant parts of whole antibodies and are not able to activate complement. Suitable peptide linkers for joining the V*_{H}* and V*_{L}* domains are those which allow the V*_{H}* and V*_{L}* domains to fold into a single polypeptide chain having an antigen binding site with a three dimensional structure similar to that of the antigen binding site of a whole antibody from which the Fv fragment is derived. Linkers having the desired properties may be obtained by the method disclosed in U.S. Patent No 4,946,778. However, in some cases a linker is absent.

ScFvs may be prepared, for example, in accordance with methods outlined in Krebber *et. al.,* 1997. Alternatively, they may be prepared by methods described in U.S. Patent No 5,091,513, European Patent No 239,400 or the articles by Winter and Milstein, 1991 and Plückthun et. al., 1996, Antibody engineering: A practical approach. 203-252.

Alternatively, the synthetic stabilised Fv fragment comprises a disulphide stabilised Fv (dsFv) in which cysteine residues are introduced into the V*_{H}* and V*_{L}* domains such that in the fully folded Fv molecule the two residues will form a disulphide bond therebetween. Suitable methods of producing dsFv are described for example in Glockshuber *et. al.* 1990, Reiter *et. al.* 1994a, Reiter *et al.* 1994b, Reiter *et. al.* 1994c, Webber *et al.* 1995.

Also contemplated as sub-immunoglobulin-sized antigen binding molecules are single variable region domains (termed dAbs) as for example disclosed in Ward *et. al.* 1989, Hamers-Casterman *et al.* 1993, Davies & Riechmann, 1994.

In other embodiments, the sub-immunoglobulin-sized antigen-binding molecule is a "minibody". In this regard, minibodies are small versions of whole antibodies, which encode in a single chain the essential elements of a whole antibody. Suitably, the minibody is comprised of the V*_{H}* and V*_{L}* domains of a native antibody fused to the hinge region and CH3 domain of the immunoglobulin molecule as, for example, disclosed in U.S. Patent No 5,837,821.

In still other embodiments, the sub-immunoglobulin-sized antigen binding molecule comprises non-immunoglobulin derived, protein frameworks. For example, reference may be made to Ku & Schultz, 1995, which discloses a four-helix bundle protein cytochrome b562 having two loops randomised to create complementarity determining regions (CDRs), which have been selected for antigen binding.

In some embodiments, the sub-immunoglobulin-sized antigen-binding molecule comprises a modifying moiety. In illustrative examples of this type, the modifying moiety modifies the effector function of the molecule. For instance, the modifying moiety may comprise a peptide for detection of the antigen-binding molecule, for example in an immunoassay. Alternatively, the modifying moiety may facilitate purification of the antigen-binding molecule. In this instance, the modifying moiety includes, but is not limited to, glutathione-S-transferase (GST), maltose binding protein (MBP) and hexahistidine (HIS₆), which are particularly useful for isolation of the antigen-binding molecule by affinity chromatography. For the purposes of purification by affinity chromatography, relevant matrices for affinity chromatography are glutathione-, amylose-, and nickel- or cobalt-conjugated resins respectively as is well known in the art.

The sub-immunoglobulin-sized antigen binding molecule may be multivalent (i.e., having more than one antigen binding site). Such multivalent molecules may be specific for one or more antigens (e.g., two target molecules expressed by a targeted cell). Multivalent molecules of this type may be prepared by dimerisation of two antibody fragments through a cysteinyl-containing peptide as, for example disclosed by Adams *et. al.,* 1993 and Cumber *et. al.,* 1992. Alternatively, dimerisation may be facilitated by fusion of the antibody fragments to amphiphilic helices that naturally dimerise (Pack and Plückthun, 1992) or by use of domains (such as the leucine zippers jun and fos) that preferentially heterodimerise (Kostelny *et. al.,* 1992). In other embodiments, the multivalent molecule comprises a multivalent single chain antibody (multi-scFv) comprising at least two scFvs linked together by a peptide linker. For example, non-covalently or covalently linked scFv dimers termed "diabodies" may be used in this regard. Multi-scFvs may be bispecific or greater depending on the number of scFvs employed having different antigen binding specificities. Multi-scFvs may be prepared for example by methods disclosed in U.S. Patent No. 5,892,020.

The compounds of formula (I) and formula (Ia) may be coupled to the cell targeting moiety by any suitable means known in the art. For example an amino or carboxy substituent on the compound of the invention may be coupled to a carboxy or amino substituent on the cell targeting moiety using general means for coupling carboxylic acids and amines. If the cell targeting moiety is an antibody or protein, care must be taken during any reaction steps, such as deprotection, to avoid denaturation of the antibody or protein.

Conjugates that comprise a compound of formula (I) or formula (Ia) and a cell-targeting moiety can be produced by any suitable technique known to persons of skill in the art. The present invention, therefore, is not dependent on, and not directed to, any one particular technique for conjugating these moieties.

The manner of attachment of a compound of formula (I) or formula (Ia) to a cell-targeting moiety should be such that the biological activity of each moiety is not substantially inhibited or impaired. A linker or spacer may be included between the moieties to spatially separate them. The linker or spacer molecule may be from about 1 to about 100 atoms in length. In some embodiments, the linker or spacer molecule comprises one or more amino acid residues (e.g., from about 1 to about 50 amino acid residues and desirably 1, 2, 3, 4, 5, 6, 7, 8, 9, 10, 15, 20 amino acid residues). Such linkers or spacers may facilitate the proper folding of the cell targeting moiety and the adoption of a desired conformation of the compound of formula (I) or formula (Ia).

Suitably, the compound of formula (I) or formula (Ia) is covalently attached to the cell-targeting moiety. Covalent attachment may be achieved by any suitable means known to persons of skill in the art. For example, a conjugate may be prepared by linking the cell targeting moiety and the compound of formula (I) or formula (Ia) using crosslinking reagents. Examples of such crosslinking agents include carbodiimides such as, but not limited to, 1-cyclohexyl-3-(2-morpholinyl-(4-ethyl)carbodiimide (CMC), 1-ethyl-3-(3-dimethylaminopropyl)carbodiimide (EDC) and 1-ethyl-3-(4-azonia-4,4-dimethylpentyl) carbodiimide. Exemplary crosslinking agents of this type are selected from the group consisting of 1-cyclohexyl-3-(2-morpholinyl-(4-ethyl)carbodiimide,(1-ethyl-3-(3-dimethylaminopropyl carbodiimide (EDC) and 1-ethyl-3-(4-azonia-4,4-dimethylpentyl)carbodiimide. Examples of other suitable crosslinking agents are cyanogen bromide, glutaraldehyde and succinic anhydride.

In general, any of a number of homobifunctional agents including a homobifunctional aldehyde, a homobifunctional epoxide, a homobifunctional imidoester, a homobifunctional N-hydroxysuccinimide ester, a homobifunctional maleimide, a homobifunctional alkyl halide, a homobifunctional pyridyl disulfide, a homobifunctional aryl halide, a homobifunctional hydrazide, a homobifunctional diazonium derivative and a homobifunctional photoreactive compound may be used. Also included are heterobifunctional compounds, for example, compounds having an amine-reactive and a sulfhydryl-reactive group, compounds with an amine-reactive and a photoreactive group and compounds with a carbonyl-reactive and a sulfhydryl-reactive group.

Homobifunctional reagents are molecules with at least two identical functional groups. The functional groups of the reagent generally react with one of the functional groups on a protein, typically an amino group. Specific examples of such homobifunctional crosslinking reagents include the bifunctional N-hydroxysuccinimide esters dithiobis(succinimidylpropionate), disuccinimidyl suberate, and disuccinimidyl tartrate; the bifunctional imidoesters dimethyl adipimidate, dimethyl pimelimidate, and dimethyl suberimidate; the bifunctional sulfhydryl-reactive crosslinkers 1,4-di-[3'-(2'-pyridyldithio)propionamido]butane, bismaleimidohexane, and bis-N-maleimido-1,8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamido)ethyl]disulfide; the bifunctional aldehydes formaldehyde, malondialdehyde, succinaldehyde, glutaraldehyde, and adipaldehyde; a bifunctional epoxide such as 1,4-butanediol diglycidyl ether, the bifunctional hydrazides adipic acid dihydrazide, carbohydrazide, and succinic acid dihydrazide; the bifunctional diazoniums o-toluidine, diazotized and bis-diazotized benzidine; the bifunctional alkylhalides N,N'-ethylene-bis(iodoacetamide), N,N'-hexamethylene-bis(iodoacetamide), N,N'-undecamethylene-bis(iodoacetamide), as well as benzylhalides and halomustards, such as α,α'-diiodo-p-xylene sulfonic acid and tri(2-chloroethyl)amine, respectively. Methods of using homobifunctional crosslinking reagents are known to practitioners in the art. For instance, the use of glutaraldehyde as a cross-linking agent is described for example by Poznansky *et. al.,* 1984. The use of diimidates as a cross-linking agent is described for example by Wang, *et. al.,* 1977.

Although it is possible to use homobifunctional crosslinking reagents for the purpose of forming a conjugate molecule according to the invention, skilled practitioners in the art will appreciate that it is more difficult to attach proteins and molecules in an ordered fashion with these reagents. In this regard, in attempting to link a protein with a compound of formula (I) or formula (Ia) by means of a homobifunctional reagent, one cannot prevent the linking of the protein to each other rather than the compound of formula (I) or formula (Ia). Accordingly, heterobifunctional crosslinking reagents are preferred because one can control the sequence of reactions, and combine proteins at will. Heterobifunctional reagents thus provide a more sophisticated method for linking two moieties. These reagents require one of the molecules to be joined, hereafter called Partner B, to possess a reactive group not found on the other, hereafter called Partner A, or else require that one of the two functional groups be blocked or otherwise greatly reduced in reactivity while the other group is reacted with Partner A. In a typical two-step process for forming heteroconjugates, Partner A is reacted with the heterobifunctional reagent to form a derivatised Partner A molecule. If the unreacted functional group of the crosslinker is blocked, it is then deprotected. After deprotecting, Partner B is coupled to derivatised Partner A to form the conjugate. Primary amino groups on Partner A are reacted with an activated carboxylate or imidate group on the crosslinker in the derivatisation step. A reactive thiol or a blocked and activated thiol at the other end of the crosslinker is reacted with an electrophilic group or with a reactive thiol, respectively, on Partner B. When the crosslinker possesses a reactive thiol, the electrophile on Partner B preferably will be a blocked and activated thiol, a maleimide, or a halomethylene carbonyl (*eg*. bromoacetyl or iodoacetyl) group. Because biological macromolecules do not naturally contain such electrophiles, they must be added to Partner B by a separate derivatisation reaction. When the crosslinker possesses a blocked and activated thiol, the thiol on Partner B with which it reacts may be native to Partner B.

An example of a heterobifunctional reagent is N-succinimidyl 3-(2-pyridyldithio)propionate (SPDP) (see for example Carlsson *et. al.,* 1978). Other heterobifunctional reagents for linking proteins include for example succinimidyl 4-(N-maleimidomethyl)cyclohexane-1-carboxylate (SMCC) (Yoshitake *et. al.,* 1979), 2-iminothiolane (IT) (Ju *et. al.,* 1978), and S-acetyl mercaptosuccinic anhydride (SAMSA) (Klotz and Heiney, 1962). All three react preferentially with primary amines (e.g., lysine side chains) to form an amide or amidine group which links a thiol to the derivatised molecule *via* a connecting short spacer arm, one to three carbon atoms long.

Another example of a heterobifunctional reagent is N-succinimidyl 3-(2-pyridyldithio)butyrate (SPDB) (Worrell *et. al.,* 1986), which is identical in structure to SPDP except that it contain a single methyl-group branch alpha to the sulfur atom which is blocked and activated by 2-thiopyridine. SMPT and SMBT described by Thorpe *et al.* 1987, contain a phenylmethyl spacer arm between an N-hydroxysuccinimide-activated carboxyl group and the blocked thiol; both the thiol and a single methyl-group branch are attached to the aliphatic carbon of the spacer arm. These heterobifunctional reagents result in less easily cleaved disulfide bonds than do unbranched crosslinkers.

Some other examples of heterobifunctional reagents containing reactive disulfide bonds include sodium S-4-succinimidyloxycarbonyl-α-methylbenzylthiosulfate, 4-succinimidyl-oxycarbony-α-methyl-(2-pyridyldithio)toluene.

Examples of heterobifunctional reagents comprising reactive groups having a double bond that reacts with a thiol group include SMCC mentioned above, succinimidyl m-maleimidobenzoate, succinimidyl 3-(maleimido)propionate, sulfosuccinimidyl 4-(p-maleimidophenyl)butyrate, sulfosuccinimidyl 4-(N-maleimidomethylcyclohexane-1-carboxylate and maleimidobenzoyl-N-hydroxysuccinimide ester (MBS).

Other heterobifunctional reagents for forming conjugates of two molecules are described for example by Rodwell *et al* in U.S. Pat. No. 4,671,958 and by Moreland et al. in U.S. Pat. No. 5,241,078.

Crosslinking of the cell-targeting moiety and the compound of formulae (I), (Ia), (Ib), (Ic) or (Id) may be accomplished by coupling a carbonyl group to an amine group or to a hydrazide group by reductive amination.

Specific antibodies may be used to target specific cells and therefore diseases or conditions that are related to unwanted or damaged cells that are targeted or the proliferation of such cells. For example, antibodies CD 19, CD20, CD22 and CD79a are able to target B cells, therefore can be used to deliver the compound of formulae (I), (Ia), (Ib), (Ic) or (Id) to a B cell to regulate apoptosis in unwanted or damaged B cells. Disorders and conditions that are characterised by unwanted or damaged B cells or the unwanted proliferation of B cells include B cell non-Hodgkins Lymphoma, B cell acute lymphoblastic leukemia (B-ALL) and autoimmune diseases related to B cells such as rheumatoid arthritis, systemic Lupus erythematosis and related arthropathies. Antibodies such as CD2, CD3, CD7 and CD5 are able to target T cells and therefore can be used to deliver the compound of formulae (I), (Ia), (Ib), (Ic) or (Id) to a T cell to regulate apoptosis in unwanted or damaged T cells. Disorders and conditions that are characterised by unwanted or damaged T cells or the unwanted proliferation of T cells include T cell acute lymphoblastic leukemia (T-ALL), T cell non-Hodgkins Lymphoma and T cell mediated autoimmune diseases such as Graft vs Host disease. Antibodies CD 13 and CD33 are able to target myeloid cells and therefore can be used to deliver the compound of formulae (I), (Ia), (Ib), (Ic) or (Id) to a myeloid cell to regulate apoptosis in unwanted or damaged myeloid cells. Diseases and conditions that are characterised by unwanted or damaged myeloid cells or the unwanted proliferation of myeloid cells include acute myelogenous leukemia (AML), chronic myelogenous leukemia (CML) and chronic myelomonocytic leukemia (CMML). The antibody CD138 is able to target plasma cells therefore can be used to deliver the compound of formulae (I), (Ia), (Ib), (Ic) or (Id) to plasma cells to regulate apoptosis in unwanted or damaged plasma cells. Diseases and conditions that are characterised by unwanted or damaged plasma cells or the unwanted proliferation of plasma cells include multiple myeloma.

Other cell targeting moieties can also be used to target specific cells. Luteinizing hormone-releasing hormone (LHRH) receptor is expressed in several types of cancer cells, such as ovarian cancer cells, breast cancer cells and prostate cancer cells, but is not expressed in healthy human viceral organs. LHRH can be used as a cell targeting moiety to deliver the compound of formulae (I), (Ia), (Ib), (Ic) or (Id) to cells expressing LHRH receptor. Disorders or conditions that are able to be treated with a conjugate comprising an LHRH-cell-targeting moiety and a compound of formulae (I), (Ia), (Ib), (Ic) or (Id) include ovarian cancer, breast cancer and prostate cancer.

The invention will now be described with reference to the following examples which illustrate some preferred aspects of the present invention. However, it is to be understood that the particularity of the following description of the invention is not to supersede the generality of the preceding description of the invention.

### EXAMPLES

### General Synthetic Procedures

### Preparation Method 1:

Amides (1) were prepared by the method shown in Scheme 4. An appropriately substituted benzoic acid was refluxed in neat SOCl₂ (150µL for 1 mmol of acid). The resulting acid chloride was dissolved in dichloromethane and treated at 0°C successively with triethylamine (1.2 equivalents) and an amine substituted with R³ (1.2 equivalents). The reaction was stirred at room temperature for 16 hours. The resulting amide was washed with 2M HCl, followed by saturated NaHCO₃, then brine. The resulting solution was then dried over MgSO₄.

### Preparation Method 2: Biphenyl compounds prepared by Suzuki coupling

Biphenyl compounds at R³ and R⁴ may be introduced using Suzuki coupling between para- or meta-halogenated phenyl derivatives and substituted phenyl boronic acids. The two starting materials (1.1 equivalent of phenyl boronic acid) are dissolved in toluene (2.2 mL for 1 mmol of halogenated phenyl derivative). Ethanol (530 µL for 1 mmol of halogenated phenyl derivative), 2N Na₂CO₃ (1 mL for 1 mmol of halogenated phenyl derivative) and 5 mol% Pd(PPh₃)₄ were added successively to the starting mixture. The reaction was stirred at 80°C until the palladium precipitates. The resulting reaction mixture was dissolved with ethyl acetate and poured onto water. The aqueous phase was extracted three times with ethyl acetate. The combined organic layers were washed with water and brine, dried over MgSO₄ and concentrated under vacuum.

### Preparation Method 3: Preparation of Carbamoyl chloride

To a stirred solution or suspension of amide from Preparation Method 1 in ether (2mL for 0.5 mmol) was added dry triethylamine (1.1 equivalent/amide). Trimethylsilyltriflate was then added (1.1 equivalent/amide). The reaction was stirred under nitrogen atmosphere at room temperature for 16 hours. After that time, an orange oil formed and was removed via a syringe. To the clear remaining solution was added at 0°C a solution of phosgene in toluene (20% in toluene, 100*µ*L for 0.1 mmol). The reaction was then slowly warmed up to room temperature and stirred over a 4 hour period. After this time the reaction flask was connected to a vacuum line and the reaction was concentrated leaving the carbamoylchloride as a thick oil residue. The compound was then used directly in the next step without further purification.

### Preparation method 4: General method for in-situ protection of amino acids

To a suspension of amino acid or amino acid hydrochloride in acetonitrile (4 mL per 1 mmol amino acid) was added successively propylene oxide (2 mL per 1 mmol amino acid) and N,O-bistrimethylsilylacetamide (1.5 equivalent per 1 mmol amino acid). The reaction mixture was stirred at room temperature under nitrogen for 30 minutes after which time it was used directly in the following step.

### Preparation Method 5: General reaction between carbamoylchloride and protected amino acid

To a solution of carbamoylchloride from Preparation Method 3 in acetonitrile (1 mL for 0.5 mmol) was added a mixture of in-situ protected amino-acid from Preparation Method 4 (1.2 equivalent amino-acid/carbamoylchloride) in acetonitrile at 0°C. The ice bath was then removed and the reaction was stirred at room temperature for 1 hour. After completion of the reaction as shown by TLC (CH₂Cl₂), the reaction mixture was diluted with ethyl acetate and poured onto 2N HCl (5mL for 1 mmol carbamoylchloride). The aqueous phase was extracted three times with ethyl acetate and the combined organic phases were washed with brine, dried over MgSO₄ and concentrated. The compounds were purified using silica gel (SiO₂) and CH₂Cl₂/MeOH/AcOH 99:0.5:0.5. The purified product was dissolved in toluene, then concentrated three times before drying under high vacuum.

### Preparation Method 6: Synthesis of Boc-protected cysteine derivatives.

According to Seko et al., (2003), cysteine was reacted in EtOH (1 mL per mmol) with 2 equivalents of NaOH 2M, 3% of tetrabutylammonium iodide and an alkyl halide (in case of alkyl iodide, tetrabutylammonium iodide was omitted) for three days at room temperature. After this time Boc₂O was added (250 *µ*L per mmol) and the reaction was stirred at room temperature for a further 24 hours. The reaction mixture was then concentrated. Cold 1N HCl was added (1.65 mL per mmol) followed by AcOEt and water. The aqueous phase was extracted 3 times with AcOEt. The combined organic layers were washed with water and brine and dried over MgSO₄. Concentration of the organic phase afforded compounds which were pure enough to be engaged in following step without further purification.

### Preparation Method 7: Synthesis of benzoylurea from Boc-protected cysteine derivatives.

The Boc-protected cysteine derivatives were dissolved in 1,4-dioxane (1.43 mL per mmol) and HCl 4N in dioxane (1.43 mL per mmol) was added. The deprotection reaction was followed by TLC and upon completion of the reaction, the mixture were concentrated. The residue was dried in vacuo and redissolved in EtOH (1 mL per mmol) and treated with 1 equivalent of NaOH 2M. To this reaction mixture was added 1.1 equivalent of a CH₃CN solution of carbamoylchloride prepared according to preparation method 3. The mixtures were concentrated down. At this stage compounds can be redissolved in MeOH and purified HPLC semi-preparative (see detail for each compound). Alternatively, the residue was dissolved in dichloromethane and treated two times with 2N HCl. The organic solution was purified directly by flash chromatography (see detail for each compound).

### Preparation Method 8: Synthesis of benzoylurea from unprotected amino-acids.

The amino acid is suspended in EtOH (1 mL per mmol) and treated with 1 equivalent of NaOH 2M (in the case of an amino acid hydrochloride salt, 2 equivalents of NaOH 2M are used). To this reaction mixture was added 1.1 equivalent of a CH₃CN solution of carbamoylchloride prepared according to preparation method 3. The mixtures were concentrated down and purified either by flash chromatography of HPLC semi-preparative (see detail for each compound).

### Preparation Method 9: Parallel synthesis of S-substituted cysteine derived benzoylureas.

Cysteine was reacted in EtOH (1 mL per mmol) with 2 equivalents of NaOH 2M, 3 % of tetrabutylammonium iodide and 1 equivalent of an alkyl halide (in case of alkyl iodide, tetrabutylammonium iodide was omitted) for three days at room temperature. To this reaction mixture was added 1 equivalent of carbamoylchloride prepared according to preparation method 3 in CH₃CN (4 mL per mmol). Stirring was applied for thirty minutes. The mixture was concentrated down. The residue was dissolved in EtOAc and treated two times with 2N HCl. The organic solution can be purified by flash chromatography (see detail for each compound). Alternatively, the desired compound may be isolated by passing through a SAX Acetate solid phase extraction column.

### Preparation Method 10: Synthesis of ethynylbenzoylureas.

(3-iodobenzoyl)urea or derivative thereof was dissolved in DMF/Et₃N 80:20 (5 mL per mmol) along with CuI (0.2 equivalent) and a substituted ethyne (1.5 equivalent). Pd(PPh₃)₂Cl₂ (0.1 equivalent) was added and stirring at room temperature was applied for 16 hours. The reaction mixture was diluted with EtOAc then filtered. The solution was washed twice with HCl 2N, then once with water, then once with brine. The organic phase was dried over MgSO₄, filtered and concentrated. The residue was passed through a SAX Acetate solid phase extraction column with MeOH 100 % then MeOH/AcOH 85:15 to obtain the desired compound.

### Preparation Method 11: Coupling amino acid-derived benzoylureas to sulfonamides.

A substituted benzoylurea derived from an amino acid was dissolved in CH₂Cl₂ (10 mL per mmol) with an arenesulfonamide (1 equivalent), DMAP (2 equivalents), and EDAC (2 equivalents). Stirring was applied at room temperature for 24 hours. The reaction mixture was diluted with EtOAc then washed twice with HCl 2N, then washed twice with water, then washed once with brine. The organic solution was dried over MgSO₄, filtered, and concentrated to yield the desired product. Reaction conditions and work up procedures were similar to those used by Oltersdorf et. al., 2005 and in US Patent Application No. 20020086887.

### Example 1: Compound (1)

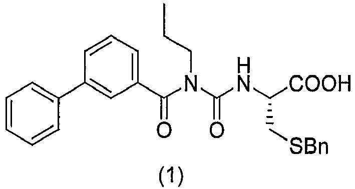

### 1A: N-n-propyl-3-bromobenzamide

Using Preparation Method 1,3-bromobenzoic acid was reacted with *n*-propylamine. The resulting reaction mixture was purified using SiO₂ with CH₂Cl₂ (100%) to CH₂Cl₂/MeOH 99:1 to give N-*n*-propyl-3-bromobenzamide as an off-white solid (79%). NMR ¹H (ppm, CDCl₃): 7.86 (s, 1H), 7.63 (d, *J*³ = 7.74 Hz, 1H), 7.50 (d, *J*³ = 7.00 Hz, 1H), 7.19-7.13 (m, 1H), 7.02 (br. s., 1H), 3.33-3.27 (m, 2H), 1.54 (sext., *J*³ = 7.34 Hz, 2H), 0.88 (t, *J*³ = 7.42 Hz, 3H).

### 1B: N-n-propyl-3-phenylbenzamide

Using Preparation Method 2, N-*n*-propyl-3-bromobenzamide from Example 1A was reacted with phenyl boronic acid. The resulting reaction mixture was purified using SiO₂ with CH₂Cl₂ (100%) to CH₂Cl₂/MeOH 90:10 to give N-*n*-propyl-3-phenylbenzamide as a white solid (96%). NMR ¹H (ppm, CDCl₃): 7.97 (t, *J*⁴ = 1.53 Hz, 1H), 7.69 (dd, *J*³ = 7.91 Hz, *J*⁴ = 1.84 Hz, 2H), 7.61-7.58 (m, 2H), 7.51-7.24 (m, 4H), 6.17 (br. s., 1H), 3.47-3.40 (m, 2H), 1.65 (sext., *J*³ = 7.32 Hz, 2H), 0.98 (t, *J*³ = 7.38 Hz, 3H).

### 1C: Compound (1)

Using Preparation Method 3, N-*n*-propyl-3-phenylbenzamide was reacted with phosgene to provide a carbamoylchloride. Using Preparation Method 4, Trimethylsilyl (TMS) protected S-benzyl-(L)-cysteine was prepared. The carbamoylchloride and TMS-protected S-benzyl-(L)-cysteine were reacted using Preparation Method 5 to provide compound (1) as a colourless glassy oil (44%). NMR ¹H (ppm, CDCl₃): 9.68 (d, *J*³ = 6.98 Hz, 1H), 7.72-7.66 (m, 2H), 7.60-7.56 (m, 2H), 7.53-7.19 (m, 10H), 6.4 (br. s., 1H), 4.74 (m, 1H), 3.78 (s, 2H), 3.70 (m, 2H), 3.03-2.87 (m, 2H), 1.56 (sext., *J*³ = 7.5 Hz, 2H), 0.73 (t, *J*³ = 7.4 Hz, 3H).

### Example 2: Compound (2)

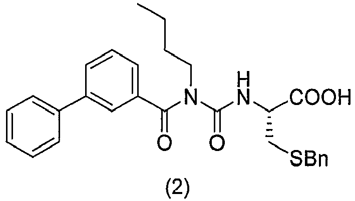

### 2A: N-n-butyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. *n-*Butylamine (247 *µ*L, 2.5 mmol) was then added into half of the acid chloride solution at 0°C. The reaction was then stirred at room temperature for 18 hours. The reaction was concentrated and water was added to the residue. The aqueous phase was extracted 3 times with CH₂Cl₂. The combined organic layers were washed with 2N HCl, saturated NaHCO₃, brine, dried over Na₂SO₄ and concentrated. Diethyl ether was added to the residue and a white solid precipitated. It was collected by filtration and rinsed with a small amount of diethyl ether. The white solid was then dried under vacuum (88 mg, 70%). NMR ¹H (ppm, CDCl₃): 7.96 (s, 1H), 7.69 (d, *J*³ = 8.01 Hz, 2H), 7.59 (d, *J*³ = 7.16 Hz, 2H), 7.51-7.34 (m, 4H), 6.11 (br. s., 1H), 3.51-3.44 (m, 2H), 1.64 (sext., *J*³ = 7.59 Hz, 2H), 1.43 (quint., *J*³ = 7.98 Hz, 2H), 0.96 (t, *J*³ = 7.32 Hz, 3H).

### 2B: Compound (2)

Using Preparation Method 3, N-*n*-butyl 3-phenylbenzamide was reacted with phosgene to provide a carbamoylchloride. Using Preparation Method 4, trimethylsilyl (TMS) protected S-benzyl-(L)-cysteine was prepared. The carbamoylchloride and TMS protected S-benzyl-(L)-cysteine were reacted using Preparation Method 5 to provide compound (2) as a colourless glassy oil (44%). NMR ¹H (ppm, CDCl₃): 9.68 (d, *J*³ = 6.92 Hz, 1H), 7.73-7.66 (m, 2H), 7.59-7.58 (m, 2H), 7.49-7.37 (m, 5H), 7.33-7.23 (m, 5H), 4.75 (m, 1H), 3.77 (s, 2H), 3.66 (m, 2H), 3.04-2.87 (m, 2H), 1.52 (q., *J*³ = 7.8 Hz, 2H), 1.12 (sext., *J*³ = 7.4 Hz, 2H), 0.72 (t, *J*³ = 7.3 Hz, 3H).

### Example 3: Compound (3)

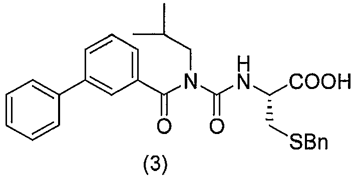

### 3A: N-isobutyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. Isobutylamine (247 *µ*L, 2.5 mmol) was then added into half of the acid chloride solution at 0°C. The reaction was then stirred at room temperature for 18 hours. The reaction was concentrated and water was added to the residue. The aqueous phase was extracted 3 times with CH₂Cl₂. The combined organic layers were washed with 2N HCl, saturated NaHCO₃, brine, dried over Na₂SO₄ and concentrated. Diethyl ether was added to the residue and a white solid precipitated. It was collected by filtration and rinsed with a small amount of diethyl ether. The white solid was then dried under vacuum (102 mg, 71%). NMR ¹H (ppm, CDCl₃): 7.97 (t, *J*⁴ = 1.71 Hz, 1H), 7.70 (dd, *J*³ = 7.65 Hz, *J*⁴ = 1.77 Hz, 2H), 7.62-7.58 (m, 2H), 7.51-7.34 (m, 4H), 6.18 (br. s., 1H), 3.33-3.29 (m, 2H), 1.91 (n, *J*³ = 6.71 Hz, 1H), 0.98 (d, *J*³ = 6.68 Hz , 6H).

### 3B: Compound (3)

Using Preparation Method 3, N-isobutyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (3) as a colourless glassy oil (73%). NMR ¹H (ppm, CDCl₃): 9.57 (d, *J*³ = 5.91 Hz, 1H), 7.67 (s, 2H), 7.57-7.55 (m, 2H), 7.49-7.36 (m, 5H), 7.31-7.19 (m, 5H), 4.72 (m, 1H), 3.75 (s, 2H), 3.67 (m, 2H), 3.03-2.86 (m, 2H), 1.87 (n., *J*³ = 6.8 Hz, 1H), 0.73 (d, *J*³ = 6.4 Hz, 6H).

### Example 4: Compound (4)

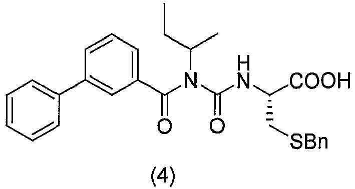

### 4A: N-(+/-)-sec-butyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. *Sec*-butylamine (253 *µ*L, 2.5 mmol) was then added into half of the acid chloride solution at 0°C. The reaction was then stirred at room temperature for 18 hours. The reaction was concentrated and water was added to the residue. The aqueous phase was extracted 3 times with CH₂Cl₂. The combined organic layers were washed with 2N HCl, saturated NaHCO₃, brine, dried over Na₂SO₄ and concentrated. Diethyl ether was added to the residue and a white solid precipitated. It was collected by filtration and rinsed with a small amount of diethyl ether. The white solid was then dried under vacuum (89 mg, 70%). NMR ¹H (ppm, CDCl₃): 7.95 (s, 1H), 7.68 (d, *J*³ = 7.51 Hz, 2H), 7.59 (d, *J*³ = 7.63 Hz, 2H), 7.50-7.33 (m, 4H), 5.97 (br. s., 1H), 4.30 (hept., *J*³ = 6.86 Hz, 1H), 1.58 (quint., *J*³ = 7.26 Hz, 1H), 1.23 (d, *J*³ = 6.56 Hz, 3H), 0.97 (t, *J*³ = 7.36 Hz, 3H).

### 4B: Compound (4)

Using Preparation Method 3, N-(+/-)-*sec*-butyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (4) as a colourless glassy solid (86%). NMR ¹H (ppm, CDCl₃), mixture of diastereoisomers: 9.39 (br. s., 1H), 8.63 and 8.54 (d, *J*³ = 6.98 and 7.03 Hz, 1H), 7.73 (s, 1H), 7.69-7.67 (m, 1H), 7.59-7.56 (m, 2H), 7.52-7.34 (m, 5H), 7.29-7.13 (m, 5H), 4.64 (m, 1H), 4.14-4.02 (m, 1H), 3.70 (s, 2H), 2.86-2.79 (m, 2H), 2.13-2.00 (m, 1H), 1.76-1.63 (m, 1H), 1.48 and 1.46 (d, *J*³ = 6.6 and 6.63 Hz, 3H), 0.87 and 0.86 (d, *J*³ = 7.4 and 7.4 Hz, 3H).

### Example 5: Compound (5)

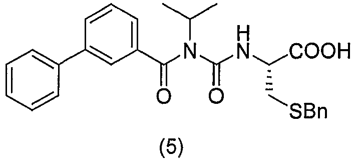

### 5A: N-isopropyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. Isopropylamine (511 *µ*L, 6 mmol) was then added into the acid chloride solution at 0°C. The reaction was then stirred at room temperature for 18 hours. The reaction was concentrated and water was added to the residue. The aqueous phase was extracted 3 times with CH₂Cl₂. The combined organic layers were washed with 2N HCl, saturated NaHCO₃, brine, dried over Na₂SO₄ and concentrated. The residue was purified by flash chromatography: SiO₂, CH₂Cl₂/AcOEt 95:5 to give a white solid (167 mg, 58%). NMR ¹H (ppm, CDCl₃): 7.96 (s, 1H), 7.69 (d, *J*³ = 7.68 Hz, 2H), 7.59 (d, *J*³ = 7.14 Hz, 2H), 7.50-7.33 (m, 4H), 5.93 (br. s., 1H), 4.14 (oct., *J*³ = 6.66 Hz, 1H), 1.27 (d, *J*³ = 6.43 Hz, 6H).

### 5B: Compound (5)

Using Preparation Method 3, N-isopropyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (5) as a colourless glassy oil (63%). NMR ¹H (ppm, CDCl₃),: 8.73 (br. s., 1H), 8.52 (d, *J*³ = 7.01 Hz, 1H), 7.73 (s, 1H), 7.71-7.63 (m, 1H), 7.59-7.56 (m, 5H), 7.28-7.09 (m, 5H), 4.67-4.60 (m, 1H), 4.37 (h., *J*³ = 6.77 Hz, 1H), 3.68 (s, 2H), 2.80 (d, *J*³ = 5.8 Hz, 2H), 1.48-1.44 (m, 6H).

### Example 6: Compound (6)

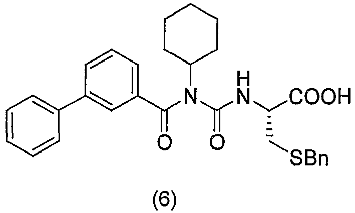

### 6A: N-cyclohexyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. Cyclohexylamine (286 *µ*L, 2.5 mmol) was then added into half of the acid chloride solution at 0°C. The reaction was then stirred at room temperature for 18 hours. The reaction was concentrated and water was added to the residue. The aqueous phase was extracted 3 times with CH₂Cl₂ and the combined organic layers were washed with 2N HCl, saturated NaHCO₃, brine, dried over Na₂SO₄ and concentrated. Diethyl ether was added to the residue and a white solid precipitated which was collected by filtration and rinsed with a small amount of diethyl ether. The white solid was then dried under vacuum (108 mg, 80%). NMR ¹H (ppm, CDCl₃): 7.95 (s, 1H), 7.68 (d, *J*³ = 7.82 Hz, 2H), 7.59 (d, *J*³ = 7.13 Hz, 2H), 7.49-7.33 (m, 4H), 6.05 (br. d., *J*³ = 6.58 Hz, 1H), 3.75-3.70 (m, 1H), 2.06-2.01 (m, 2H), 1.78-1.61 (m, 3H), 1.49-1.36 (m, 2H), 1.30-1.14 (m, 3H).

### 6B: Compound (6)

Using Preparation Method 3, N-cyclohexyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (6) as a white solid (60%). NMR ¹H (ppm, CDCl₃),: 8.8 (br. s., 1H), 7.92 (d, *J*³ = 7.09 Hz, 1H), 7.74 (m, 1H), 7.66 (d.t., *J*³ = 7.15 Hz, *J*⁴ = 1.7 Hz, 2H), 7.55 (d, *J*³ = 6.95 Hz, 2H), 7.50-7.33 (m, 5H), 7.29-7.12 (m, 5H), 4.58-4.52 (m, 1H), 4.05-3.98 (m, 1H), 3.61 (s, 2H), 2.76-2.64 (m, 2H), 2.19-2.12 (m, 2H), 1.84-1.77 (m, 4H), 1.56 (br. s., 1H), 1.34 (br. s., 3H).

### Example 7: Compound (7)

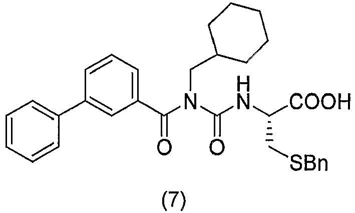

### 7A: N-cyclohexylmethyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. Cyclohexylmethylamine (325 *µ*L, 2.5 mmol) was then added into half of the acid chloride solution at 0°C. The reaction mixture was then stirred at room temperature for 18 hours. The reaction mixture was concentrated and water was added to the residue. The aqueous phase was extracted 3 times with CH₂Cl₂. The combined organic layers were washed with 2N HCl, saturated NaHCO₃, brine, dried over Na₂SO₄ and concentrated. Diethyl ether was added to the residue and a white solid precipitated which was collected by filtration and rinsed with a small amount of diethyl ether. The white solid was then dried under vacuum (115 mg, 78%). NMR ¹H (ppm, CDCl₃): 7.95 (s, 1H), 7.69 (d, *J*³ = 7.80 Hz, 2H), 7.59 (d, *J*³ = 8.22 Hz, 2H), 7.50-7.33 (m, 4H), 6.22 (br. s., 1H), 3.31 (t, *J*³ = 6.29 Hz, 2H), 1.80-1.53 (m, 6H), 1.31-1.10 (m, 3H), 1.05-0.84 (m, 2H).

### 7B: Compound (7)

Using Preparation Method 3, N-cyclohexylmethyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (7) as a glassy colourless oil (86%). NMR ¹H (ppm, CDCl₃),: 9.92 (br. s., 1H), 9.60 (d; *J*³ = 7.07 Hz, 1H), 7.72-7.70 (m, 2H), 7.62-7.59 (m, 2H), 7.54-7.44 (m, 4H), 7.41-7.21 (m, 6H), 4.83-4.77 (m, 1H), 3.79 (s, 2H), 3.72 (d, *J*³ = 6.84 Hz, 2H), 3.06-2.89 (m, 2H), 1.62-1.59 (m, 6H), 1.22-0.99 (m, 3H), 0.76-0.64 (m, 2H).

### Example 8: Compound (8)

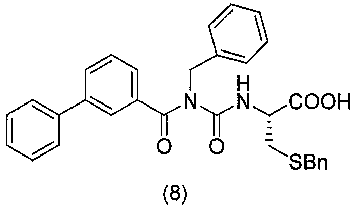

### 8A: N-benzyl 3-phenylbenzamide

Oxalyl chloride (132 *µ*L, 1.5 mmol) was added over a 10 minute period to a mixture of 3-phenylbenzoic acid (200 mg, 1 mmol) dissolved in a mixture THF/DMF (3.5 mL/58 *µ*L). After the addition, the reaction was stirred at room temperature for 2.5 hours. Benzylamine (268 *µ*L, 2.5 mmol) was then added into half of the acid chloride solution at 0°C. The reaction mixture was then stirred at room temperature for 18 hours. The reaction mixture was concentrated and water was added to the residue. The aqueous phase was extracted 3 times with CH₂Cl₂ and the combined organic layers were washed with 2N HCl, saturated NaHCO₃, brine, dried over Na₂SO₄ and concentrated. Diethyl ether was added to the residue and a white solid precipitated which was collected by filtration and rinsed with a small amount of diethyl ether. The white solid was then dried under vacuum (105 mg, 73%). NMR ¹H (ppm, CDCl₃): 8.01 (s, 1H), 7.72 (t, *J*³ = 7.89 Hz, 2H), 7.58 (d, *J*³ = 7.14 Hz, 2H), 7.49-7.24 (m, 9H), 6.57 (br. s., 1H), 4.64 (d, *J*³ = 5.29 Hz, 2H).

### 8B: Compound (8)

Using Preparation Method 3, N-benzyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (8) as a colourless glassy oil (66%). NMR ¹H (ppm, CDCl₃),: 9.81 (d, *J*³ = 7.15 Hz, 1H), 9.33 (br. s., 1H), 7.66 (d.t., *J*³ = 7.86 Hz, *J*⁴ = 1.44 Hz, 1H), 7.49 (t, *J*⁴ = 1.54 Hz, 1H), 7.46-7.16 (m, 10H), 7.05 (d.d., *J*³ = 7.91 Hz, *J*⁴ = 1.97 Hz, 2H), 5.02 (s, 2H), 4.86-4.79 (m, 1H), 3.79 (s, 2H), 3.07-2.90 (m, 2H).

### Example 9: Compound (11)

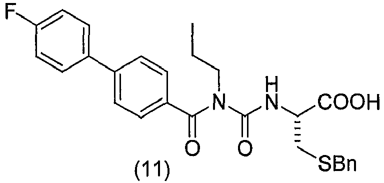

### 9A: N-n-propyl 4-bromobenzamide

Using Preparation Method 1, 4-bromobenzoic acid was reacted with *n*-propylamine. The resulting reaction mixture was purified using SiO₂ with 100% CH₂Cl₂ to give a white solid (67%). NMR ¹H (ppm, CDCl₃): 7.61 (d, *J*³ = 8.60 Hz, 2H), 7.54 (d, *J*³ = 8.57 Hz, 2H), 6.06 (br. s., 1H), 3.43-3.37 (m, 2H), 1.62 (sext., *J*³ = 7.25 Hz, 2H), 0.97 (t, *J*³ = 7.39 Hz, 3H).

### 9B: N-n-propyl 4-(4'-fluoro)-phenylbenzamide

Using Preparation Method 2, N-*n*-propyl 4-bromobenzamide was reacted with 4-fluorophenylboronic acid. The resulting reaction mixture was purified using SiO₂ with CH₂Cl₂/Petroleum Ether 80:20 to CH₂Cl₂/AcOH 80:20 to give a white solid (89%). NMR ¹H (ppm, CDCl₃): 7.81 (d, *J*³ = 8.18 Hz, 2H), 7.58 (d, *J*³ = 8.09 Hz, 2H), 7.56-7.53 (m, 2H), 7.13 (t, *J*³ = 8.54 Hz, 2H), 6.12 (br. s., 1H), 3.47-3.40 (m, 2H), 1.65 (sext., *J*³ = 7.26 Hz, 2H), 0.99 (t, *J*³ = 7.44 Hz, 3H).

### 9C: Compound (11)

Using Preparation Method 3, N-*n*-propyl 4-(4'-fluoro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (11) as a colourless glassy oil (81%). NMR ¹H (ppm, CDCl₃): 9.63 (d, *J*³ = 7.04 Hz, 1H), 9.43 (br. s., 1H), 7.62-7.52 (m, 6H), 7.35-7.23 (m, 5H), 7.14 (t, *J*³ = 8.64 Hz, 2H), 4.80-4.74 (m, 1H), 3.78 (s, 2H), 3.73 (m, 2H), 3.04-2.87 (m, 2H), 1.57 (sext., *J*³ = 7.45 Hz, 2H), 0.75 (t, *J*³ = 7.34 Hz, 3H).

### Example 10: Compound (12)

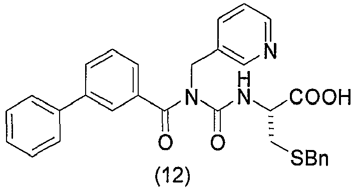

### 10A: N-(3-pyridylmethyl)-3-bromobenzamide

Using Preparation Method 1, 3-bromobenzoic acid was reacted with 3-pyridylmethylamine. The resulting reaction mixture was purified using SiO₂ with CH₂Cl₂/MeOH 95:5 to give N-(3-pyridylmethyl)-3-bromobenzamide as thick yellow oil (73%). NMR ¹H (ppm, CDCl₃): 8.44 (s, 1H), 8.41 (d, *J*⁴ = 3.9 Hz, 1H), 7.92 (s, 1H), 7.69 (d, *J*³ = 7.8 Hz, 1H), 7.64 (d, *J*³ = 7.8 Hz, 1H), 7.57-7.54 (m, 2H), 7.24-7.18 (m, 2H), 4.55 (d, *J*³ = 5.9 Hz, 2H).

### 10B: N-(3-pyridylmethyl)-3-phenylbenzamide

Using Preparation Method 2, N-(3-pyridylmethyl)-3-bromobenzamide was reacted with phenylboronic acid. The resulting reaction mixture was purified using SO₂ with CH₂Cl₂/MeOH 98:2 to 90:10. A colourless oil was obtained (87%). NMR ¹H (ppm, CDCl₃): 8.87 (s, 1H), 8.73 (d, *J*³ = 4.5 Hz, 1H), 8.28 (t, *J*⁴ = 2.0 Hz, 1H), 8.03-7.94 (m, 3H), 7.89 (d, *J*³ = 6.9 Hz, 2H), 7.75-7.50 (m, 5H), 6.17 (br. t., 1H), 4.91 (d, *J*³ = 5.9 Hz, 2H).

### 10C: Compound (12)

Using Preparation Method 3, N-(3-pyridylmethyl)-3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5. Work-up was followed without washing with HCl 2N. Compound (12) was obtained as a white solid (14%). NMR ¹H (ppm, CDCl₃): 9.35 (br. s., 1H), 8.45 (br. s., 2H), 7.65-7.24 (m, 16H), 5.15-4.90 (m, 2H), 4.68 (br. s., 1H), 3.71 (br. s., 2H), 2.95 (br. s., 2H).

### Example 11: Compound (13)

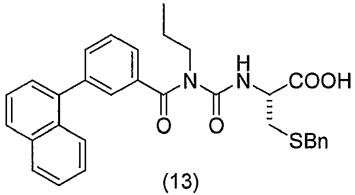

### 11A: N-n-propyl-3-(1-naphthyl)-benzamide

Using Preparation Method 2, N-*n*-propyl-3-bromobenzamide from Example 1A was reacted with 1-naphthylboronic acid. The resulting reaction mixture was purified using SiO₂ with CH₂Cl₂/Petroleum ether 80:20 to CH₂Cl₂/AcOH 80:20 to give a thick yellow oil (75%). NMR ¹H (ppm, CDCl₃): 7.92-7.78 (m, 5H), 7.67-7.40 (m, 6H), 6.16 (br. s., 1H), 3.50-3.36 (m, 2H), 1.64 (sext., *J*³ = 7.33 Hz, 2H), 0.97 (t, *J*³ = 7.38 Hz, 3H).

### 11B: Compound (13)

Using Preparation Method 3, N-*n*-propyl-3-(1-naphthyl)-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (13) as a colourless glassy oil (87%). NMR ¹H (major stereoisomer, ppm, CDCl₃): 10.76 (br. s., 1H), 9.72 (d, *J*³ = 6.99 Hz, 1H), 7.91 (t, *J*³ = 8.05 Hz, 2H), 7.82 (d, *J*³ = 8.18 Hz, 1H), 7.64-7.48 (m, 5H), 7.43 (t, *J*³ = 6.68 Hz, 2H), 7.34-7.21 (m, 4H), 7.17 (t, *J*³ = 7.57 Hz, 2H), 4.82-4.76 (m, 1H), 3.78 (s, 2H), 3.77 (m, 2H), 3.04-2.88 (m, 2H), 1.61 (sext., *J*³ = 7.46 Hz, 2H), 0.79 (t, *J*³ = 7.33 Hz, 3H).

### Example 12: Compound (14)

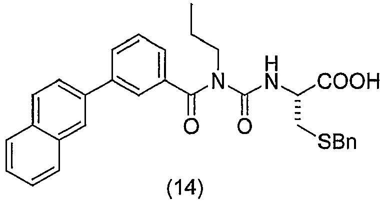

### 12A: N-n-propyl-3-(2-naphthyl)-benzamide

Using Preparation Method 2, N-*n*-propyl-3-bromobenzamide from Example 1A was reacted with 2-naphthylboronic acid. The resulting reaction mixture was purified using SiO₂ with CH₂Cl₂/Petroleum ether 80:20 to CH₂Cl₂/AcOH 80:20 to give a white solid (57%). NMR ¹H (ppm, CDCl₃): 8.12 (t, *J*⁴ = 1.65 Hz, 1H), 8.06 (s, 1H), 7.91 (t, *J*³ = 8.37 Hz, 2H), 7.83 (d, *J*³ = 7.81 Hz, 2H), 7.73 (td, *J*³ = 8.51 Hz, *J*⁴ = 1.79 Hz, 2H), 7.55-7.46 (m, 3H), 6.18 (br. s., 1H), 3.49-3.43 (m, 2H), 1.67 (sext., *J*³ = 7.31 Hz, 2H), 1.00 (t, *J*³ = 7.37 Hz, 3H).

### 12B: Compound (14)

Using Preparation Method 3, N-*n*-propyl-3-(2-naphthyl)-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (14) as a colourless glassy oil (88%). NMR ¹H (ppm, CDCl₃): 9.76 (d, *J*³ = 6.98 Hz, 1H), 9.67 (br. s., 1H), 8.07 (s, 1H), 7.94 (t, *J*³ = 8.49 Hz, 2H), 7.85 (d, *J*³ = 7.32 Hz, 3H), 7.74 (d.d., *J*³ = 8.50 Hz, *J*⁴ = 1.5 Hz, 1H), 7.60-7.46 (m, 4H), 7.37-7.25 (m, 5H), 4.85-4.79 (m, 1H), 3.82 (s, 2H), 3.77 (m, 2H), 3.08-2.92 (m, 2H), 1.63 (sext., *J*³ = 7.42 Hz, 2H), 0.78 (t, *J*³ = 7.34 Hz, 3H).

### Example 13: Compound (15)

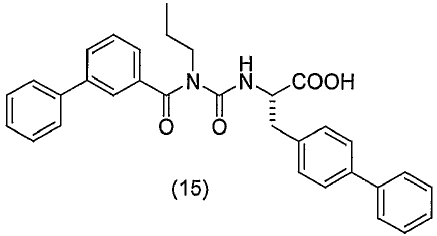

Using Preparation Method 3, N-*n*-propyl-3-phenylbenzamide from Example 1B was reacted with phosgene to provide a carbamoylchloride. Using Preparation Method 4, TMS protected (L)-4,4'-biphenylalanine was prepared. The carbamoylchloride and TMS protected (L)-4,4'-biphenylalanine were reacted using Preparation Method 5 to provide compound (15) as a colourless glassy oil (70%). NMR ¹H (ppm, CDCl₃): 10.1 (br. s., 1H), 9.53 (d, *J*³ = 6.91 Hz, 1H), 7.69 (d., *J*³ = 7.82 Hz, 1H), 7.64 (s, 1H), 7.59-7.56 (m, 6H), 7.52-7.32 (m, 10H), 4.93-4.86 (m, 1H), 3.67 (m, 2H), 3.40-3.16 (m, 2H), 1.52 (sext., *J*³ = 7.38 Hz, 2H), 0.70 (t, *J*³ = 7.36 Hz, 3H).

### Example 14: Compound (16)

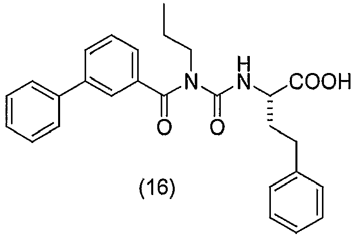

Using Preparation Method 3, N-*n*-propyl-3-phenylbenzamide from Example 1B was reacted with phosgene to provide a carbamoylchloride. Using Preparation Method 4, TMS protected (L)-homophenylalanine was prepared. The carbamoylchloride and TMS protected (L)-homophenylalanine were reacted using Preparation Method 5 to provide compound (16) as a colourless glassy oil (92%). NMR ¹H (ppm, CDCl₃): 10.28 (br. s., 1H), 9.56 (d, *J*³ = 7.02 Hz, 1H), 7.73-7.69 (m, 2H), 7.62-7.60 (m, 2H), 7.56-7.37 (m, 6H), 7.32-7.21 (m, 3H), 4.65-4.59 (m, 1H), 3.73 (m, 2H), 2.80 (t, *J*³ = 8.00 Hz, 2H), 2.39-2.08 (m, 2H), 1.59 (sext., *J*³ = 7.46 Hz, 2H), 0.76 (t, *J*³ = 7.37 Hz, 3H).

### Example 15: Compound (17)

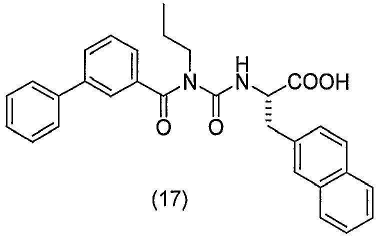

Using Preparation Method 3, N-*n*-propyl-3-phenylbenzamide from Example 1B was reacted with phosgene to provide a carbamoylchloride. Using Preparation Method 4, TMS protected (L)-2-naphthylalanine was prepared. The carbamoylchloride and TMS protected (L)-2-naphthylalanine were reacted using Preparation Method 5 to provide compound (17) as a colourless glassy oil (89%). NMR ¹H (ppm, CDCl₃): 11.00 (br. s., 1H), 9.56 (d, *J*³ = 6.99 Hz, 1H), 7.84-7.80 (m, 3H), 7.76 (s, 1H), 7.60-7.57 (m, 3H), 7.50-7.40 (m, 7H), 7.38-7.32 (m, 1H), 5.01-4.94 (m, 1H), 3.67-3.62 (m, 2H), 3.55-3.26 (m, 2H), 1.46 (sext., *J*³ = 7.38 Hz, 2H), 0.67 (t, *J*³ = 7.36 Hz, 3H).

### Example 16: Compound (18)

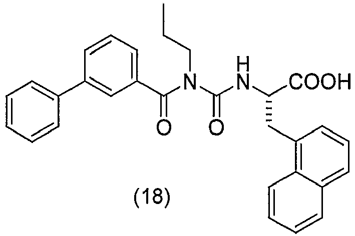

Using Preparation Method 3, N-*n*-propyl-3-phenylbenzamide from Example 1B was reacted with phosgene to provide a carbamoylchloride. Using Preparation Method 4, TMS protected (L)-1-naphthylalanine was prepared. The carbamoylchloride and TMS protected (L)-1-naphthylalanine were reacted using Preparation Method 5 to provide compound (18) as a colourless glassy oil (90%). NMR ¹H (ppm, CDCl₃): 10.9 (br. s., 1H), 9.56 (d, *J*³ = 6.52 Hz, 1H), 8.19 (d., *J*³ = 8.62 Hz, 1H), 7.87 (d.d., *J*³ = 8.03 Hz, *J*⁴ = 1.11 Hz, 1H), 7.79 (d.d., *J*³ = 6.81 Hz, *J*⁴ = 2.33 Hz, 1H), 7.67 (d.d.d., *J*³ = 7.72 Hz, *J*⁴ = 2.78 Hz, *J*⁴ = 1.02 Hz, 1H), 7.62-7.56 (m, 4H), 7.53-7.37 (m, 8H), 5.00-4.97 (m, 1H), 3.94-3.3.87 (m, 1H), 3.63-3.58 (m, 2H), 3.52-3.45 (m, 1H), 1.46 (sext., *J*³ = 7.55 Hz, 2H), 0.68 (t, *J*³ = 7.35 Hz, 3H).

### Example 17: Compound (19)

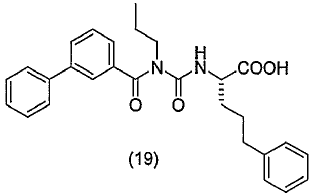

Using Preparation Method 3, N-*n*-propyl-3-phenylbenzamide from Example 1B was reacted with phosgene to provide a carbamoylchloride. Using Preparation Method 4, TMS protected (L)-2-amino-5-phenyl-pentanoic acid was prepared. The carbamoylchloride and TMS protected (L)-2-amino-5-phenyl-pentanoic acid were reacted using Preparation Method 5 to provide compound (19) as a colourless glassy oil (97%). NMR ¹H (ppm, CDCl₃): 9.44 (d, *J*³ = 7.00 Hz, 1H), 9.1 (br. s., 1H), 7.70 (d.d.d., *J*³ = 7.75 Hz, *J*⁴ = 2.98 Hz, *J*⁴ = 1.31 Hz, 1H), 7.67 (t., *J*⁴ = 1.57 Hz, 1H), 7.60-7.57 (m, 2H), 7.52 (d, *J*³ = 7.54 Hz, 1H), 7.48-7.38 (m, 5H), 7.29-7.22 (m, 1H), 7.19-7.14 (m, 3H), 4.61-4.55 (m, 1H), 3.71-3.67 (m, 2H), 2.67 (t, *J*³ = 7.76 Hz, 2H), 2.08-1.92 (m, 2H), 1.92-1.72 (m, 2H), 1.55 (sext., *J*³ = 7.52 Hz, 2H), 0.73 (t, *J*³ = 7.36 Hz, 3H).

### Example 18: Compound (20)

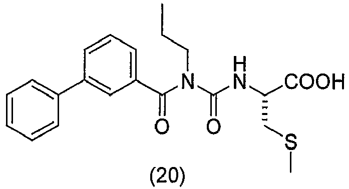

Using Preparation Method 3, N-*n*-propyl-3-phenylbenzamide from Example 1B was reacted with phosgene to provide a carbamoylchloride. Using Preparation Method 4, TMS protected S-methyl-(L)-cysteine was prepared. The carbamoylchloride and TMS protected S-methyl-(L)-cysteine were reacted using Preparation Method 5 to provide compound (20) as a colourless glassy oil (82%). NMR ¹H (ppm, CDCl₃): 9.85 (d, *J*³ = 6.85 Hz, 1H), 7.70 (dt, *J*³ = 7.71 Hz, *J*⁴ = 1.79 Hz, 1H), 7.66 (t, *J*⁴ = 1.34 Hz, 1H), 7.60-7.56 (m, 2H), 7.54-7.33 (m, 5H), 4.80-4.74 (m, 1H), 3.71 (m, 2H), 3.12-3.00 (m, 2H), 2.19 (s, 3H), 1.56 (sext., *J*³ = 7.53 Hz, 2H), 0.73 (t, *J*³ = 7.39 Hz, 3H).

### Example 19: Compound (21)

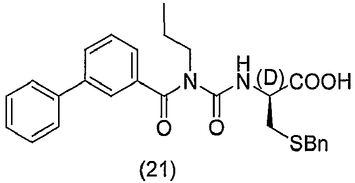

Using Preparation Method 3, N-*n*-propyl-3-phenylbenzamide from Example 1B was reacted with phosgene to provide a carbamoylchloride. Using Preparation Method 4, TMS protected S-benzyl-(D)-cysteine was prepared. The carbamoylchloride and TMS protected S-benzyl-(D)-cysteine were reacted using Preparation Method 5 to provide compound (21) as a colourless glassy oil (78%). NMR ¹H (ppm, CDCl₃): 9.70 (d, *J*³ = 7.00 Hz, 1H), 9.2 (br. s., 1H), 7.73-7.67 (m, 2H), 7.62-7.57 (m, 2H), 7.54-7.38 (m, 6H), 7.36-7.27 (m, 4H), 4.80-4.74 (m, 1H), 3.79 (s, 2H), 3.71 (m, 2H), 3.04-2.88 (m, 2H), 1.58 (sext., *J*³ = 7.53 Hz, 2H), 0.74 (t, *J*³ = 7.35 Hz, 3H).

### Example 20: Compound (22)

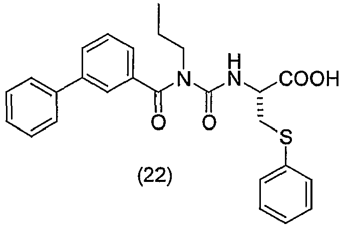

Using Preparation Method 3, N-*n*-propyl-3-phenylbenzamide from Example 1B was reacted with phosgene to provide a carbamoylchloride. Using Preparation Method 4, TMS protected S-phenyl-(L)-cysteine was prepared. The carbamoylchloride and TMS protected S-phenyl-(L)-cysteine were reacted using Preparation Method 5 to provide compound (22) as a colourless glassy oil. NMR ¹H (ppm, CDCl₃): 9.74 (d, *J*³ = 6.65 Hz, 1H), 7.70 (d, *J*³ = 7.81 Hz, 1H), 7.62-7.57 (m, 3H), 7.53-7.44 (m, 4H), 7.38 (t, *J*³ = 6.83, 2H), 7.28-7.22 (m, 4H), 4.77-4.72 (m, 1H), 3.63 (m, 2H), 3.58-3.24 (m, 2H), 1.50 (sext., *J*³ = 7.53 Hz, 2H), 0.70 (t, *J*³ = 7.36 Hz, 3H).

### Example 21: Compound (23)

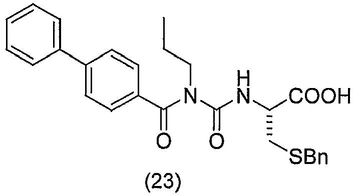

### 21A: N-n-propyl-4-phenylbenzamide

Using Preparation Method 2, N-*n*-propyl-4-bromobenzamide from Example 9A was reacted with phenylboronic acid. The resulting reaction mixture was purified using SiO₂ with CH₂Cl₂/Petroleum Ether 80:20 to CH₂Cl₂/AcOH 80:20 to give a white solid (71%). NMR ¹H (ppm, CDCl₃): 7.82 (d, *J*³ = 8.32 Hz, 2H), 7.63 (d, *J*³ = 8.69 Hz, 2H), 7.59 (d, *J*³ = 7.14 Hz, 2H), 7.45 (t, *J*³ = 7.09 Hz, 2H), 7.39-7.34 (m, 1H), 6.14 (br. s., 1H), 3.47-3.41 (m, 2H), 1.65 (sext., *J*³ = 7.18 Hz, 2H), 0.99 (t, *J*³ = 7.37 Hz, 3H).

### 21B: Compound (23)

Using Preparation Method 3, N-*n*-propyl-4-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (23) as a colourless glassy oil (82%). NMR ¹H (ppm, CDCl₃): 10.29 (br. s., 1H), 9.66 (d, *J*³ = 7.05 Hz, 1H), 7.67 (d, *J*³ = 8.3 Hz, 2H), 7.62 (d, *J*³ = 7.00 Hz, 2H), 7.54 (d, *J*³ = 8.32 Hz, 2H), 7.47 (t, *J*³ = 7.00 Hz, 2H), 7.42-7.23 (m, 4H), 7.18-7.13 (m, 2H), 4.82-4.75 (m, 1H), 3.79 (s, 2H), 3.74 (m, 2H), 3.04-2.88 (m, 2H), 1.59 (sext., *J*³ = 7.47 Hz, 2H), 0.76 (t, *J*³ = 7.37 Hz, 3H).

### Example 22: Compound (24)

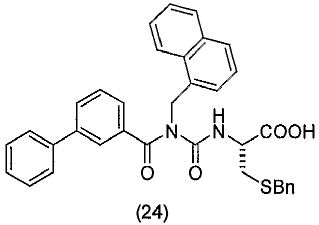

### 22A: N-naphthalenemethyl-3-bromobenzamide

Using Preparation Method 1, 3-bromobenzoic acid was reacted with 1-naphthylenemethylamine. The resulting reaction mixture was purified using SiO₂ with CH₂Cl₂ 100% to CH₂Cl₂/Ethyl Acetate 95:5 to give a white solid (78%). NMR ¹H (ppm, CDCl₃): 8.05 (d, *J*³ = 7.42 Hz, 1H), 7.91-7.88 (m, 2H), 7.84 (d, *J*³ = 8.14 Hz, 1H), 7.64 (d, *J*³ = 7.74 Hz, 1H), 7.60-7.42 (m, 5H), 7.25 (t, *J*³ = 7.85 Hz, 1H), 6.26 (br. s., 1H), 5.07 (d, *J*³ = 5.24 Hz, 1H).

### 22B: N-naphthalenemethyl-3-phenylbenzamide

Using Preparation Method 2, N-naphthalenemethyl-3-bromobenzamide was reacted with phenylboronic acid. The resulting reaction mixture was purified using SiO₂ with CH₂Cl₂/Petroleum Ether 95:5 to CH₂Cl₂/Ethyl Acetate 90:10 to give a white solid (100%). NMR ¹H (ppm, CDCl₃): 8.08-8.04 (m, 2H), 7.88-7.85 (m, 1H), 7.80 (d, *J*³ = 8.07 Hz, 1H), 7.67 (t, *J*³ = 7.95 Hz, 2H), 7.56-7.46 (m, 5H), 7.43-7.33 (m, 5H), 6.88 (t, *J*³ = 5.04 Hz, 1H), 5.03 (d, *J*³ = 5.36 Hz, 1H). NMR ¹³C (ppm, CDCl₃): 167.2, 141.5, 140.0, 134.7, 133.8, 133.6, 131.4, 130.0, 128.8, 128.7, 128.71, 128.67, 128.5, 127.6, 127.0, 126.6, 126.5, 125.9, 125.8, 125.6, 125.3, 123.4, 42.2.

### 22C: Compound (24)

Using Preparation Method 3, N-naphthalenemethyl-3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (24) as a colourless glassy oil (87%). NMR ¹H (ppm, CDCl₃),: 10.18 (br. s., 1H), 9.97 (d, *J*³ = 7.25 Hz, 1H), 7.84 (d., *J*³ = 7.73 Hz, 1H), 7.77 (d, *J*³ = 7.98 Hz, 1H), 7.67-7.64 (d, *J*³ = 8.14 Hz, 1H), 7.91-7.23 (m., 13H), 7.18 (d, *J*³ = 6.9 Hz, 2H), 7.052-7.02 (m, 2H), 5.50 (s, 2H), 4.89-4.83 (m, 1H), 3.81 (s, 2H), 3.10-2.93 (m, 2H).

### Example 23: Compound (25)

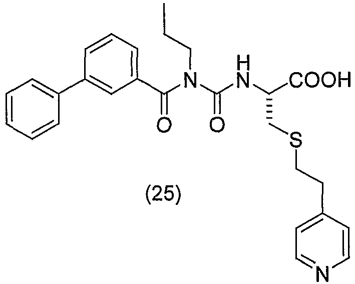

Using Preparation Method 3, N-*n*-propyl-3-phenylbenzamide from Example 1B was reacted with phosgene to provide a carbamoylchloride. Using Preparation Method 4, TMS protected S-ethylpyridyl-(L)-cysteine was prepared. The carbamoylchloride and TMS protected S-ethylpyridyl-(L)-cysteine were reacted using Preparation Method 5 to provide compound (25) as a colourless glassy oil (63%). NMR ¹H (ppm, CDCl₃): 9.69 (br. s., 1H), 8.29 (br. s., 2 H), 7.69-7.66 (m, 2H), 7.58-7.55 (m, 2H), 7.51-7.33 (m, 7H), 4.82 (br. s., 1H), 3.70 (br. s., 2H), 3.70 (br. m., 1H), 3.14-3.10 (m, 1H), 2.96-2.74 (m, 4H), 1.56 (br. sext., *J*³ = 6.89 Hz, 2H), 0.73 (br. t., *J*³ = 6.50 Hz, 3H).

### Example 24: Compound (26)

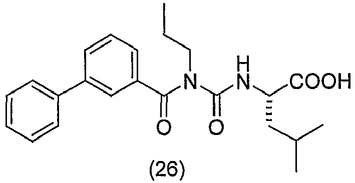

Using Preparation Method 3, N-*n*-propyl-3-phenylbenzamide from Example 1B was reacted with phosgene to provide a carbamoylchloride. Using Preparation Method 4, TMS protected (L)-leucine was prepared. The carbamoylchloride and TMS protected (L)-leucine were reacted using Preparation Method 5 to give a colourless glassy oil (89%). NMR ¹H (ppm, CDCl₃): 9.35 (d, *J*³ = 6.82 Hz, 1H), 7.69 (d, *J*³ = 7.64 Hz, 1H), 7.65 (s, 1H), 7.58 (d., *J*³ = 7.33 Hz, 2H), 7.53-7.34 (m, 5H), 4.56-4.52 (m, 1H), 3.69 (m, 2H), 1.80-1.68 (m, 2H), 1.58 (sext., *J*³ = 7.52 Hz, 2H), 0.99-0.96 (m, 6H), 0.72 (t, *J*³ = 7.37 Hz, 3H).

### Example 25: Compound (27)

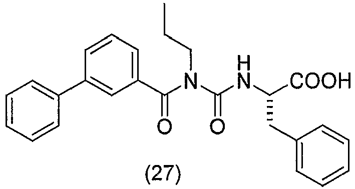

Using Preparation Method 3, N-*n*-propyl-3-phenylbenzamide from Example 1B was reacted with phosgene to provide a carbamoylchloride. Using Preparation Method 4, TMS protected (L)-phenylalanine was prepared. The carbamoylchloride and TMS protected (L)-phenylalanine were reacted using Preparation Method 5 to give a colourless glassy oil (85%). NMR ¹H (ppm, CDCl₃): 9.45 (d, *J*³ = 6.8 Hz, 1H), 9.44 (br. s., 1H), 7.69 (d, *J*³ = 7.8 Hz, 1H), 7.62-7.57 (m, 3H), 7.38 (t, *J*³ = 7.3 Hz, 2H), 7.33-7.26 (m, 5H), 4.87-4.80 (m, 1H), 3.66 (m, 2H), 3.34-3.11 (m, 2H), 1.51 (sext., *J*³ = 7.3 Hz, 2H), 0.70 (t, *J*³ = 7.3 Hz, 3H).

### Example 26: Compound (28)

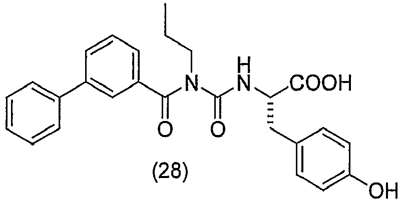

Using Preparation Method 3, N-*n*-propyl-3-phenylbenzamide from Example 1B was reacted with phosgene to provide a carbamoylchloride. Using Preparation Method 4, but using two equivalents of (N,O)-bistrimethylsilylacetamide, TMS protected (L)-tyrosine was prepared. The carbamoylchloride and TMS protected (L)-tyrosine were reacted using Preparation Method 5 to give a colourless glassy oil (85%). NMR ¹H (ppm, CDCl₃): 9.43 (d, *J*³ = 6.86 Hz, 1H), 7.68 (d, *J*³ = 7.82 Hz, 1H), 7.61 (s, 1H), 7.59 (d., *J*³ = 7.03 Hz, 2H), 7.51-7.34 (m, 5H), 7.06 (d, *J*³ = 8.25 Hz, 2H), 6.71 (d, *J*³ = 8.28 Hz, 2H), 4.80-4.73 (m, 1H), 3.65 (m, 2H), 3.18-3.03 (m, 2H), 1.51 (sext., *J*³ = 7.44 Hz, 2H), 0.69 (t, *J*³ = 7.34 Hz, 3H).

### Example 27: Compound (29)

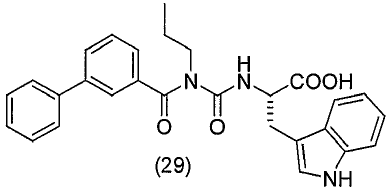

Using Preparation Method 3, N-*n*-propyl-3-phenylbenzamide from Example 1B was reacted with phosgene to provide a carbamoylchloride. Using Preparation Method 4, TMS protected (L)-tryptophan was prepared. The carbamoylchloride and TMS protected (L)-tryptophan were reacted using Preparation Method 5 to give a colourless glassy oil (85%). NMR ¹H (ppm, CDCl₃): 9.95 (br. s., 1H), 9.49 (d, *J*³ = 6.32 Hz, 1H), 8.31 (s, 1H), 7.67 (d, *J*³ = 9.17 Hz, 1H), 7.64 (d, *J*³ = 7.83 Hz, 1H), 7.57 (d, *J*³ = 7.25 Hz, 4H), 7.50-7.36 (m, 4H), 7.32-7.25 (m, 2H), 7.16-7.08 (m, 2H), 4.91-4.85 (m, 1H), 3.65 (m, 2H), 3.49-3.30 (m, 2H), 1.52 (sext., *J*³ = 7.40 Hz, 2H), 0.69 (t, *J*³ = 7.36 Hz, 3H).

### Example 28: Compound (30)

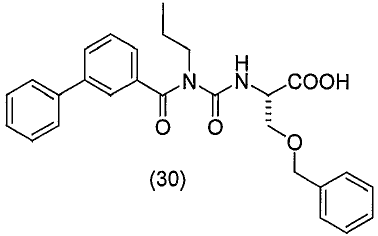

Using Preparation Method 3, N-*n*-propyl-3-phenylbenzamide from Example 1B was reacted with phosgene to provide a carbamoylchloride. Using Preparation Method 4, TMS protected O-benzyl-(L)-serine was prepared. The carbamoylchloride and TMS protected O-benzyl-(L)-serine were reacted using Preparation Method 5 to give a colourless glassy oil (72%). NMR ¹H (ppm, CDCl₃): 9.66 (d, *J*³ = 7.32 Hz, 1H), 9.19 (br. s., 1H), 7.72-7.69 (m, 2H), 7.60 (d, *J*³ = 7.02 Hz, 1H), 7.54-7.38 (m, 5H), 7.34-7.27 (m, 5H), 4.79-4.75 (m, 1H), 4.58 (s, 2H), 4.01-3.96 (m, 1H), 3.82-3.77 (m, 2H), 3.71 (m, 2H), 1.58 (sext., *J*³ = 7.39 Hz, 2H), 0.74 (t, *J*³ = 7.33 Hz, 3H).

### Example 29: Compound (31)

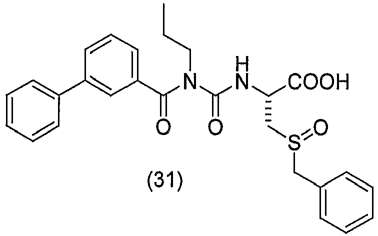

Meta-chloroperbenzoic acid (70 mg, Tech grade 77% purity) was added to a solution of compound (1) (134 mg, 0.28 mmol) in 1 mL of dry THF at 0°C. TLC showed complete reaction after 20 minutes. After concentration under vacuum, the residue was purified by flash chromatography using SiO₂ with CH₂Cl₂/MeOH/AcOH 99:0.5:0.5 to 89.5:10:0.5 to give compound (31) as a colourless film (66 mg, 48%). NMR ¹H (ppm, CDCl₃): 9.71-9.64 (m, 1H), 7.63-7.23 (m, 14H), 4.94 (br. s., 1H), 4.28-4.07 (br. m., 2H), 3.64-3.17 (br. m., 4H), 1.50 (br. m., 2H), 0.68 (br. m., 3H).

### Example 30: Compound (32)

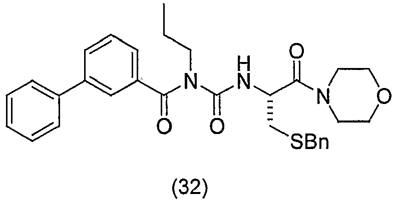

A mixture of compound (1) from Example 1 (121 mg, 0.25 mmol), 1-ethyl-3-(3-dimethylaminopropyl)carbodiimide hydrochloride (EDCI) (73 mg, 0.38 mmol), DMAP (2.53 mg), and morpholine (44.3 *µ*L, 0.5 mmol) in 2 mL of dry DMF was stirred for 16 hours at room temperature. The reaction was then poured into 1N HCl. The acidic aqueous phase was extracted three times with ethyl acetate. The combined organic layers were washed with 1N HCl, water and brine, dried over Na₄SO₄ and concentrated. Purification by flash chromatography using SiO₂, CH₂Cl₂/MeOH 98:2 to 85:15 gave a yellow oil. NMR ¹H (ppm, CDCl₃): 9.42 (d, *J*³ = 7.96 Hz, 1H), 7.69-7.25 (m, 14H), 4.99-4.92 (m, 1H), 3.68-3.74 (m, 2H), 3.71 (m, 2H), 3.68-3.33 (br. m., 8H), 2.86-2.63 (m, 2H), 1.55 (hept., *J*³ = 7.52 Hz, 2H), 0.74 (t, *J*³ = 7.37 Hz, 3H).

### Example 31: Compound (33)

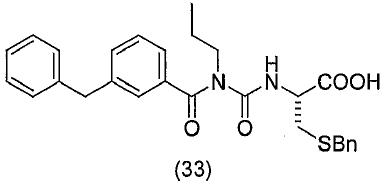

### 31A: 3-phenylmethylbenzoic acid

3-Phenylmethylbenzoic acid was prepared according to the method of Van Herwijnen *et. al.,* 2001. 3-Phenylmethylbenzoic acid (904 mg, 4 mmol), powdered NaOH (700 mg), hydrazine hydrate (0.7 mL), and tri(ethylene glycol) (20 mL) were stirred at 190°C for four hours. Upon cooling, the reaction mixture was washed twice with diethyl ether. Concentrated HCl was added dropwise to the aqueous phase until an acid solution was obtained. The resulting precipitate was filtered off, washed with water and dried to give pale yellow flakes (780 mg, 92%). NMR ¹H (ppm, CDCl₃) 7.96-7.93 (m, 2H), 7.43-7.17 (m, 7H), 4.04 (s, 2H).

### 31B: N-n-propyl-(3-phenylmethyl)benzamide

Using Preparation Method 1, 3-phenylmethylbenzoic acid was reacted with propylamine. The resulting reaction mixture was purified using SiO₂, CH₂Cl₂/Petroleum Ether 80:20 to CH₂Cl₂/Ethyl Acetate 80:20 to give a white solid (53%). NMR ¹H (ppm, CDCl₃): 7.61 (s, 1H), 7.56 (dt, *J*³ = 6.84, *J*⁴= 1.93 Hz, 1H), 7.32-7.26 (m, 4H), 7.21-7.15 (m, 3H), 6.10 (br. s., 1H), 4.00 (s, 2H), 3.44-3.36 (m, 2H), 1.61 (sext., *J*³ = 7.23 Hz, 2H), 0.96 (t, *J*³ = 7.36 Hz, 3H).

### 31C: Compound (33)

Using Preparation Method 3, N-*n*-propyl -(3-phenylmethyl)benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (33) as a colourless glassy oil (72%). NMR ¹H (ppm, CDCl₃): 9.68 (br. s., 1H), 9.66 (d, *J*³ = 6.94 Hz, 1H), 7.38-7.20 (m, 14H), 4.75-4.69 (m, 1H), 4.01 (s, 2H), 3.76 (s, 2H), 3.58 (m, 2H), 3.00-2.84 (m, 2H), 1.47 (sext., *J*³ = 7.52 Hz, 2H), 0.66 (t, *J*³ = 7.38 Hz, 3H).

### Example 32: Compound (34)

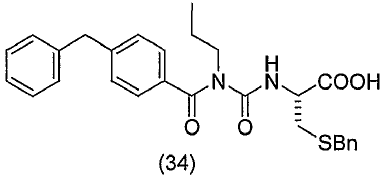

### 32A: 4-phenylmethyl benzoic acid

4-Phenylmethylbenzoic acid was prepared according to the method of Van Herwijnen *et. al.,* 2001. 4-Benzoylbenzoic acid (452 mg, 2mmol), powdered NaOH (350 mg), hydrazine hydrate (0.35 mL) and tri(ethylene glycol) (15 mL) were stirred at 190°C for four hours.

Upon cooling, the reaction mixture was washed twice with diethyl ether. Concentrated HCl was added dropwise to the aqueous phase until an acidic solution was obtained. The resulting precipitate was filtered off, washed with water and dried to give white crystals (259 mg, 61%). NMR ¹H (ppm, CDCl₃) 8.02, (d, *J³* = 8.1 Hz, 2H), 7.32-7.16 (m, 7H), 4.04 (s, 2H).

### 32B: N-n-propyl-(4-phenylmethyl)benzamide

Using Preparation Method 1, 4-phenylmethylbenzoic acid was reacted with propylamine. The resulting reaction mixture was purified using SiO₂, CH₂Cl₂/Petroleum Ether 80:20 to CH₂Cl₂/Ethyl Acetate 80:20 to give a white solid (69%). NMR ¹H (ppm, DMSO-d₆): 7.66 (d , *J*³ = 8.22, 1H), 7.33-7.08 (m, 7H), 6.02 (br. s., 1H), 4.00 (s, 2H), 3.43-3.36 (m, 2H), 1.61 (sext., *J*³ = 7.32 Hz, 2H), 0.96 (t, *J*³ = 7.35 Hz, 3H).

### 32C: Compound (34)

Using Preparation Method 3, N-*n*-propyl(4-phenylmethyl)benzoic acid was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (34) as a colourless glassy oil (75%). NMR ¹H (ppm, CDCl₃): 10.27 (br. s., 1H), 9.62 (d, *J*³ = 6.99 Hz, 1H), 7.38 (d, *J*³ = 8.13 Hz, 2H), 7.33-7.22 (m, 12H), 4.76-4.70 (m, 1H), 4.02 (s, 2H), 3.76 (s, 2H), 3.67 (m, 2H), 3.01-2.85 (m, 2H), 1.53 (sext., *J*³ = 7.47 Hz, 2H), 0.72 (t, *J*³ = 7.38 Hz, 3H).

### Example 33: Compound (35)

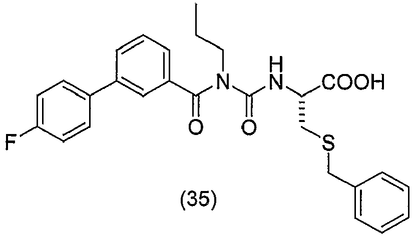

### 33A: N-n-propyl 3-((4'-fluoro)-phenyl)benzamide

Using Preparation Method 2, N-*n*-propyl-3-bromobenzamide from Example 1A was reacted with 4-fluorophenylboronic acid. The resulting reaction mixture was purified using SiO₂ with CH₂Cl₂/Petroleum Ether 80:20 to CH₂Cl₂/Ethyl Acetate 80:20 to give a white solid (70%). NMR ¹H (ppm, CDCl₃): 7.94 (t, *J*⁴ = 2.1 Hz, 1H), 7.66 (tt, *J*³ = 7.8 Hz, *J*⁴ = 0.9 Hz, 2H), 7.58-7.53 (m, 2H), 7.47 (t, *J*³ = 7.8 Hz, 1H), 7.13 (t, *J*³ = 9 Hz, 2H), 6.12 (br. s., 1H), 3.47-3.40 (m, 2H), 1.65 (sext, *J*³ = 7.5 Hz, 2H), 0.99 (t, *J*³ = 7.44 Hz, 3H).

### 33B: Compound (35)

Using Preparation Method 3, N-*n*-propyl-3-((4'-fluoro)-phenyl)benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (35) as a colourless glassy oil (86%). NMR ¹H (ppm, CDCl₃): 9.93 (br. s., 1H), 9.67 (d, *J*³ = 6.92 Hz, 1H), 7.67-7.63 (m, 2H), 7.57-7.48 (m, 3H), 7.42 (d, *J*³ = 7.61 Hz, 1H), 7.32-7.11 (m, 7H), 4.80-4.74 (m, 1H), 3.79 (s, 2H), 3.71 (m, 2H), 3.04-2.88 (m, 2H), 1.58 (sext., *J*³ = 7.35 Hz, 2H), 0.74 (t, *J*³ = 7.34 Hz, 3H).

### Example 34: Compound (36)

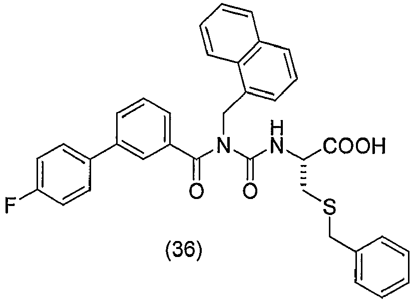

### 34A: N-naphthalenemethyl 3-(4'-fluorophenyl)benzamide

Using Preparation Method 2, N-naphthalenemethyl-3-bromobenzamide from Example 21A was reacted with 4-fluorophenyl boronic acid. The resulting reaction mixture was purified using SiO₂ with CH₂Cl₂ 100% to CH₂Cl₂/Ethyl Acetate 95:5 to give a white solid (71%). NMR ¹H (ppm, CDCl₃): 8.08 (dd, *J*³ = 9 Hz, *J*⁴ = 2.1 Hz, 1H), 7.95 (t, *J*⁴ = 2.1 Hz 1H), 7.90-7.87 (m, 1H), 7.83 (d, *J*³ = 8.04 Hz, 1H), 7.65 (dt, *J*³ = 9.3 Hz, *J*⁴ = 1.2 Hz, 1H), 7.62 (dq, *J*³ = 7.8 Hz, *J*⁴ = 1.2 Hz, 1H), 7.57-7.48 (m, 5H), 7.46-7.39 (m, 2H), 7.09 (t, *J*³ = 8.70 Hz, 2H), 6.46, (br. s., 1H), 5.08 (d, *J*³ = 5.27 Hz, 1H).

### 34B: Compound (36)

Using Preparation Method 3, N-naphthalenemethyl 3-(4'-fluorophenyl)-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (36) to give a colourless glassy oil (72%). NMR ¹H (ppm, CDCl₃): 9.64 (d., *J*³ = 7.25 Hz, 1H), 8.1 (br. s., H), 7.84 (d, *J*³ = 6.9 Hz, 1H), 7.77 (d, *J*³ = 7.71 Hz, 1H), 9.63 (d, *J*³ = 8.42 Hz, 1H), 7.49-7.29 (m, 13H), 6.90 (d, *J*³ = 6.6 Hz, 1H), 4.81 (br. s., 1H), 5.54-5.42 (m, 2H), 4.88-4.82 (m, 2H), 3.80 (s, 2H), 3.10-2.92 (m, 2H).

### Example 35: Compound (37)

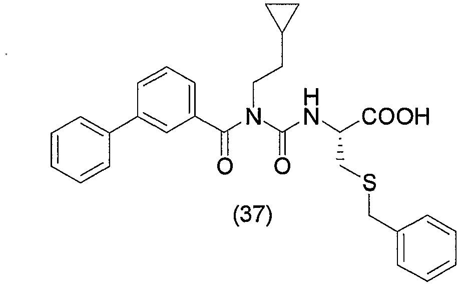

### 35A: N-(2-cyclopropyl)ethyl-3-phenylbenzamide

Using Preparation Method 1, 3-phenylbenzoic acid was reacted with (2-cyclopropyl)ethylamine. The resulting reaction mixture was purified using SiO₂ with CH₂Cl₂ 100 %. A white crystalline solid was obtained (69%). NMR ¹H (ppm, CDCl₃): 7.97 (s, 1H), 7.69 (d, *J*³ = 7.7 Hz, 2H), 7.60 (d, *J*³ = 7.2 Hz, 2H), 7.51-7.33 (m, 4H), 6.29 (br. s., 1H), 3.60-3.53 (m, 2H), 1.54 (quart., *J*³ = 7.0 Hz, 2H), 0.78-0.67 (m, 1H), 0.52-0.46 (m, 2H), 0.14-0.06 (m, 2H).

### 35B: Compound (37)

Using Preparation Method 3, N-(2-cyclopropyl)ethyl-3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (37) as a colourless oil (35%). NMR ¹H (ppm, CDCl₃): 9.71 (d, *J*³ = 6.9 Hz, 1H), 7.72-7.23 (m, 14H), 4.75-4.68 (m, 1H), 3.86 (t, *J*³ = 7.2 Hz, 2H), 3,78 (s, 2H), 3.02-2.87 (m, 2H), 1.42 (quart., *J*³ = 6.9 Hz, 2H), 0.40-0.27 (m, 3H), (-0.11)-(-0.15) (m, 2H).

### Example 36: Compound (38)

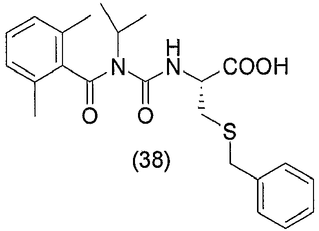

### 36A: N-isopropyl-2,6-dimethylbenzamide

Using Preparation Method 1, 2,6-dimethylbenzoic acid was reacted with isopropylamine. The resulting reaction mixture was purified using SiO₂ with CH₂Cl₂/MeOH 99:1. A white crystalline solid was obtained (73%). NMR ¹H (ppm, CDCl₃): 7.15-7.10 (m, 1H), 6.99 (d, *J*³ = 7.57 Hz, 2H), 5.44 (br. s., 1H), 4.40-4.24 (m, 1H), 2.31 (s, 6H), 1.24 (d, *J*³ = 6.58 Hz, 6H).

### 36B: Compound (38)

Using Preparation Method 3, *N*-isopropyl-2,6-dimethylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (38) as a colourless glassy oil (35%). NMR ¹H (ppm, CDCl₃): 9.98 (d, *J*³ = 6.76 Hz, 1H), 7.33-7.27 (m, 5H), 7.06-7.04 (m, 3H), 4.78-4.72 (m, 1H), 3.88-3.79 (m, 1H), 3.79 (s, 2H), 3.05-2.87 (m, 2H), 2.30 (s, 6H), 1.39 (d, *J*³ = 6.69 Hz, 6H).

### Example 37: Compound (39)

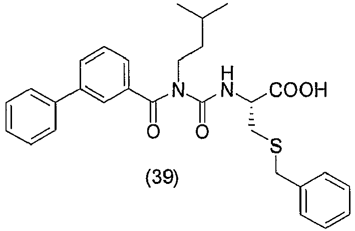

### 37A: N-isoamyl-3-phenylbenzamide

Using Preparation Method 1, 3-phenylbenzoic acid was reacted with isoamylamine. The resulting reaction mixture was purified using SiO₂ with CH₂Cl₂ 100%. An off-white crystalline solid was obtained (61%). NMR ¹H (ppm, CDCl₃): 7.96 (s, 1H), 7.69 (d, *J*³ = 8.3 Hz, 2H), 7.59 (d, *J*³ =7.2 Hz, 2H), 7.50-7.33 (m, 4H), 6.10 (br. s., 1H), 3.52-3.45 (m, 2H), 1.76-1.64 (m, 1H), 1.52 (quart., 2H), 0.95 (d, *J*³ = 6.6 Hz, 6H).

### 37B: Compound (39).

Using Preparation Method 3, N-isoamyl-3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (39) as a colourless oil (62%). NMR ¹H (ppm, CDCl₃): 9.72 (d, *J*³ = 6.8 Hz, 1H), 7.68-7.23 (m, 14H), 4.77-4.70 (m, 1H), 3.78-3.70 (m, 4H), 3.03-2.87 (m, 2H), 1.47-1.32 (m, 3H), 0.67 (d, *J*³ = 6.1 Hz, 6H).

### Example 38: Compound (40)

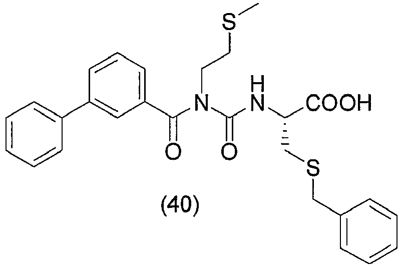

### 38A: N-(2-methylthio)ethyl-3-phenylbenzamide

Using Preparation Method 1, 3-phenylbenzoic acid was reacted with 2-(methylthio)ethylamine. The resulting reaction mixture was purified using SiO₂ with CH₂Cl₂ 100%. An white crystalline solid was obtained (65%). NMR ¹H (ppm, CDCl₃): 8.00 (s, 1H), 7.72 (d, *J*³ = 7.7 Hz, 2H), 7.60 (d, *J*³ =7.2 Hz, 2H), 7.52-7.34 (m, 4H), 6.62 (br. s., 1H), 3.72-3.65 (m, 2H), 2.77 (t, *J*³ = 6.3 Hz, 2H), 2.14 (s, 3H).

### 38B: Compound (40)

Using Preparation Method 3, N-(2-methylthio)ethyl-3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (40) as a colourless oil (62%). NMR ¹H (ppm, CDCl₃): 9.62 (d, *J*³ = 6.9 Hz, 1H), 7.71-7.11 (m, 14H), 4.75-4.68 (m, 1H), 3.97 (t, *J*³ = 7.0 Hz, 2H), 3.78 (s, 2H), 3.02-2.87 (m, 2H), 2.63 (t, *J*³ = 6.9 Hz, 2H), 1.79 (s, 3H).

### Example 39: Compound (42)

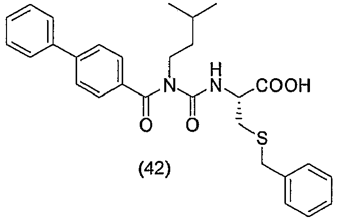

### 39A: N-isoamyl-4-phenylbenzamide

Using Preparation Method 1, 4-phenylbenzoic acid was reacted with isoamylamine. The resulting reaction mixture was purified using SiO₂ with CH₂Cl₂ 100%. A white crystalline solid was obtained (29%). NMR ¹H (ppm, CDCl₃): 7.81 (d, *J*³ = 8.3 Hz, 2H), 7.65-7.58 (m, 4H), 7.47-7.34 (m, 3H), 6.06 (br. s., 1H), 3.53-3.36 (m, 2H), 1.74-1.65 (m, 1H), 1.52 (quart., *J*³ = 6.7 Hz, 2H), 0.96 (d, *J*³ = 6.6 Hz).

### 39B: Compound (42)

Using Preparation Method 3, N-isoamyl-4-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (42) as a colourless oil (55 %). NMR ¹H (ppm, CDCl₃): 9.70 (d, *J*³ = 6.8 Hz, 1H), 7.68-7.15 (m, 14H), 4.76-4.69 (m, 1H), 3.79-3.73 (m, 4H), 3.01-2.86 (m, 2H), 1.47-1.34 (m, 3H), 0.70 (d, *J*³ = 6.0 Hz, 6H).

### Example 40: Compound (43)

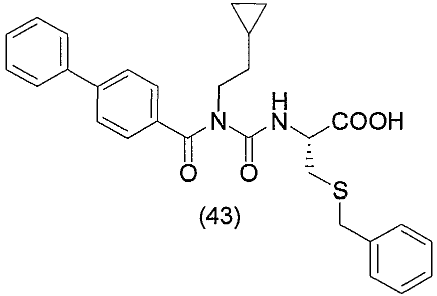

### 40A: N-(2-cyclopropyl)ethyl-4-phenylbenzamide

Using Preparation Method 1, 4-phenylbenzoic acid was reacted with (2-cyclopropyl)ethylamine. The resulting reaction mixture was purified using SiO₂ with CH₂Cl₂ 100%. A white crystalline solid was obtained (29%). NMR ¹H (ppm, CDCl₃): 7.82 (d, *J*³ = 8.2 Hz, 2H), 7.64 (d, *J*³ = 8.4 Hz, 2H), 7.60 (d, *J*³ = 7.5 Hz, 2H), 7.47-7.34 (m, 3H), 6.26 (br. s., 1H), 3.60-3.53 (m, 2H), 1.54 (quart., *J*³ = 6.8 Hz, 2H), 0.76-0.71 (m, 1H), 0.51-0.48 (m, 2H), 0.13-0.11 (m, 2H).

### 40B: Compound (43)

Using Preparation Method 3, N-(2-cyclopropyl)ethyl-4-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (43) as a colourless oil (47%). NMR ¹H (ppm, CDCl₃): 9.70 (d, *J*³ = 6.9 Hz, 1H), 7.68-7.15 (m, 14H), 4.73-4.69 (m, 1H), 3.88 (t, *J*³ = 7.2 Hz, 2H), 3,78 (s, 2H), 3.02-2.86 (m, 2H), 1.44 (quart., *J*³ = 6.9 Hz, 2H), 0.46-0.29 (m, 3H), (-0.08)-(-0.12) (m, 2H).

### Example 41: Compound (44)

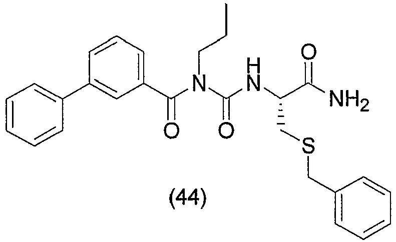

Compound (1) from example 1A (171 mg, 0.36 mmol) was dissolved in dry CH₂Cl₂ (2.5 mL) with DMF (0.25 mL). The solution was cooled to 0°C and stirred under nitrogen. Oxalyl chloride (46 *µ*L, 70 mg, 0.55 mmol) was added via syringe and stirring was applied at room temperature for two hours. The solvent and any excess oxalyl chloride were removed under reduced pressure. The orange residue was dissolved in dry CH₂Cl₂ (1 mL) and added via syringe to ammonia solution (25%, 2 mL) at 0°C. The mixture was stirred for one hour, and then neutralized with 10% citric acid. The product was extracted with CH₂Cl₂ three times, and the organic layers were washed with water, then brine, and were then dried over MgSO₄ and concentrated. The product was purified using SiO₂ with CH₂Cl₂/EtOAc 90:10. A colourless oil was obtained (72mg, 42%). NMR ¹H (ppm, CDCl₃): 9.44 (d, *J*³ = 7.2 Hz, 1H), 7.66-7.19 (m, 14H), 6.44 (br. s., 1H), 6.03 (br. s., 1H), 4.60-4.53 (m, 1H), 3.79 (s, 2H), 3.69 (t, *J*³ = 7.5 Hz, 2H), 2.96-2.80 (m, 2H), 1.56 (sext., *J*³ = 7.5 Hz, 2H), 0.74 (t, *J*³ = 7.4 Hz, 3H).

### Example 42: Compound (45)

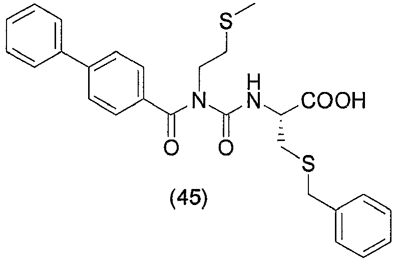

### 42A: N-(2-methylthio)ethyl-4-phenylbenzamide

Using Preparation Method 1, 4-phenylbenzoic acid was reacted with 2-(methylthio)ethylamine. The resulting reaction mixture was purified using SiO₂ with CH₂Cl₂ 100%. A white crystalline solid was obtained. NMR ¹H (ppm, CDCl₃): 7.50-7.35 (m, 3H), 7.85 (d, *J*³ = 8.1 Hz, 2H), 7.65 (d, *J*³ = 8.4 Hz, 2H), 7.60 (d, *J*³ = 8.0 Hz, 2H), 6.61 (br. s., 1H), 3.72-3.65 (m, 2H), 2.77 (t, *J*³ = 6.4 Hz, 2H), 2.15 (s, 3H).

### 42B: Compound (45)

Using Preparation Method 3, N-(2-methylthio)ethyl-4-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (45) as an off-white solid (38%). NMR ¹H (ppm, CDCl₃): 9.55 (d, *J*³ = 6.9 Hz, 1H), 7.68-7.23 (m, 14H), 4.76-4.69 (m, 1H), 4.00 (t, *J*³ = 6.9 Hz, 2H), 3.78 (s, 2H), 3.02-2.87 (m, 2H), 2.65 (t, *J*³ = 7.0 Hz, 2H), 1.85 (s, 3H).

### Example 43: Compound (46)

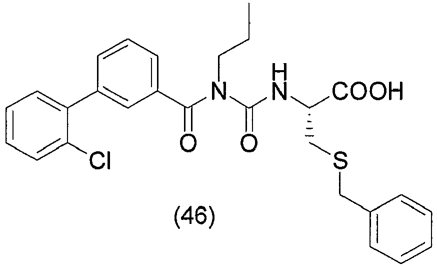

### 43A: N-n-propyl-3-(2'-chloro)-phenylbenzamide

Using Preparation Method 2, N-*n*-propyl-3-bromobenzamide from example 1A was reacted with 2-chloro-phenyl boronic acid. The resulting reaction mixture was purified using SiO₂ with Petroleum ether/CH₂Cl₂ 5:95 to CH₂Cl₂ 100% to AcOEt/CH₂Cl₂ 5:95. A white crystalline solid was obtained (79%). NMR ¹H (ppm, CDCl₃): 7.80 (s, 1H), 7.76 (d. t., *J*³ = 7.7 Hz, *J*⁴ = 1.3 Hz, 1H), 7.52 (d. t., *J*³ = 7.8 Hz, *J*⁴ = 1.4 Hz, 1H), 7.44-7.38 (m, 2H), 7.29-7.20 (m, 3H), 6.61 (br. s., 1H), 3.41-3.33 (m, 2H), 1.56 (sex., *J*³ = 7.2 Hz, 2H), 0.92 (t, *J*³ = 7.4 Hz, 3H).

### 43B: Compound (46).

Using Preparation Method 3, N-*n*-propyl-3-(2'-chloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (46) as a colourless oil (46%). NMR ¹H (ppm, CDCl₃): 9.68 (d, *J*³ =6.8 Hz, 1H), 7.55-7.17 (m, 13H), 4.74-4.67 (m, 1H), 3.78 (s, 2H), 3.73 (t, *J*³ = 7.5 Hz, 2H), 3.02-2.86 (m, 2H), 1.55 (sext., *J*³ = 7.5 Hz, 2H), 0.73 (t, *J*³ = 7.4 Hz, 3H).

### Example 44: Compound (47)

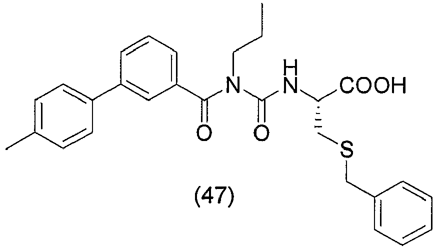

### 44A: N-n-propyl-3-(4'-tolyl)-benzamide

Using Preparation Method 2, N-*n*-propyl-3-bromobenzamide from example 1A was reacted with 4-tolylboronic acid. The resulting reaction mixture was purified using SiO₂ with AcOEt/CH₂Cl₂ 10:90. A white crystalline solid was obtained (78%). NMR ¹H (ppm, CDCl₃): 7.99 (s, 1H), 7.69 (d, *J*³ = 7.7 Hz, 1H), 7.64 (d, *J*³ = 7.8 Hz, 1H), 7.46 (d, *J*³ = 7.9 Hz, 2H), 7.39 (d, *J*³ = 7.7 Hz, 1H), 7.21 (d, *J*³ = 7.6 Hz, 2H), 6.74 (br. s., 1H), 3.42-3.35 (m, 2H), 2.36 (s, 3H), 1.61 (sexy., *J*³ = 7.2 Hz, 2H), 0.94 (t, *J*³ = 7.4 Hz, 3H).

### 44B: Compound (47)

Using Preparation Method 3, N-*n*-propyl-3-(4'-tolyl)-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (47) as a colourless solid (71%). NMR ¹H (ppm, CDCl₃): 9.73 (d, *J*³ = 6.9 Hz, 1H), 9.64 (br. s., 1H), 7.70-7.68 (m, 2H), 7.52-7.47 (m, 3H), 7.41-7.23 (m, 8H), 4.82-4.76 (m, 1H), 3.79 (s, 2H), 3.72 (t, *J*³ = 7.5 Hz, 2H), 3.05-2.89 (m, 2H), 2.40 (s, 3H), 1.58 (sext., *J*³ = 7.4 Hz, 2H), 0.74 (t, *J*³ = 7.3 Hz, 3H).

### Example 45: Compound (48)

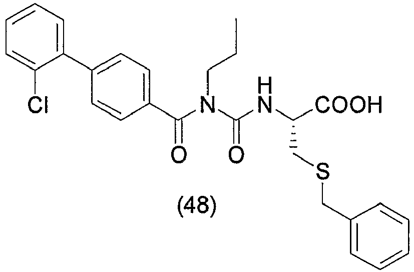

### 45A: N-n-propyl-4-(2'-chloro)-phenylbenzamide

Using Preparation Method 2, N-*n*-propyl-4-bromobenzamide from example 9A was reacted with 2-chloro-phenyl boronic acid. The resulting reaction mixture was purified using SiO₂ with Petroleum ether/CH₂Cl₂ 5:95 to CH₂Cl₂ 100% to AcOEt/CH₂Cl₂ 5:95. A white crystalline solid was obtained (81%). NMR ¹H (ppm, CDCl₃): 7.82 (d, *J*³ = 8.53 Hz, 2H), 7.46-7.43 (m, 3H), 7.28-7.26 (m, 3H), 6.59 (br. s., 1H), 3.44-3.37 (m, 2H), 1.63 (sext., *J*³ = 7.2 Hz, 2H), 0.92 (t, *J*³ = 7.4 Hz, 3H).

### 45B: Compound (48)

Using Preparation Method 3, N-*n*-propyl-4-(2'-chloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (48) as a colourless oil (63%). NMR ¹H (ppm, CDCl₃): 9.63 (d, J³ =6.9 Hz, 1H), 7.52-7.23 (m, 13H), 4.76-4.69 (m, 1H), 3.79 (s, 2H), 3.72 (t, J³ = 7.5 Hz, 2H), 3.02-2.87 (m, 2H), 1.57 (sext., J³ = 7.4 Hz, 2H), 0.76 (t, J³ = 7.4 Hz, 3H).

### Example 46: Compound (49)

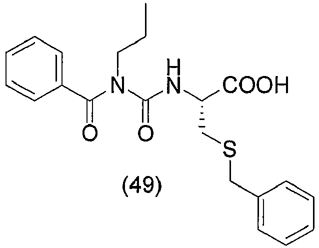

### 46A: N-n-propylbenzamide

A solution of benzoyl chloride (116 *µ*L, 1 mmol) in 1 mL of dry CH₂Cl₂ was treated successively at 0°C with triethylamine (167 *µ*L, 1.2 mmol) and *n*-propylamine (124 *µ*L, 1.5 mmol). The reaction was stirred at room temperature for two hours. The resulting reaction mixture was diluted with CH₂Cl₂ washed with 2M HCl, followed by saturated NaHCO₃, then brine. The resulting solution was then dried over MgSO₄. The residue was purified using SiO₂ with CH₂Cl₂/Petroluem ether 95:5 to CH₂Cl₂ 100%. A white solid was obtained (166 mg, quantitative yield). NMR ¹H (ppm, CDCl₃): 7.74 (d, *J*³ = 8.1 Hz, 2H), 7.41 (t. t., *J*³ = 7.2 Hz, *J*⁴ = 1.3 Hz, 1H), 7.33 (t. t., *J*³ *=* 7.6 Hz, *J*⁴ = 1.6 Hz, 2H), 5.23 (br. s., 1H), 3.36-3.30 (m, 2H), 1.57 (sext., *J*³ = 7.2 Hz, 2H), 0.90 (t, *J*³ = 7.4 Hz, 3H).

### 46B: Compound (49)

Using Preparation Method 3, N-*n*-propylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (49) as a colourless oil (78%). NMR ¹H (ppm, CDCl₃): 9.67 (d, *J*³ = 6.7 Hz, 1H), 7.48-7.22 (m, 10H), 4.74-4.67 (m, 1H), 3.78 (s, 2H), 3.65 (t, *J*³ = 7.5 Hz, 2H), 3.02-2.86 (m, 2H), 1.53 (sext., *J*³ =7.5 Hz, 2H), 0.71 (t, *J*³ = 7.4 Hz, 3H).

### Example 47: Compound (50)

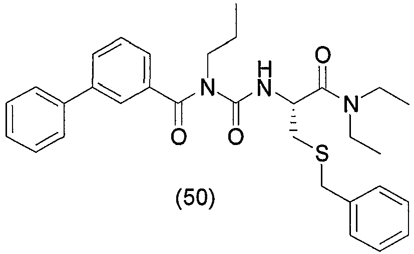

Compound (1) from example 1A (171 mg, 0.36 mmol) was dissolved in dry CH₂Cl₂ (2.5 mL) with DMF (0.25 mL). The solution was cooled to 0°C and stirred under nitrogen. Oxalyl chloride (46 *µ*L, 70 mg, 0.55 mmol) was added via syringe and stirring was applied at room temperature for two hours. The solvent and any excess oxalyl chloride were removed under reduced pressure. The orange residue was dissolved in dry CH₂Cl₂ (1 mL) and added via syringe to a solution of diethylamine (83 *µ*L, 59 mg, 0.8 mmol) in dry CH₂Cl₂ (2 mL) at 0°C. The mixture was stirred for 16 hours, and then neutralized with 10% citric acid. The product was extracted with CH₂Cl₂ three times, and the organic layers were washed with water, then brine, and were then dried over MgSO₄ and concentrated. The product was purified using SiO₂ with CH₂Cl₂/EtOAc 90:10. A colourless oil was obtained (38 mg, 20%). NMR ¹H (ppm, CDCl₃): 9.25 (d, *J*³ = 8.2 Hz, 1H), 7.71-7.22 (m, 14H), 5.01-4.94 (m, 1H), 3.77-3.67 (m, 4H), 3.56-3.40 (m, 2H), 3.36-3.18 (m, 2H), 2.85-2.65 (m, 2H), 1.57 (sext., *J*³ = 7.4 Hz, 2H), 1.16 (t, *J*³ = 7.2 Hz, 3H), 1.10 (t, *J*³ = 7.1 Hz, 3H), 0.75 (t, *J*³ = 7.4 Hz, 3H).

### Example 48: Compound (52)

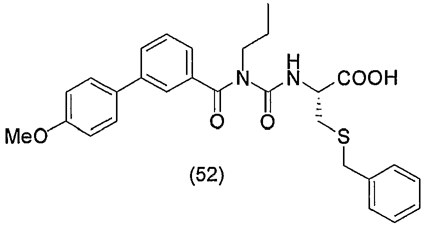

### 48A: N-n-propyl-3-(4'-methoxy)-phenylbenzamide

Using Preparation Method 2, N-*n*-propyl-3-bromobenzamide from Example 1A was reacted with 4-methoxyphenylboronic acid. The resulting reaction mixture was purified using SiO₂ with AcOEt/CH₂Cl₂ 10:90. A white crystalline solid was obtained (52%). NMR ¹H (ppm, CDCl₃): 7.93 (s, 1H), 7.75-7.62 (m, 2H), 7.54 (d, *J*³ = 8.8 Hz, 2H), 7.45 (t, *J*³ = 7.9 Hz, 1H), 6.97 (d, *J*³ = 8.8 Hz, 2H), 6.15 (br. s., 1H), 3.84 (s, 3H), 3.47-3.40 (m, 2H), 1.65 (sext., *J*³ = 7.3 Hz, 2H), 0.99 (t, *J*³ = 7.4 Hz, 3H).

### 48B: Compound (52)

Using Preparation Method 3, N-in-propyl-3-(4'-methoxy)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (52) as a colourless oil (32%). NMR ¹H (ppm, CDCl₃): 9.70 (d, *J*³ = 7.0 Hz, 1H), 9.19 (br. s., 1H), 7.66-7.20 (m, 11H), 6.98 (d, *J*³ = 8.7 Hz, 2H), 4.79-4.73 (m, 1H), 3.84 (s, 3H), 3.78 (s, 2H), 3.70 (t, *J*³ = 7.5 Hz, 2H), 3.03-2.87 (m, 2H), 1.56 (sext., *J*³ = 7.4 Hz, 2H), 0.73 (t, *J*³ = 7.4 Hz, 3H).

### Example 49: compound (55)

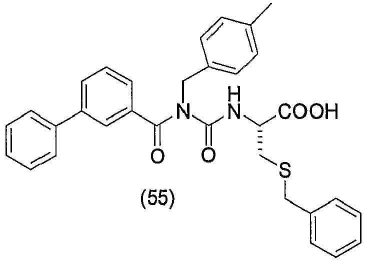

### 49A: Biphenyl-3-carboxylic acid 4-methyl-benzylamide

Using preparation method 1, 3-phenylbenzoic acid (125 mg, 0.63 mmol) was reacted with 4-methylbenzylamine (96 mg, 0.7 mmol). The product was purified by flash chromatography on SiO₂ using CH₂Cl₂/hexanes 50:50 then CH₂Cl₂ 100%. White crystals were obtained (115 mg, 58 %). NMR ¹H (ppm, CDCl₃): 7.99 (s, 1H), 7.73-7.69 (m, 2H), 7.58 (dd, *J³* = 7.0 Hz, *J⁴* = 1.4 Hz, 2H), 7.48 (t, *J³* = 7.8 Hz, 1H), 7.43 (t, *J³* = 7.6 Hz, 2H), 7.35 (tt, *J³* = 7.2 Hz, *J⁴* = 2.4 Hz, 1H), 7.29 (d, *J³* = 8.6 Hz, 2H), 6.88 (d, *J³* = 8.7 Hz, 2H), 6.36 (br. s, 1H), 4.59 (d, *J³* = 5.5 Hz, 2H), 3.79 (s, 3H).

### 49B: compound (55)

Using Preparation Method 3, compound 49A (79 mg, 0.25 mmol) was reacted with phosgene to provide a carbamoylchloride. Using Preparation Method 4, TMS protected S-benzyl-(L)-cysteine (64 mg, 0.3 mmol) was prepared. The carbamoylchloride and TMS protected S-benzyl-(L)-cysteine were reacted using Preparation Method 5 to provide compound (55) as a colourless oil (60 mg, 43 %). NMR ¹H (ppm, CDCl₃): 9.78 (d, *J³* = 7.1 Hz, 1H), 8.60 (br. s, 1H), 7.66 (d, *J³* = 7.8 Hz, 1H), 7.47-7.20 (m, 13H), 6.94 (d, *J³* = 8.6 Hz, 2H), 6.76 (d, *J³* = 8.7 Hz, 2H), 4.95 (s, 2H), 4.85-4.78 (m, 1H), 3.77 (s, 2H), 3.72 (s, 3H), 3.06-2.89 (m, 2H).

### Example 50: compound (56)

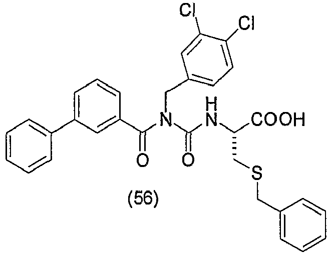

### 50A: Biphenyl-3-carboxylic acid 3,4-dichloro-benzylamide

Using preparation method 1, 3-phenylbenzoic acid (125 mg, 0.63 mmol) was reacted with 3,4-dichlorobenzylamine (123 mg, 0.7 mmol). The product was purified by flash chromatography on SiO₂ using CH₂Cl₂/hexanes 50:50 then CH₂Cl₂ 100%. White crystals were obtained (111 mg, 50 %). NMR ¹H (ppm, CDCl₃): 8.00 (s, 1H), 7.73 (dd, *J³* = 7.7 Hz, *J⁴* = 1.6 Hz, 2H), 7.59 (d, *J³* = 7.3 Hz, 2H), 7.50 (t, *J³* = 7.7 Hz, 1H), 7.46-7.34 (m, 6H), 7.19 (dd, *J³* = 8.2 Hz, *J⁴* = 1.8 Hz, 1H), 6.54 (br. s, 1H), 4.61 (d, *J³* = 5.9 Hz, 2H).

### 50B: compound (56)

Using Preparation Method 3, compound 50A (89 mg, 0.25 mmol) was reacted with phosgene to provide a carbamoylchloride. Using Preparation Method 4, TMS protected S-benzyl-(L)-cysteine (64 mg, 0.3 mmol) was prepared. The carbamoylchloride and TMS protected S-benzyl-(L)-cysteine were reacted using Preparation Method 5 to provide compound (56) as white glassy solid (106 mg, 72 %). NMR ¹H (ppm, CDCl₃): 9.71 (d, *J³* = 7.1 Hz, 1H), 8.69 (br. s, 1H), 7.69 (d, *J³* = 7.7 Hz, 1H), 7.48-7.16 (m, 14H), 7.13 (d, *J⁴* = 1.8 Hz, 1H), 6.90 (dd, *J³* = 8.3 Hz, *J⁴* = 1.8 Hz, 1H), 4.94 (s, 2H), 4.83-4.77 (m, 1H), 3.78 (s, 2H), 3.06-2.89 (m, 2H).

### Example 51: Compound (57)

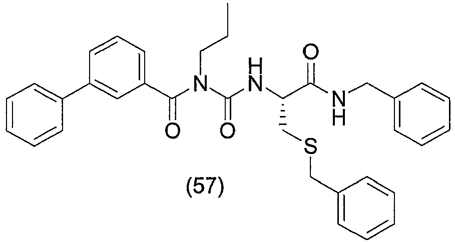

Compound (1) from Example 1A (171 mg, 0.36 mmol) was dissolved in dry CH₂Cl₂ (2.5 mL) with DMF (0.25 mL). The solution was cooled to 0°C and stirred under nitrogen. Oxalyl chloride (46 *µ*L, 70 mg, 0.55 mmol) was added via syringe and stirring was applied at room temperature for two hours. The solvent and any excess oxalyl chloride were removed under reduced pressure. The orange residue was dissolved in dry CH₂Cl₂ (1 mL) and added via syringe to a solution of benzylamine (88 *µ*L, 86 mg, 0.8 mmol) in dry CH₂Cl₂ (2 mL) at 0°C. The mixture was stirred for 16 hours, and then neutralized with 10% citric acid. The product was extracted with CH₂Cl₂ three times, and the organic layers were washed with water, then brine, and were then dried over MgSO₄ and concentrated. The product was purified using SiO₂ with CH₂Cl₂/EtOAc 90:10. A colourless oil was obtained (81 mg, 44%). NMR ¹H (ppm, CDCl₃): 9.45 (d, *J*³ = 7.3 Hz, 1H), 7.71-7.19 (m, 19H), 6.71 (t, *J*³ = 5.6 Hz, 1H), 4.61-4.39 (m, 3H), 3.76 (s, 2H), 3.68 (t, *J*³ = 7.6 Hz, 2H), 3.01-2.84 (m, 2H), 1.53 (sext., *J*³ = 7.5 Hz, 2H), 0.72 (t, *J*³ = 7.4 Hz, 3H).

### Example 52: Compound (58)

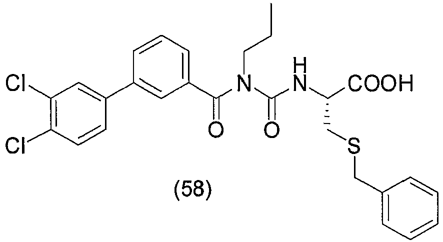

### 52A: N-n-propyl-3-(3',4'-dichloro)-phenylbenzamide

Using Preparation Method 2, N-*n*-propyl-3-bromobenzamide from Example 1A was reacted with 3,4-dichlorophenylboronic acid. The resulting reaction mixture was purified using SiO₂ with AcOEt/CH₂Cl₂ 10:90. A white crystalline solid was obtained (83%). NMR ¹H (ppm, CDCl₃): 7.93 (d, *J*⁴ = 1.6 Hz, 1H), 7.72-7.69 (m, 1H), 7.64 (d, *J*⁴ = 2.0 Hz, 1H), 7.62-7.58 (m, 1H), 7.47-7.42 (m, 2H), 7.37 (d. d., *J*³ = 8.4 Hz, *J*⁴ = 2,1 Hz, 1H), 6.51 (br. s., 1H), 3.44-3.35 (m, 2H), 1.63 (sext., *J*³ = 7.3 Hz, 2H), 0.96 (t, *J*³ = 7.4 Hz, 3H).

### 52B: Compound (58)

Using Preparation Method 3, N-*n*-propyl-3-(3',4'-dichloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (58) as a colourless oil (82%). NMR ¹H (ppm, CDCl₃): 10.65 (br. s., 1H), 9.63 (d, *J*³ = 7.1 Hz, 1H), 7.66-7.14 (m, 12H), 4.81-4.75 (m, 1H), 3.78 (s, 2H), 3.69 (t, *J*³ = 7.5 Hz, 2H), 3.04-2.88 (m, 2H), 1.57 (sext., *J*³ = 7.5 Hz, 2H), 0.74 (t, *J*³ = 7.4 Hz, 3H).

### Example 53: Compound (59)

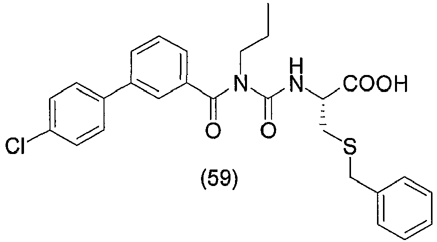

### 53A: N-n-propyl-3-(4'-chloro)-phenylbenzamide

Using Preparation Method 2, N-*n*-propyl-3-bromobenzamide from Example 1A was reacted with 4-chlorophenylboronic acid. The resulting reaction mixture was purified using SiO₂ with AcOEt/CH₂Cl₂ 10:90. A white crystalline solid was obtained (79%). NMR ¹H (ppm, CDCl₃): 7.95 (s, 1H), 7.69 (d, *J*³ = 7.7 Hz, 1H), 7.57 (d, *J*³ = 7.7 Hz, 1H), 7.43 (d. t., *J*³ = 8.6 Hz, *J*⁴ = 2.2 Hz, 2H), 7.35 (t., *J*³ = 7.7 Hz, 1H), 7.31 (d. t., *J*³ = 8.6 Hz, *J*⁴ = 2,2 Hz, 2H), 6.95 (br. s., 1H), 3.39-3.32 (m, 2H), 1.59 (sext., *J*³ = 7.3 Hz, 2H), 0.91 (t, *J*³ = 7.3 Hz, 3H).

### 53B: Compound (59)

Using Preparation Method 3, N-*n*-propyl-3-(4'-chloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (59) as a colourless oil (80%). NMR ¹H (ppm, CDCl₃): 10.0 (br. s., 1H), 9.65 (d, *J*³ = 7.0 Hz, 1H), 7.66-7.23 (m, 13H), 4.81-4.75 (m, 1H), 3.78 (s, 2H), 3.69 (t, *J*³ = 7.4 Hz, 2H), 3.04-2.87 (m, 2H), 1.57 (sext., *J*³ = 7.3 Hz, 2H), 0.73 (t, *J*³ = 7.4 Hz, 3H).

### Example 54: compound (60)

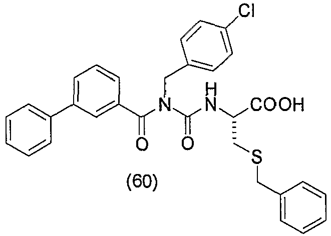

### 54A: Biphenyl-4-carboxylic acid 4-chloro-benzylamide

Using preparation method 1, 3-phenylbenzoic acid (125 mg, 0.63 mmol) was reacted with 4-chlorobenzylamine (99 mg, 0.7 mmol). The product was purified by flash chromatography on SiO₂ using CH₂Cl₂/hexanes 50:50 then CH₂Cl₂ 100%. White crystals were obtained (103 mg, 51 %). NMR ¹H (ppm, CDCl₃): 7.99 (t, *J⁴* = 1.6 Hz, 1H), 7.72 (dd, *J³* = 7.1 Hz, *J⁴* = 1.1 Hz, 2H), 7.58 (dd, *J³* = 7.1 Hz, *J⁴* = 1.5 Hz, 2H), 7.51-7.21 (m, 8H), 6.48 (br. s, 1H), 4.62 (d, *J³* = 5.8 Hz, 2H).

### 54B: compound (60)

Using Preparation Method 3, compound 54A (80 mg, 0.25 mmol) was reacted with phosgene to provide a carbamoylchloride. Using Preparation Method 4, TMS protected S-benzyl-(L)-cysteine (64 mg, 0.3 mmol) was prepared. The carbamoylchloride and TMS protected S-benzyl-(L)-cysteine were reacted using Preparation Method 5 to provide compound (60) as white glassy solid (98 mg, 70 %). NMR ¹H (ppm, CDCl₃): 9.76 (d, *J³* = 6.9 Hz, 1H), 8.95 (br. s, 1H), 7.67 (d, *J³* = 7.5 Hz, 1H), 7.45-7.16 (m, 15H), 6.97 (d, *J³* = 8.0 Hz, 2H), 4.96 (s, 2H), 4.82-4.80 (m, 1H), 3.78 (s, 2H), 3.07-2.89 (m, 2H).

### Example 55: compound (61)

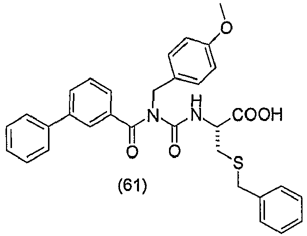

### 55A: Biphenyl-3-carboxylic acid 4-methoxy-benzylamide

Using preparation method 1,3-phenylbenzoic acid (125 mg, 0.63 mmol) was reacted with 4-methoxybenzylamine (123 mg, 0.7 mmol). The product was purified by flash chromatography on SiO₂ using CH₂Cl₂/hexanes 50:50 then CH₂Cl₂ 100%. White crystals were obtained (89 mg, 47 %). NMR ¹H (ppm, CDCl₃): 7.99 (t, *J⁴* = 1.5 Hz, 1H), 7.73-7.69 (m, 2H), 7.59 (dd, *J³* = 8.1 Hz, *J⁴* = 1.0 Hz, 2H), 7.48 (t, *J³* = 7.7 Hz, 1H), 7.44 (t, *J³* = 7.6 Hz, 2H), 7.35 (tt, *J³* = 7.2 Hz, *J⁴* = 2.4 Hz, 1H), 7.26 (d, *J³* = 7.3 Hz, 2H), 7.16 (d, *J³* = 7.8 Hz, 2H), 6.36 (br. s, 1H), 4.62 (d, *J³* = 5.5 Hz, 2H), 2.33 (s, 3H).

### 55B: compound (61)

Using Preparation Method 3, compound 53A (75 mg, 0.25 mmol) was reacted with phosgene to provide a carbamoylchloride. Using Preparation Method 4, TMS protected S-benzyl-(L)-cysteine (64 mg, 0.3 mmol) was prepared. The carbamoylchloride and TMS protected S-benzyl-(L)-cysteine were reacted using Preparation Method 5 to provide compound (61) as a yellow oil (47 mg, 35 %). NMR ¹H (ppm, CDCl₃): 9.83 (d, *J³* = 7.1 Hz, 1H), 8.70 (br. s, 1H), 7.65 (d, *J³* = 7.8 Hz, 1H), 7.46-7.21 (m, 13H), 7.05 (d, *J³* = 7.9 Hz, 2H), 6.93 (d, *J³* = 7.9 Hz, 2H), 4.97 (s, 2H), 4.86-4.80 (m, 1H), 3.78 (s, 2H), 3.07-2.89 (m, 2H), s, 2.35 (s, 3H).

### Example 56: Compound (62)

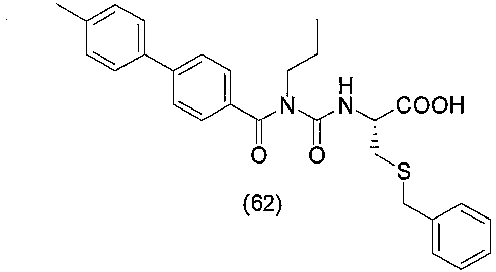

### 56A: N-n-propyl-4-(4'-tolyl)-benzamide

Using Preparation Method 2, N-*n*-propyl-4-bromobenzamide from Example 9A was reacted with 4-tolylboronic acid. The resulting reaction mixture was purified using SiO₂ with AcOEt/CH₂Cl₂ 10:90. A white crystalline solid was obtained (72%). NMR ¹H (ppm, CDCl₃): 7.81 (d, *J*³ = 8.3 Hz, 2H), 7.61 (d, *J*³ = 8.4 Hz, 2H), 7.49 (d, *J*³ = 8.1 Hz, 2H), 7.25 (d, *J*³ = 7.9 Hz, 2H), 6.25 (br. s., 1H), 3.46-3.39 (m, 2H), 2.39 (s, 3H), 1.65 (sext., *J*³ = 7.3 Hz, 2H), 0.98 (t, *J*³ = 7.4 Hz, 3H).

### 56B: Compound (62)

Using Preparation Method 3, N-*n*-propyl-4-(4'-tolyl)-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (62) as a colourless solid (74%). NMR ¹H (ppm, CDCl₃): 9.64 (d, *J*³ = 7.2 Hz, 1H), 8.72 (br. s., 1H), 7.63 (d, *J*³ = 8.4 Hz, 2H), 7.51 (d, *J*³ = 6.6 Hz, 2H), 7.49 (d, *J*³ = 6.6 Hz, 2H), 7.33-7.18 (m, 7H), 4.77-4.73 (m, 1H), 3.78 (s, 2H), 3.71 (t, *J*³ = 7.5 Hz, 2H), 3.02-2.92 (m, 2H), 2.40 (s, 3H), 1.56 (sext., *J*³ = 7.4 Hz, 2H), 0.75 (t, *J*³ = 7.3 Hz, 3H).

### Example 57: Compound (63)

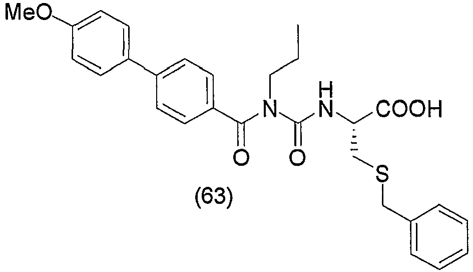

### 57A: N-n-propyl-4-(4'-methoxy)-phenylbenzamide

Using Preparation Method 2, N-*n*-propyl-4-bromobenzamide from Example 9A was reacted with 4-methoxyphenylboronic acid. The resulting reaction mixture was purified using SiO₂ with AcOEt/CH₂Cl₂ 10:90. A white crystalline solid was obtained (56%). NMR ¹H (ppm, CDCl₃): 7.79 (d, *J*³ = 8.4 Hz, 2H), 7.58 (d, *J*³ = 8.5 Hz, 2H), 7.53 (d, *J*³ = 8.8 Hz, 2H), 6.97 (d, *J*³ = 8.8 Hz, 2H), 6.29 (br. s., 1H), 3.84 (s, 3H), 3.45-3.39 (m, 2H), 1.64 (sext., *J*³ = 7.4 Hz, 2H), 0.98 (t, *J*³ = 7.4 Hz, 3H).

### 57B: Compound (63)

Using Preparation Method 3, N-*n*-propyl-4-(4'-methoxy)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (63) as a colourless solid (64%). NMR ¹H (ppm, CDCl₃): 9.56 (d, *J*³ = 7.2 Hz, 1H), 8.84 (br. s., 1H), 7.69-7.19 (m, 11H), 6.99 (d, *J*³ = 9.0 Hz, 2H), 4.80-4.74 (m, 1H), 3.84 (s, 3H), 3.77 (s, 2H), 3.73 (t, *J*³ = 7.5 Hz, 2H), 3.03-2.85 (m, 2H), 1.58 (sext., *J*³ = 7.4 Hz, 2H), 0.75 (t, *J*³ = 7.4 Hz, 3H).

### Example 58: Compound (64)

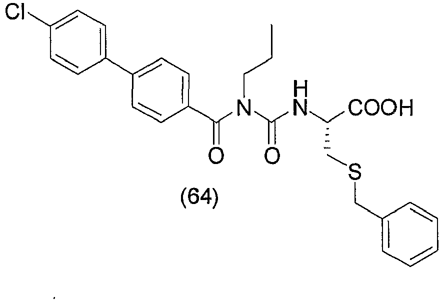

### 58A: N-n-propyl-4-(4'-chloro)-phenylbenzamide

Using Preparation Method 2, N-*n*-propyl-4-bromobenzamide from example 9A was reacted with 4-chlorophenylboronic acid. The resulting reaction mixture was purified using SiO₂ with AcOEt/CH₂Cl₂ 10:90. A white crystalline solid was obtained (50%). NMR ¹H (ppm, CDCl₃): 7.81 (d, *J*³ = 8.2 Hz, 2H), 7.56 (d, *J*³ = 8.2 Hz, 2H), 7.49 (d, *J*³ = 8.4 Hz, 2H), 7.39 (d, *J*³ = 8.6 Hz, 2H), 6.34 (br. s., 1H), 3.45-3.38 (m, 2H), 1.64 (sext., *J*³ = 7.3 Hz, 2H), 0.97 (t, *J*³ = 7.4 Hz, 3H).

### 58B: Compound (64)

Using Preparation Method 3, N-*n*-propyl-4-(4'-chloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (64) as a colourless oil (61%). NMR ¹H (ppm, CDCl₃): 9.62 (d, *J*³ = 7.0 Hz, 1H), 9.24 (br. s., 1H), 7.64 (d, *J*³ = 8.1 Hz, 2H), 7.54-7.50 (m, 4H), 7.42 (d, *J*³ = 8.6 Hz, 2H), 7.33-7.14 (m, 5H), 4.80-4.74 (m, 1H), 3.78 (s, 2H), 3.71 (t, *J*³ = 7.4 Hz, 2H), 3.03-2.87 (m, 2H), 1.57 (sext., *J*³ = 7.4 Hz, 2H), 0.75 (t, *J*³ = 7.4 Hz, 3H).

### Example 59: Compound (65)

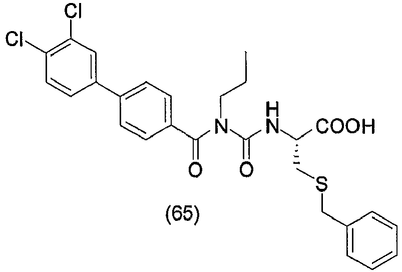

### 59A: N-n-propyl-4-(3',4'-dichloro)-phenylbenzamide

Using Preparation Method 2, N-*n*-propyl-4-bromobenzamide from example 9A was reacted with 3,4-dichlorophenylboronic acid. The resulting reaction mixture was purified using SiO₂ with AcOEt/CH₂Cl₂ 10:90. A white crystalline solid was obtained (92%). NMR ¹H (ppm, CDCl₃): 7.83 (d, *J*³ = 8.5 Hz, 2H), 7.67 (d, *J*⁴ = 2.0 Hz, 1H), 7.58 (d, *J*³ = 8.5 Hz, 2H), 7.51 (d, *J*³ = 8.3 Hz, 1H), 7.41 (d.d., *J*³ = 8.3 Hz, *J*⁴ = 2.1 Hz, 1H), 6.17 (br. s., 1H), 3.47-3.40 (m, 2H), 1.65 (sext., *J*³ = 7.3 Hz, 2H), 0.99 (t, *J*³ = 7.4 Hz, 3H).

### 59B: Compound (65)

Using Preparation Method 3, N-*n*-propyl-4-(3',4'-dichloro)-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (65) as a colourless oil (12%). NMR ¹H (ppm, CDCl₃): 9.37 (br. s., 1H), 7.56-7.13 (m, 7H), 4.61 (br. s., 1H), 3.68-3.44 (m, 4H), 3.01-2.81 (m, 2H), 1.48-1.45 (m, 2H), 0.65 (t, *J*³ = 7.2 Hz, 3H).

### Example 60: Compound (66)

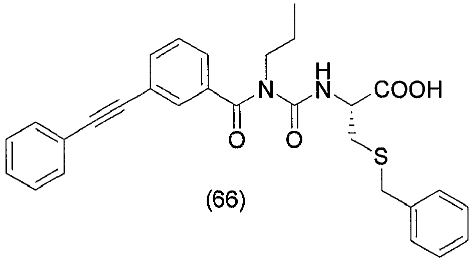

### 60A: N-n-propyl-3-iodobenzamide

Using Preparation Method 1, 3-iodobenzoic acid was reacted with *n*-propylamine. The resulting reaction mixture was purified using SiO₂ with CH₂Cl₂/petroleum ether 95:5 to CH₂Cl₂/AcOEt 95:5 to give N-*n*-propyl-3-iodobenzamide as an off-white solid (82%). NMR ¹H (ppm, CDCl₃): 8.07 (t, *J*⁴ = 1.6 Hz, 1H), 7.80 (d. t., *J*³ = 7.9 Hz, *J*⁴ = 1.1 Hz, 1H), 7.69 (d. t., *J*³ = 7.8 Hz, *J*⁴ = 1.1 Hz, 1H), 7.15 (t, *J*³ = 7.8 Hz, 1H), 6.06 (br. s., 1H), 3.43-3.36 (m, 2H), 1.62 (sext., *J*³ = 7.3 Hz, 2H), 0.97 (t, *J*³ = 7.4 Hz, 3H).

### 60B: N-n-propyl-3-phenylethynyl-benzamide

A mixture of N-*n*-propyl-3-iodobenzamide, phenylacetylene (1.5 eq.) and Pd(PPh₃)₂Cl₂ (5 mol%) in piperidine (3 eq.) was heated at 70°C for 30 minutes in a sealed tube. The solidified residue was dissolved with CH₂Cl₂ and water and poured onto HCl 2N. The acidic phase was extracted three times with CH₂Cl₂. The combined organic layers were washed once with HCl 2N, twice with saturated NaHCO₃, once with water and once with brine. The organic layer was then dried over MgSO₄ and concentrated. The resulting residue was purified using SiO₂ with CH₂Cl₂/petroleum ether 80:20 to CH₂Cl₂ 100% to give N-*n*-propyl-3-phenylethynyl-benzamide as a yellow solid (Quantitative yield). NMR ¹H (ppm, CDCl₃): 7.88 (s, 1H), 7.74-7.68 (m, 1H), 7.63-7.60 (m, 1H), 7.53-7.50 (m, 2H), 7.42-7.33 (m, 4H), 6.22 (br. s., 1H), 3.44-3.38 (m, 2H), 1.64 (sext., *J*³ = 7.3 Hz, 2H), 0.98 (t, *J*³ = 7.3 Hz, 3H).

### 60C: Compound (66)

Using Preparation Method 3, N-*n*-propyl-3-phenylethynyl-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (66) as a colourless oil (68%). NMR ¹H (ppm, CDCl₃): 9.62 (d, *J*³ = 6.9 Hz, 1H), 7.64-7.21 (m, 14H), 4.75-4.69 (m, 1H), 3.78 (s, 2H), 3.67 (t, *J*³ = 7.5 Hz, 2H), 3.02-2.87 (m, 2H), 1.54 (sext., *J*³ = 7.4 Hz, 2H), 0.74 (t, *J*³ = 7.4 Hz, 3H).

### Example 61: Compound (67)

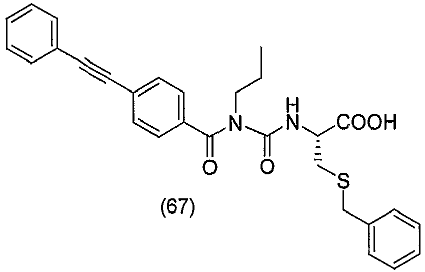

### 61A: N-n-propyl-4-phenylethynyl-benzamide

A mixture of N-*n*-propyl-4-bromobenzamide from Example 9A, phenylacetylene (1.5 eq.) and Pd(PPh₃)₂Cl₂ (5 mol%) in piperidine (3 eq.) was heated at 70°C for 30 minutes in a sealed tube. The solidified residue was dissolved with CH₂Cl₂ and water and poured onto HCl 2N. The acidic phase was extracted three times with CH₂Cl₂. The combined organic layers were washed once with HCl 2N, twice with saturated NaHCO₃, once with water and once with brine. The organic layer was then dried over MgSO₄ and concentrated. The resulting residue was purified using SiO₂ with CH₂Cl₂/petroleum ether 80:20 to CH₂Cl₂ 100% to give N-*n*-propyl-3-phenylethynyl-benzamide as a yellow solid (Quantitative yield). NMR ¹H (ppm, CDCl₃): 7.73 (d, *J*³ = 8.4 Hz, 2H), 7.63-7.51 (m, 5H), 7.36-7.32 (m, 2H), 6.10 (br. s., 1H), 3.45-3.37 (m, 2H), 1.64 (sext., *J*³ = 7.2 Hz, 2H), 0.99 (t, *J*³ = 7.4 Hz, 3H).

### 61B: compound (67)

Using Preparation Method 3, N-*n*-propyl-4-phenylethynyl-benzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (67) as a colourless oil (60%). NMR ¹H (ppm, CDCl₃): 9.60 (d, *J*³ = 6.6 Hz, 1H), 7.61-7.24 (m, 14H), 4.75-4.69 (m, 1H), 3.78 (s, 2H), 3.66 (t, *J*³ = 7.4 Hz, 2H), 3.02-2.87 (m, 2H), 1.54 (sext., *J*³ = 7.4 Hz, 2H), 0.73 (t, *J*³ = 7.4 Hz, 3H).

### Example 62: Compound (70)

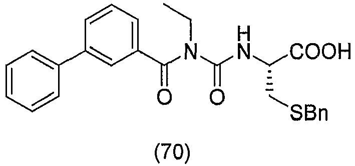

### 62A: Methyl 3-phenylbenzoate

Using Preparation Method 2, Methyl 3-bromobenzoate (3.86 g, 18.6 mmol) was reacted with phenyl boronic acid (2.4 g, 20.5 mmol) in the presence of 5 mol% Pd(PPh₃)₄, 18 mL of 2N Na₂CO₃, 9.4 mL of Ethanol and 37 mL of toluene. The resulting reaction mixture was purified using SiO₂ with hexane/diethyl ether 98:2 to give a colourless thick oil (389 g, 96%). NMR ¹H (ppm, CDCl₃): 8.29 (s, 1H), 8.03 (d, *J*³ = 7.8 Hz, 1H), 7.79 (d, *J*³ = 7.8 Hz, 1H), 7.63 (d, *J*³ = 7.2 Hz, 2H), 7.54-7.26 (m, 4H), 3.96 (s, 3H). MS: M+1 463.2.

### 62B: N-ethyl 3-phenylbenzamide

Trimethylaluminium (920 *µ*L of 2M solution in toluene, 1.84 mmol) was added drop wise to a suspension of ethylamine hydrochloride (151 mg, 1.84 mmol) in 2.76 mL of dry toluene at 0°C. The reaction was then warmed to room temperature and stirred until no gas evolution was observed. The clear solution was then canulated onto a solution of Methyl 3-phenylbenzoate in 6 mL of toluene. The reaction was then heated to 80°C and stirred for 18 hours. The reaction mixture was then carefully treated with 5% HCl at 0°C. The aqueous layer was extracted three times with ethyl acetate. The combined organic layers were washed with brine, dried over Na₂SO₄ and concentrated. The oil obtained was purified by flash chromatography using SiO₂ with CH₂Cl₂/MeOH 99:1 to give a pale yellow oil (129 mg, 62%). NMR ¹H (ppm, CDCl₃): 8.0 (s, 1H), 7.72 (d, *J*³ = 8.1 Hz, 2H), 7.61 (d, *J*³ = 5.4 Hz, 2H), 7.52-7.35 (m, 4H), 6.14 (br. s., 1H), 3.58-3.49 (m, 2H), 1.28 (t, *J*³ = 7.5 Hz, 3H).

### 62C: Compound (70)

Using Preparation Method 3, N-ethyl 3-phenylbenzamide was reacted with phosgene to provide a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to provide compound (70) as a colourless glassy oil (65%). NMR ¹H (ppm, CDCl₃): 9.72 (d, *J*³ = 6.9 Hz, 1H), 9.57 (br. s., 1H), 7.75-7.73 (m, 2H), 7.70-7.69 (m, 2H), 7.61-7.22 (m, 10H), 4.79 (m, 1H), 3.84-3.78 (m, 4H), 3.04-2.95 (m, 2H), 1.16 (t, 3H).

### Example 63: Compound (71)

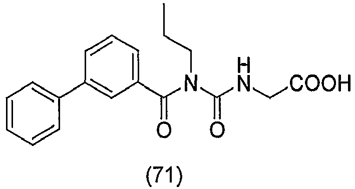

Using Preparation Method 3, N-*n*-propyl-3-phenylbenzamide from Example 1B was reacted with phosgene to provide a carbamoylchloride. Using Preparation Method 4, TMS protected glycine was prepared. The carbamoylchloride and TMS protected glycine were reacted using Preparation Method 5 to give a colourless glassy oil (82%). NMR ¹H (ppm, CDCl₃): 9.50 (t, *J*³ = 5.14 Hz, 1H), 7.70 (d, *J*³ = 6.52 Hz, 1H), 7.64 (s, 1H), 7.59-7.37 (m, 7H), 4.17 (d, *J*³ = 5.41 Hz, 2H), 3.69 (m, 2H), 1.55 (sext., *J*³ = 7.49 Hz, 2H), 0.72 (t, *J*³ = 7.41 Hz, 3H).

### Example 64: Compound (72)

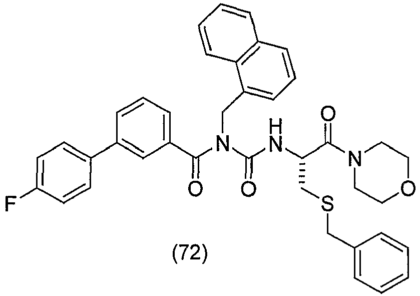

A mixture of compound (36) from Example (34) (105 mg, 0.18 mmol), EDCI (52 mg, 0.36 mmol), DMAP (1.8 mg), and morpholine (31 *µ*L, 0.27 mmol) in 1.8 mL of dry DMF was stirred for 16 hours at room temperature. The reaction was then poured onto 1N HCl. The acidic aqueous phase was extracted three times with ethyl acetate and the combined organic layers were washed with 1N HCl, water and brine and dried over Na₄SO₄. After concentration the reaction product was purified by flash chromatography using SiO₂ with CH₂Cl₂/MeOH 98:2 to give compound (72) as a pale yellow oil (27 mg, 23%). NMR ¹H (ppm, CDCl₃): 9.75 (d., *J*³ = 8.15 Hz, 1H), 7.85 (d, *J*³ = 7.87 Hz, 1H), 7.78 (d, *J*³ = 7.84 Hz, 1H), 9.65 (d, *J*³ = 7.85 Hz, 1H), 7.49-7.23 (m, 13H), 6.90 (d, *J*³ = 6.95 Hz, 1H), 5.53-5.37 (m, 2H), 5.04-4.93 (m, 1H), 3.83-3.72 (m, 2H), 3.67-3.54 (m, 4H), 3.52-3.44 (m, 2H), 3.40-3.25 (m, 2H), 2.87-2.70 (m, 2H).

### Example 65: Compound (74)

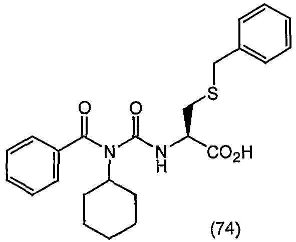

### 65A: N-cyclohexylbenzamide

Cyclohexylamine (8.5 mL, 0.1 mole) was added drop wise to a solution of benzoylchloride (1.17 mL, 10 mmol) in 35 mL of dry THF at 0°C. The reaction was then stirred at room temperature for 3 hours. After that time water was added and the mixture was poured onto crushed ice. A white solid precipitated. It was collected by filtration and rinsed thoroughly with water. The solid was then dried under vacuum (1.63 g, 80 %). NMR ¹H (ppm, CDCl₃): 7.72 (d, *J*³ = 6.78 Hz, 2H), 7.48-7.36 (m, 3H), 6.02 (br. d., *J*³ = 6.58 Hz, 1H), 4.00-3.89 (m, 1H), 2.03-1.99 (m, 2H), 1.79-1.71 (m, 3H), 1.44-1.37 (m, 2H), 1.25-1.15 (m, 3H).

### 65B: Compound (74)

Using Preparation Method 3, N-cyclohexylbenzamide was reacted with phosgene to give a carbamoylchloride. Using Preparation Method 4, TMS protected S-benzyl-(L)-cysteine was prepared. The carbamoylchloride and TMS protected S-benzyl-(L)-cysteine were reacted using Preparation Method 5 to give a colourless glassy oil (65%). NMR ¹H (ppm, CDCl₃),: 10.51 (br. s., 1H), 7.82 (d, *J*³ = 7.11 Hz, 1H), 7.5 (d.d., *J*³ *=* 6.55 Hz, *J*⁴ = 1.61 Hz, 2H), 7.45-7.33 (m, 3H), 7.31-7.19 (m, 5H), 4.57-4.50 (m, 1H), 4.01-3.92 (m, 1H), 3.64 (s, 2H), 2.69 (br. s., 2H), 2.14-2.05 (m, 2H), 1.86-1.77 (m, 4H), 1.54 (br. s., 1H), 1.12 (br. s., 3H).

### Example 66: Compound (75)

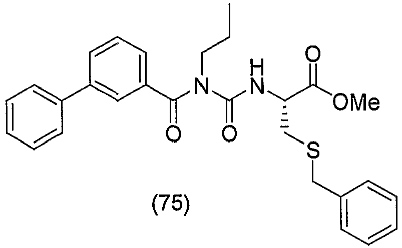

Compound (1) from example 1A (171 mg, 0.36 mmol) was dissolved in dry CH₂Cl₂ (2.5 mL) with DMF (0.25 mL). The solution was cooled to 0°C and stirred under nitrogen. Oxalyl chloride (46 *µ*L, 70 mg, 0.55 mmol) was added via syringe and stirring was applied at room temperature for two hours. The solvent and any excess oxalyl chloride were removed under reduced pressure. The orange residue was dissolved in CH₂Cl₂ (1 mL) and was added dropwise to a mixture of methanol (0.5 mL) in CH₂Cl₂ (2 mL). After stirring for 16 hours at room temperature, the reaction mixture was diluted with 10% citric acid and extracted three times with CH₂Cl₂. The combined organic layers were washed with water and then brine, dried over MgSO₄ and concentrated. The oil obtained was purified using SiO₂ with CH₂Cl₂/EtOAc 90:10. A colourless oil was obtained (89 mg, 51%). NMR ¹H (ppm, CDCl₃): 9.65 (d, *J*³ = 7.2 Hz, 1H), 7.71-7.20 (m, 14H), 4.80-4.74 (m, 1H), 3.77 (s, 2H), 3.76 (s, 3H), 3.70 (t, *J*³ = 7.5 Hz, 2H), 2.99-2.84 (m, 2H), 1.57 (sext., *J*³ = 7.5 Hz, 2H), 0.74 (t, *J*³ = 7.4 Hz, 3H).

### Example 67: Compound (80)

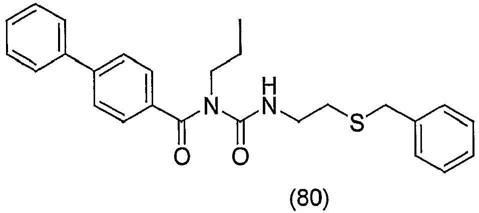

### 67A: 2-(N-Boc-amino)-ethanol

To a solution of ethanolamine (500 mg, 8.2 mmol) in 10 mL of dry CH₂Cl₂ was added DMAP (50 mg, 0.4 mmol), triethylamine (1.25 mL, 9 mmol) and Boc₂O (1.96 g, 90 mmol). The reaction was stirred overnight at room temperature. The reaction mixture was then washed with 10 % citric acid, saturated NaHCO₃ and brine, dried over MgSO₄ and concentrated. A clear oil was obtained which was used without further purification (1 g, 76%). NMR ¹H (ppm, CDCl₃): 5.07 (br. s., 1H), 3.64-3.63 (m, 2H), 3.24-3.23 (m, 2H), 2.9 (br. s., 1H), 1.40 (s, 9H).

### 67B: N-Boc-2-Tosyl-ethylamine

Pyridine (1.5 mL, 18.5 mmol) was added to 2-(N-Boc-amino)-ethanol (280 mg, 1.7 mmol) at 0°C. Tosyl chloride (1.65g, 8.7 mmol) was then added and the reaction was allowed to stir at 0°C overnight. The resulting cloudy reaction was diluted with Et₂O and washed with water, saturated NaHCO₃ and brine, dried over MgSO₄ and concentrated. The resulting white solid was purified using SiO₂ with AcOEt/petroleum ether 20:80 to 30:70. A white solid was obtained (250 mg, 46%). NMR ¹H (ppm, CDCl₃): 7.77 (d, *J*³ = 8.3 Hz, 2H), 7.33 (d, *J*³ = 8.0 Hz, 2H), 4.82 (br. s., 1H), 4.05 (t, *J*³ = 5.08 Hz, 2H), 3.38-3.33 (m, 2H), 2.43 (s, 3H), 1.39 (s, 9H).

### 67C: N-Boc-2-Benzylsulfanyl-ethylamine

N-Boc-2-Tosyl-ethylamine (100 mg, 0.32 mmol) was added to a mixture of benzylmercaptan (45 mL, 0.38 mmol) and Cs₂CO₃ (68 mg, 0.21 mmol) in 1.6 mL of dry DMF. The reaction was stirred for 18 hours after which time the reaction mixture was poured onto water. The aqueous layer was extracted three times with AcOEt. The combined organic layers were washed with water, saturated NaHCO₃ and brine, dried over Na₂SO₄ and concentrated. The residue was purified using SiO₂ with AcOEt/Petroleum ether 5:95 to 15:75. A yellow oil was obtained (72 mg, 83%). NMR ¹H (ppm, CDCl₃): 7.31-7.25 (m, 5H), 4.79 (br. s., 1H), 3.70 (s, 2H), 3.25 (m, 2H), 2.55-2.53 (m, 2H), 1.43 (s, 9H).

### 67D: 2-Benzylsulfanyl-ethylammonium trifluoroacetate

N-Boc-2-Benzylsulfanyl-ethylamine (56 mg, 0.21 mmol) in 1 mL dry CH₂Cl₂ was treated with 0.5 mL of TFA at 0°C. After reaction completion (checked by TLC), the reaction mixture was concentrated in vacuo. The residue was dissolved in toluene and concentrated. This procedure was repeated two more times and the residue was dried under high vacuum and used directly in the next step without further purification.

### 67E: compound (80)

Using Preparation Method 3, N-*n*-propyl-3-phenylbenzamide (42 mg, 0.175 mmol) from Example 1B was reacted with phosgene to provide a carbamoylchloride. To a solution of carbamoylchloride (0.175 mmol) in 2 mL of acetonitrile was added a solution of 2-benzylsulfonyl-ethylammonium trifluoroacetate (56 mg, 0.21 mmol) and triethylamine (33 *µ*L, 0.22 mmol) in 1 mL of acetonitrile at 0°C. The reaction was then stirred at room temperature for 16 hours. The reaction mixture was then diluted with ethyl acetate and poured onto 2N HCl. The aqueous phase was extracted three times with ethyl acetate. The combined organic phases were washed with brine, dried over MgSO₄ and concentrated. The residue was purified using Silica gel with CH₂Cl₂ 100% to give compound (80) as a colourless oil (55 mg, 73%). NMR ¹H (ppm, CDCl₃): 9.18 (br. s., 1H), 7.68-7.30 (m, 14H), 3.75 (s, 2H), 3.69 (t, *J*³ = 7.5 Hz, 2H), 3.54-3.47 (m, 2H), 2.63 (t, *J*³ = 6.8 Hz, 2H), 1.57 (sext., *J*³ = 7.4 Hz, 2H), 0.73 (t, *J*³ = 7.4 Hz, 3H).

### Example 68: compound (81)

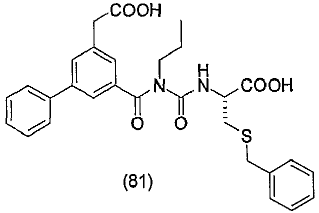

### 68A: 5-Bromo-isophthalic acid dimethyl ester

Prepared from 5-Amino-isophthalic acid dimethyl ester according to Brummer *et al.,* 1998.

### 68B: 5-Bromo-isophthalic acid monomethyl ester

Prepared from 68A according to Chen *et al.,* 2000.

### 68C: 5-Bromo-N-propyl-isophthalamic acid methyl ester

Using Preparation Method 1, 68B (350 mg, 1.3 mmol) was reacted with *n*-propylamine (160 *µ*L, 1.95 mmol). The resulting reaction mixture was purified using SiO₂ with CH₂Cl₂/AcOEt 99:1 to 95:5 to give 64C as a white solid (257 mg, 66%). NMR ¹H (ppm, CDCl₃): 8.27 (s, 2H), 8.13 (s., 1H), 6.30 (br. s., 1H), 3.92 (s, 3H), 3.44-3.37 (m, 2H), 1.65 (sext., *J*³ = 7.2 Hz, 2H), 0.97 (t, *J*³ = 7.4 Hz, 3H).

### 68D: 5-Propylcarbamoyl-biphenyl-3-carboxylic acid methyl ester

Using Preparation Method 2, 68C (247 mg, 0.82 mmol) was reacted with phenyl boronic acid (109 mg, 0.9 mmol) in the presence of 5 mol% Pd(PPh₃)₄, 0.82 mL of 2N Na₂CO₃, 0.43 mL of Ethanol and 1.8 mL of toluene. The resulting reaction mixture was purified using SiO₂ with CH₂Cl₂/AcOEt 98:2 to 90:10 to give a greenish oil (284 g, quant.). NMR ¹H (ppm, CDCl₃): 8.37 (t, *J*⁴ = 1.64 Hz, 1H), 8.27 (d. t., *J*³ = 6.03 Hz, *J*⁴ = 1.75 Hz, 1H), 7.63 (d, *J*³ = 6.97 Hz, 1H), 7.49-7.36 (m, 5H), 6.27 (br. s., 1H), 3.49-3.42 (m, 2H), 1.64 (sext., *J*³ = 7.25 Hz, 2H), 1.00 (t., *J*³ = 7.37 Hz, 3H).

### 68E: 5-Propylcarbamoyl-biphenyl-3-carboxylic acid

A solution of NaOH (44 mg, 1.1 mmol) in 372 *µ*L of MeOH was added to a solution of 68D (284 mg, 0.96 mmol) in 1.8 mL of acetone. The reaction was stirred at room temperature for 16 hrs after which time 10 mg of NaOH and 200 *µ*L of MeOH were added. The reaction was stirred for 72 hrs. The reaction mixture was then concentrated *in vacuo* and the residue obtained was dissolved in water and a few drops of concentrated HCl were added till pH ∼1. An off-white solid appeared. It was collected by filtration and extensively rinsed with water. The solid was then dried in vacuo (194 mg, 72%). NMR ¹H (ppm, CD₃OD) 8.43 (t., *J*⁴ = 1.63 Hz, 1H), 8.38 (t., *J*⁴ = 1.73 Hz, 1H), 8.27 (t., *J*⁴ = 1.78 Hz, 1H), 7.71-7.67 (m, 2H), 7.48 (m, 2H), 7.39 (t.t., *J*³ = 7.34 Hz, *J*⁴ = 1.36 Hz 1H), 3.39-3.34 (m, 2H), 1.65 (sext., *J*³ = 7.18 Hz, 2H), 0.98 (t., *J*³ = 7.34 Hz, 3H).

### 68F: 5-(2-Diazo-acetyl)-biphenyl-3-carboxylic acid propylamide

68E (194 mg, 0.69 mmol) was suspended in 1.5 mL of toluene with 11 *µ*L of dry DMF. Oxalyl chloride (72 *µ*L, 0.82 mmol) was then added dropwise at 0°C leading to a gas evolution. After the addition, the reaction was stirred at room temperature for 2 hours. After that time, the reaction mixture was concentrated. The residue was dissolved in THF. At 0°C, triethylamine (95 *µ*L, 0.69 mmol) was added followed by trimethylsilyldiazomethane (582 *µ*L of a 2 M solution in Et₂O). The reaction was then stirred at room temperature for 16 hours. The reaction mixture was then concentrated and the residue was purified using SiO₂ with CH₂Cl₂/AcOEt 98:2 to 85:15. An orange oil was obtained (100 mg, 48%). NMR ¹H (ppm, CDCl₃): 8.14 (t., *J*⁴ = 1.68 Hz, 1H), 8.06 (t., *J*⁴ = 1.60 Hz, 1H), 8.03 (t., *J*⁴ = 1.68 Hz, 1H), 7.59-7.55 (m, 2H), 7.46-7.34 (m, 3H), 6.51 (br.s., 1H), 6.01 (s, 1H), 3.46-3.39 (m, 2H), 1.64 (sext., *J*³ = 7.24 Hz, 2H), 0.97 (t., *J*³ = 7.37 Hz, 3H).

### 68G: (5-Propylcarbamoyl-biphenyl-3-yl)-acetic acid methyl ester

A freshly prepared solution of Ag(PhCO₂) in NEt₃ (0.5 g in 5 mL, filtered) was added to a solution of 68F (100mg, 0.33 mmol) in 1 mL of dry MeOH. The reaction vessel was then sonicated for 2 minutes. The reaction mixture was then filtered through a pad of celite and the celite was rinsed with MeOH. The filtrate was concentrated and the residue was purified using SiO₂ with CH₂Cl₂/AcOEt 98:2 to 90:10. A yellowish oil was obtained (50 mg, 50%). NMR ¹H (ppm, CDCl₃): 7.86 (s, 1H), 7.61-7.60 (m, 3H), 7.57 (s, 1H), 7.46-7.33 (m, 3H), 6.16 (br. s, 1H), 3.72 (s, 2H), 3.70 (s, 3H), 3.46-3.39 (m, 2H), 1.64 (sext., *J*³ = 7.43 Hz, 2H), 0.98 (t., *J*³ = 7.50 Hz, 3H).

### 68H: (5-Propylcarbamoyl-biphenyl-3-yl)-acetic acid

A solution of LiOH (800 *µ*L of a 2 M solution in H₂O) was added to a solution of 68G (50 mg, 0.16 mmol) in 4 mL of a 3:1 mixture MeOH/H₂O. After 1 hour, the reaction was complete as shown by TLC. The reaction mixture was then concentrated and the residue was dissolved in water and HCl 10% was then added. As no product precipitated, the aqueous solution was extracted 3 times with AcOEt. The combined organic layers were washed with brine, dried over MgSO₄ and concentrated. A white foamy solid was obtained and used in the next step without further purification (47 mg, quantitative). NMR ¹H (ppm, DMSO-d₆): 7.86 (s, 1H), 7.61-7.60 (m, 3H), 7.57 (s, 1H), 7.46-7.33 (m, 3H), 6.16 (br. s, 1H), 3.72 (s, 2H), 3.70 (s, 3H), 3.46-3.39 (m, 2H), 1.64 (sext., *J*³ = 7.43 Hz, 2H), 0.98 (t., *J*³ = 7.50 Hz, 3H).

### 68I: compound (81)

Using Preparation Method 3 with 2.2 equivalents of TMSOTf and NEt₃, 68H was reacted with phosgene to provide a carbamoylchloride. Using Preparation Method 4, Trimethylsilyl (TMS) protected S-benzyl-(L)-cysteine was prepared. The carbamoylchloride and TMS-protected S-benzyl-(L)-cysteine were reacted using Preparation Method 5 to provide compound (81) as a white solid (40%). NMR ¹H (ppm, CDCl₃): 9.56 (d, *J*³ = 7.12 Hz, 1H), 9.5 (br. s, 1H), 7.59-7.55 (m, 4H), 7.44-7.36 (m, 4H), 7.30-7.24 (m, 5H), 4.74 (m, 1H), 3.76 (s, 2H), 3.74 (s, 2H), 3.66 (m, 2H), 3.01-2.86 (m, 2H), 1.57 (sext., *J*³ = 7.44 Hz, 2H), 0.73 (t, *J*³ = 7.28 Hz, 3H).

### Example 69: compound (82)

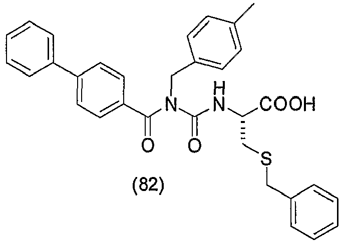

### 69A: N-(4-methyl)-benzyl-4-phenylbenzamide

Using preparation method 1, 4-phenylbenzoic acid (125 mg, 0.63 mmol) was reacted with 4-methylbenzylamine (85 mg, 0.7 mmol). The product was purified by flash chromatography on SiO₂ using CH₂Cl₂/hexanes 50:50 then pure CH₂Cl₂. White crystals were obtained (107 mg, 57 %). NMR ¹H (ppm, CDCl₃): 7.84 (d, *J³* = 8.1 Hz, 2H), 7.63 (d, *J³* = 8.2 Hz, 2H), 7.59 (d, *J³* = 7.2 Hz, 2H), 7.44 (t, *J³* = 7.3 Hz, 2H), 7.36 (t, *J³* = 7.2 Hz, 1H), 7.26 (d, *J³* = 7.6 Hz, 2H), 7.16 (d, *J³* = 7.6 Hz, 2H), 6.34 (br. s, 1H), 4.62 (d, *J³* = 5.3 Hz, 2H), 1.55 (s, 3H).

### 69B: compound (82)

Using preparation method 3, compound 69A (75 mg, 0.25 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. The product was purified by flash chromatography on SiO₂, using CH₂Cl₂ 100%, then CH₂Cl₂/CH₃COOH 99.5:0.5, then CH₂Cl₂/CH₃COOH/MeOH 99:0.5:0.5. A yellow oil was obtained (47 mg, 35 %). NMR ¹H (ppm, CDCl₃): 9.46 (br. s, 1H), 7.40-6.86 (m, 18H), 5.05-4.99 (m, 1H), 4.62 (br. s, 2H), 3.62 (br. s, 2H), 3.03-2.86 (m, 2H), 2.15 (s, 3H).

### Example 70: compound (83)

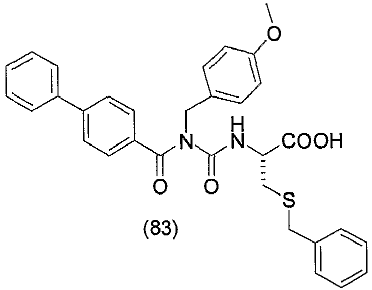

### 70A: N-(4-methoxy)-benzyl-4-phenylbenzamide

Using preparation method 1, 4-phenylbenzoic acid (125 mg, 0.63 mmol) was reacted with 4-methoxybenzylamine (96 mg, 0.7 mmol). The product was purified by flash chromatography on SiO₂ using CH₂Cl₂/hexanes 50:50 then pure CH₂Cl₂. White crystals were obtained (87 mg, 44 %). NMR ¹H (ppm, CDCl₃): 7.84 (d, *J³* = 8.3 Hz, 2H), 7.63 (d, *J³* = 8.4 Hz, 2H), 7.59 (d, *J³* = 7.1 Hz, 2H), 7.44 (t, *J³* = 7.3 Hz, 2H), 7.36 (t, *J³* = 7.2 Hz, 1H), 7.29 (d, *J³* = 8.6 Hz, 2H), 6.88 (d, *J³* = 8.6 Hz, 2H), 6.34 (br. s, 1H), 4.59 (d, *J³* = 5.4 Hz, 2H), 3.80 (s, 3H).

### 70B: compound (83)

Using preparation method 3, compound 70A (79 mg, 0.25 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. The product was purified by flash chromatography on SiO₂, using CH₂Cl₂ 100%, then CH₂Cl₂/CH₃COOH 99.5:0.5, then CH₂Cl₂/CH₃COOH/MeOH 99:0.5:0.5. A colourless oil was obtained (26 mg, 19 %). NMR ¹H (ppm, CDCl₃): 9.63 (br. s, 1H), 7.56-6.73 (m, 18H), 4.96 (d, *J³* = 5.8 Hz, 2H), 4.77-4.73 (m, 1H), 3.76-3.72 (m, 5H), 3.02-2.88 (m, 2H).

### Example 71: compound (84)

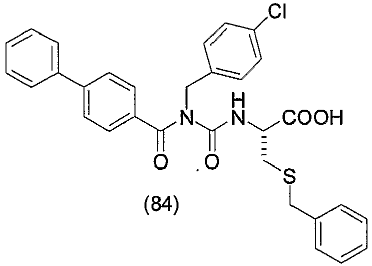

### 71A: N-(4-chloro)-benzyl-4-phenylbenzamide

Using preparation method 1, 4-phenylbenzoic acid (125 mg, 0.63 mmol) was reacted with 4-chlorobenzylamine (99 mg, 0.7 mmol). The product was purified by flash chromatography on SiO₂ using CH₂Cl₂/hexanes 50:50 then CH₂Cl₂ 100%. White crystals were obtained (102 mg, 50 %). NMR ¹H (ppm, CDCl₃): 7.86-7.84 (m, 2H), 7.66-7.58 (m, 4H), 7.47-7.23 (m, 7H), 6.42 (br. s, 1H), 4.63 (br. s, 2H).

### 71B: Compound (84)

Using preparation method 3, compound 71A (80 mg, 0.25 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. The product was purified by flash chromatography on SiO₂, using CH₂Cl₂ 100%, then CH₂Cl₂/CH₃COOH 99.5:0.5, then CH₂Cl₂/CH₃COOH/MeOH 99:0.5:0.5. A colourless oil was obtained (18 mg, 13 %). NMR ¹H (ppm, CDCl₃): 9.62 (d, *J³* = 7.0 Hz, 1H), 7.59-7.55 (m, 4H), 7.47-7.37 (m, 5H), 7.30-7.20 (m, 7H), 6.99 (d, *J³* = 8.3 Hz, 2H), 4.98 (s, 2H), 4.79-4.72 (m, 1H), 3.77 (s, 2H), 3.04-2.87 (m, 2H).

### Example 72: compound (85)

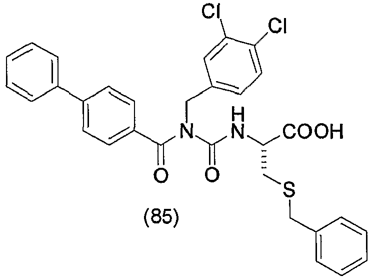

### 72A: Biphenyl-4-carboxylic acid 3,4-dichloro-benzylamide

Using preparation method 1, 4-phenylbenzoic acid (125 mg, 0.63 mmol) was reacted with 3,4-dichlorobenzylamine (123 mg, 0.7 mmol). The product was purified by flash chromatography on SiO₂ using CH₂Cl₂/hexanes 50:50 then CH₂Cl₂ 100%. White crystals were obtained (210 mg, 94 %). NMR ¹H (ppm, CDCl₃): 7.87-7.82 (m, 2H), 7.67-7.56 (m, 4H), 7.48-7.36 (m, 5H), 7.19 (tt, *J³* = 7.8 Hz, *J⁴* = 2.1 Hz, 1H), 6.51 (br. s, 1H), 4.61 (m, 2).

### 72B: compound (85)

Using preparation method 3, compound 72A (89 mg, 0.25 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. The product was purified by flash chromatography on SiO₂, using CH₂Cl₂ 100%, then CH₂Cl₂/CH₃COOH 99.5:0.5, then CH₂Cl₂/CH₃COOH/MeOH 99:0.5:0.5. A yellow oil was obtained (77 mg, 52 %). NMR ¹H (ppm, CDCl₃): 9.60 (d, *J³* = 7.2 Hz, 1H), 7.60 (d, *J³* = 8.5 Hz, 2H), 7.57 (d, *J³* = 7.5 Hz, 2H), 7.47-7.38 (m, 5H), 7.33-7.24 (m, 6H), 7.12 (d, *J⁴* = 2.0 Hz, 1H), 6.92 (dd, *J³* = 8.1 Hz, *J⁴* = 2.4 Hz, 1H), 4.96 (s, 2H), 4.79-4.73 (m, 1H), 3.77 (s, 2H), 3.04-2.87 (m, 2H).

### Example 73: compound (86)

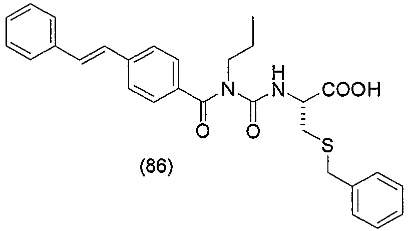

### 73A: 4-Styryl-benzoic acid methyl ester

Methyl 4-(bromomethyl)benzoate (458 mg, 2 mmol) was added to dry toluene (8 mL) along with triphenylphosphine (609 mg, 2.2 mmol). Under nitrogen, reflux conditions were applied for three hours and the mixture was then permitted to cool to room temperature. The fine white solid was filtered off, washed thoroughly with EtOAc, and dried under vacuum (967 mg). This solid was stirred in dry THF (4 mL) under nitrogen, and potassium t-butoxide (246 mg, 2.18 mmol) was added. The bright orange mixture was heated under reflux for one hour. Upon cooling, benzaldehyde (202 µL, 212 mg, 1.2 mmol) in dry THF (2 mL) was added. Further heating under reflux was applied for thirty minutes. The reaction mixture was diluted with water and extracted with EtOAc. The organic layers were combined, washed with water and then brine, dried over MgSO₄ and concentrated. The product was purified by flash chromatography on SiO₂ using EtOAc/hexanes 10:90. A white solid was obtained (97 mg, 20 %).

### 73B: 4-Styryl-benzoic acid

73A (80 mg, 0.34 mmol) was suspended in EtOH (0.5 mL) and aqueous NaOH (1 M, 2 mL) and was heated to 80°C for one hour. Upon cooling to room temperature, the solution was acidified with HCl (concentrated), was cooled to 0°C, and was filtered. The white crystalline precipitate was dried under vacuum (75 mg, quant.). NMR ¹H (ppm, CDCl₃): 7.93 (d, *J³* = 8.3 Hz, 2H), 7.56-7.53 (m, 4H), 7.33 (t, *J³* = 7.4 Hz, 2H), 7.27-7.11 (m, 3H).

### 73C: N-Propyl-4-styryl-benzamide

Using preparation method 1, 73B (67 mg, 0.3 mmol) was reacted with *n*-propylamine (27 *µ*L, 19.5 mg, 0.33 mmol). The product was purified by flash chromatography on SiO₂ using CH₂Cl₂/EtOAc 80:20. White crystals were obtained (48 mg, 61 %). NMR ¹H (ppm, CDCl₃): 7.74 (d, *J³* = 8.4 Hz, 2H), 7.53 (d, *J³* = 8.1 Hz, 2H), 7.51 (d, *J³* = 6.4 Hz, 2H), 7.35 (t, *J³* = 7.1 Hz, 2H), 7.27 (tt, *J³* = 7.2 Hz, *J⁴* = 2.4 Hz, 1H), 7.17 (d, *J³* = 16 Hz, 1H), 7.09 (d, *J³* = 16 Hz, 1H), 6.21 (br. s, 1H), 3.45-3.38 (m, 2H), 1.64 (sext., *J³* = 7.3 Hz, 2H), 0.98 (t, *J³* = 7.4 Hz, 3H).

### 73D: compound (86)

Using preparation method 3, compound 73C (48 mg, 0.18 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. Purification using CH₂Cl₂ 100%, then CH₂Cl₂/CH₃COOH 99.5:0.5, then CH₂Cl₂/CH₃COOH/MeOH 99:0.5:0.5 gave compound (86) as a yellow oil (35 mg, 39 %). NMR ¹H (ppm, CDCl₃): 9.62 (d, *J³* = 7.0 Hz, 1H), 7.58-7.12 (m, 16H), 4.77-4.71 (m, 1H), 3.78 (s, 2H), 3.73-3.68 (m, 2H), 1.56 (sext., J³ = 7.4 Hz, 2H), 0.74 (t, *J³* = 7.4 Hz, 3H).

### Example 74: compound (87)

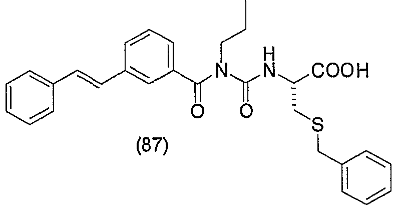

### 74A: 3-Styryl-benzoic acid methyl ester

Methyl 3-bromobenzoate (458 mg, 2 mmol), 1-styreneboronic acid pinacoyl ester (484 mg, 2.1 mmol), Na₂CO₃ (222 mg, 2.1 mmol), tetrakis(triphenylphosphino)palladium (116 mg, 0.1 mmol), water (4 mL) and 1,2-dimethoxyethane (6 mL) were stirred with reflux conditions for one hour, under nitrogen. The reaction mixture was diluted with water and was extracted three times with EtOAc. The organic layers were combined, washed with water and then brine, dried over MgSO₄ and concentrated. The product was purified by trituration with Et₂O. White crystals were obtained (393 mg, 82 %). NMR ¹H (ppm, CDCl₃): 8.19 (t, *J⁴* = 1.6 Hz, 1H), 7.91 (dt, *J³* = 7.7 Hz, *J⁴* = 1.3 Hz, 1H), 7.67 (d, *J³* = 7.8 Hz, 1H), 7.52 (d, *J³* = 7.3 Hz, 2H), 7.42 (t, *J³* = 7.9 Hz, 1H), 7.36 (t, *J³* = 7.4 Hz, 2H), 7.30-7.26 (m, 1H), 7.19 (d, *J³* = 16.3 Hz, 1H), 7.11 (d, *J³* = 16.4 Hz, 1H), 3.06 (s, 3H).

### 74B: 3-Styryl-benzoic acid

74A (357 mg, 1.5 mmol) was suspended in EtOH (2 mL) and aqueous NaOH (1 M, 6 mL) and was heated to 80°C for thirty minutes. Upon cooling to room temperature, the solution was acidified with HCl (concentrated), was cooled to 0°C, and was filtered. The white crystalline precipitate was dried under vacuum (303 mg, 90 %). NMR ¹H (ppm, CDCl₃): 8.26 (s, 1H), 7.99 (d, *J³* = 7.7 Hz, 1H), 7.73 (d, *J³* = 8.2 Hz, 1H), 7.53 (d, *J³* = 7.3 Hz, 2H), 7.46 (td, *J³* = 7.7 Hz, *J⁴* = 2.2 Hz, 1H), 7.37 (t, *J³* = 7.4 Hz, 2H), 7.30-7.24 (m, 1H), 7.21 (d, *J³* = 17 Hz, 1H), 7.13 (d, *J³* = 16 Hz, 1H).

### 74C: N-Propyl-3-styryl-benzamide

Using preparation method 1, 74B (224 mg, 1 mmol) was reacted with *n*-propylamine (90 *µ*L, 65 mg, 1.1 mmol). The product was purified by flash chromatography on SiO₂ using CH₂Cl₂/hexanes 40:60 to CH₂Cl₂/hexanes 60:40. White crystals were obtained (154 mg, 58 %). NMR ¹H (ppm, CDCl₃): 7.92 (s, 1H), 7.60 (t, *J³* = 7.7 Hz, 2H), 7.51 (d, *J³* = 7.8 Hz, 2H), 7.40 (t, *J³* = 7.6 Hz, 1H), 7.36 (t, *J³* = 7.5 Hz, 2H), 7.29-7.26 (m, 1H), 7.18 (d, *J³* = 16.4 Hz, 1H), 7.10 (d, *J³* = 16.3 Hz, 1H), 6.12 (br. s, 1 H), 3.47-3.40 (m, 2H), 1.65 (sext., *J³* = 7.2 Hz, 2H), 1.00 (t, *J³* = 7.4 Hz, 3H).

### 74D: compound (87)

Using preparation method 3, compound 74C (66 mg, 0.25 mmol) was reacted with phosgene to give a carbamoyl chloride. Using preparation method 4, the carbamoyl chloride and TMS- protected S-benzyl-(L)-cysteine (64 mg, 0.3 mmol) were reacted using preparation method 5. Purification using CH₂Cl₂ 100%, then CH₂Cl₂/CH₃COOH 99.5:0.5, then CH₂Cl₂/CH₃COOH/MeOH 99:0.5:0.5 gave compound (87) as a colourless oil (68 mg, 54 %). NMR ¹H (ppm, CDCl₃): 9.69 (d, *J³* = 7.0 Hz, 1H), 7.61-7.24 (m, 14H), 7.16 (d, *J³* = 18.3 Hz, 1H), 7.08 (d, *J³* = 16.2 Hz, 1H), 4.80-4.74 (m, 1H), 3.79 (s, 2H), 3.70 (t, *J³* = 7.5 Hz, 2H), 3.04-2.87 (m, 2H), 1.57 (sext., *J³* = 7.4 H, 2H), 0.74 (t, *J³* = 7.4 Hz, 3H).

### Example 75: compound (88)

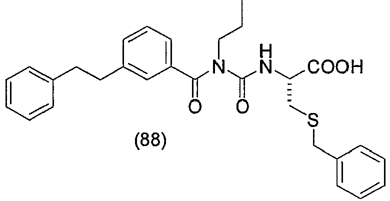

### 75A: 3-Trifluoromethanesulfonyloxy-benzoic acid methyl ester

Methyl 3-hydroxybenzoate (1.52 g, 10 mmol) was dissolved in dry CH₂Cl₂ (25 mL) along with 4-dimethylaminopyridine (244 mg, 2 mmol) and 2,4,6-collidine (1.6 mL, 1.45 g, 12 mmol). The solution was immersed in a dry ice/CH₃CN bath, and stirred under nitrogen. Triflic anhydride (2 mL, 3.36 g, 12 mmol) was added by dropping funnel. The reaction mixture was permitted to reach room temperature and stirring was maintained for thirty minutes. The reaction mixture was quenched with citric acid (10 %) and was extracted three times with CH₂Cl₂. The combined organic layers were washed with water and then brine, were dried over MgSO₄ and were concentrated. The product was purified by flash chromatography on SiO₂ using EtOAc/hexanes 3:97. A colourless oil was obtained (2.50 g, 88 %). NMR ¹H (ppm, CDCl₃): 8.09 (d, *J³* = 10.9 Hz, 1H), 7.92 (s, 1H), 7.57 (t, *J³* = 9.2 Hz, 1H), 7.46 (d, *J³* = 8.3 Hz, 1H), 3.94 (s, 3H).

### 75B: 3-Phenethyl-benzoic acid methyl ester

75A (1.14 g, 4 mmol), N-methylpyrrolidinone (2.2 mL), Fe(acac)₃ (70 mg, 0.2 mmol) and dry THF (25 mL) were stirred under nitrogen at room temperature. Phenethylmagnesium bromide (1.0 M in THF, 5 mL) was added by syringe. After stirring for fifteen minutes, HCl (1 M, 10 mL) was slowly added. The mixture was diluted with water and extracted three times with EtOAc. The organic layers were combined, washed with water and then brine, were dried over MgSO₄ and were concentrated. Purification was achieved using flash chromatography on SiO₂ with hexanes/toluene 50:50. A colourless oil was obtained (660 mg, 69 %). NMR ¹H (ppm, CDCl₃): 7.96-7.85 (m, 2H), 7.33-7.15 (m, 7H), 3.91 (s, 3H), 2.97-2.93 (m, 4H).

### 75C: 3-Phenethyl-benzoic acid

75B (660 mg, 2.75 mmol) was suspended in EtOH (2 mL) and aqueous NaOH (1 M, 6 mL) and was heated to 80°C for thirty minutes. Upon cooling to room temperature, the solution was acidified with HCl (concentrated), was cooled to 0°C, and was filtered. The white crystalline precipitate was dried under vacuum (356 mg, 57 %). NMR ¹H (ppm, CDCl₃): 7.96-7.91 (m, 2H), 7.40-7.36 (m, 2H), 7.30-7.15 (m, 5H), 3.02-2.90 (m, 4H).

### 75D: 3-Phenethyl-N-propyl-benzamide

Using preparation method 1, 75C (226 mg, 1 mmol) was reacted with *n*-propylamine (90 µL, 65 mg, 1.1 mmol). No purification was necessary. White crystals were obtained (107 mg, 40 %). NMR ¹H (ppm, CDCl₃): 7.96-7.89 (m, 2H), 7.56-7.12 (m, 7H), 6.03 (br. s, 1H), 3.42-3.38 (m, 2H), 3.04-2.87 (m, 4H), 1.63 (sext., *J³* = 7.3 Hz, 2H), 0.98 (t, *J³* = 7.4 Hz, 3H).

### 75E: compound (88)

Using preparation method 3, compound 75D (67 mg, 0.25 mmol) was reacted with phosgene to give a carbamoyl chloride. Using preparation method 4, the carbamoyl chloride and TMS- protected S-benzyl-(L)-cysteine cysteine (60 mg, 0.28 mmol) were reacted using preparation method 5. Purification using CH₂Cl₂ 100%, then CH₂Cl₂/CH₃COOH 99.5:0.5, then CH₂Cl₂/CH₃COOH/MeOH 99:0.5:0.5 gave compound (88) as a colourless oil was obtained (45 mg, 36 %). NMR ¹H (ppm, CDCl₃): 9.69 (d, *J³* = 6.9 Hz, 1H), 7.41-7.11 (m, 14H), 4.76-4.79 (m, 1H), 3.78 (s, 2H), 3.58 (t, *J³* = 7.5 Hz, 2H), 3.02-2.86 (m, 2H), 1.50 (sext., *J³* = 7.5 Hz, 2H), 0.70 (t, *J³* = 7.4 Hz, 3H).

### Example 76: compound (89)

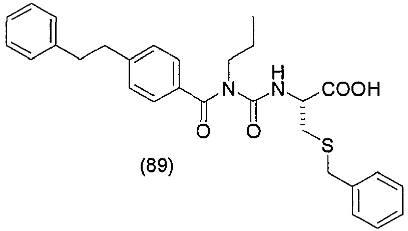

### 76A: 4-Trifluoromethanesulfonyloxy-benzoic acid methyl ester

Methyl 4-hydroxybenzoate (1.52 g, 10 mmol) was dissolved in dry CH₂Cl₂ (25 mL) along with 4-dimethylaminopyridine (244 mg, 2 mmol) and 2,4,6-collidine (1.6 mL, 1.45 g, 12 mmol). The solution was immersed in a dry ice/CH₃CN bath, and stirred under nitrogen. Triflic anhydride (2 mL, 3.36 g, 12 mmol) was added by syringe. The reaction mixture was permitted to reach room temperature and stirring was maintained for thirty minutes. The reaction mixture was quenched with NaHCO₃ (conc.) and was extracted three times with CH₂Cl₂. The combined organic layers were washed with water and then brine, were dried over MgSO₄ and were concentrated. The product was purified by flash chromatography on SiO₂ using hexanes/toluene 50:50. A colourless oil was obtained (2.52 g, 88 %). NMR ¹H (ppm, CDCl₃): 8.13 (d, *J³* = 8.9 Hz, 2H), 7.34 (d, *J³* = 8.9 Hz, 2H), 3.93 (s, 3H).

### 76B: 4-Phenethyl-benzoic acid methyl ester

76A (2.27 g, 8 mmol), N-methylpyrrolidinone (4.4 mL), Fe(acac)₃ (140 mg, 0.4 mmol) and dry THF (50 mL) were stirred under nitrogen at room temperature. Phenethylmagnesium bromide (1.0 M in THF, 10 mL) was added by syringe. After stirring for fifteen minutes, HCl (1 M, 20 mL) was slowly added. The mixture was diluted with water and extracted three times with EtOAc. The organic layers were combined, washed with water and then brine, were dried over MgSO₄ and were concentrated. Purification was achieved using flash chromatography on SiO₂ with hexanes/toluene 50:50. A colourless oil was obtained (1.575 g, 82 %). NMR ¹H (ppm, CDCl₃): 7.94 (d, *J³* = 8.2 Hz, 2H), 7.29-7.13 (m, 7H), 3.89 (s, 3H), 3.01-2.89 (m, 4H).

### 76C: 4-Phenethyl-benzoic acid

76B (1.20 g, 5 mmol) was suspended in EtOH (4 mL) and aqueous NaOH (1 M, 10 mL) and was heated to 75°C for one hour. Upon cooling to room temperature, the solution was acidified with HCl (conc.), was cooled to 0°C, and was filtered. The white crystalline precipitate was dried under vacuum (835 mg, 74 %). NMR ¹H (ppm, CDCl₃): 8.00 (d, *J³* = 8.2 Hz, 2H), 7.29-7.13 (m, 9H), 3.01-2.91 (m, 4H).

### 76D: 4-Phenethyl-N-propyl-benzamide

Using preparation method 1, 76C (226 mg, 1 mmol) was reacted with *n*-propylamine (90 µL, 65 mg, 1.1 mmol). No purification was necessary. White crystals were obtained (103 mg, 39 %). NMR ¹H (ppm, CDCl₃): 7.65 (d, *J³* = 8.1 Hz, 2H), 7.28-7.12 (m, 7H), 6.05 (br. s, 1H), 3.44-3.38 (m, 2H), 3.01-2.92 (m, 4H), 1.63 (sext., *J³* = 7.3 Hz, 2H), 0.98 (t, *J³* = 7.4 Hz, 3H).

### 76E: compound (89)

Using preparation method 3, compound 76D (67 mg, 0.25 mmol) was reacted with phosgene to give a carbamoyl chloride. Using preparation method 4, the carbamoyl chloride and TMS- protected S-benzyl-(L)-cysteine cysteine (60 mg, 0.28 mmol) were reacted using preparation method 5. Purification using CH₂Cl₂ 100%, then CH₂Cl₂/CH₃COOH 99.5:0.5, then CH₂Cl₂/CH₃COOH/MeOH 99:0.5:0.5 gave compound (89) as a colourless oil was obtained (20 mg, 16 %). NMR ¹H (ppm, CDCl₃): 9.66 (br. s, 1H), 7.36-7.11 (m, 14H), 4.71 (br. s, 1H), 3.76 (s, 2H), 3.64 (br. s, 2H) 2.92 (br. s, 6H), 1.50 (br. s, 2H), 0.70 (br. s, 3H).

### Example 77: compound (90)

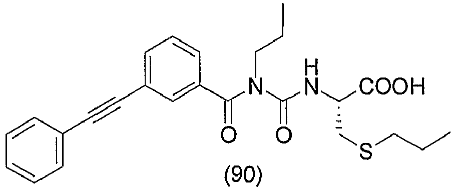

### 77A: Boc-(S)-propyl-L-cysteine

Using preparation method 6, cysteine (242 mg, 2 mmol) was reacted with propyl iodide (219 *µ*L, 2.2 mmol) to provide Boc-(S)-propyl-L-cysteine as thick pale yellow oil (474 mg, 90%). NMR ¹H (ppm, DMSO-d₆): 4.27 (br. t., *J*³ = 6.97 Hz, 1H), 3.00-2.77 (m, 2H), 2.54 (t., *J*³ = 7.19 Hz, 2H), 1.59 (sex., *J*³ = 7.28 Hz, 2H), 1.45 (s, 9H), 0.98 (t., *J*³ = 7.31 Hz, 3H).

### 77B: Compound (90)

Using preparation method 3, compound from example 60B (445 mg, 1.7 mmol) was reacted with phosgene to provide a carbamoylchloride. This carbamoylchloride was dissolved in acetonitrile to obtain a 0.4 M solution. Using preparation method 7, compound 77A (58 mg, 0.22 mmol) was deprotected and reacted with 660 *µ*L of the carbamoylchloride solution. The reaction was then stirred at room temperature for 10 min. Purification by flash chromatography using CH₂Cl₂/Pet. Et. 80:20 then CH₂Cl₂ 100% then CH₂Cl₂/MeOH/AcOH 99:0.5:0.5 to give compound (90) as a pale yellow oil (25 mg, 25%). NMR ¹H (ppm, CDCl₃): 9.62 (d, *J*³ = 6.7 Hz, 1H), 7.63-7.60 (m, 2H), 7.54-7.51 (m, 2H), 7.43-7.40 (m, 2H), 7.36-7.34 (m, 3H), 4.78-4.71 (m, 1H), 3.70-3.62 (m, 2H), 3.13-3.00 (m, 2H), 2.57 (t., *J*³ = 7.24 Hz, 2H), 1.68-1.48 (m, 4H), 0.97 (t, *J*³ = 7.30 Hz, 3H), 0.74 (t, *J*³ = 7.35 Hz, 3H). MS (-ESI): M-H- 451.1.

### Example 78: compound (91)

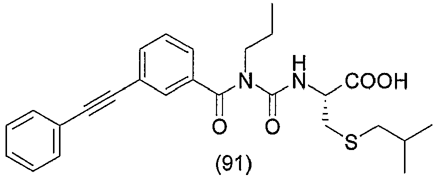

### 78A: Boc-(S)-isobutyl-L-cysteine

Using preparation method 6, cysteine (242 mg, 2 mmol) was reacted with 2-methyl-1-bromopropane (239 *µ*L, 2.2 mmol) to provide Boc-(S)-isobutyl-L-cysteine as thick pale yellow oil (510 mg, 92%). NMR ¹H (ppm, DMSO-d₆): 4.27 (br. t., *J*³ = 7.06 Hz, 1H), 2.99-2.77 (m, 2H), 2.44 (d, *J*³ = 6.81 Hz, 2H), 1.75 (hept., *J*³ = 6.71 Hz, 1H), 1.44 (s, 9H), 0.99 (d, *J*³ = 6.62 Hz, 6H).

### 78B: Compound (91)

Using preparation method 7, compound 78A (62 mg, 0.22 mmol) was deprotected and reacted with 670 *µ*L of the carbamoylchloride solution from example 77B. The reaction was then stirred at room temperature for 10 min. Purification by HPLC semi-preparative to give compound (91) as a colourless oil (25 mg, 24%). NMR ¹H (ppm, CDCl₃): 9.62 (d, *J*³ = 6.84 Hz, 1H), 7.64-7.61 (m, 2H), 7.54-7.50 (m, 2H), 7.43-7.40 (m, 2H), 7.36-7.33 (m, 3H), 4.78-4.72 (m, 1H), 3.70-3.65 (m, 2H), 3.10-2.99 (m, 2H), 2.47 (d, *J*³ = 6.85 Hz, 2H), 1.80 (hept., *J*³ = 6.69 Hz, 1H), 1.54 (sex., *J*³ = 7.51 Hz, 2H), 0.97 (d, *J*³ = 6.64 Hz, 6H), 0.74 (t, *J*³ = 7.37 Hz, 3H). MS (+ESI): M+H⁺ 467.1.

### Example 79: compound (92)

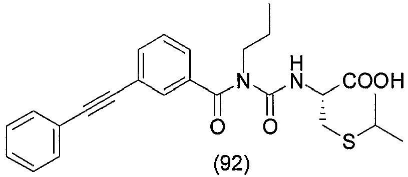

### 79A: Boc-(S)-isopropyl-L-cysteine

Using preparation method 6, cysteine (242 mg, 2 mmol) was reacted with 2-bromopropane (207 *µ*L, 2.2 mmol) to provide Boc-(S)-propyl-L-cysteine as thick pale yellow oil (480 mg, 91%). NMR ¹H (ppm, DMSO-d₆): 4.28 (br. t., *J*³ = 6.95 Hz, 1H), 3.03-2.80 (m, 3H), 1.44 (s, 9H), 1.24 (d, *J*³ = 6.8 Hz, 6H).

### 79B: Compound (92)

Using preparation method 7, compound 79A (56 mg, 0.21 mmol) was deprotected and reacted with 640 *µ*L of the carbamoylchloride solution from example 77B. The reaction was then stirred at room temperature for 10 min. Purification by HPLC semi-preparative to give compound (92) as a colourless yellow oil (25 mg, 24%). NMR ¹H (ppm, CDCl₃): 9.60 (d, *J*³ = 6.80 Hz, 1H), 7.63-7.60 (m, 2H), 7.54-7.50 (m, 2H), 7.46-7.40 (m, 2H), 7.36-7.32 (m, 3H), 4.78-4.72 (m, 1H), 3.70-3.65 (m, 2H), 3.14-2.94 (m, 3H), 1.54 (sex., *J*³ = 7.48 Hz, 2H), 1.27 (d, *J*³ = 6.69 Hz, 6H), 0.74 (t, *J*³ = 7.37 Hz, 3H). MS (-ESI): M-H⁻ 451.1.

### Example 80: compound (93)

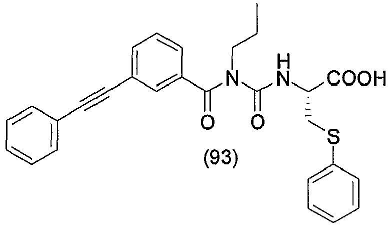

Using preparation method 8, carbamoylchloride from example 77B was reacted with S-phenyl-L-cysteine. Purification by flash chromatography using CH₂Cl₂/Pet.Et. 80:20, then CH₂Cl₂ 100% then CH₂Cl₂/MeOH/AcOH 99:0.5:0.5 gave compound (93) as a pale yellow oil (43 mg, 44%). NMR ¹H (ppm, CDCl₃): 9.66 (d, *J*³ = 6.86 Hz, 1H), 7.63-7.60 (m, 1H), 7.56-7.52 (m, 3H), 7.47-7.40 (m, 2H), 7.42-7.34 (m, 5H), 7.39-7.21 (m, 3H), 4.80-4.74 (m, 1H), 3.62-3.57 (m, 2H), 3.56-3.31 (m, 2H), 1.48 (sex., *J*³ = 7.46 Hz, 2H), 0.71 (t, *J*³ = 7.31 Hz, 3H). MS (-ESI): M-H⁻ 402.0.

### Example 81: compound (94)

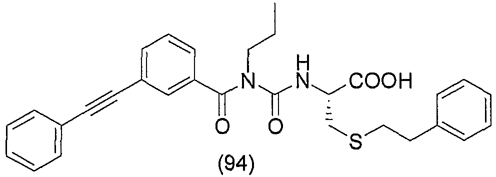

### 81A: Boc-(S)-phenethyl-L-cysteine

Using preparation method 6, cysteine (242 mg, 2 mmol) was reacted with 2-bromopropane (207 *µ*L, 2.2 mmol) to provide Boc-(S)-phenethyl-L-cysteine as thick pale yellow oil (586 mg, 90%). NMR ¹H (ppm, DMSO-d₆): 7.24-7.13 (m, 5H), 4.30-4.26 (m, 1H), 3.00-2.76 (m, 6H), 1.41 (s, 9H).

### 81B: Compound (94)

Using preparation method 7, compound 81A (78 mg, 0.24 mmol) was deprotected and reacted with 528 *µ*L of the carbamoylchloride solution from example 77B. The reaction was then stirred at room temperature for 10 min. Purification by flash chromatography using CH₂Cl₂/Pet.Et. 80:20, then CH₂Cl₂ 100% then CH₂Cl₂/MeOH/AcOH 99:0.5:0.5 gave compound (94) as a pale yellow oil (63 mg, 51%). NMR ¹H (ppm, CDCl₃): 9.67 (d, *J*³ = 6.88 Hz, 1H), 7.63-7.58 (m, 2H), 7.54-7.51 (m, 2H), 7.42 (t, *J*³ = 7.58 Hz, 1H), 7.36-7.34 (m, 3H), 7.26-7.7.24 (m, 1H), 7.21-7.17 (m, 3H), 4.80-4.74 (m, 1H), 3.69-3.64 (m, 2H), 3.15-3.00 (m, 2H), 2.92-2.80 (m, 4H), 1.49 (sex., *J*³ = 7.43 Hz, 2H), 0.72 (t, *J*³ = 7.34 Hz, 3H). MS (-ESI): M-H⁻ 513.7.

### Example 82: compound (95)

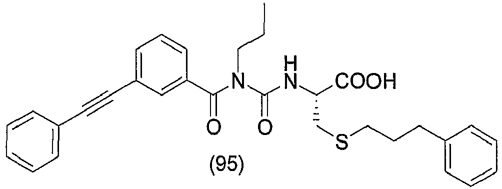

### 82A: Boc-(S)-phenylpropyl-L-cysteine

Using preparation method 6, cysteine (242 mg, 2 mmol) was reacted with 1-bromo-3-phenylpropane (334 *µ*L, 2.2 mmol) to provide Boc-(S)-phenylpropyl-L-cysteine as thick pale yellow oil (638 mg, 94%). NMR ¹H (ppm, DMSO-d₆): 7.26-7.10 (m, 5H), 4.27-4.24 (m, 1H), 3.02-2.78 (m, 2H), 2.69 (t, *J*³ = 7.32, 2H), 2.55 (t, *J*³ = 7.31 Hz, 2H), 1.84 (q, *J*³ = 7.43 Hz, 2H), 1.41 (s, 9H).

### 82B: Compound (95)

Using preparation method 7, compound 82A (48 mg, 0.14 mmol) was deprotected and reacted with 423 *µ*L of the carbamoylchloride solution from example 77B. The reaction was then stirred at room temperature for 10 min. Purification by semi-preparative HPLC to give compound (95) as colourless oil (29 mg, 39%). NMR ¹H (ppm, CDCl₃): 9.66 (d, *J*³ = 6.76 Hz, 1H), 7.64-7.59 (m, 2H), 7.54-7.50 (m, 2H), 7.46-7.33 (m, 5H), 7.28-7.23 (m, 2H), 7.18-7.14 (m, 3H), 4.78-4.72 (m, 1H), 3.70-3.64 (m, 2H), 3.13-3.00 (m, 2H), 2.69 (t, *J*³ = 7.40 Hz, 2H), 2.60 (t, *J*³ = 7.18 Hz, 2H), 1.91 (q, *J*³ = 7.57 Hz, 2H), 1.53 (sex., *J*³ = 7.49 Hz, 2H), 0.73 (t, *J*³ = 7.35 Hz, 3H). MS (-ESI): M-H⁻ 529.2.

### Example 83: compound (96)

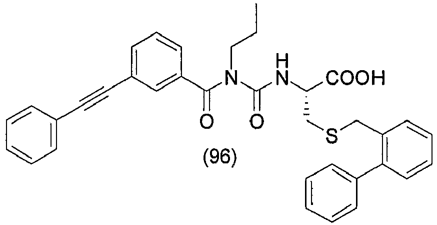

### 83A: Boc-(S)-biphenyl-2-ylmethyl-L-cysteine

Using preparation method 6, cysteine (35.5 mg, 0.25 mmol) was reacted with 2- biphenyl-2-ylmethylbromide (50.5 *µ*L, 0.28 mmol) to provide Boc-(S)-biphenyl-2-ylmethyl-L-cysteine as thick pale yellow oil (252 mg, 65%). NMR ¹H (ppm, CDCl₃): 9.86 (br. s, 1H), 7.42-7.18 (m, 9H), 5.20 (d, *J*³ = 7.05 Hz, 1H), 4.44 (br s., 1H), 3.77-3.67 (m, 2H), 2.96 (m, 2H), 1.43 (s, 9H).

### 83B: Compound (96)

Using preparation method 7, compound 83A (47 mg, 0.12 mmol) was deprotected and reacted with 360 *µ*L of the carbamoylchloride solution from example 77B. The reaction was then stirred at room temperature for 10 min. Purification by semi-preparative HPLC to give compound (96) as colourless oil (6 mg, 9%). NMR ¹H (ppm, CDCl₃): 9.55 (d, *J*³ = 6.70 Hz, 1H), 7.64-7.58 (m, 2H), 7.54-7.50 (m, 2H), 7.46-7.23 (m, 14H), 4.59-4.53 (m, 1H), 3.76 (s, 2H), 3.67-3.62 (m, 2H), 3.07-3.85 (m, 2H), 1.52 (sex., *J*³ = 7.48 Hz, 2H), 0.73 (t, *J*³ = 7.40 Hz, 3H). MS (+ESI): M+H⁺ 577.1.

### Example 84: compound (97)

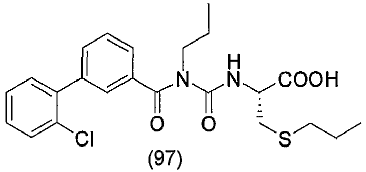

Using preparation method 3, compound from example 43A (527 mg, 1.93 mmol) was reacted with phosgene to provide a carbamoylchloride. This carbamoylchloride was dissolved in acetonitrile to obtain a 2 M solution. Using preparation method 7, compound 77A (45 mg, 0.17 mmol) was deprotected and reacted with 374 *µ*L of the carbamoylchloride solution. The reaction was then stirred at room temperature for 10 min. Purification by semi-preparative HPLC afforded compound (97) as a colourless oil. NMR ¹H (ppm, CDCl₃): 9.67 (d, *J*³ = 6.81 Hz, 1H), 7.54-7.51 (m, 3H), 7.48-7.44 (m, 2H), 7.32-7.28 (m, 3H), 4.93 (br. s, 1H), 4.77-4.71 (m, 1H), 3.75-3.70 (m, 2H), 3.12-2.98 (m, 2H), 2.56 (t., *J*³ = 7.27 Hz, 2H), 1.66-1.49 (m, 4H), 0.96 (t, *J*³ = 7.30 Hz, 3H), 0.73 (t, *J*³ = 7.34 Hz, 3H). MS (+ESI): M+H⁺ 463.5.

### Example 85: compound (98)

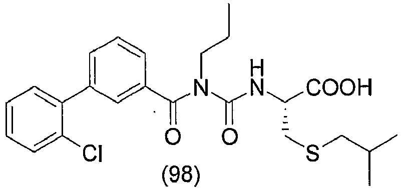

Using preparation method 3, compound from example 43A (112 mg, 0.407 mmol) was reacted with phosgene to provide a carbamoylchloride. This carbamoylchloride was dissolved in acetonitrile to obtain a 2 M solution. Using preparation method 7, compound 78A (102 mg, 0.37 mmol) was deprotected and reacted with 814 *µ*L of the carbamoylchloride solution. The reaction was then stirred at room temperature for 10 min. Purification by semi-preparative HPLC afforded compound (98) as a colourless oil. NMR ¹H (ppm, CDCl₃): 9.67 (d, *J*³ = 6.73 Hz, 1H), 7.54-7.44 (m, 5H), 7.32-7.28 (m, 3H), 4.93 (br. s, 1H), 4.76-4.70 (m, 1H), 4.4 (br. s., 2H), 3.75-3.70 (m, 2H), 3.11-2.98 (m, 2H), 2.47 (d, *J*³ = 6.82 Hz, 2H), 1.80 (hept., *J*³ = 6.69 Hz, 1H), 1.54 (sex., *J*³ = 7.46 Hz, 2H), 0.96 (d, *J*³ = 6.63 Hz, 6H), 0.73 (t, *J*³ = 7.36 Hz, 3H). MS (+ESI): M+H⁺ 477.5.

### Example 86: compound (99)

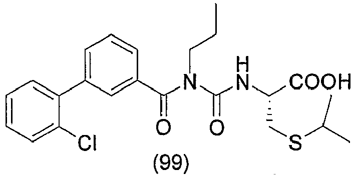

Using preparation method 3, compound from example 43A (69 mg, 0.26 mmol) was reacted with phosgene to provide a carbamoylchloride. This carbamoyl chloride was dissolved in acetonitrile to obtain a 2 M solution. Using preparation method 7, compound 79A (60 mg, 0.23 mmol) was deprotected and reacted with 506 *µ*L of the carbamoylchloride solution. The reaction was then stirred at room temperature for 10 min. Purification by semi-preparative HPLC afforded compound (99) as a colourless oil. NMR ¹H (ppm, CDCl₃): 9.67 (d, *J*³ = 6.76 Hz, 1H), 7.54-7.51 (m, 3H), 7.48-7.43 (m, 2H), 7.32-7.30 (m, 3H), 4.78-4.72 (m, 1H), 3.75-3.70 (m, 2H), 3.07 (t, *J*³ = 6.55 Hz, 2H), 3.01 (hept., *J*³ = 6.67 Hz, 1H), 1.55 (sex., *J*³ = 7.52 Hz, 2H), 1.26 (d, *J*³ = 6.69 Hz, 6H), 0.73 (t, *J*³ = 7.39 Hz, 3H). MS (+ESI): M+H⁺ 463.1.1.

### Example 87: compound (100)

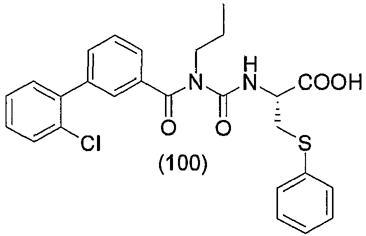

Using preparation method 3, compound from example 43A (61 mg, 0.22 mmol) was reacted with phosgene to provide a carbamoylchloride. This carbamoylchloride was dissolved in acetonitrile to obtain a 2 M solution. Using preparation method 8, this carbamoylchloride was reacted with S-phenyl-L-cysteine. Purification by semi-preparative HPLC afforded compound (100) as a colourless oil. NMR ¹H (ppm, CDCl₃): 9.72 (d, *J*³ = 6.75 Hz, 1H), 7.52-7.41 (m, 7H), 7.32-7.31 (m, 2H), 7.28-7.17 (m, 4H), 4.80-4.71 (m, 1H), 4.5 (br. s., 2H), 3.67-3.62 (m, 2H), 3.57-3.38 (m, 2H), 1.49 (sex., *J*³ = 7.55 Hz, 2H), 0.70 (t, *J*³ = 7.37 Hz, 3H). MS (+ESI): M+H⁺ 497.5.

### Example 88: compound (101)

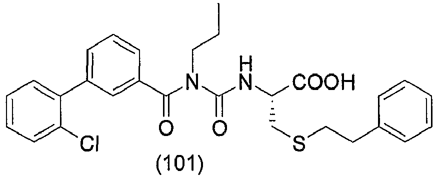

Using preparation method 3, compound from example 43A (72 mg, 0.27 mmol) was reacted with phosgene to provide a carbamoylchloride. This carbamoylchloride was dissolved in acetonitrile to obtain a 2 M solution. Using preparation method 7, compound 81A (77 mg, 0.24 mmol) was deprotected and reacted with 528 *µ*L of the carbamoylchloride solution. The reaction was then stirred at room temperature for 10 min. Purification by flash chromatography using CH₂Cl₂/Pet.Et. 80:20, then CH₂Cl₂ 100% then CH₂Cl₂/MeOH/AcOH 99:0.5:0.5 gave compound (101) as a pale yellow oil. NMR ¹H (ppm, CDCl₃): 9.73 (d, *J*³ = 6.90 Hz, 1H), 8.05 (br. s., 1H), 7.56-7.44 (m, 5H), 7.33-7.31 (m, 3H), 7.27-7.23 (m, 2H), 7.19-7.15 (m, 3H), 4.82-4.76 (m, 1H), 3.75-3.70 (m, 2H), 3.16-2.97 (m, 2H), 2.92-2.80 (m, 4H), 1.52 (sex., *J*³ = 7.49 Hz, 2H), 0.73 (t, *J*³ = 7.35 Hz, 3H). MS (-ESI): M-H⁻ 523.3.

### Example 89: compound (102)

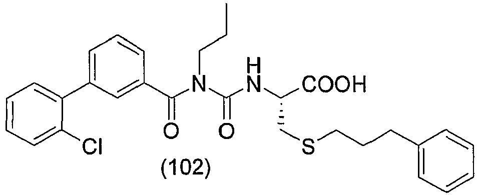

Using preparation method 3, compound from example 43A (118 mg, 0.43 mmol) was reacted with phosgene to provide a carbamoylchloride. This carbamoylchloride was dissolved in acetonitrile to obtain a 2 M solution. Using preparation method 7, compound 82A (131 mg, 0.39 mmol) was deprotected and reacted with 858 *µ*L of the carbamoylchloride solution. The reaction was then stirred at room temperature for 10 min. Purification by flash chromatography using CH₂Cl₂/Pet.Et. 80:20, then CH₂Cl₂ 100% then CH₂Cl₂/MeOH/AcOH 99:0.5:0.5 gave compound (102) as a pale yellow oil. NMR ¹H (ppm, CDCl₃): 9.72 (d, *J*³ = 6.86 Hz, 1H), 9.00 (br. s., 1H), 7.53-7.44 (m, 5H), 7.33-7.30 (m, 3H), 7.27-7.25 (m, 2H), 7.17-7.15 (m, 2H), 4.81-4.75 (m, 1H), 3.76-3.71 (m, 2H), 3.15-3.01 (m, 2H), 2.70 (t, *J*³ = 7.38 Hz, 2H), 2.61 (t, *J*³ = 7.17 Hz, 2H), 1.91 (q, *J*³ = 7.47 Hz, 2H), 1.56 (sex., *J*³ = 7.41 Hz, 2H), 0.74 (t, *J*³ = 7.35 Hz, 3H). MS (-ESI): M-H⁺: 537.5.

### Example 90: compound (103)

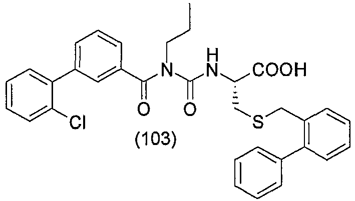

Using preparation method 3, compound from example 43A (45 mg, 0.17 mmol) was reacted with phosgene to provide a carbamoylchloride. This carbamoylchloride was dissolved in acetonitrile to obtain a 2 M solution. Using preparation method 7, compound 83A (58 mg, 0.15 mmol) was deprotected and reacted with 330 *µ*L of the carbamoylchloride solution. The reaction was then stirred at room temperature for 10 min. Purification by semi-preparative HPLC gave compound (103) as colourless oil. NMR ¹H (ppm, CDCl₃): 9.60 (d, *J*³ = 6.68 Hz, 1H), 7.54-7.44 (m, 5H), 7.40-7.23 (m, 12H), 4.67 (br. s., 1H), 4.58-4.52 (m, 1H), 3.75 (s, 2H), 3.73-3.68 (m, 2H), 3.06-2.84 (m, 2H), 1.50 (sex., *J*³ = 7.59 Hz, 2H), 0.71 (t, *J*³ = 7.34 Hz, 3H). MS (-ESI): M-H⁻ 585.7.

### Example 91: compound (104)

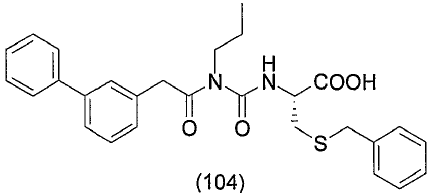

### 91A: 1-Biphenyl-3-yl-2-diazo-ethanone

A mixture of 3-phenylbenzoic acid (595 mg, 3 mmol) and thionyl chloride (1 mL) was heated at 60°C for 2.5 hours. After this time, the thionyl chloride was removed under vaccum. Toluene was then added and the resulting solution was concentrated. This procedure was repeated two more times. The oily residue was then dissolved in dry THF. At 0°C, triethylamine (304 *µ*L, 3 mmol) and trimethylsilyldiazomethane were added successively. The reaction was then stirred for 20 hours at room temperature. The reaction was concentrated. Purification of the residue by flash chromatography using CH₂Cl₂/AcOEt 95:5 to 85:15 gave the compound as a yellow thick oil (141 mg, 21%). NMR ¹H (ppm, CDCl₃): 7.98 (t., *J*³ = 1.37 Hz, 1H), 7.74-7.67 (m, 2H), 7.60-7.57 (m, 2H), 7.50-7.24 (m, 4H), 5.95 (s, 1H). MS (+ESI): M+H⁺: 222.9.

### 91B: Biphenyl-3-yl-acetic acid methyl ester

2 mL of a freshly prepared and filtered solution of Ag(PhCOO) in triethylamine (500 mg in 5mL), was added to a solution of compound 91A in 2 mL of methanol. The dark solution was sonicated for 2 minutes. The reaction mixture was then filtered through a pad of silica gel. The residue was rinsed with methanol. The combined filtrates were concentrated and the residue was purified by flash chromatography using Pet. Et./AcOEt 98:2 to 96:4 to give the compound as a colourless oil (141 mg, quant.). NMR ¹H (ppm, CDCl₃): 7.58 (d., *J*³ = 7.22 Hz, 2H), 7.50-7.48 (m, 2H), 7.45-7.42 (m, 2H), 7.40 (d., *J*³ = 2.51 Hz, 1H), 7.37-7.31 (m, 1H), 7.28-7.25 (m, 1H), 3.70 (s, 3H), 3.69 (s, 2H).

### 91C: Biphenyl-3-yl-acetic acid

3.5 mL of a 2 M LiOH solution in methanol was added to a solution of compound 91B in 3:1 MeOH/H₂O solution. The reaction was stirred at room temperature for 20 hours. The mixture was concentrated under vacuum and the residue dissolved in water. 10% HCl was then added leading to a white precipitate. The solids were collected, washed thoroughly with water and dried (125 mg, 95%). NMR ¹H (ppm, MeOD): 7.58 (d., *J*³ = 7.26 Hz, 2H), 7.52-7.47 (m, 2H), 7.43-7.37 (m, 3H), 7.34-7.24 (m, 2H), 3.66 (s, 2H). MS (-ESI): M-H⁺: 211.

### 91D: 2-Biphenyl-3-yl-N-propyl-acetamide

EDAC.HCl (114 mg, 0.59 mmol) was added to a mixture of compound 91C (125 mg, 0.59 mmol), propylamine (49 *µ*L, 0.59 mmol) and HOBT (80 mg, 0.59 mmol) in 1 mL of CH₃CN. The reaction was stirred for 72 hours. After this time, the mixture was diluted with AcOEt. The organic layer was washed three times with HCl 2N, three times with NaOH 2N, dried over MgSO₄ and concentrated. A with solid was obtained (100 mg, 67 %). NMR ¹H (ppm, CDCl₃): 7.57 (d., *J*³ = 7.05 Hz, 2H), 7.52-7.32 (m, 6H), 7.23 *J*³ = 7.94 Hz, 1H), 5.65 (br. s., 1H), 3.62 (s, 2H), 3.20-3.13 (m, 2H), 1.44 (sex. *J*³ = 7.21 Hz, 2H), 0.83 (t, *J*³ = 7.43 Hz, 3H). NMR ¹³C (ppm, CDCl₃): 170.9, 141.9, 140.5, 135.5, 129.3, 128.8, 128.2, 127.5, 127.0, 126.0, 43.8, 41.3, 22.7, 11.2. MS (+ESI): M+H⁺: 254.3.

### 91E: compound (104)

Using Preparation Method 8, compound 91D (51 mg, 0.2 mmol) and S-benzyl-(L)-cysteine (96 mg, 0.22 mmol) were reacted. Purification using CH₂Cl₂ 100% then CH₂Cl₂/MeOH 99.5:0.5 then CH₂Cl₂/MeOH/AcOH 99:0.5:0.5 provided compound (104) as a colourless oil (34 mg, 35%). NMR ¹H (ppm, CDCl₃): 9.87 (d, *J*³ = 6.40 Hz, 1H), 7.57-7.50 (m, 3H), 7.44-7.39 (m, 3H), 7.35-7.31 (m, 1H), 7.27-7.20 (m, 6H), 4.90 (br. s., 1H), 4.68-4.62 (m, 2H), 3.95 (s, 2H), 3.76-3.70 (m, 2H), 3.72 (s, 2H), 2.96-2.78 (m, 2H), 1.65 (sex., *J*³ = 7.71 Hz, 2H), 0.93 (t, *J*³ = 7.26 Hz, 3H). MS (-ESI): M-H⁺: 489.9.

### Example 92: compound (105)

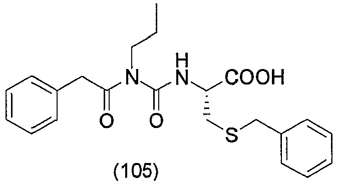

### 92A: 2-Phenyl-N-propyl-acetamide

EDAC.HCl (959 mg, 5 mmol) was added to a mixture of phenylacetic acid (681 mg, 5 mmol), propylamine (411 *µ*L, 5 mmol) and HOBT (676 mg, 5 mmol) in 5 mL of CH₃CN. The reaction was stirred for 72 hours. After this time, the mixture was diluted with AcOEt. The organic layer was washed three times with HCl 2N, three times with NaOH 2N, dried over MgSO₄ and concentrated. A with solid was obtained (576 mg, 65 %). NMR ¹H (ppm, CDCl₃): 7.39-7.24 (m, 5H), 5.38 (br. s., 1H), 3.57 (s, 2H), 3.20-3.13 (m, 2H), 1.45 (sex. *J*³ = 7.19 Hz, 2H), 0.83 (t, *J*³ = 7.40 Hz, 3H).

### 92B: compound (105)

Using Preparation Method 8, compound 92A (36 mg, 0.2 mmol) and S-benzyl-(L)-cysteine (96 mg, 0.22 mmol) were reacted. Purification using CH₂Cl₂ 100% then CH₂Cl₂/MeOH 99.5:0.5 then CH₂Cl₂/MeOH/AcOH 99:0.5:0.5 provided compound (105) as a colourless oil (26 mg, 31%). NMR ¹H (ppm, CDCl₃): 9.87 (d, *J*³ = 6.88 Hz, 1H), 7.37-7.18 (m, 10H), 6.9 (br. s., 1H), 4.69-4.63 (m, 2H), 3.88 (s, 2H), 3.72 (s, 2H), 3.72-3.67 (m, 2H), 2.96-2.78 (m, 2H), 1.62 (sex., *J*³ = 7.06 Hz, 2H), 0.92 (t, *J*³ = 7.35 Hz, 3H). MS (-ESI): M-H⁺: 413.3.

### Example 93: compound (106)

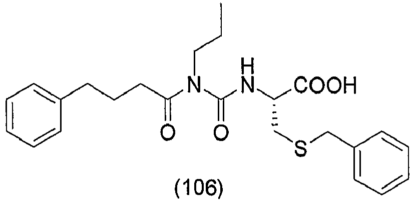

### 93A: 4-Phenyl-N-propyl-butyramide

EDAC.HCl (959 mg, 5 mmol) was added to a mixture of phenylacetic acid (821 mg, 5 mmol), propylamine (411 *µ*L, 5 mmol) and HOBT (676 mg, 5 mmol) in 5 mL of CH₃CN. The reaction was stirred for 72 hours. After this time, the mixture was diluted with AcOEt. The organic layer was washed three times with HCl 2N, three times with NaOH 2N, dried over MgSO₄ and concentrated. A with solid was obtained (698 mg, 68 %). NMR ¹H (ppm, CDCl₃): 7.26-7.21 (m, 2H), 7.17-7.12 (m, 3H), 6.02 (br. s., 1H), 3.18-3.12 (m, 2H), 2.61 (t, *J*³ = 7.49 Hz, 2H), 2.15 (t, *J*³ = 7.14 Hz, 2H), 1.93 (q, *J*³ = 7.36 Hz, 2H), 1.47 (sex. *J*³ = 7.32 Hz, 2H), 0.88 (t, *J*³ = 7.37 Hz, 3H).

### 93B: compound (106)

Using Preparation Method 8, compound 93A (44 mg, 0.2 mmol) and S-benzyl-(L)-cysteine (96 mg, 0.22 mmol) were reacted. Purification using CH₂Cl₂ 100% then CH₂Cl₂/MeOH 99.5:0.5 then CH₂Cl₂/MeOH/AcOH 99:0.5:0.5 provided compound (106) as a colourless oil (32 mg, 36%). NMR ¹H (ppm, CDCl₃): 9.97 (d, *J*³ = 6.88 Hz, 1H), 7.33-7.24 (m, 6H), 7.23-7.16 (m, 4H), 4.69-4.63 (m, 2H), 3.74 (s, 2H), 3.59-3.54 (m, 2H), 2.97-2.81 (m, 2H), 2.69 (t, *J*³ = 7.39 Hz, 2H), 2.50 (t, *J*³ = 7.25 Hz, 2H), 2.02 (q, *J*³ = 7.28 Hz, 2H), 1.51 (sex. *J*³ = 7.87 Hz, 2H), 0.84 (t, *J*³ = 7.35 Hz, 3H). MS (-ESI): M-H⁺: 442.0.

### Example 94: compound (107)

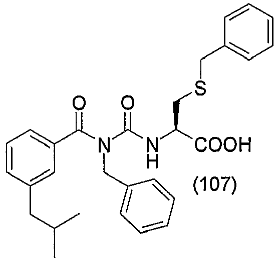

### 94A: 3-Isobutyl-benzoic acid methyl ester

Compound from example 76A (1.62 g, 6 mmol), N-methylpyrrolidinone (3.4 mL), Fe(acac)₃ (140 mg, 0.4 mmol) and dry THF (35 mL) were stirred under nitrogen at room temperature. Isobutylmagnesium bromide (2.0 M in Et₂O, 3.6 mL) was added by syringe.

After stirring for fifteen minutes, HCl (2 M, 20 mL) was slowly added. The mixture was diluted with water and extracted three times with EtOAc. The organic layers were combined, washed with water and then brine, were dried over MgSO₄ and were concentrated. Purification was achieved using flash chromatography on SiO₂ with hexanes/toluene 75:25. A colourless oil was obtained (1.07 g, 93 %). NMR ¹H (ppm, CDCl₃): 7.94-7.89 (m, 2H), 7.39-7.33 (m, 2H), 3.89 (s, 3H), 2.51 (d, *J³* = 7.2 Hz, 2H), 1.94-1.85 (m, 1H), 0.89 (t, *J³* = 6.6 Hz, 6H).

### 94B: 3-Isobutyl-benzoic acid

94A (1.07 g, 5.5 mmol) was suspended in EtOH (10 mL) and aqueous NaOH (1 M, 20 mL) and was heated to 80°C for forty-five minutes. Upon cooling to room temperature, the solution was acidified with HCl (conc.), was cooled to 0°C, and was filtered. The white crystalline precipitate was dried under vacuum (262 mg, 27 %). NMR ¹H (ppm, CDCl₃): 7.94-7.89 (m, 2H), 7.39-7.33 (m, 2H), 2.53 (d, *J³* = 7.2 Hz, 2H), 1.94-1.85 (m, 1H), 0.90 (t, *J³* = 6.6 Hz, 6H).

### 94C: 3-Isobutyl-N-benzyl-benzamide

Using preparation method 1, 94B (131 mg, 0.74 mmol) was reacted with benzylamine (120 µL, 118 mg, 1.1 mmol). Purification by flash chromatography on SiO₂ using CH₂Cl₂/hexanes 50:50 then CH₂Cl₂ 100% afforded white crystals (160 mg, 81 %). NMR ¹H (ppm, CDCl₃): 7.58-7.54 (m, 2H), 7.36-7.24 (m, 7H), 6.35 (br. s, 1H), 4.64 (d, *J³* = 5.7 Hz, 2H), 2.50 (d, *J³* = 7.2 Hz, 2H), 1.92-1.82 (m, 1H), 0.88 (t, *J³* = 6.6 Hz, 6H).

### 94D: compound (107)

Using Preparation Method 8, compound 94C (67 mg, 0.25 mmol) and S-benzyl-(L)-cysteine (60 mg, 0.28 mmol) were reacted. Purification using CH₂Cl₂ 100% then CH₂Cl₂/MeOH 99.5:0.5 then CH₂Cl₂/MeOH/AcOH 99:0.5:0.5 provided compound (107) as a colourless oil (74 mg, 59 %). NMR ¹H (ppm, CDCl₃): 9.79 (d, *J³* = 7.1 Hz, 1H), 7.45 (br. s, 1H), 7.31-7.01 (m, 14H), 4.97 (s, 2H), 4.82-4.75 (m, 1H), 3.77 (s, 2H), 3.02-2.87 (m, 2H), 2.38 (d, *J³* = 7.2 Hz, 2H), 1.75-1.66 (m, 1H), 0.82 (d, *J³* = 6.6 Hz, 6H). MS (-ESI): M-H⁺: 503.7.

### Example 95: compound (108)

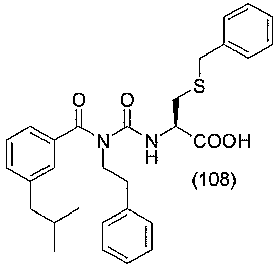

### 95A: 3-Isobutyl-N-phenethyl-benzamide

Using preparation method 1, 94B (131 mg, 0.74 mmol) was reacted with phenethylamine (138 *µ*L, 133 mg, 1.1 mmol). Purification by flash chromatography on SiO₂ using CH₂Cl₂/hexanes 50:50 then CH₂Cl₂ 100% afforded white crystals were obtained (177 mg, 86 %). NMR ¹H (ppm, CDCl₃): 7.50 (s, 1H), 7.47 (dt, *J³* = 7.0 Hz, J⁴ = 1.8 Hz, 1H), 7.33-7.21 (m, 7H), 6.28 (br. s, 1H), 3.72-3.66 (m, 2H), 2.92 (t, *J³* = 7.0 Hz, 2H), 2.48 (d, *J³* = 7.2 Hz, 2H), 1.90-1.81 (m, 1H), 0.88 (d, *J³* = 6.6 Hz, 6H).

### 95B: compound (108)

Using Preparation Method 8, compound 95A (70 mg, 0.25 mmol) and S-benzyl-(L)-cysteine (60 mg, 0.28 mmol) were reacted. Purification using CH₂Cl₂ 100% then CH₂Cl₂/MeOH 99.5:0.5 then CH₂Cl₂/MeOH/AcOH 99:0.5:0.5 provided compound (108) as a colourless oil (27 mg, 21 %). NMR ¹H (ppm, CDCl₃): 9.72 (d, *J³* = 7.1 Hz, 1H), 7.32-7.15 (m, 10H), 7.06 (d, *J³* = 7.2 Hz, 1H), 6.99 (s, 1H), 6.90-6.88 (m, 2H), 6.32 (br. s, 1H), 4.81-4.75 (m, 1H), 3.93 (t, *J³* = 7.3 Hz, 2H), 3.80 (s, 2H), 3.05-2.77 (m, 2H), 2.79 (t, *J³* = 7.3 Hz, 2H), 2.46 (d, *J³* = 7.2 Hz, 2H), 1.89-1.79 (m, 1H), 0.87 (d, *J³* = 7.5 Hz, 6H). MS (-ESI): M-H⁺: 517.5.

### Example 96: compound (109)

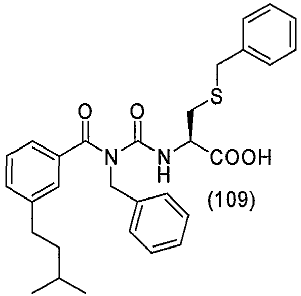

### 96A: 3-Isopentyl-benzoic acid methyl ester

Compound from example 76A (1.42 g, 5 mmol), N-methylpyrrolidinone (2.8 mL), Fe(acac)₃ (120 mg, 0.34 mmol) and dry THF (30 mL) were stirred under nitrogen at room temperature. Isopentylmagnesium bromide (0.46 M in THF, 13 mL) was added by syringe. After stirring for fifteen minutes, HCl 2N (25 mL) was slowly added. The mixture was diluted with water and extracted three times with EtOAc. The organic layers were combined, washed with water and then brine, were dried over MgSO₄ and were concentrated. Purification was achieved using flash chromatography on SiO₂ with hexanes/EtOAc 98:2. A colourless oil was obtained (848 mg, 82 %). NMR ¹H (ppm, CDCl₃): 7.85-7.81 (m, 2H), 7.37-7.29 (m, 2H), 3.90 (s, 3H), 2.67-2.61 (m, 2H), 1.62-1.46 (m, 3H), 0.92 (d, *J3* = 6.3 Hz, 6H).

### 96B: 3-Isopentyl-benzoic acid

96A (824 mg, 4 mmol) was suspended in EtOH (4 mL) and aqueous NaOH (1 M, 20 mL) and was heated under reflux conditions for sixty minutes. Upon cooling to room temperature, the solution was acidified with HCl (conc.), was cooled to 0°C, and was filtered. The white crystalline precipitate was dried under vacuum (633 mg, 82 %). NMR ¹H (ppm, CDCl₃): 7.94-7.91 (m, 2H), 7.42 (d, *J³* = 7.6 Hz, 1H), 7.36 (t, *J³* = 7.7 Hz, 1H), 2.69-2.64 (m, 2H), 1.62-1.49 (m, 3H), 0.94 (d, *J³* = 6.3 Hz, 6H).

### 96C: 3-Isopentyl-N-benzyl-benzamide

Using preparation method 1, 96B (288 mg, 1.5 mmol) was reacted with benzylamine (240 µL, 236 mg, 2.2 mmol). Purification by flash chromatography on SiO₂ using CH₂Cl₂/hexanes 50:50 then CH₂Cl₂ 100% afforded white crystals (316 mg, 74 %). NMR ¹H (ppm, CDCl₃): 7.62 (s, 1H), 7.56-7.53 (m, 1H), 7.36-7.27 (m, 7H), 6.35 (br. s, 1H), 4.64 (d, *J³* = 5.7 Hz, 2H), 2.66-2.60 (m, 2H), 1.64-1.46 (m, 3H), 0.92 (d, *J³* = 6.3 Hz, 6H).

### 96D: compound (109)

Using Preparation Method 8, compound 96C (71 mg, 0.25 mmol) and S-benzyl-(L)-cysteine (60 mg, 0.28 mmol) were reacted. Purification using CH₂Cl₂ 100% then CH₂Cl₂/MeOH 99.5:0.5 then CH₂Cl₂/MeOH/AcOH 99:0.5:0.5 provided compound (109) as a colourless oil (55 mg, 42 %). NMR ¹H (ppm, CDCl₃): 9.80 (d, *J³* = 7.2 Hz, 1H), 7.43 (br. s, 1H), 7.35-7.13 (m, 11H), 7.08 (s, 1H), 7.00 (d, *J³* = 6.4 Hz, 2H), 4.96 (s, 2H), 4.82-4.75 (m, 1H), 3.77 (s, 2H), 3.04-2.87 (m, 2H), 2.53-2.47 (m, 2H), 1.54-1.45 (m, 1H), 1.34 (quart., *J³* = 7.6 Hz, 2H), 0.88 (d, *J³* = 6.5 Hz, 6H). MS (-ESI): M-H⁺: 517.5.

### Example 97: compound (110)

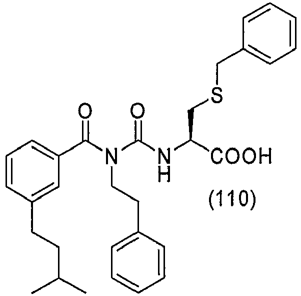

### 97A: 3-Isopentyl-N-phenethyl-benzamide

Using preparation method 1, 96B (288 mg, 1.5 mmol) was reacted with phenethylamine (276 µL, 266 mg, 2.2 mmol). Purification by flash chromatography on SiO₂ using CH₂Cl₂/hexanes 50:50 then CH₂Cl₂ 100% afforded white crystals (330 mg, 74 %). NMR ¹H (ppm, CDCl₃): 7.52 (s, 1H), 7.44-7.41 (m, 1H), 7.34-7.22 (m, 7H), 6.09 (br. s, 1H), 3.74-3.67 (m, 2H), 2.92 (t, *J³* = 6.9 Hz, 2H), 2.64-2.59 (m, 2H), 1.63-1.40 (m, 3H), 0.92 (d, *J³* = 6.3 Hz, 6H).

### 97B: compound (110)

Using Preparation Method 8, compound 97A (74 mg, 0.25 mmol) and S-benzyl-(L)-cysteine (60 mg, 0.28 mmol) were reacted. Purification by flash chromatography on SiO₂ using CH₂Cl₂ 100% then CH₂Cl₂/MeOH 99.5:0.5 then CH₂Cl₂/MeOH/AcOH 99:0.5:0.5 provided compound (110) as a colourless oil (77 mg, 58 %). NMR ¹H (ppm, CDCl₃): 9.73 (d, *J³* = 7.0 Hz, 1H), 7.38-7.16 (m, 10H), 7.10-6.89 (m, 4H), 6.12 (br. s, 1H), 4.82-4.75 (m, 1H), 3.92 (t, *J³* = 7.2 Hz, 2H), 3.80 (s, 2H), 3.05-2.88 (m, 2H), 2.80 (t, *J³* = 7.2 Hz, 2H), 2.62-2.56 (m, 2H), 1.59-1.43 (m, 3H), 0.92 (d, *J³* = 6.4 Hz, 6H). MS (-ESI): M-H⁺: 531.5.

### Example 98: compound (111)

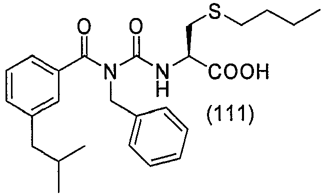

Using preparation method 9, (L)-cysteine (30 mg, 0.25 mmol) was treated with 1-iodobutane (28 µL, 46 mg, 0.25 mmol), and then treated with the carbamoylchloride of 94C. Purification by flash chromatography on SiO₂ using CH₂Cl₂ 100% then CH₂Cl₂/MeOH 99.5:0.5 then CH₂Cl₂/MeOH/AcOH 99:0.5:0.5 provided compound (111) as a colourless oil (37 mg, 31 %). NMR ¹H (ppm, CDCl₃): 9.77 (d, *J³* = 7.0 Hz, 1H), 7.34-7.13 (m, 6H), 7.06 (s, 1H), 6.99 (d, *J³* = 6.3 Hz, 2H), 6.39 (br. s, 1H), 4.96 (s, 2H), 4.82-4.76 (m, 1H), 3.14-2.99 (m, 2H), 2.58 (t, *J³* = 7.3 Hz, 2H), 2.37 (d, *J³* = 7.2 Hz, 2H), 1.76-1.64 (m, 1H), 1.56 (quint., *J³* = 7.5 Hz, 2H), 1.38 (sext., *J³* = 7.4 Hz, 2H), 0.88 (t, *J³* = 7.2 Hz, 3H), 0.81 (d, *J³* = 6.6 Hz, 6H). MS (-ESI): M-H⁺: 469.4.

### Example 99: compound (112)

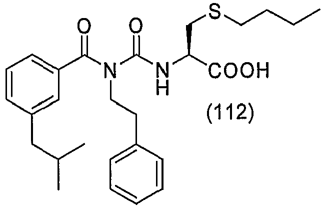

Using preparation method 9, cysteine (30 mg, 0.25 mmol) was treated with 1-iodobutane (28 µL, 46 mg, 0.25 mmol), and then treated with the carbamoylchloride of 95A. Purification by flash chromatography on SiO₂ using CH₂Cl₂ 100% then CH₂Cl₂/MeOH 99.5:0.5 then CH₂Cl₂/MeOH/AcOH 99:0.5:0.5 provided compound (112) as a colourless oil (24 mg, 20 %). NMR ¹H (ppm, CDCl₃): 9.72 (d, *J³* = 6.9 Hz, 1H), 7.30-7.16 (m, 5H), 7.03 (d, *J³* = 7.3 Hz, 1H), 6.99 (s, 1H), 6.90-6.87 (m, 2H), 5.71 (br. s, 1H), 4.82-4.76 (m, 1H), 3.92 (t, *J³* = 7.3 Hz, 2H), 3.16-3.00 (m, 2H), 2.81 (t, *J³* = 7.3 Hz, 2H), 2.61 (t, *J³* = 7.3 Hz, 2H), 2.46 (d, *J³* = 7.1 Hz, 2H), 1.88-1.78 (m, 1H), 1.56 (quint., *J³* = 7.4 Hz, 2H), 1.39 (sext., *J³* = 7.5 Hz, 2H), 0.92-0.86 (m, 9H). MS (-ESI): M-H⁺: 483.5.

### Example 100: compound (113)

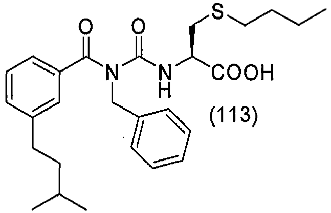

Using preparation method 9, cysteine (30 mg, 0.25 mmol) was treated with 1-iodobutane (28 µL, 46 mg, 0.25 mmol), and then treated with the carbamoylchloride of 96C. Purification by flash chromatography on SiO₂ using CH₂Cl₂ 100% then CH₂Cl₂/MeOH 99.5:0.5 then CH₂Cl₂/MeOH/AcOH 99:0.5:0.5 provided compound (113) as a colourless oil (38 mg, 31 %). NMR ¹H (ppm, CDCl₃): 9.78 (d, *J³* = 6.9 Hz, 1H), 7.24-7.14 (m, 6H), 7.07 (s, 1H), 6.99 (d, *J³* = 6.8 Hz, 2H), 6.70 (br. s, 1H), 4.95 (s, 2H), 4.82-4.76 (m, 1H), 3.14-3.00 (m, 2H), 2.58 (t, *J³* = 7.2 Hz, 2H), 2.52-2.47 (m, 2H), 1.76-1.64 (m, 1H), 1.61-1.29 (m, 7H), 0.91-0.86 (m, 9H). MS (-ESI): M-H⁺: 483.5.

### Example 101: compound (114)

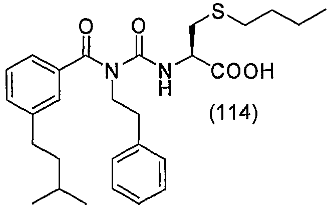

Using preparation method 9, cysteine (30 mg, 0.25 mmol) was treated with 1-iodobutane (28 µL, 46 mg, 0.25 mmol), and then treated with the carbamoylchloride of 97A. Purification by flash chromatography on SiO₂ using CH₂Cl₂ 100% then CH₂Cl₂/MeOH 99.5:0.5 then CH₂Cl₂/MeOH/AcOH 99:0.5:0.5 provided compound (114) as a colourless oil (48 mg, 39 %). NMR ¹H (ppm, CDCl₃): 9.73 (d, *J³* = 6.9 Hz, 1H), 8.02 (br. s, 1H), 7.29-7.16 (m, 5H), 7.02 (d, *J³* = 3.7 Hz, 1H), 6.98 (s, 1H), 6.90 (d, *J³* = 4.8 Hz, 2H), 4.84-4.77 (m, 1H), 3.92 (t, *J³* = 7.1 Hz, 2H), 3.15-3.01 (m, 2H), 2.80 (t, *J³* = 7.1 Hz, 2H), 2.63-2.56 (m, 4H), 1.63-1.33 (m, 7H), 0.92-0.87 (m, 9H). MS (-ESI): M-H⁺: 497.4.

### Example 102: compound (115)

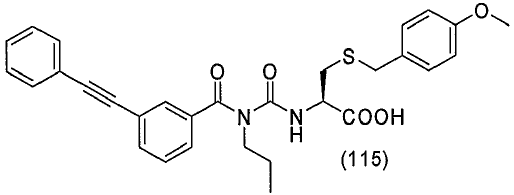

Using preparation method 8, the carbamoylchloride of 60C was reacted with (S)-(4-methoxybenzyl)-(L)-cysteine (22 mg, 0.09 mmol). Purification using a SAX Acetate solid phase extraction column with MeOH 100% then MeOH/AcOH 85:15 provided compound (115) as a colourless oil (16 mg, 34 %). NMR ¹H (ppm, CDCl₃): 9.61 (d, *J³* = 6.9 Hz, 1H), 7.63-7.33 (m, 9H), 7.23 (d, *J³* = 8.5 Hz, 2H), 6.83 (d, *J³* = 8.6 Hz, 2H), 4.80 (br. s), 4.76-4.70 (m, 1H), 3.77 (s, 3H), 3.74 (s, 2H), 3.70-3.65 (m, 2H), 3.01-2.86 (m, 2H), 1.54 (sext., *J³* = 7.4 Hz, 2H), 0.74 (t, *J³* = 7.4 Hz, 3H). MS (-ESI): M-H⁺: 529.3.

### Example 103: compound (116)

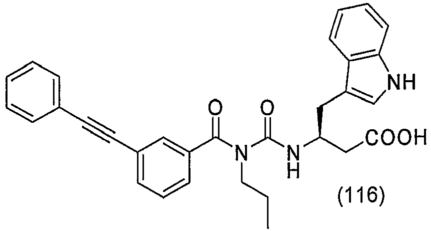

Using preparation method 8, the carbamoylchloride of 60C was reacted with (L)-β-homotryptophan hydrochloride (22 mg, 0.09 mmol). Purification using a SAX Acetate solid phase extraction column with MeOH 100% then MeOH/AcOH 85:15 provided compound (116) as a colourless oil (8 mg, 18 %). NMR ¹H (ppm, CDCl₃): 9.19 (d, *J³* = 7.8 Hz, 1H), 8.11 (br. s, 1H), 7.68 (d, *J³* = 7.4 Hz, 1H), 7.59 (d, *J³* = 7.7 Hz, 1H), 7.53-7.10 (m, 12H), 4.67-4.58 (m, 1H), 3.64-3.59 (m, 2H), 3.31-3.06 (m, 2H), 2.72-2.58 (m, 2H), 1.48 (sext., *J³* = 7.5 Hz, 2H), 0.70 (t, *J³* = 7.4 Hz, 3H). MS (-ESI): M-H⁺: 506.3.

### Example 104: compound (117)

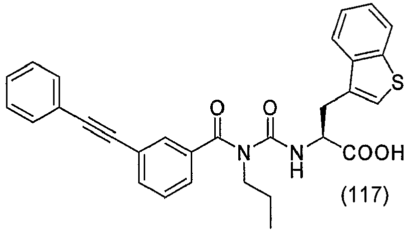

Using Preparation Method 3, compound 60B (26 mg, 0.1 mmol) was reacted with phosgene to provide a carbamoylchloride. Using Preparation Method 4, (L)-3-benzothienylalanine (23 mg, 0.105 mmol) was protected. The carbamoylchloride and TMS protected (L)-3-benzothienylalanine were reacted using Preparation Method 5. Purification using a SAX Acetate solid phase extraction column with MeOH 100% then MeOH/AcOH 85:15 provided compound (117) as a colourless oil (28 mg, 55 %). NMR ¹H (ppm, CDCl₃): 9.50 (d, *J³* = 6.7 Hz, 1H), 7.84 (t, *J³* = 7.1 Hz, 2H), 7.61-7.51 (m, 4H), 7.42-7.12 (m, 8H), 6.00 (br. s, 1H), 4.96-4.89 (m, 1H), 3.62-3.34 (m, 4H), 1.46 (sext., *J³* = 7.4 Hz, 2H), 0.68 (t, *J³* = 7.4 Hz, 3H). MS (-ESI): M-H⁺: 509.2.

### Example 105: compound (118)

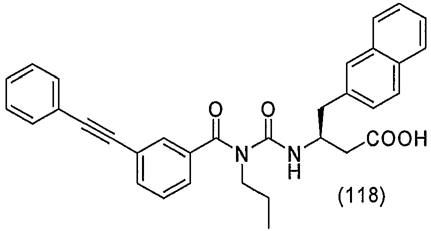

Using Preparation Method 3, compound 60B (26 mg, 0.1 mmol) was reacted with phosgene to provide a carbamoylchloride. Using Preparation Method 4, (L)-3-amino-4-(2-naphthyl)-butyric acid hydrochloride (28 mg, 0.105 mmol) was protected. The carbamoylchloride and TMS protected (S)-3-amino-4-(2-naphthyl)-butyric acid were reacted using Preparation Method 5. Purification using a SAX Acetate solid phase extraction column with MeOH 100% then MeOH/AcOH 85:15 provided compound (118) as a colourless oil (16 mg, 31 %). NMR ¹H (ppm, CDCl₃): 9.24 (d, *J³* = 8.0 Hz, 1H), 7.80-7.15 (m, 16H), 4.66-4.54 (m, 1H), 3.59-3.54 (m, 2H), 3.19-3.06 (m, 2H), 2.67 (d, *J³* = 5.7 Hz, 2H), 1.38 (sext., *J³* = 7.5 Hz, 2H), 0.63 (t, *J³* = 7.4 Hz, 3H). MS (-ESI): M-H⁺: 517.4.

### Example 106: compound (119)

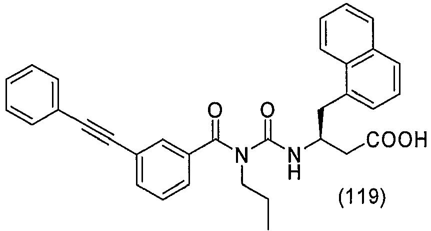

Using Preparation Method 3, compound 60B (26 mg, 0.1 mmol) was reacted with phosgene to provide a carbamoylchloride. Using Preparation Method 4, (L)-3-amino-4-(1-naphthyl)-butyric acid hydrochloride (28 mg, 0.105 mmol) was protected. The carbamoylchloride and TMS protected (S)-3-amino-4-(1-naphthyl)-butyric acid were reacted using Preparation Method 5. Purification using a SAX Acetate solid phase extraction column with MeOH 100% then MeOH/AcOH 85:15 provided compound (119) as a colourless oil (24 mg, 46 %). NMR ¹H (ppm, CDCl₃): 9.30 (d, *J³* = 7.8 Hz, 1H), 8.28 (d, *J³* = 8.4 Hz, 1H), 7.83 (d, *J³* = 8.1 Hz, 1H), 7.74 (dd, *J³* = 7.3 Hz, *J⁴* = 2.0 Hz, 1H), 7.60-7.15 (m, 13H), 5.80 (br. s, 1H), 4.71-4.65 (m, 1H), 3.62-3.32 (m, 4H), 2.73-2.59 (m, 2H), 1.44 (sext., *J³* = 7.4 Hz, 2H), 0.69 (t, *J³* = 7.4 Hz, 3H). MS (-ESI): M-H⁺: 517.3.

### Example 107: compound (120)

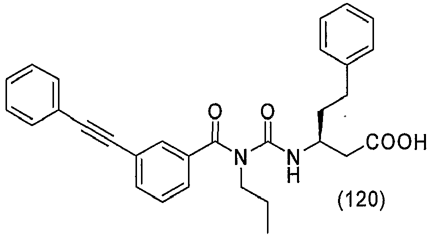

Using Preparation Method 3, compound 60B (26 mg, 0.1 mmol) was reacted with phosgene to provide a carbamoylchloride. Using Preparation Method 4, (L)-3-amino-5-phenylpentanoic acid hydrochloride (24 mg, 0.105 mmol) was protected. The carbamoylchloride and TMS protected (S)-3-amino-5-phenylpentanoic acid were reacted using Preparation Method 5. Purification using a SAX Acetate solid phase extraction column with MeOH 100% then MeOH/AcOH 85:15 provided compound (120) as a colourless oil (15 mg, 31 %). NMR ¹H (ppm, CDCl₃): 9.15 (d, *J³* = 8.2 Hz, 1H), 7.59 (d, *J³* = 8.4 Hz, 2H), 7.53-7.50 (m, 2H), 7.42-7.22 (m, 6H), 7.16 (d, *J3* = 7.4 Hz, 4H), 5.01 (br. s, 1H), 4.27-4.24 (m, 1H), 3.66-3.61 (m, 2H), 2.72-2.61 (m, 4H), 2.04-1.91 (m, 2H), 1.52 (sext., *J³* = 7.4 Hz, 2H), 0.72 (t, *J³* = 7.4 Hz, 3H). MS (-ESI): M-H⁺: 481.4.

### Example 108: compound (121)

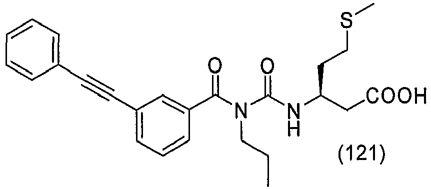

Using Preparation Method 3, compound 60B (26 mg, 0.1 mmol) was reacted with phosgene to provide a carbamoylchloride. Using Preparation Method 4, (L)-β-homomethionine hydrochloride (21 mg, 0.105 mmol) was protected. The carbamoylchloride and TMS protected (L)-β-homomethionine were reacted using Preparation Method 5. Purification using a SAX Acetate solid phase extraction column with MeOH 100% then MeOH/AcOH 85:15 provided compound (121) as a colourless oil (15 mg, 33 %). NMR ¹H (ppm, CDCl₃): 7.60 (d, *J³* = 9.0 Hz, 1H), 7.53-7.50 (m, 2H), 7.42 (t, *J³* = 7.6 Hz, 1H), 7.37-7.33 (m, 4H), 4.42-4.31 (m, 1H), 3.68-3.63 (m, 2H), 2.75-2.60 (m, 2H), 2.55 (br. s, 2H), 2.10 (br. s, 3H), 2.01-1.87 (m, 2H), 1.53 (sext., *J³* = 7.4 Hz, 2H), 0.69 (t, *J³* = 7.4 Hz, 3H). MS (-ESI): M-H⁺: 451.3.

### Example 109: compound (122)

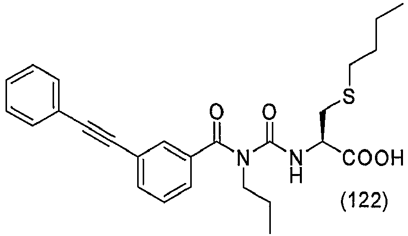

Using preparation method 9, (L)-cysteine (12 mg, 0.1 mmol) was treated with 1-bromobutane (11 µL, 14 mg, 0.1 mmol), and then treated with the carbamoylchloride prepared in 60C. Purification using a SAX Acetate solid phase extraction column with MeOH 100% then MeOH/AcOH 85:15 provided compound (122) as a colourless oil (15 mg, 32 %). NMR ¹H (ppm, CDCl₃): 9.61 (d, *J³* = 6.8 Hz, 1H), 7.63-7.60 (m, 2H), 7.53-7.50 (m, 2H), 7.45-7.33 (m, 5H), 4.97 (br. s, 1H), 4.77-4.71 (m, 1H), 3.70-3.65 (m, 2H), 3.13-2.99 (m, 2H), 2.58 (t, *J³* = 7.3 Hz, 2H), 1.61-1.50 (m, 4H), 1.38 (sext., *J³* = 7.4 Hz, 2H), 0.89 (t, *J³* = 7.3 Hz, 3H), 0.74 (t, *J³* = 7.4 Hz, 3H). MS (-ESI): M-H⁺: 465.5.

### Example 110: compound (123)

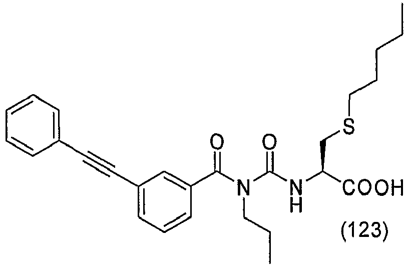

Using preparation method 9, (L)-cysteine (12 mg, 0.1 mmol) was treated with 1-bromopentane (12 µL, 15 mg, 0.1 mmol), and then treated with the carbamoylchloride prepared in 60C. Purification using a SAX Acetate solid phase extraction column with MeOH 100% then MeOH/AcOH 85:15 provided compound (123) as a colourless oil (10 mg, 21 %). NMR ¹H (ppm, CDCl₃): 9.59 (d, *J³* = 6.7 Hz, 1H), 7.63-7.60 (m, 2H), 7.53-7.50 (m, 2H), 7.45-7.33 (m, 5H), 4.75-4.69 (m, 1H), 4.42 (br. s, 1H), 3.70-3.65 (m, 2H), 3.13-2.99 (m, 2H), 2.58 (t, *J³* = 7.4 Hz, 2H), 1.60-1.50 (m, 4H), 1.36-1.31 (m, 4H), 0.87 (t, *J³* = 7.0 Hz, 3H), 0.73 (t, *J³* = 7.4 Hz, 3H). MS (-ESI): M-H⁺: 479.3.

### Example 111: compound (124)

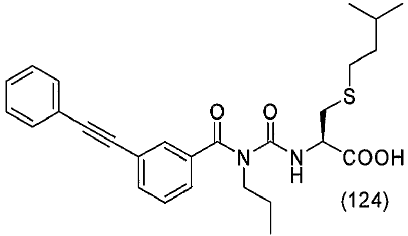

Using preparation method 9, (L)-cysteine (12 mg, 0.1 mmol) was treated with 1-bromo-3-methylbutane (12.5 µL, 15 mg, 0.1 mmol), and then treated with the carbamoylchloride prepared in 60C. Purification using a SAX Acetate solid phase extraction column with MeOH 100% then MeOH/AcOH 85:15 provided compound (124) as a colourless oil (11 mg, 23 %). NMR ¹H (ppm, CDCl₃): 9.57 (d, J³ = 6.7 Hz, 1H), 7.62-7.59 (m, 2H), 7.53-7.50 (m, 2H), 7.44-7.32 (m, 5H), 4.81 (br. s, 1H), 4.71-4.65 (m, 1H), 3.68-3.63 (m, 2H), 3.13-2.95 (m, 2H), 2.60-2.55 (m, 2H), 1.71-1.42 (m, 5H), 0.87 (d, *J³* = 6.5 Hz, 6H), 0.72 (t, *J³* = 7.4 Hz, 3H). MS (-ESI): M-H⁺: 479.4.

### Example 112: compound (125)

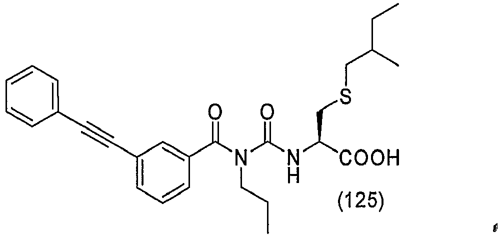

Using preparation method 9, (L)-cysteine (12 mg, 0.1 mmol) was treated with 1-bromo-2-methylbutane (12.5 µL, 15 mg, 0.1 mmol), and then treated with the carbamoylchloride prepared in 60C. Purification using a SAX Acetate solid phase extraction column with MeOH 100% then MeOH/AcOH 85:15 provided compound (125) as a colourless oil (7 mg, 15 %). NMR ¹H (ppm, CDCl₃): 9.62 (d, *J³* = 6.9 Hz, 1H), 7.63-7.60 (m, 2H), 7.53-7.50 (m, 2H), 7.46-7.36 (m, 5H), 4.75-4.71 (m, 1H), 3.70-3.65 (m, 2H), 3.05-3.03 (m, 2H), 2.61-2.43 (m, 2H), 1.55-1.30 (m, 4H), 1.24-1.12 (m, 1H), 0.96 (d, *J³* = 6.5 Hz, 3H), 0.87 (t, *J³* = 7.3 Hz, 3H), 0.74 (t, *J³* = 7.3 Hz, 3H). MS (-ESI): M-H⁺: 479.3.

### Example 113: compound (126)

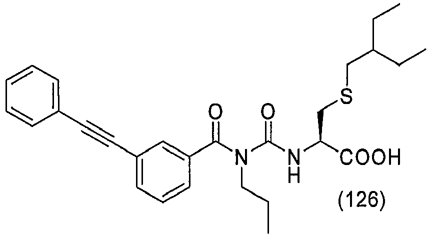

Using preparation method 9, (L)-cysteine (12 mg, 0.1 mmol) was treated with 1-bromo-2-ethylbutane (14 µL, 16.5 mg, 0.1 mmol), and then treated with the carbamoylchloride prepared in 60C. Purification using a SAX Acetate solid phase extraction column with MeOH 100% then MeOH/AcOH 85:15 provided compound (126) as a colourless oil (8 mg, 16 %). NMR ¹H (ppm, CDCl₃): 9.58 (d, *J³* = 6.3 Hz, 1H), 7.62-7.59 (m, 2H), 7.53-7.50 (m, 2H), 7.45-7.34 (m, 5H), 4.70-4.66 (m, 1H), 3.68-3.63 (m, 2H), 3.17-2.91 (m, 2H), 2.56 (d, *J³* = 4.9 Hz, 2H), 1.53 (sext., *J³* = 7.6 Hz, 2H), 1.48-1.24 (m, 5H), 0.87 (t, *J³* = 6.9 Hz, 6H), 0.73 (t, *J³ =* 7.3 Hz, 3H). MS (-ESI): M-H⁺: 493.3.

### Example 114: compound (127)

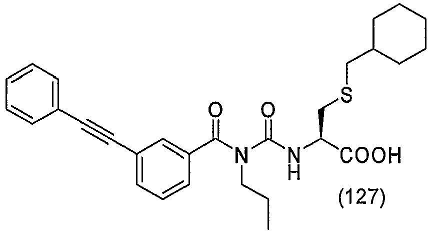

Using preparation method 9, (L)-cysteine (12 mg, 0.1 mmol) was treated with (bromomethyl)cyclohexane (14 µL, 18 mg, 0.1 mmol), and then treated with the carbamoylchloride prepared in 60C. Purification using a SAX Acetate solid phase extraction column with MeOH 100% then MeOH/AcOH 85:15 provided compound (127) as a colourless oil (11 mg, 22 %). NMR ¹H (ppm, CDCl₃): 9.55 (d, *J³* = 6.0 Hz, 1H), 7.62-7.59 (m, 2H), 7.53-7.50 (m, 2H), 7.44-7.34 (m, 5H), 5.17 (br. s, 1H), 4.71-4.65 (m, 1H), 3.68-3.63 (m, 2H), 3.16-3.00 (m, 2H), 2.49 (d, *J³* = 9.5 Hz, 2H), 1.83-1.36 (m, 8H), 1.23-1.12 (m, 3H), 0.96-0.86 (m, 4H), 0.72 (t, *J³* = 7.3 Hz, 3H). MS (-ESI): M-H⁺: 505.2.

### Example 115: compound (128)

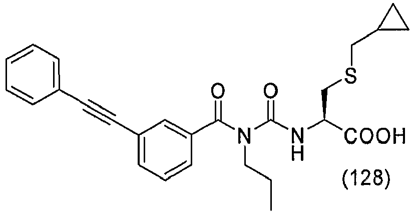

Using preparation method 9, (L)-cysteine (12 mg, 0.1 mmol) was treated with (bromomethyl)cyclopropane (10 µL, 14 mg, 0.1 mmol), and then treated with the carbamoylchloride prepared in 60C. Purification using a SAX Acetate solid phase extraction column with MeOH 100% then MeOH/AcOH 85:15 provided compound (128) as a colourless oil (7 mg, 15 %). NMR ¹H (ppm, CDCl₃): 9.61 (d, *J³* = 6.5 Hz, 1H), 7.63-7.60 (m, 2H), 7.53-7.50 (m, 2H), 7.45-7.35 (m, 5H), 4.78-4.72 (m, 1H), 3.70-3.65 (m, 2H), 3.21-3.04 (m, 2H), 2.54 (d, *J³* = 6.9 Hz, 2H), 1.54 (sext., *J³* = 7.1 Hz, 2H), 1.05-0.95 (m, 1H), 0.74 (t, *J³* = 7.3 Hz, 3H), 0.57 (d, *J³* = 8.1 Hz, 2H), 0.22 (d, *J³* = 4.4 Hz, 2H). MS (-ESI): M-H⁺: 463.2.

### Example 116: compound (129)

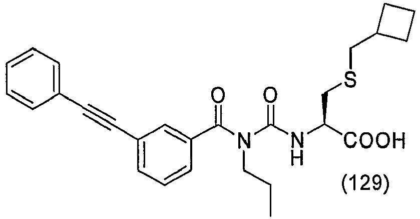

Using preparation method 9, (L)-cysteine (12 mg, 0.1 mmol) was treated with (bromomethyl)cyclobutane (11 µL, 15 mg, 0.1 mmol), and then treated with the carbamoylchloride prepared in 60C. Purification using a SAX Acetate solid phase extraction column with MeOH 100% then MeOH/AcOH 85:15 provided compound (129) as a colourless oil (6 mg, 13 %). NMR ¹H (ppm, CDCl₃): 9.60 (d, *J³* = 7.1 Hz, 1H), 7.63-7.60 (m, 2H), 7.53-7.50 (m, 2H), 7.46-7.35 (m, 5H), 4.75-4.70 (m, 1H), 3.70-3.65 (m, 2H), 3.20-2.97 (m, 2H), 2.66 (d, *J³* = 7.5 Hz, 2H), 2.49 (sept., *J³* = 7.5 Hz, 1H), 1.92-1.68 (m, 4H), 1.54 (sext., *J³* = 7.4 Hz, 2H), 0.74 (t, *J³* = 7.3 Hz, 3H). MS (-ESI): M-H⁺: 477.3.

### Example 117: compound (130)

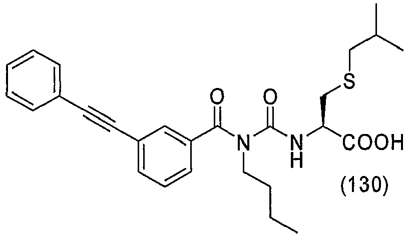

### 117A: Methyl 3-iodobenzoate

3-iodobenzoic acid (7.44 g, 30 mmol) was suspended in dry MeOH (40 mL) under nitrogen at 0°C. SOCl₂ (3.3 mL) was added over 5 minutes. Stirring continued at room temperature for 16 hours, after which the reaction mixture was concentrated. The residue was dissolved in EtOAc and was washed twice with NaHCO₃ (conc.). The organic solution was dried over MgSO₄, filtered and concentrated to yield a white crystalline solid (7.32 g, 93 %). NMR ¹H (ppm, CDCl₃): 8.36 (t, *J⁴* = 1.6 Hz, 1H), 7.98 (d, *J³* = 7.8 Hz, 1H), 7.86 (d, *J³* = 7.9 Hz, 1H), 7.16 (t, *J³* = 7.8 Hz, 1H), 3.90 (s, 3H).

### 117B: Methyl 3-phenylethynyl-benzoate

Methyl 3-iodobenzoate, phenylacetylene (1.5 eq.) and Pd(PPh₃)₂Cl₂ (5 mol%) in piperidine (3 eq.) was heated at 70°C for 30 minutes. The solidified residue was dissolved with CH₂Cl₂ and water and poured onto HCl 2N. The acidic phase was extracted three times with CH₂Cl₂. The combined organic layers were washed twice with HCl 2N, once with water and once with brine. The organic layer was then dried over MgSO₄ and concentrated. The resulting residue was purified using SiO₂ with petroleum spirit/toluene 70:30 to give methyl 3-phenylethynyl-benzoate as a white solid (quantitative yield). NMR ¹H (ppm, CDCl₃): 8.20 (t, *J⁴* = 1.7 Hz, 1H), 7.98 (d, *J³* = 7.9 Hz, 1H), 7.69 (d, *J³* = 7.6 Hz, 1H), 7.54-7.50 (m, 2H), 7.42 (t, *J³* = 7.8 Hz, 1H), 7.36-7.31 (m, 3H), 3.92 (s, 3H).

### 117C: 3-phenylethynyl-benzoic acid

Compound from example 117B (1.18 g, 5 mmol) was suspended in EtOH (10 mL) containing NaOH 2N (15 mL) and was stirred at 80°C for sixty minutes. Upon cooling to room temperature the solution was acidified with HCl (conc.). The white precipitate was filtered off and dried under vacuum to give 3-phenylethynylbenzoic acid as a white solid (1.04 g, 94 %). NMR ¹H (ppm, CDCl₃): 8.27 (t, *J⁴* = 1.4 Hz, 1H), 8.06 (dt, *J³* = 7.8 Hz, J⁴ = 1.3 Hz, 1H), 7.75 (dt, *J³* = 7.8 Hz, *J⁴* = 1.3 Hz, 1H), 7.56-7.53 (m, 2H), 7.47 (t, *J³* = 7.8 Hz, 1H), 7.37-7.34 (m, 3H).

### 117D: N-n-butyl-3-phenylethynyl-benzamide

Using preparation method 1, compound from example 117C (222 mg, 1 mmol) was treated with *n*-butylamine (110 µL, 1.1 mmol). Purification by flash chromatography with SiO₂ using CH₂Cl₂/EtOAc 97:3 gave N-*n*-butyl-3-phenylethynyl-benzamide as a white flaky solid (230 mg, 83 %). NMR ¹H (ppm, CDCl₃): 7.87 (s, 1H), 7.73 (d, *J³* = 7.8 Hz, 1H), 7.62 (d, *J³* = 7.6 Hz, 1H), 7.54-7.50 (m, 2H), 7.40 (t, *J³* = 7.8 Hz, 1H), 7.35-7.33 (m, 3H), 6.09 (br. s, 1H), 3.49-3.42 (m, 2H), 1.65-1.51 (m, 2H), 1.41 (sext., *J³* = 7.2 Hz, 2H), 0.95 (t, *J³* = 7.3 Hz, 3H).

### 117E: (S)-isobutyl-(L)-cysteine hydrochloride

Compound from example 78A was stirred in HCl 4N in 1,4-dioxane for 24 hours. The solvent was removed to obtain (S)-isobutyl-(L)-cysteine hydrochloride as a white solid. Quantitative yield.

### 117F: compound (130)

Using preparation method 3, 117D (69 mg, 0.25 mmol) was reacted with phosgene to provide a carbamoylchloride. Using preparation method 4, (S)-isobutyl-(L)-cysteine hydrochloride (64 mg, 0.3 mmol) was protected. The carbamoylchloride and TMS protected amino acid were reacted using Preparation Method 5. Purification by flash chromatography using SiO₂ with CH₂Cl₂ 100% then CH₂Cl₂/MeOH 99.5:0.5 then CH₂Cl₂/MeOH/AcOH 99:0.5:0.5 gave compound (130) as a pale yellow oil (22 mg, 18 %). NMR ¹H (ppm, CDCl₃): 9.61 (d, *J³* = 6.8 Hz, 1H), 8.76 (br. s, 1H), 7.61-7.59 (m, 2H), 7.52-7.50 (m, 2H), 7.45-7.34 (m, 5H), 4.77-4.74 (m, 1H), 3.72-3.67 (m, 2H), 3.10-2.98 (m, 2H), 2.47 (d, *J³* = 6.8 Hz, 2H), 1.84-1.74 (m, 1H), 1.50 (quin., *J³* = 6.6 Hz, 2H), 1.14 (sext., *J³* = 7.3 Hz, 2H), 0.97 (d, *J³* = 6.5 Hz, 6H), 0.75 (t, *J³* = 7.3 Hz, 3H). MS (+ESI): M+H⁺: 481.7.

### Example 118: compound (131)

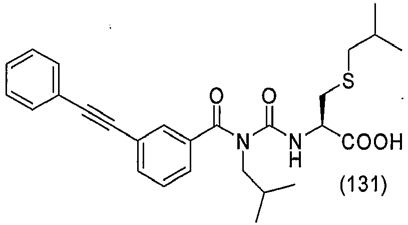

### 118A: N-isobutyl-3-phenylethynyl-benzamide

Using preparation method 1, compound from example 117C (222 mg, 1 mmol) was treated with isobutylamine (110 µL, 1.1 mmol). Purification by flash chromatography with SiO₂ using CH₂Cl₂/EtOAc 97:3 gave N-isobutyl-3-phenylethynyl-benzamide as a white flaky solid (192 mg, 69 %). NMR ¹H (ppm, CDCl₃); 7.87 (s, 1H), 7.73 (d, *J³* = 7.7 Hz, 1H), 7.63 (d, *J³* = 7.7 Hz, 1H), 7.53-7.51 (m, 2H), 7.41 (t, *J³* = 7.6 Hz, 1H), 7.35-7.33 (m, 3H), 6.16 (br. s, 1H), 3.30-3.26 (m, 2H), 1.94-1.83 (m, 1H), 0.98 (d, *J³* = 6.7 Hz, 6H).

### 118B: compound (131)

Using preparation method 3, 118A (69 mg, 0.25 mmol) was reacted with phosgene to provide a carbamoylchloride. Using preparation method 4, (S)-isobutyl-(L)-cysteine hydrochloride (64 mg, 0.3 mmol) was protected. The carbamoylchloride and TMS protected amino acid were reacted using Preparation Method 5. Purification by flash chromatography using SiO₂ with CH₂Cl₂ 100% then CH₂Cl₂/MeOH 99.5:0.5 then CH₂Cl₂/MeOH/AcOH 99:0.5:0.5 gave compound (131) as a pale yellow oil (21 mg, 17 %). NMR ¹H (ppm, CDCl₃): 9.50 (d, *J³* = 6.8 Hz, 1H), 7.72-7.70 (m, 2H), 7.53-7.50 (m, 2H), 7.42-7.34 (m, 5H), 4.75-4.72 (m, 1H), 3.64 (d, *J³* = 7.2 Hz, 2H), 3.10-2.98 (m, 2H), 2.46 (d, *J³* = 6.7 Hz, 2H), 1.87-1.74 (m, 2H), 0.96 (d, *J³* = 6.6 Hz, 6H), 0.75 (t, *J³* = 6.6 Hz, 6H). MS (+ESI): M+H⁺: 481.6.

### Example 119: compound (132)

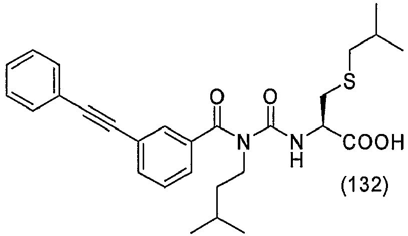

### 119A: N-isopentyl-3-phenylethynyl-benzamide

Using preparation method 1, compound from example 117C (222 mg, 1 mmol) was treated with isopentylamine (130 µL, 1.1 mmol). Purification by flash chromatography with SiO₂ using CH₂Cl₂/EtOAc 97:3 gave N-isopentyl-3-phenylethynyl-benzamide as a white flaky solid (184 mg, 63 %). NMR ¹H (ppm, CDCl₃): 7.86 (s, 1H), 7.73 (dt, *J³* = 7.7 Hz, *J⁴* = 1.3 Hz, 1H), 7.62 (dt, *J³* = 7.7 Hz, *J⁴* = 1.3 Hz, 1H), 7.54-7.51 (m, 2H), 7.40 (t, *J³* = 7.6 Hz, 1H), 7.35-7.32 (m, 3H), 6.06 (br. s, 1H), 3.51-3.44 (m, 2H), 1.73-1.64 (m, 1H), 1.51 (quart., *J³* = 7.3 Hz, 2H), 0.95 (d, *J³* = 6.7 Hz, 6H).

### 119B: compound (132)

Using preparation method 3, 119A (73 mg, 0.25 mmol) was reacted with phosgene to provide a carbamoylchloride. Using preparation method 4, (S)-isobutyl-(L)-cysteine hydrochloride (64 mg, 0.3 mmol) was protected. The carbamoylchloride and TMS protected amino acid were reacted using Preparation Method 5. Purification by flash chromatography using SiO₂ with CH₂Cl₂ 100% then CH₂Cl₂/MeOH 99.5:0.5 then CH₂Cl₂/MeOH/AcOH 99:0.5:0.5 gave compound (132) as a pale yellow oil (18 mg, 15 %). NMR ¹H (ppm, CDCl₃): 9.64 (d, *J³* = 6.8 Hz, 1H), 7.61-7.59 (m, 2H), 7.53-7.50 (m, 2H), 7.45-7.33 (m, 5H), 4.78-4.72 (m, 1H), 3.73-3.67 (m, 2H), 3.10-2.88 (m, 2H), 2.47 (d, *J³* = 6.7 Hz, 2H), 1.84-1.71 (m, 1H), 1.51-1.38 (m, 3H), 0.97 (d, *J³* = 6.5 Hz, 6H), 0.75 (t, *J³* = 6.6 Hz, 6H). MS (+ESI): M+H⁺: 495.6.

### Example 120: compound (133)

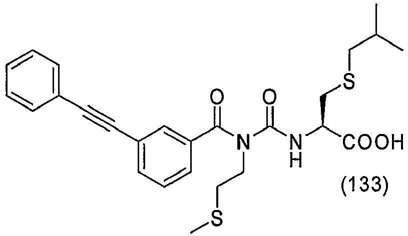

### 120A: N-(2-methylthio)ethyl-3-phenylethynyl-benzamide

Using preparation method 1, compound from example 117C (222 mg, 1 mmol) was treated with (2-methylthio)ethylamine (100 µL, 1.1 mmol). Purification by flash chromatography with SiO₂ using CH₂Cl₂/EtOAc 97:3 gave N-(2-methylthio)ethyl-3-phenylethynyl-benzamide as a white flaky solid (187 mg, 63 %). NMR ¹H (ppm, CDCl₃): 7.91 (t, *J⁴* = 1.5 Hz, 1H), 7.75 (dt, *J³* = 7.8 Hz, *J⁴* = 1.5 Hz, 1H), 7.64 (dt, *J³* = 7.8 Hz, *J⁴* = 1.3 Hz, 1H), 7.54-7.51 (m, 2H), 7.42 (t, *J³* = 7.8 Hz, 1H), 7.36-7.33 (m, 3H), 6.59 (br. s, 1H), 3.70-3.64 (m, 2H), 2.76 (t, *J³* = 6.3 Hz, 2H), 2.14 (s, 3H).

### 120B: compound (133)

Using preparation method 3, 120A (73 mg, 0.25 mmol) was reacted with phosgene to provide a carbamoylchloride. Using preparation method 4, (S)-isobutyl-(L)-cysteine hydrochloride (64 mg, 0.3 mmol) was protected. The carbamoylchloride and TMS protected amino acid were reacted using Preparation Method 5. Purification by flash chromatography using SiO₂ with CH₂Cl₂ 100 % then CH₂Cl₂/MeOH 99.5:0.5 then CH₂Cl₂/MeOH/AcOH 99:0.5:0.5 gave compound (133) as a pale yellow oil (22 mg, 18 %). NMR ¹H (ppm, CDCl₃): 9.54 (d, *J³* = 6.9 Hz, 1H), 8.41 (br. s, 1H), 7.63-7.61 (m, 2H), 7.53-7.50 (m, 2H), 7.44-7.34 (m, 5H), 4.78-4.72 (m, 1H), 3.95 (t, *J³* = 6.7 Hz, 2H), 3.10-2.90 (m, 2H), 2.63 (t, *J³* = 6.7 Hz, 2H), 2.47 (d, *J³* = 6.7 Hz, 2H), 1.84-1.72 (m, 4H), 0.97 (d, *J³* = 6.5 Hz, 6H). MS (+ESI): M+H⁺: 499.5.

### Example 121: compound (134)

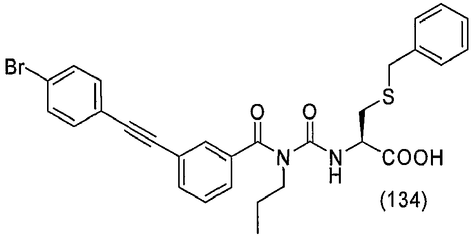

### 121A:

Using preparation method 3, N-*n*-propyl-3-iodobenzamide (1.156 g, 4 mmol) was reacted with phosgene to provide a carbamoylchloride. Using preparation method 4, (S)-benzyl-(L)-cysteine hydrochloride was protected. The carbamoyl chloride and TMS protected amino acid were reacted using preparation method 5. Purification by flash chromatography using SiO₂ with CH₂Cl₂/MeOH 95:5 gave the compound as a pale amber oil (1.92 mg, 91 %). NMR ¹H (ppm, CDCl₃): 9.52 (d, *J³* = 7.0 Hz, 1H), 8.73 (br. s, 1H), 7.82-7.79 (m, 2H), 7.40 (d*, J³* = 7.7 Hz, 1H), 7.32-7.21 (m, 5H), 7.17(t, *J³* = 7.9 Hz, 1H), 4.76-4.70 (m, 1H), 3.76 (s, 2H), 3.64-3.59 (m, 2H), 3.01-2.85 (m, 2H), 1.53 (sext., *J³* = 7.5 Hz, 2H), 0.73 (t, *J³* = 7.4 Hz, 3H). MS (+ESI): M+H⁺: 527.3.

### 121B: compound (134)

Using preparation method 10, 121A (100 mg, 0.19 mmol) was reacted with 4-bromophenylacetylene (54 mg, 0.3 mmol). Purification by passing through a SAX Acetate solid phase extraction column with MeOH 100 % then MeOH/AcOH 85:15 gave compound (134) as an amber oil (74 mg, 67 %). NMR ¹H (ppm, CDCl₃): 9.60 (d, *J³* = 6.9 Hz, 1H), 8.72 (br. s, 1H), 7.60-7.22 (m, 13H), 4.77-4.71 (m, 1H), 3.78 (s, 2H), 3.69-3.64 (m, 2H), 3.02-2.86 (m, 2H), 1.54 (sext., *J³* = 7.4 Hz, 2H), 0.73 (t, *J³* = 7.4 Hz, 3H). MS (+ESI): M+H⁺: 581.5.

### Example 122: compound (135)

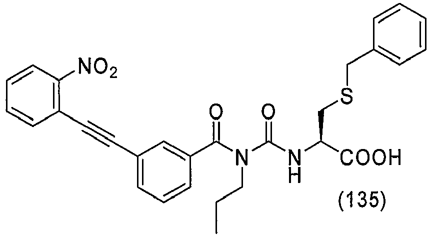

Using preparation method 10, 121A (100 mg, 0.19 mmol) was reacted with 2-nitrophenylacetylene (44 mg, 0.3 mmol). Purification by passing through a SAX Acetate solid phase extraction column with MeOH 100 % then MeOH/AcOH 85:15 gave compound (135) as a yellow oil (68 mg, 66 %). NMR ¹H (ppm, CDCl₃): 9.65-9.56 (m, 1H), 9.25 (br. s, 1H), 7.72-7.22 (m, 13H), 4.76-4.71 (m, 1H), 3.78 (s, 2H), 3.71-3.64 (m, 2H), 2.98-2.86 (m, 2H), 1.63-1.51 (m, 2H), 0.74 (t, *J³* = 7.3 Hz, 3H). MS (+ESI): M+H⁺: 546.6.

### Example 123: compound (136)

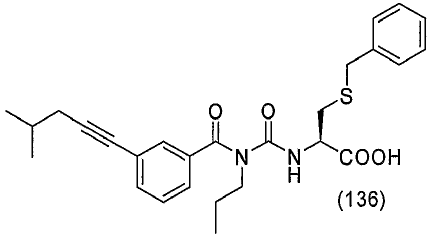

Using preparation method 10, 121A (100 mg, 0.19 mmol) was reacted with 4-methylpent-1-yne (35 µL, 25 mg, 0.3 mmol). Purification by passing through a SAX Acetate solid phase extraction column with MeOH 100 % then MeOH/AcOH 85:15 gave compound (136) as a colourless oil (65 mg, 71 %). NMR ¹H (ppm, CDCl₃): 9.63 (d, *J³* = 6.9 Hz, 1H), 8.92 (br. s, 1H), 7.49-7.46 (m, 2H), 7.38-7.21 (m, 7H), 4.76-4.70 (m, 1H), 3.77 (s, 2H), 3.66-3.61 (m, 2H), 3.01-2.85 (m, 2H), 2.29 (d, *J³* = 6.5 Hz, 2H), 1.96-1.85 (m, 1H), 1.52 (sext., *J³* = 7.4 Hz, 2H), 1.03 (d, *J³* = 6.6 Hz, 6H), 0.72 (t, *J³* = 7.4 Hz, 3H). MS (+ESI): M+H⁺: 481.8.

### Example 124: compound (137)

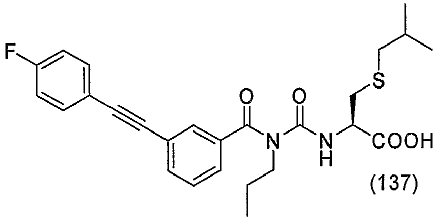

### 124A:

Using preparation method 3, N-*n*-propyl-3-iodobenzamide (820 mg, 2.84 mmol) was reacted with phosgene to provide a carbamoylchloride. Using preparation method 4, (S)-isobutyl-(L)-cysteine hydrochloride was protected. The carbamoyl chloride and TMS protected amino acid were reacted using preparation method 5. Purification by flash chromatography using SiO₂ with CH₂Cl₂/MeOH 95:5 gave the compound as a pale amber oil (1.190 g, 85 %). NMR ¹H (ppm, CDCl₃): 9.53 (d, *J³* = 6.3 Hz, 1H), 7.85-7.79 (m, 2H), 7.41 (d, *J³* = 7.5 Hz, 1H), 7.18 (t, *J³* = 7.7 Hz, 1H), 4.75-4.69 (m, 1H), 3.60-3.55 (m, 2H), 3.06-2.96 (m, 2H), 2.47 (d, *J³* = 6.7 Hz, 2H), 1.84-1.75 (m, 1H), 1.54 (sext., *J³* = 7.6 Hz, 2H), 0.97 (d, *J³* = 6.6 Hz, 6H), 0.74 (t, *J³* = 7.5 Hz, 3H). MS (+ESI): M+H⁺: 493.1.

### 124B:

Using preparation method 10, 124A (49 mg, 0.1 mmol) was reacted with 4-fluorophenylacetylene (18 mg, 0.15 mmol). Purification by passing through a SAX Acetate solid phase extraction column with MeOH 100 % then MeOH/AcOH 85:15 gave compound (137) as a golden oil (15 mg, 31 %). NMR ¹H (ppm, CDCl₃): 9.62 (d, *J³* = 6.9 Hz, 1H), 7.64-7.32 (m, 8H), 4.77-4.71 (m, 1H), 3.70-3.65 (m, 2H), 3.12-2.99 (m, 2H), 2.48 (d, *J³* = 6.9 Hz, 2H), 1.87-1.73 (m, 1H), 1.54 (sext., *J³* = 7.4 Hz, 2H), 0.97 (d*, J³* = 6.6 Hz, 6H), 0.74 (t, *J³* = 7.4 Hz, 3H). MS (+ESI): M+H⁺: 485.3.

### Example 125: compound (138)

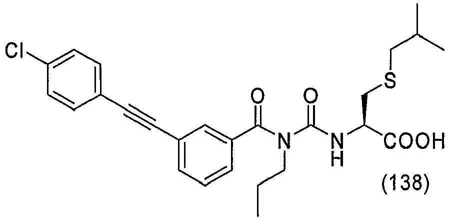

Using preparation method 10, 124A (49 mg, 0.1 mmol) was reacted with 4-chlorophenylacetylene (21 mg, 0.15 mmol). Purification by passing through a SAX Acetate solid phase extraction column with MeOH 100 % then MeOH/AcOH 85:15 gave compound (138) as a golden solid (31 mg, 62 %). NMR ¹H (ppm, CDCl₃): 9.59 (d, *J³* = 6.9 Hz, 1H), 7.61-7.30 (m, 8H), 4.78-4.71 (m, 1H), 3.69-3.64 (m, 2H), 3.12-2.98 (m, 2H), 2.47 (d, *J³* = 6.9 Hz, 2H), 1.84-1.75 (m, 1H), 1.54 (sext*., J³* = 7.4 Hz, 2H), 0.96 (d*, J³* = 6.6 Hz, 6H), 0.73 (t, *J³* = 7.3 Hz, 3H). MS (+ESI): M+H⁺: 501.3.

### Example 126: compound (139)

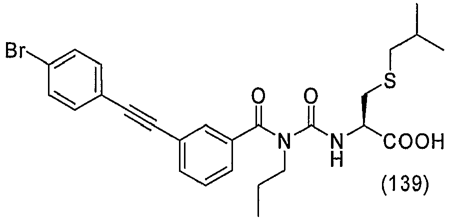

Using preparation method 10, 124A (49 mg, 0.1 mmol) was reacted with 4-bromophenylacetylene (27 mg, 0.15 mmol). Purification by passing through a SAX Acetate solid phase extraction column with MeOH 100 % then MeOH/AcOH 85:15 gave compound (139) as a golden solid (20 mg, 37 %). NMR ¹H (ppm, CDCl₃): 9.59 (d, *J³* = 6.9 Hz, 1H), 7.62-7.59 (m, 2H), 7.49-7.36 (m, 6H), 4.78-4.72 (m, 1H), 3.69-3.64 (m, 2H), 3.12-2.99 (m, 2H), 2.47 (d, *J³* = 6.9 Hz, 2H), 1.87-1.73 (m, 1H), 1.54 (sext., *J³* = 7.5 Hz, 2H), 0.97 (d, *J³* = 6.6 Hz, 6H), 0.73 (t, *J³* = 7.4 Hz, 3H). MS (+ESI): M+H⁺: 547.2.

### Example 127: compound (140)

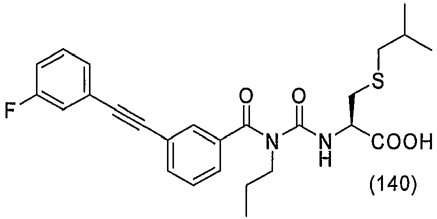

Using preparation method 10, 124A (49 mg, 0.1 mmol) was reacted with 3-fluorophenylacetylene (18 mg, 0.15 mmol). Purification by passing through a SAX Acetate solid phase extraction column with MeOH 100 % then MeOH/AcOH 85:15 gave compound (140) as a golden solid (37 mg, 76 %). NMR ¹H (ppm, CDCl₃): 9.60-9.51 (m, 1H), 8.05-7.04 (m, 8H), 4.78-4.71 (m, 1H), 3.70-3.60 (m, 2H), 3.09-2.99 (m, 2H), 2.48-2.43 (m, 2H), 1.81-1.75 (m, 1H), 1.54 (sext., *J³* = 7.0 Hz, 2H), 0.97 (d, *J³* = 6.5 Hz, 6H), 0.74 (t, *J³* = 7.4 Hz, 3H). MS (+ESI): M+H⁺: 485.1.

### Example 128: compound (141)

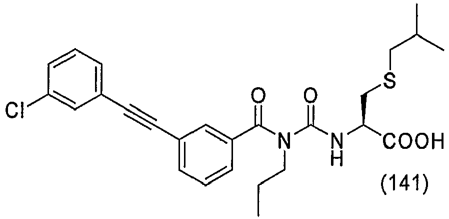

Using preparation method 10, 124A (49 mg, 0.1 mmol) was reacted with 3-chlorophenylacetylene (18 mg, 0.15 mmol). Purification by passing through a SAX Acetate solid phase extraction column with MeOH 100 % then MeOH/AcOH 85:15 gave compound (141) as a golden solid (20 mg, 40 %). NMR ¹H (ppm, CDCl₃): 9.58 (d*, J³* = 6.9 Hz, 1H), 8.54 (br. s, 1H), 7.62-7.59 (m, 2H), 7.51-7.23 (m, 6H), 4.79-4.73 (m, 1H), 3.70-3.64 (m, 2H), 3.12-2.99 (m, 2H), 2.47 (d, *J³* = 6.6 Hz, 2H), 1.86-1.73 (m, 1H), 1.54 (sext., *J³* = 7.5 Hz, 2H), 0.97 (d, *J³* = 6.6 Hz, 6H), 0.74 (t, *J³* = 7.4 Hz, 3H). MS (+ESI): M+H⁺: 501.2.

### Example 129: compound (142)

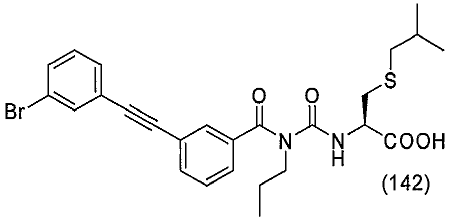

Using preparation method 10, 124A (49 mg, 0.1 mmol) was reacted with 3-bromophenylacetylene (27 mg, 0.15 mmol). Purification by passing through a SAX Acetate solid phase extraction column with MeOH 100 % then MeOH/AcOH 85:15 gave compound (142) as an orange solid (32 mg, 59 %). NMR ¹H (ppm, CDCl₃): 9.58 (d, *J³* = 6.9 Hz, 1H), 8.69 (br. s, 1H), 7.67-7.59 (m, 3H), 7.48-7.41 (m, 4H), 7.21 (t, *J³* = 7.8 Hz, 1H), 4.79-4.73 (m, 1H), 3.70-3.64 (m, 2H), 3.12-2.99 (m, 2H), 2.47 (d, *J³* = 6.6 Hz, 2H), 1.86-1.73 (m, 1H), 1.54 (sext., *J³* = 7.5 Hz, 2H), 0.97 (d, *J³* = 6.6 Hz, 6H), 0.74 (t, *J³* = 7.4 Hz, 3H). MS (+ESI): M+H⁺: 547.2.

### Example 130: compound (143)

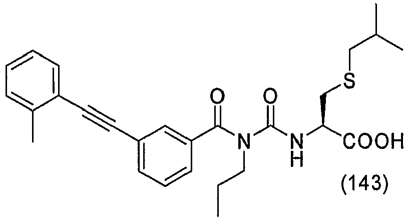

Using preparation method 10, 124A (49 mg, 0.1 mmol) was reacted with 2-ethynyltoluene (17.4 mg, 0.15 mmol). Purification by passing through a SAX Acetate solid phase extraction column with MeOH 100 % then MeOH/AcOH 85:15 gave compound (143) as an off-white solid (20 mg, 42 %). NMR ¹H (ppm, CDCl₃): 9.63 (d, *J³* = 6.8 Hz, 1H), 8.40 (br. s, 1H), 7.63-7.60 (m, 2H), 7.49-7.37 (m, 3H), 7.27-7.13 (m, 3H), 4.79-4.73 (m, 1H), 3.70-3.65 (m, 2H), 3.13-2.99 (m, 2H), 2.51-2.47 (m, 5H), 1.84-1.74 (m, 1H), 1.55 (sext., *J³* = 7.4 Hz, 2H), 0.97 (d, *J³* = 6.6 Hz, 6H), 0.74 (t, *J³* = 7.4 Hz, 3H). MS (+ESI): M+H⁺: 481.3.

### Example 131: compound (144)

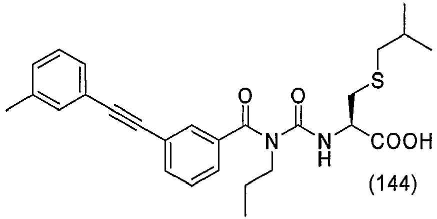

Using preparation method 10, 124A (49 mg, 0.1 mmol) was reacted with 3-ethynyltoluene (17.4 mg, 0.15 mmol). Purification by passing through a SAX Acetate solid phase extraction column with MeOH 100 % then MeOH/AcOH 85:15 gave compound (144) as an amber solid (29 mg, 60 %). NMR ¹H (ppm, CDCl₃): 9.61 (d*, J³* = 6.9 Hz, 1H), 8.41 (br. s, 1H), 7.61-7.59 (m, 2H), 7.45-7.31 (m, 4H), 7.26-7.14 (m, 2H), 4.79-4.73 (m, 1H), 3.70-3.65 (m, 2H), 3.12-2.99 (m, 2H), 2.47 (d, *J³* = 7.0 Hz, 2H), 2.34 (s, 3H), 1.84-1.75 (m, 1H), 1.54 (sext., *J³* = 7.4 Hz, 2H), 0.97 (d, *J³* = 6.6 Hz, 6H), 0.74 (t, *J³* = 7.4 Hz, 3H). MS (+ESI): M+H⁺: 493.1.

### Example 132: compound (145)

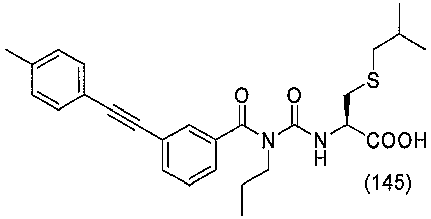

Using preparation method 10, 124A (49 mg, 0.1 mmol) was reacted with 4-ethynyltoluene (17.4 mg, 0.15 mmol). Purification by passing through a SAX Acetate solid phase extraction column with MeOH 100 % then MeOH/AcOH 85:15 gave compound (145) as an amber solid (12 mg, 25 %). NMR ¹H (ppm, CDCl₃): 9.62 (d, *J³* = 5.9 Hz, 1H), 7.61-7.10 (m, 8H), 5.85 (br. s, 1H), 4.77-4.71 (m, 1H), 3.70-3.65 (m, 2H), 2.36-2.34 (m, 5H), 1.82-1.77 (m, 1H), 1.54 (sext., *J³* = 7.4 Hz, 2H), 0.97 (d, *J³* = 6.5 Hz, 6H), 0.73 (t, *J³* = 7.4 Hz, 3H). MS (+ESI): M+H⁺: 481.3.

### Example 133: compound (146)

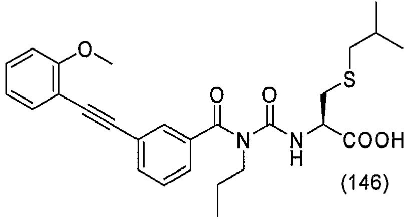

Using preparation method 10, 124A (49 mg, 0.1 mmol) was reacted with 2-methoxyphenylacetylene (20 mg, 0.15 mmol). Purification by passing through a SAX Acetate solid phase extraction column with MeOH 100 % then MeOH/AcOH 85:15 gave compound (146) as an amber oil (7 mg, 14 %). NMR ¹H (ppm, CDCl₃): 9.56 (d, *J³* = 5.4 Hz, 1H), 7.65-6.89 (m, 8H), 4.76-4.70 (m, 1H), 4.56 (br. s, 1H), 3.91-3.88 (m, 5H), 3.71-3.66 (m, 2H), 1.81-1.72 (m, 1H), 1.54 (sext., *J³* = 7.4 Hz, 2H), 0.97 (d, *J³* = 6.5 Hz, 6H), 0.74 (t, *J³* = 7.4 Hz, 3H). MS (+ESI): M+H⁺: 497.3.

### Example 134: compound (147)

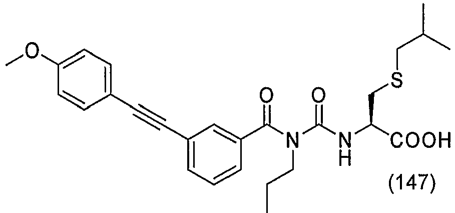

Using preparation method 10, 124A (49 mg, 0.1 mmol) was reacted with 4-methoxyphenylacetylene (20 mg, 0.15 mmol). Purification by passing through a SAX Acetate solid phase extraction column with MeOH 100 % then MeOH/AcOH 85:15 gave compound (147) as an amber solid (5 mg, 10 %). NMR ¹H (ppm, CDCl₃): 9.62 (d, *J³* = 6.7 Hz, 1H), 7.65 (d*, J³* = 6.7 Hz, 2H), 7.47-7.35 (m, 4H), 6.87 (d*, J³* = 8.7 Hz, 2H), 5.90 (br. s, 1H), 4.78-4.72 (m, 1H), 3.82 (s, 3H), 3.70-3.65 (m, 2H), 3.13-3.00 (m, 2H), 2.48 (d, *J³* = 6.6 Hz, 2H), 1.85-1.76 (m, 1H), 1.54 (sext., *J³* = 7.3 Hz, 2H), 0.97 (d, *J³* = 6.6 Hz, 6H), 0.74 (t, *J³* = 7.4 Hz, 3H). MS (+ESI): M+H⁺: 497.3.

### Example 135: compound (148)

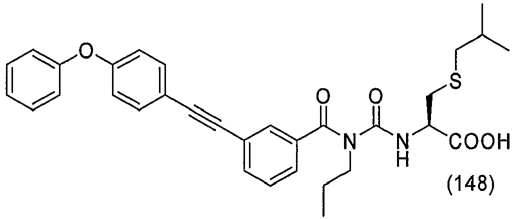

Using preparation method 10, 124A (49 mg, 0.1 mmol) was reacted with 4-phenoxyphenylacetylene (29.1 mg, 0.15 mmol). Purification by passing through a SAX Acetate solid phase extraction column with MeOH 100 % then MeOH/AcOH 85:15 gave compound (148) as an amber oil (33 mg, 59 %). NMR ¹H (ppm, CDCl₃): 9.61 (d, *J³* = 6.8 Hz, 1H), 8.37 (br. s, 1H), 7.60-7.33 (m, 8H), 7.14 (t, *J³* = 8.1 Hz, 1H), 7.04-6.90 (m, 4H), 4.79-4.73 (m, 1H), 3.70-3.65 (m, 2H), 3.12-2.99 (m, 2H), 2.47 (d, *J³* = 6.9 Hz, 2H), 1.84-1.75 (m, 1H), 1.54 (sext., *J³* = 7.3 Hz, 2H), 0.97 (d, *J³* = 6.6 Hz, 6H), 0.73 (t, *J³* = 7.3 Hz, 3H). MS (+ESI): M+H⁺: 559.4.

### Example 136: compound (149)

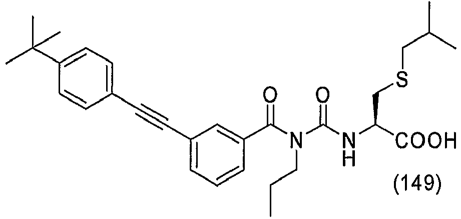

Using preparation method 10, 124A (49 mg, 0.1 mmol) was reacted with 4*-tert-*butylphenylacetylene (25 mg, 0.15 mmol). Purification by passing through a SAX Acetate solid phase extraction column with MeOH 100 % then MeOH/AcOH 85:15 gave compound (149) as a colourless oil (3 mg, 6 %). NMR, ¹H (ppm, CDCl₃): 9.62 (d*, J³* = 6.8 Hz, 1H), 7.62-7.35 (m, 8H), 4.75-4.71 (m, 1H), 3.70-3.65 (m, 2H), 3.12-2.99 (m, 2H), 2.48 (d, *J³* = 6.7 Hz, 2H), 1.84-1.75 (m, 1H), 1.54 (sext., *J³* = 7.3 Hz, 2H), 1.31 (s, 9H), 0.97 (d, *J³* = 6.4 Hz, 6H), 0.74 (t, *J³* = 7.2 Hz, 3H). MS (+ESI): M+H⁺: 523.7.

### Example 137: compound (150)

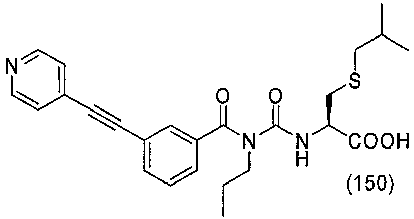

Using preparation method 10, 124A (49 mg, 0.1 mmol) was reacted with 4-ethynylpyridine hydrochloride (21 mg, 0.15 mmol). Purification by passing through a SAX Acetate solid phase extraction column with MeOH 100 % then MeOH/AcOH 85:15 gave compound (150) as a red oil (10 mg, 21 %). NMR ¹H (ppm, CDCl₃): 9.50-9.35 (m, 1H), 7.81-7.14 (m, 8H), 4.76-4.70 (m, 1H), 3.70-3.65 (m, 2H), 3.12-2.99 (m, 2H), 2.46 (d, J³ = 6.9 Hz, 2H), 1.82-1.73 (m, 1H), 1.54 (sext., J³ = 7.2 Hz, 2H), 0.95 (d, J³ = 6.6 Hz, 6H), 0.74 (m, 3H). MS (+ESI): M+H⁺: 468.2.

### Example 138: compound (151)

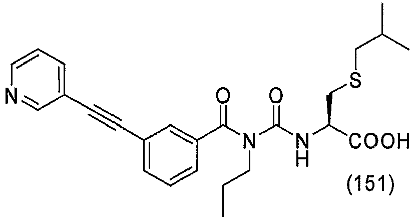

Using preparation method 10, 124A (49 mg, 0.1 mmol) was reacted with 3-ethynylpyridine (15 mg, 0.15 mmol). Purification by passing through a SAX Acetate solid phase extraction column with MeOH 100 % then MeOH/AcOH 85:15 gave compound (151) as an amber oil (32 mg, 69 %). NMR ¹H (ppm, CDCl₃): 9.34 (d, *J³* = 6.8 Hz, 1H), 8.06 (d, *J³* = 7.6 Hz, 1H), 7.65-7.43 (m, 7H), 4.78-4.72 (m, 1H), 3.69-3.64 (m, 2H), 3.12-2.99 (m, 2H), 2.46 (d, *J³* = 6.6 Hz, 2H), 1.85-1.72 (m, 1H), 1.55 (sext., *J³* = 7.3 Hz, 2H), 0.95 (d, *J³* = 6.5 Hz, 6H), 0.75 (t, *J³* = 7.3 Hz, 3H). MS (+ESI): M+H⁺: 468.3.

### Example 139: compound (152)

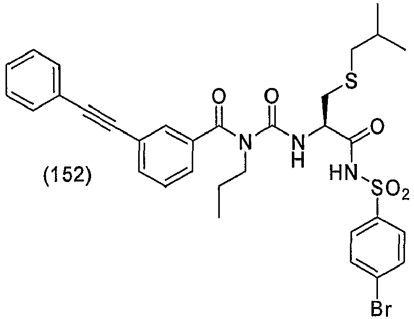

Compound from example 78B (47 mg, 0.1 mmol) was reacted with 4-bromobenzenesulfonamide (24 mg, 0.1 mmol) as described in preparation method 11, to obtain compound (152) as a colourless oil (51 mg, 75 %). NMR ¹H (ppm, CDCl₃): 9.83 (s, 1H), 9.59 (d, *J³* = 6.2 Hz, 1H), 8.14 (d, *J³* = 8.3 Hz, 2H), 7.72 (d, *J³* = 8.3 Hz, 2H), 7.60-7.34 (m, 9H), 4.55-4.52 (m, 1H), 3.71-3.65 (m, 2H), 2.99-2.87 (m, 2H), 2.39 (d, *J³* = 6.7 Hz, 2H), 1.78-1.70 (m, 1H), 1.52 (sext., *J³* = 7.5 Hz, 2H), 0.93 (d, *J³* = 6.6 Hz, 6H), 0.74 (t, *J³* = 7.4 Hz, 3H). MS (+ESI): M+H⁺: 686.3.

### Example 140: compound (153)

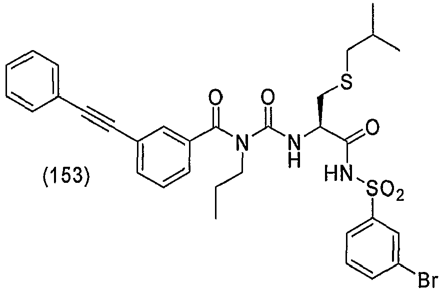

Compound from example 78B (47 mg, 0.1 mmol) was reacted with 3-bromobenzenesulfonamide (24 mg, 0.1 mmol) as described in preparation method 11, to obtain compound (153) as a colourless oil (61 mg, 89 %). NMR ¹H (ppm, CDCl₃): 9.90 (s, 1H), 9.58 (d, *J³* = 6.5 Hz, 1H), 8.18 (s, 1H), 8.03 (d, *J³* = 7.6 Hz, 1H), 7.85 (d, *J³* = 7.6 Hz, 1H), 7.72-7.62 (m, 2H), 7.53-7.50 (m, 2H), 7.43-7.33 (m, 6H), 4.53-4.47 (m, 1H), 3.70-3.65 (m, 2H), 2.97-2.82 (m, 2H), 2.38 (d, *J³* = 6.8 Hz, 2H), 1.78-1.70 (m, 1H), 1.52 (sext., *J³* = 7.5 Hz, 2H), 0.93 (d, *J³* = 6.6 Hz, 6H), 0.74 (t, *J³* = 7.4 Hz, 3H). MS (+ESI): M+H⁺: 686.5.

### Example 141: compound (154)

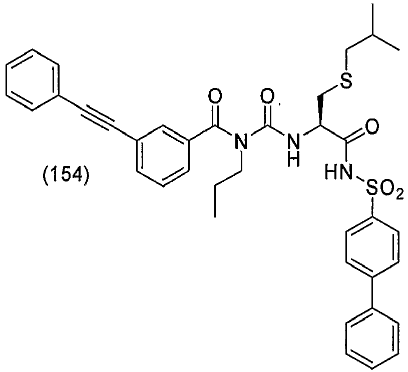

### 141A: 4-phenylbenzenesulfonamide

Using preparation method 2, 4-bromobenzenesulfonamide (472 mg, 2 mmol) was reacted with phenylboronic acid (268 mg, 2.2 mmol). Purification by washing with hot toluene followed by filtration gave 4-phenylbenzenesulfonamide as an off-white crystalline solid (264 mg, 55 %). NMR ¹H (ppm, CDCl₃): 7.87 (d, *J³* = 8.6 Hz, 2H), 7.83 (d, *J³* = 8.7 Hz, 2H), 7.70 (d*, J³* = 7.2 Hz, 2H), 7.48 (t, *J³* = 7.3 Hz), 7.40 (t, *J³* = 7.1 Hz, 1H).

### 141B: compound (154)

Compound from example 78B (47 mg, 0.1 mmol) was reacted with 4-phenylbenzenesulfonamide (23 mg, 0.1 mmol) as described in preparation method 11, to obtain compound (154) as a colourless oil (53 mg, 78 %). NMR ¹H (ppm, CDCl₃): 9.86 (s, 1H), 9.57 (d*, J³* = 6.3 Hz, 1H), 7.93 (d, *J³* = 8.6 Hz, 2H), 7.67-7.59 (m, 5H), 7.53-7.50 (m, 2H), 7.43 (t, *J³* = 7.6 Hz, 1H), 7.39-7.33 (m, 8H), 4.54-4.48 (m, 1H), 3.70-3.65 (m, 2H), 2.97-2.83 (m, 2H), 2.37 (d, *J³* = 6.8 Hz, 2H), 1.77-1.68 (m, 1H), 1.49 (sext., *J³* = 7.4 Hz, 2H), 0.92 (d*, J³* = 6.6 Hz, 6H), 0.73 (t, *J³* = 7.4 Hz, 3H). MS (+ESI): M+H⁺: 682.6.

### Example 142: compound (155)

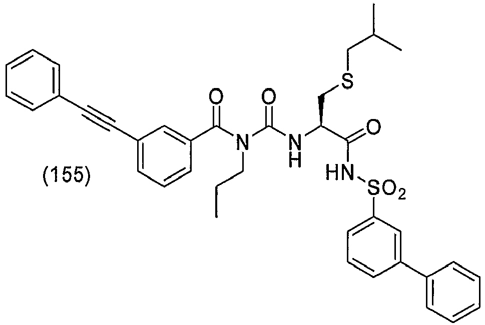

### 142A: 3-phenylbenzenesulfonamide

Using preparation method 2, 3-bromobenzenesulfonamide (236 mg, 1 mmol) was reacted with phenylboronic acid (134 mg, 1.1 mmol). Purification by flash chromatography on SiO₂ with CH₂Cl₂ gave 3-phenylbenzenesulfonamide as an off-white crystalline solid (155 mg, 66 %). NMR ¹H (ppm, d₆-DMSO): 8.11 (s, 1H), 7.84 (d, *J³* = 7.8 Hz, 1H), 7.70 (d, *J³* = 7.8 Hz, 1H), 7.56-7.53 (m, 2H), 7.48 (t, *J³* = 7.8 Hz, 1H), 7.43-7.34 (m, 3H), 5.10 (br. s, 2H).

### 142B: compound (155)

Compound from example 78B (47 mg, 0.1 mmol) was reacted with 3-phenylbenzenesulfonamide (23 mg, 0.1 mmol) as described in preparation method 11, to obtain compound (155) as a colourless oil (48 mg, 70 %). NMR ¹H (ppm, CDCl₃): 9.85 (s, 1H), 9.56 (d, *J³* = 6.5 Hz, 1H), 8.28 (s, 1H), 8.05 (d, *J³* = 7.9 Hz, 1H), 7.83 (d, *J³* = 7.9 Hz, 1H), 7.62-7.34 (m, 15H), 4.54-4.48 (m, 1H), 3.64-3.57 (m, 2H), 2.97-2.83 (m, 2H), 2.37 (d, *J³* = 6.8 Hz, 2H), 1.76-1.67 (m, 1H), 1.49 (sext., *J³* = 7.4 Hz, 2H), 0.90 (d, *J³* = 6.6 Hz, 6H), 0.69 (t, *J³* = 7.4 Hz, 3H). MS (+ESI): M+H⁺: 683.9.

### Example 143: compound (156)

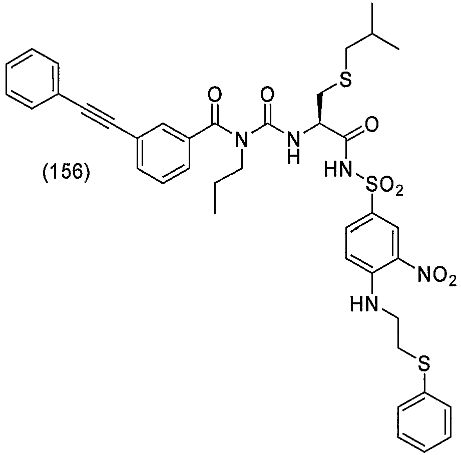

Compound from example 78B (47 mg, 0.1 mmol) was reacted with 3-nitro-4-(2-phenylthioethyl)aminobenzenesulfonamide (35 mg, 0.1 mmol) as described in preparation method 11, to obtain compound (156) as a yellow oil (68 mg, 85 %). NMR ¹H (ppm, CDCl₃): 9.89 (s, 1H), 9.58 (d, *J³* = 6.5 Hz, 1H), 8.79 (d, *J⁴* = 1.7 Hz, 1H), 8.65 (d, *J³* = 5.6 Hz, 1H), 8.02 (dd, *J³* = 9.2 Hz, J4 = 2.1 Hz, 1H), 7.61-7.24 (m, 14 H), 6.78 (d, *J³* = 9.2 Hz, 1H), 4.53-4.47 (m, 1H), 3.70-3.65 (m, 2H), 3.57-3.50 (m, 2H), 3.18 (t, *J³* = 6.6 Hz, 2H), 2.94-2.85 (m, 2H), 2.40 (d*, J³* = 6.8 Hz, 2H), 1.78-1.69 (m, 1H), 1.49 (sext., *J³* = 7.4 Hz, 2H), 0.92 (d*, J³* = 6.6 Hz, 6H), 0.72 (t, *J³* = 7.4 Hz, 3H). MS (+ESI): M+H⁺: 802.3.

### Constrained analogues

### Example 144: compound (157)

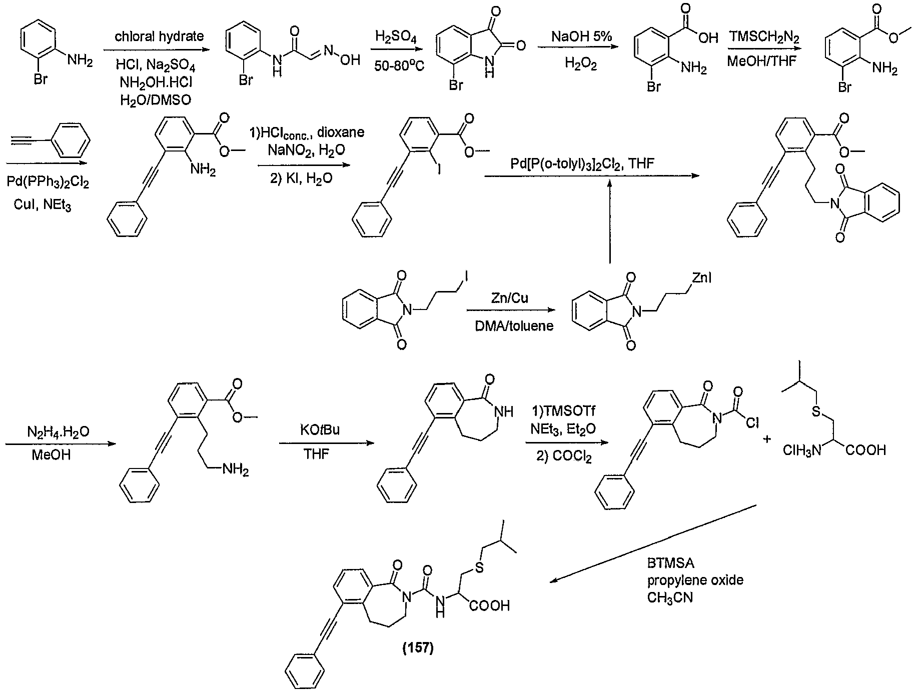

144A: *N*-(2-Bromo-phenyl)-2-hydroxyimino-acetamide. Chloral hydrate (11.6 g, 70 mmol) was added to a solution of sodium sulfate (18 g, 127 mmol) in 150 mL of water. HCl_{conc}. (6 mL) was added to a suspension of 2-bromo aniline (10 g, 58 mmol) in 50 mL of water. A small amount of DMSO was added until the solution had cleared up. This mixture was then added to the previous solution followed by a solution of hydroxylamine hydrochloride (15 g, 216 mmol) in 70 mL of water. The mixture was heated slowly to reflux (over 90 minutes). Refluxed was then maintained for 10 minutes. The reaction was then cooled down to room temperature and filtered. The light brown solids were washed thoroughly with water and dried *in vacuo.* 7.95 g of solids was obtained (56%). NMR ¹H (ppm, CDCl₃): 8.90 (br. s., 1H), 8.39 (d, J³ = 8.1 Hz, 1H), 7.93 (br. s., 1H), 7.54 (d, J³ = 8.1 Hz, 1H), 7.32 (t, J³ = 7.8 Hz, 1H), 6.99 (t, J³ = 7.5 Hz, 1H).

144B. 7-bromoisatin. *N-*(2-Bromo-phenyl)-2-hydroxyimino-acetamide from example 144A (7.8 g, 32 mmol) was added in small portions to 41 mL of sulfuric acid at 60°C so as to keep the reaction temperature under 80°C. After addition the temperature was raised to 80°C and the reaction was stirred at this temperature for 1 hr. The mixture was than cooled to room temperature and poured onto crushed ice. The red solids formed were isolated by filtration, rinsed thoroughly with water and dried *in vacuo.* 6.36 g of solid was obtained (88%). NMR ¹H (ppm, acetone-d⁶): 10.2 (br. s., 1H), 7.79 (d, J³ = 8.1 Hz, 1H), 7.54 (d, J³ = 7.3 Hz, 1H), 7.09 (t, J³ = 8.01 Hz, 1H).

144C. 2-Amino-3-bromo-benzoic acid. H₂O₂ (30%, 141 mL) was added dropwise to a mixture of 7-bromoisatin from example 144B (6.3g, 28.1 mmol) sodium hydroxide (5 % in water, 141 mL). After the addition was finished, the reaction was stirred at 50°C for 30 minutes. After that time 30 mL HCl 1N were added till pH 4. A white solid precipitated. It was collected by filtration and dried *in vacuo.* 2.66 g of solids were obtained (44%). NMR ¹H (ppm, DMSO-d⁶): 7.85 (m, 1H), 7.55 (m, 1H), 6.5 (m, 1H).

144D. Methyl 2-Amino-3-bromo-benzoate. Trimethylsylildiazomethane (2M solution in THF, 5.6 mL, 13.4 mmol) was added to a solution of 2-Amino-3-bromo-benzoic acid from example 144B (2.32 g, 11.1 mmol) in 1 mL of dry THF at 0°C. After 30 minutes at room temperature, the reaction mixture was concentrated and the crude residue was purified by flash chromatography on SiO₂ using Pet. Et./AcOEt 99:1 then 96:4 to afford a white solid (2.23 g, 86%). NMR ¹H (ppm, CDCl₃): 7.83 (d, J³ = 7.97 Hz, 1H), 7.55 (d, J³ = 7.7 Hz, 1H), 6.51 (t, J³ = 7.9 Hz, 1H), 3.87 (s, 3H). MS (+ ESI): M+H⁺ 230.0.

144E. Methyl 2-Amino-3-phenylethynyl-benzoate. Methyl 2-Amino-3-bromo-benzoate from example 144D (2.13 g, 9.26 mmol) and phenyl acetylene (4.33 mL, 37 mmol) were dissolved in 72 mL of triethylamine. CuI (109 mg, 0.6 mmol) and Pd(PPh₃)Cl₂ (224 mg, 0.32 mmol). The reaction was then stirred at 90°C for 20 hours. After this time, the reaction was concentrated. The residue was diluted with AcOEt and the organic layer was washed three times with HCl 12 %, then water and brine. It was dried over Na₂SO₄ and concentrated. The crude product was purified by flash chromatography on SiO₂ using Pet. Et./CH₂Cl₂ 90:10 to 70:30. 2.35g of yellow oil was obtained (quantitative yield). NMR ¹H (ppm, CDCl₃): 7.87 (dd, J³ = 8.03 Hz, J⁴ = 3.2 Hz, 1H), 7.56-7.49 (m, 3H), 7.39-7.31 (m, 3H), 6.61 (t, J³ = 7.9 Hz, 1H), 5.65 (br. s., 1H), 3.87 (s, 3H). MS (+ ESI): M+H⁺ 252.1.

144F. Methyl 2-iodo-3-phenylethynyl-benzoate. Methyl 2-Amino-3-phenylethynyl-benzoate from example 144E (2.35 g, 9.35 mmol) was treated with 18.4 mL of concentrated HCl. 1 mL of dioxane was added to dissolve the precipitate. NaNO₂ (715 mg, 10.4 mmol) in solution in 12 mL of water was added dropwise at 0°C. The reaction was stirred at 0°C for 1 hr. KI (16 g, 93.3 mmol) in solution in 13 mL of water was added and the reaction was then stirred at room temperature for 20 hours. After that time, the reaction mixture was concentrated. Dichloromethane and saturated NaHCO₃ were added. The aqueous layer was extracted 3 times with dichloromethane. The combined organic layers were washed with 10 % sodium thiosulfate (two times), water and brine and dried over Na₂SO₄. After concentration the residue was purified by flash chromatography on SiO₂ using Pet. Et./AcOEt 98:2 then 95:5. 920 mg of brown oil were obtained (27%). NMR ¹H (ppm, CDCl₃): 7.62-7.58 (m, 3H), 7.39-7.31 (m, 3H), 7.53 (dd, J³ = 7.7 Hz, J⁴ = 1.7 Hz, 1H), 7.37-7.32 (m, 4H), 3.94 (s, 3H).

144G. 2-[3-naphthylaminopropyl]-3-phenylethynyl-benzoic acid methyl ester. 1-iodo-3-naphtylaminopropane from example 144F (262 mg, 0.82 mmol) was placed in a flame dried schlenck flask and dissolved in a mixture of 4 mL of dry toluene and 0.5 mL of dry dimethylacetamide. 118 mg of Zn/Cu complex was then added and the mixture was placed in a sonicator bath. Sonication was applied for 2 hours after which TLC showed complete conversion of starting material. The reaction was left to decant and the supernatant was added to a solution of Methyl 2-iodo-3-phenylethynyl-benzoate (100 mg, 0.26 mmol) and Pd[P(o-tolyl)₃]Cl₂ (10.8 mg, 0.41 mmol) in 0.8 mL of dry THF. The reaction was stirred for 1 hour at room temperature. Saturated ammonium chloride was added and the reaction was diluted with AcOEt. HCl 2N was then added. The aqueous layer was extracted three times with AcOEt. The combined organic phases were washed with HCl 2N (two times), water and brine, then dried over Na₂SO₄ and concentrated. The residue was purified by flash chromatography on SiO₂ using Pet. Et./AcOEt 95:5 then 80:20. 92 mg of a white solid were obtained (80 %). NMR ¹H (ppm, CDCl₃): 7.78-7.75 (m, 3H), 7.66-7.60 (m, 3H), 7.49-7.46 (m, 2H), 7.31-7.29 (m, 3H), 7.21 (t, J³ = 7.9 Hz, 1H), 3.85 (t, J³ = 6.7 Hz, 1H), 3.82 (s, 3H), 3.26-3.21 (m, 2H), 2.14-2.04 (m, 2H).

144H. 2-[3-aminopropyl]-3-phenylethynyl-benzoic acid methyl ester. Hydrazine monohydrate (138 µL, 2.8 mmol) was added to a solution of 2-[3-naphthylaminopropyl]-3-phenylethynyl-benzoic acid methyl ester from example 144G (92 mg, 0.22 mmol) in 0.4 mL of methanol. The reaction was stirred at room temperature for 72 hours. HCl 6N was added till pH 1. The mixture was then concentrated to remove methanol and the solid residue was suspended in HCl 1N. It was filtered and the solid were rinsed with more HCl 1N. The acidic phase was washed three times with dichloromethane. Potassium carbonate was then added till pH 12 and the basic phase was extracted three times with dichloromethane. The combined organic layers were dried over Na₂SO₄ and concentrated. The white solid obtained was used in the next step without further purification.). MS (+ ESI): M+H⁺ 294.3.

144I. 6-Phenylethynyl-2,3,4,5-tetrahydro-benzo[c]azepin-1-one. 2-[3-aminopropyl]-3-phenylethynyl-benzoic acid methyl ester from example 144H (0.22 mmol) was dissolved in dry THF. Potassium terbutoxide (198 mg, 1.76 mmol) was added in one portion at 0°C. The ice bath was removed and the reaction was stirred at room temperature for 12 hours. Saturated ammonium chloride was added and the reaction was extracted three times with AcOEt. The combined organic layers were washed with water and brine, dried over Na₂SO₄ and concentrated. The residue was purified by preparative TLC using Pet. Et./AcOEt 60:40. 92 mg of a white solid were obtained. NMR ¹H (ppm, CDCl₃): 7.67-7.63 (m, 2H), 7.54-7.50 (m, 2H), 7.36-7.25 (m, 3H), 7.25-7.13 (m, 1H), 6.54 (br. s., 1H), 3.22-3.11 (m, 3H), 2.11-2.03 (m, 3H). MS (+ ESI): M+H⁺ 262.1.

### 144J. Compound (157)

Using preparation method 3, 144I was reacted with phosgene to provide a carbamoylchloride. Using preparation method 4, (S)-isobutyl-(L)-cysteine hydrochloride was protected. The carbamoylchloride and TMS protected amino acid were reacted using Preparation Method 5.

### Example 145: compound (158)

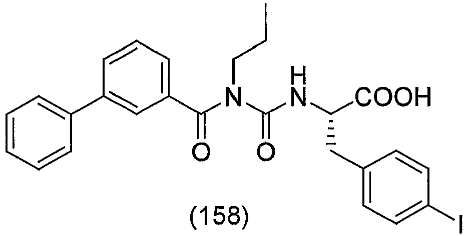

This synthesis was performed on Lantern™ solid phase.

### 145A:

A solution of N-Fmoc-4-iodo-(L)-phenylalanine (62 mg, 0.24 mmol), diisopropylcarbodiimide (18.6 µL, 0.12 mmol) and DMAP (1.1 mg, 9 µmol) in dry DMF was added to HMP-lantern (2, 15 µmol loading each). The reaction was stood at room temperature for 2 hours. After that time, the lanterns were rinsed with DMF (3×3 min.) and CH₂Cl₂ (3×3 min.). The lanterns were then air-dried.

### 145B:

The lanterns were then treated two times with 1 mL of a 20% piperidine solution in DMF (1×5min., 1×25 min). After this, the lanterns were rinsed with DMF (3×5min.), MeOH (3x5 min.) and CH₂Cl₂ (3×5 min.). The lanterns were dried in vacuo.

### 145C:

To the deprotected lantern-supported compounds was added 0.5 mL of CH₃CN and 100 µL of propylene oxide. This mixture was treated at 0°C with were treated with a solution of carbamoylchloride from Example 1B using Preparation Method 3 (0.3 mmol) in 0.5 mL of CH₃CN. The reaction was stood at room temperature for 16 hours. After that time, the lanterns were rinsed with CH₃CN (3×3 min.), MeOH (3×3 min.) and CH₂Cl₂ (3×3 min.).

### 145D: Compound (158):

On one lantern (15 µmol theoretical loading): The lantern was treated with a 20% TFA solution in CH₂Cl₂. The reaction was stood at room temperature for 1 hour. After this time the lantern was removed and the remaining solution was concentrated. The remaining residue was dissolved in a mixture CH₃CN/H₂O 90:10 and freeze-dried to give compound (158) as a white powder. HPLC analysis, (1 mL gradient 0% MeOH to 100% MeOH, run time: 18 minutes; retention time: 11.17 minutes) showed high purity was achieved. NMR ¹H (CDCl₃, ppm): 9.51 (d, *J*³ = 6.5 Hz, 1H), 7.71-7.37 (m, 11H), 7.01 (d, *J*³ = 8.3 Hz, 1H), 4.77-4.76 (m, 1H), 3.67-3.62 (m, 2H), 3.23-3.07 (m, 2H), 1.49 (sext., *J*³ = 7.71 Hz, 2H), 0.69 (t, *J*³ = 7.4 Hz, 3H).

### Example 146: Compound (15)

In this example, compound (15) from Example 13 was prepared using a solid phase synthesis on lantern using the following procedure.

### 146A:

One lantern from Example 64C (15 *µ*M theoretical loading) was swelled in 320 µL of dry DMF for 10 min. 80 *µ*L of dry THF and 400 *µ*L of dry diisopropylethylamine were added, followed by phenyl boronic acid (18mg, 0.15 mmol) and Pd(Ph₃)₄ (3.5 mg, 20 mol%). The reaction was then stood under a nitrogen atmosphere for 18 hours. After this time, the lantern was successively rinsed with DMF (3×3 min.), sodium diethyldithiocarbamate DMF solution (sodium diethyldithiocarbamate 5 mg/mL, diisopropylethylamine 5 *µ*L/mL, 3×3 min.), MeOH (3×3 min.) and CH₂Cl₂ (3×3 min.). The lantern was then air dried.

### 146B: compound (15)

On the lantern from Example 65A (15 *µ*mol theoretical loading): The lantern was treated with 1 mL of a 20% TFA solution in CH₂Cl₂. The reaction was stood at room temperature for 1 hour. After this time the lantern was removed and rinsed with CH₂Cl₂ for 5 minuntes. The two solutions were combined and concentrated to give compound (15) as a yellowish film. Mass spectrometry (electrospray, positive ion): [M+H]⁺: 507.2 (molecular weight: 506.22). HPLC analysis, (1 mL gradient 0% MeOH to 100% MeOH, run time: 18 minutes; retention time: 11.32 minutes) showed high purity was achieved and identical retention time compared to a sample of compound (15) from Example 13.

### Biological Examples:

Measurement of competition of benzoylurea compounds with Bim26-mer for a Bcl-2 binding site.

Alphascreen (Amplified Luminescent Proximity Homogenous Assay) is a bead based technology which measures the interaction between molecules. The assay consists of two hydrogel coated beads which, when bought into close proximity by a binding interaction, allow the transfer of singlet oxygen from a donor bead to an acceptor bead.

Upon binding and excitation with laser light at 680 nm, a photosensitiser in the donor bead converts ambient oxygen to a more excited singlet state. This singlet oxygen then diffuses across to react with a chemiluminescer in the acceptor bead. Fluorophores within the same bead are activated resulting in the emission of light at 580-620 nm.

Screening of the benzoylurea test compounds was performed using the Alphascreen GST (glutathione s-transferase) detection kit system. Test compounds were titrated into the assay which consisted of GST tagged Bcl_{w} ΔC29 protein (0.05 nM Final concentration) and Biotinylated Bim BH3-26 peptide, Biotin-DLRPEIRIAQELRRIGDEFNETYTRR (3.0 nM Final concentration). For the GST tagged Bcl-x_{L} assay, GST tagged Bcl-x_{L} ΔC25 protein (0.6 nM Final concentration) and Biotinylated Bim BH3-26 peptide, Biotin-DLRPEIRIAQELRRIGDEFNETYTRR (5.0 nM Final concentration) were used. To this reaction mix anti-GST coated acceptor beads and Streptavidin coated donor beads, both at 15µg/ml Final concentration, were added and the assay mixture incubated for 4 hours at room temperature before reading. Similarly when the Bcl-2 protein was Mcl-1, GST tagged Mcl-1 protein (0.4 nM Final concentration) and Biotinylated Bak BH3 peptide, Biotin-PSSTMGQVGRQLAIIGDDINRRYDSE-OH (4.0 nM Final concentration) were used.

### Detailed protocol:

1) prepare a 384 well with 4.75 µL of buffer and 0.25 µL of compounds (20 mM in DMSO) per well.
2) Mix the binding partners, in one tube add Bcl-w, Bcl-x_{L} or Mcl-1 and the acceptor beads, in the second tube add Biotinylated BH3 peptide and the donor beads.
3) Pre-incubate the two pairs of binding partners for 30 minutes.
4) Add 1 µL of acceptor beads:Bcl-w, Bcl-x_{L} or Mcl-1 protein mix to each well.
5) Seal the plate and incubate at room temperature for 30 minutes.
6) Add 10 µL of donor bead:BH3 peptide mix to each well.
7) Seal the plate, cover with foil and incubate for 4 hours.

Assay buffer contained 50mM Hepes pH 7.4, 10mM DTT, 100mM NaCl, 0.05% Tween and 0.1 mg/ml casein. Bead dilution buffer contained 50mM Tris, pH 7.5, 0.01% Tween and 0.1 mg/ml casein. The final DMSO concentration in the assay was 0.5%. Assays were performed in 384 well white Optiplates and analysed on the PerkinElmer Fusion alpha plate reader (Ex680, Em520-620nM).

The GST Alphascreen detection kit and Optiplates were purchased from PerkinElmer.

The results of the binding assay are shown in Table 3.

**Table 3: Binding affinities of Benzoylurea Compounds**

| Compound No. | Bcl-wΔ29 IC₅₀ (µM) | Bcl-x_{L}Δ25 IC₅₀ (µM) | Mcl-1 IC₅₀ (µM) |
|---|---|---|---|
| (1) | 38^{a} | 70.1 | - |
| (2) | 57 | 56.8 | - |
| (3) | 65 | 65.8 | - |
| (4) | 149 | 148 | - |
| (5) | 266 | 152.8 | - |
| (6) | 73 | 54.2 | - |
| (7) | 37 | 45.2 | - |
| (8) | 46 | 36.4 | - |
| (9) | 14^{a} | - | - |
| (10) | 26^{a} | - | - |
| (11) | 104 | - | - |
| (12) | 46^{a} | - | - |
| (13) | 31 | - | - |
| (14) | 36 | - | - |
| (15) | 41 | - | - |
| (16) | 130 | - | - |
| (17) | 55 | - | - |
| (18) | 60 | - | - |
| (19) | 132 | - | - |
| (21) | 84 | - | - |
| (22) | 80 | - | - |
| (23) | 76 | - | - |
| (24) | 31.2 | - | - |
| (25) | 261 | - | - |
| (27) | 194 | - | - |
| (28) | 300 | - | - |
| (29) | 88 | - | - |
| (30) | 147 | - | - |
| (31) | 165 | - | - |
| (33) | 72 | - | - |
| (34) | 72 | - | - |
| (35) | 66 | - | - |
| (36) | 47 | - | - |
| (37) | 60 | - | - |
| (38) | 297 | - | - |
| (39) | 50 | - | - |
| (40) | 63 | - | - |
| (42) | 49 | - | - |
| (43) | 57 | - | - |
| (45) | 69 | - | - |
| (46) | 38 | - | - |
| (47) | 62 | - | - |
| (48) | 68 | - | - |
| (52) | 96 | - | - |
| (58) | 48 | - | - |
| (59) | 68 | - | - |
| (62) | 48 | - | - |
| (63) | 136 | - | - |
| (64) | 74 | - | - |
| (65) | 60 | - | - |
| (66) | 58 | - | - |
| (67) | 92 | - | - |
| (70) | 153 | - | - |
| (71) | n.b.^{b} | - | - |
| (72) | n.b.^{b} | - | - |
| (81) | 144 | - | - |
| (82) | 45 | 27 | 169 |
| (83) | 35 | 14 | 77 |
| (84) | 27 | 11 | 61 |
| (85) | 40 | n.b.^{b} | 95 |
| (86) | 82 | 283 | n.b.^{b} |
| (87) | 99 | 151 | 161 |
| (88) | 49 | 51 | 154 |
| (89) | 50 | 86 | 107 |
| (90) | 39 | 22 | 70 |
| (91) | 32 | 16 | 92 |
| (92) | 67 | 36 | n.b.^{b} |
| (93) | 26 | 27 | 53 |
| (94) | 19 | 23 | 46 |
| (95) | 35 | 73 | 94 |
| (96) | 52 | 153 | 133 |
| (97) | 68 | 199 | 264 |
| (98) | 32 | 93 | 107 |
| (99) | 60 | 163 | 168 |
| (100) | 18 | 48 | 52 |
| (101) | 19 | 42 | 55 |
| (102) | 18 | 50 | 51 |
| (103) | 18 | 53 | 36 |
| (104) | 205 | 187 | 267 |
| (105) | 71 | 183 | 164 |
| (106) | 102 | 182 | 251 |
| (107) | 37 | 70 | 73 |
| (108) | 49 | 87 | 72 |
| (109) | 29 | 81 | 65 |
| (110) | 37 | 85 | 64 |
| (111) | 36 | 115 | 86 |
| (112) | 40 | 119 | 112 |
| (113) | 28 | 94 | 65 |
| (114) | 32 | 85 | 58 |
| (115) | - | 32 | - |
| (116) | - | 19 (26) | - |
| (117) | - | 35 (44) | - |
| (118) | - | 34 (39) | - |
| (119) | - | 67 (52) | - |
| (120) | - | 44 (46) | - |
| (121) | - | 141 (165) | - |
| (122) | - | 26 | - |
| (123) | - | 30 | - |
| (124) | - | 33 | - |
| (125) | - | 44 | - |
| (126) | - | 90 | - |
| (127) | - | 40 | - |
| (128) | - | 53 | - |
| (129) | - | 37 | - |
| (130) | - | 39 | - |
| (131) | - | 20 | - |
| (132) | - | 24 | - |
| (133) | - | 27 | - |
| (134) | - | 51 | 181 |
| (135) | - | 32 | 57 |
| (136) | - | 23 | 204 |
| (137) | - | 9.5 | 132 |
| (138) | - | 26 | 146 |
| (139) | - | 28 | 80 |
| (140) | - | 44 | n.b.^{b} |
| (141) | - | 28 | 84 |
| (142) | - | 32 | 70 |
| (143) | - | 30 | 74 |
| (144) | - | 39 | 66 |
| (145) | - | 48 | 92 |
| (146) | - | 46 | n.b.^{b} |
| (147) | - | 50 | 85 |
| (148) | - | 172 | 71 |
| (149) | - | 107 | - |
| (150) | - | n.b.^{b} | - |
| (151) | - | 212 | - |
| (152) | - | 48 | - |
| (153) | - | 40 | - |
| (154) | - | 42 | - |
| (155) | - | 53 | - |
| (156) | - | 10 | - |

| | | | |
|---|---|---|---|
| a. Tested in a separate experiment; b. n.b. not binding in the assay conditions c. - not tested | | | |

### Cell based assay

The efficacy of the compounds of the present invention can also be determined in cell based killing assays using a variety of cell lines and mouse tumor models. For example, their activity on cell viability can be assessed on a panel of cultured tumorigenic and non-tumorigenic cell lines, as well as primary mouse or human cell populations, e.g. lymphocytes. For these assays, 5,000-20,000 cells are cultured at 37°C and 10% CO₂ in appropriate growth media, eg: 100 µL Dulbecco's Modified Eagle's medium supplemented with 10% foetal calf serum, asparaginase and 2-mercaptoethanol in the case of pre-B *Eµ-Myc* mouse tumors in 96 well plates. Cell viability and total cell numbers can be monitored over 1-7 days of incubation with 1 nM-100 µM of the compounds to identify those that kill at IC50<10 µM. Cell viability is determined by the ability of the cells to exclude propidum iodide (10 µg/mL by immunofluorescence analysis of emission wavelengths of 660-675 nm on a flow cytometer (BD FACScan). Alternatively, a high throughput colorimetric assay such as the Cell Titre 96. AQueous Non-Radioactive Cell Proliferation Assay (Promega) may be used. Cell death by apoptosis is confirmed by preincubation of the cells with 50 µM of a caspase inhibitor such as zVAD-*fmk.* Drug internalisation is confirmed by confocal microscopy of conjugates labelled with a fluorochrome such as Fitc.

The conjugates of the present invention can also be evaluated for the specificity of their targets and mode of action *in vivo.* For example, if a conjugate comprises a compound of the invention that binds with high selectivity to Bcl-2, it should not kill cells lacking Bcl-2. Hence, the specificity of action can be confirmed by comparing the activity of the compound in wild-type cells with those lacking Bcl-2, derived from Bcl-2-deficient mice.

### Antibody Production

Antibodies suitable for preparation of conjugates may be prepared by techniques known in the art. See, for example, Galfre *et. al.,* 1977.

### Coupling antibodies and compounds of the invention

The antibody is reacted with NHS-activated maleimide-ACP, sulfosuccinimidyl-4-[N-maleimidomethyl]cyclohexane-1-carboxylate, 4-succinimidyloxycarbonyl-α-methyl-α-(2-pyridyldithio)toluene or LC-SMPT to prepare an antibody decorated with multiple linkers. The antibody is then reacted with a thiol group on a thiol containing compound of the invention.

### References

G. P. Adams, J. E. McCartney, M. S. Tai, H. Oppermann, J. S. Huston, W. F. Stafford 3rd, M. A. Bookman, I. Fand, L. L. Houston, L. M. Weiner, Cancer Res., 1993, 53: 4026-34.
Ausubel et. al., Current Protocols in Molecular Biology, John Wiley & Sons Inc., 1994-1998.
J. B. Baell, D. C. S. Huang, Biochem. Pharmacol. 2002, 64, 851-863.
P. Bouillet, S. Cory, L. C. Zhang, A. Strasser, J. M. Adams, Developmental Cell. 2001, 1, 645.
N. D. Brewis, A. Phelan, N. Normand, E. Choolun, P. O'Hare, Mol. Ther., 2003, 7(2), 262-70.
Brummer et al. Synthesis, 1998, 1742**.**
J. Carlsson, H. Dreven, R. Axen, Biochem. J., 2001, 173: 723-37.
Chen et. al., O.P.P.I. Briefs, 2000, 32(4):381.
Coligan et. al., Current Protocols in Immunology, John Wiley & Sons, Inc., 1991.
S. Cory, J. A. Adams, Nature Reviews/Cancer 2002, 2, 647.
A. J. Cumber, E. S. Ward, G. Winter, G. D. Parnell, E. J. Wawrzynczak, J. Immunol., 1992, 149: 120-126.
J. Davies, L. Riechmann, FEBS Lett., 1994, 339: 285-290.
Deardon, C., Biodrug, 2002, 1b: 283-301.
D. J. Dunican and P. Doherty, Biopolymers (Peptide Science), 2001, 60: 45-60.
S. S. Dharap and T. Minko, Pharm. Res., 2003, 20 (6): 889-896.
A. Egle, P. Bouillet, A. W. Harris, S. Cory, Blood, 2003, 102, 135A.
G. Galfre, S. C. Howe, C. Milstein, G. W. Butcher, J. C. Howard, Nature, 1977, 266: 550-552.
R. Glockshuber, M. Malia, I. Pfitzinger, A. Plückthun, Biochem., 1990, 29: 1363-1367.
A. C. Goulet, V. S. Goldmacher, J. M. Lambert, C. Baron, D. D. Roy, E. Kouassi, Blood, 1997, 90 (6): 2364-75.
D. R. Green, G. I. Evan, Cancer Cell. 2002, 1, 19.
C. Hamers-Casterman, T. Atarhouch, S. Muyldermans, G. Robinson, C. Hamers, E. B. Songa, N. Bendahman, R. Hamers, Nature, 1993, 363: 446-448.
M. G. Hinds, M. Lackmann, G. L. Skea, P. J. Harrison, D. C. S. Huang, C. L. Day, EMBO J. 2003, 22, 1497.
J. H. Horner, O. M. Musa, A. Bouvier, M. Newcomb, Journal of the American Chemical Society 1998, 120, 7738*.*
R. Ju, J. M. Lambert, L. R. Pierce, R. R. Traut, Biochemistry, 1978, 17: 5399-5406.
S. H. Kaufmann, M. O. Hengartner, Trendy in Cell Biology. 2001, 11, 526.
I. M. Klotz and R. E. Heiney, Arch. Biochem. Biophys., 1962, 96: 605-612.
G. Köhler and C. Milstein, Nature, 1975, 256: 495-497.
S. A. Kostelny, M. S. Cole, J. Y. Tso, J. Immunol., 1992, 148: 1547-1553.
A. Krebber, S. Bomhauser, J. Burmester, A. Honnerger, J. Wiluda, H. R. Bosshard, A. Plückthun, J. Immunol. Methods, 1997, 201(1): 35-55.
J. Ku, P. G. Schultz, Proc. Natl. Acad. Sci. USA, 1995, 92: 6552-6556.
A. Letai, M. C. Bassik, L. D. Walensky, M. D. Sorcinelli, S. Weiler, S. J. Korsmeyer, Cancer cell, 2002, 2(3): 183-92.
X. Liu, S. Dai, Y. Zhu, P. Marrack, J. Kappler, Immunity. 2003, 19, 341.
D. L. Ludwig, D. S. Pereira, Z. Zhu, D. J. Hicklin, P. Bohlen, Oncogene, 2003, 22 (56): 9097-9106.
A. J. Marks, M. Cooper, K. Orchard, N. I. Folarin, K. Ganeshaguru, A. V. Hoffbrand, A. B. Mehta, R. G. Wickremasinghe, Abstract, Blood, (11) 247, Part 1, November 16, 2003.
S. W. Muchmore, M. Sattler, H. Liang, R. P. Meadows, J. E. Harlan, H. S. Yoon, D. Nettesheim, B. S. Chang, C. B. Thompson, S. L. Wong, S. L. Ng, S. W. Fesik, Nature. 1996, 381, 335.
E. Negishi and L. Anastasia, Chem. Rev., 2003, 103, p1979.
T. Oltersdorf, S. W. Elmore, A. R. Shoemaker, R.C. Armstrong, D. J. Augeri, B.A. Belli, M. Bruncko, T. L. Deckwerth, J. Dinges, P. J. Hajduk, M. K. Joseph, S. Kitada, S. J. Korsmeyer, A. R. Kunzer, A. Letai, C. Li, M. J. Mitten, D. G. Nettesheim, S. Ng, P. M. Nimmer, J. M. O'Connor, A. Oleksijew, A. M. Petros, J. C. Reed, W. Shen, S. K. Tahir, C. B. Thompson, K. J. Tomaselli, B. Wang, M. D. Wendt, H. Zhang, S. W. Fesik, S. H. Rosenberg, Nature, 2005, 435, 677-681.
P. Pack, A. Pluckthun, Biochem., 1992, 31: 1579-1584.
G. A. Patani and E. J. LaVoie, Chem. Rev., 1996, 96, 3147-3176.
A. M. Petros, D. G. Nettesheim, Y. Wang, E. T. Olejniczak, R. P. Meadows, J. Mack, K. Swift, E. D. Matayoshi, H. Zhang, C. B. Thompson, S. W. Fesik, Protein Science. 2000, 9, 2528.
Plückthun et. al., Antibody Engineering: A practical approach. 203-252, 1996.
M. J. Poznansky, R. Singh, B. Singh, G. Fantus, Science, 1984, 223: 1304-1306.
Y. Reiter, U. Brinkmann, S. H. Jung, B. Lee, P. G. Kasprzyk, C. R. King, I. Pastan, J. Biol. Chem.,1994a, 269: 18327-18331.
Y. Reiter, U. Brinkmann, R. J. Kreitman, S. H. Jung, B. Lee, I. Pastan, Biochem., 1994b, 33: 5451-5459.
Y. Reiter, L. H. Pai, U. Brinkmann, Q. C. Wang, I. Pastan, Cancer Res., 1994c, 54: 2714-2718.
P. Sapra and T. M. Allen, Cancer Res., 2002, 62 (24): 7190-4.
M. Sattler, H. Liang, D. Nettesheim, R. P. Meadows, J. E. Harlan, M. Eberstadt, H. S. Yoon, S. B. Shuker, B. S. Chang, A. J. Minn, C. B. Thompson, S. W. Fesik, Science. 1997, 275, 983*.*
A. D. Schimmer, D. W. Hedley, S. Chow, N. A. Pham, A. Chakrabartty, D. Bouchard, T. W. Mak, M. R. Trus, M. D. Minden, Cell Death Differ., 2001, 8(7): 725-733.
T. Seko, M. Kato, H. Kohno, S. Ono, K. Hashimura, H. Takimizu, K. Nakai, H. Maegawa, N. Katsube, M. Toda, Bioorg. Med. Chem., 2003, 11:1901-1913.
S. Shangary and D. E. Johnson, Biochemistry, 2002, 41(30): 9485-95.
E. L. Snyder, B. R. Meade, C. C. Saenz, S. F. Dowdy, PloS Biology, 2004, 2, 0186-0193.
P. E. Thorpe, P. M. Wallace, P. P. Knowles, M. G. Relf, A. N. Brown, G. J. Watson, R. E. Knyba, E. J. Wawrzynczak, D. C. Blokey, Cancer Res., 1987, 47: 5924-5931.
F. N. Uckun, W. E. Evans, C. J. Forsyth, K. G. Waddick, L. T. Ahlgren, L. M. Chelstrom, A. Burkhardt, J. Bolen, D. E. Meyers, Science, 1995, 267 (5199): 886-898.
H. W. G. Van Herwijnen and U. H. Brinker, Journal of Organic Chemistry, 2001, 66(8), 2874-2876.
D. Wang, S. Moore, Biochemistry, 1977, 16: 2937-2941.
D. Wang, Q. Li, W. Hudson, E. Berren, F. Uckun and J. H. Kersey, Bioconjug. Chem., 1997, 8 (6): 878-84.
J. L. Wang, Z. J. Zhang, S. Choksi, S. Shan, Z. Lu, C. M. Croce, E. S. Alnemri, R. Korngold, Z. Huang, Cancer Res., 2000, 60(6): 498-502.
E. S. Ward, D. Gussow, A. D. Griffiths, P. T. Jones, G. Winter, Nature, 1989, 341: 544-546.
K. O. Webber, Y. Reiter, U. Brinkmann, R. Kreitman, I. Pastan, Mol. Immunol. 1995, 32: 249-258.
G. Winter and C. Milstein, Nature, 1991, 349: 293-9.
N. R. Worrell, A. J. Cumber, G. D. Parnell, A. Mirza, J. A. Forrester, W. C. Ross, Anticancer Drug Design, 1986, 1: 179-188.
H. Yin and A. D. Hamilton, Bioorg. Med. Chem. Lett., 2004, 14, 1375-1379.
S. Yoshitake, Y. Yamada, E. Ishikawa, R. Masseyeff, Eur. J. Biochem., 1979, 101: 395-399.
J. Y. Zhang, Nature Reviews/Drug Discovery 2002, 1, 10 1.

## Claims

1. A compound of formula (I): 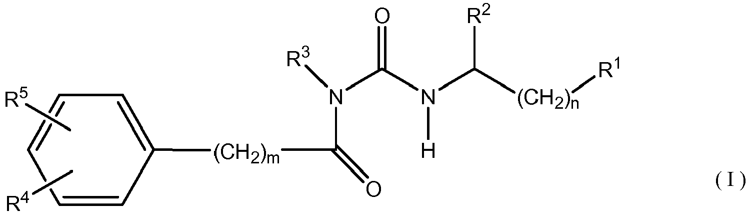 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, -(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and a group
R^{a}-(CHR')ₓ-A-(CH₂)_{y}-
wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 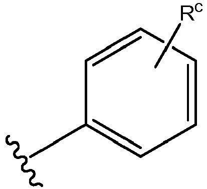 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group
R^{d}-(CH₂)ₚ-W-(CH₂)_{q}-
wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)pNH(C₁₋₆alkyl), -(CH₂)ₚN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)pC(R⁷)₃, -(CH₂)pOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen;
and pharmaceutically acceptable salts and prodrugs selected from N-α-acyloxy amides, N-(acyloxyalkoxycarbonyl) amines and α-acyloxyalkyl esters of alcohols or phenols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is CO₂H, m and n are 0 and R⁵ is H, R³ is not n-propyl.

2. A compound according to claim 1, wherein R¹ is CO₂H, tetrazole, tetrazolate or a benzene acylsulfonamide.

3. A compound according to claim 2, wherein R¹ is CO₂H.

4. A compound according to claim 1, wherein R² is R^{a}-(CHR')ₓ-A-(CH₂)_{y}- in which R^{a} is H, optionally substituted cycloalkyl or optionally substituted aryl, x is 0 or 1 to 4, R' is H or C₁₋₃alkyl, A is O, S or SO and y is 1 to 3.

5. A compound according to claim 1, wherein R² is R^{a}-(CHR')ₓ-A-(CH₂)_{y}- in which R^{a} is optionally substituted aryl or optionally substituted heteroaryl, R' is H, the sum of x and y is 1 to 4 and A is a covalent bond.

6. A compound according to claim 1, wherein R³ is C₁₋₆alkyl, optionally substituted cycloalkyl or a group R^{d}-(CH₂)ₚ-W-(CH₂)_{q}- in which R^{d} is optionally substituted cycloalkyl, optionally substituted aryl, optionally substituted heterocyclyl or optionally substituted heteroaryl, W is a covalent bond and the sum of p and q is 1 to 3, or R^{d} is H, W is O or S and the sum of p and q is 2 to 4.

7. A compound according to claim 1, wherein R⁴ is C₁₋₆alkyl, C₂₋₆alkenyl, C₂₋₆alkynyl, 3- or 4-aryl, aryl(C₁₋₃alkyl)-, aryl(C₂₋₃alkenyl)- or aryl(C₂₋₃alkynyl)-wherein aryl is optionally substituted with one or more halogen, C₁₋₆alkyl, C₁₋₆alkoxy, trifluoromethyl, hydroxy(C₁₋₆alkyl), CN or C₁₋₆acyl.

8. A compound according to claim 7, wherein R⁴ is 3- or 4-phenyl, naphthyl or phenyl(ethynyl) in which the phenyl or naphthyl groups are optionally substituted.

9. A compound according to claim 1, wherein R⁵ is hydrogen, halogen, methyl or methoxy.

10. A compound according to claim 9, wherein R⁵ is hydrogen.

11. A compound according to claim 1, wherein m is 0.

12. A compound according to claim 1, wherein n is 0.

13. A compound according to claim 1, wherein said compound is a compound of formula (II) 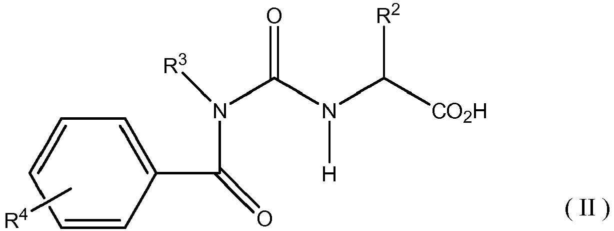 wherein R² and R³ are as defined for formula (I) in claim 1 and R⁴ is C₁₋₆alkyl, C₂₋₆alkenyl, C₂₋₆alkynyl, 3-phenyl, 3-(2-naphthyl), 3-(1-naphthyl), 3-benzyl, 4-phenyl, 4-benzyl, 3-(phenylethynyl) or 4-(phenylethynyl), wherein each phenyl, naphthyl or benzyl group is optionally substituted with one or more substituents selected from C₁₋₆alkyl, C₂₋₆alkenyl, C₂₋₆alkynyl, C₁₋₆alkoxy, C₂₋₆alkenyloxy and halogen, and pharmaceutically acceptable salts or prodrugs selected from N-α-acyloxy amides, N-(acyloxyalkoxycarbonyl) amines and α-acyloxyalkyl esters of alcohols or phenols; with the proviso that (i) when R² is C₆H₅-CH₂S-CH₂- and R⁴ is 3-phenyl, R³ is not ethyl and (ii) when R² is CH₃CH(CH₃)CH₂SCH₂ and R⁴ is 3-phenethynyl, R³ is not n-propyl.

14. A compound according to claim 1, wherein said compound is a compound of formula (IIa) 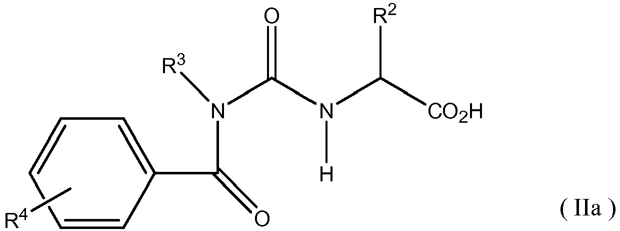
wherein R² is defined as above for formula (I) in claim 1,
R³ is selected from C₃₋₆alkyl, C₂₋₆alkenyl, C₂₋₆alkynyl, cycloalkyl, aryl, heterocyclyl, heteroaryl and a group
R^{d}-(CH₂)ₚ-W-(CH₂)₄-
wherein W is selected from a covalent bond, O, S or NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is C₁₋₆alkyl, C₂₋₆alkenyl, C₂₋₆alkynyl, 3-phenyl, 3-(2-naphthyl), 3-(1-naphthyl), 3-benzyl, 4-phenyl, 4-benzyl, 3-(phenylethynyl) or 4-(phenylethynyl), wherein each phenyl, naphthyl or benzyl group is optionally substituted with one or more substituents selected from C₁₋₆alkyl, C₂₋₆alkenyl, C₂₋₆alkynyl, C₁₋₆alkoxy, C₂₋₆alkenyloxy and halogen, and pharmaceutically acceptable salts or prodrugs selected from N-α-acyloxy amides, N-(acyloxyalkoxycarbonyl) amines and α-acyloxyalkyl esters of alcohols or phenols; with the proviso that (ii) when R² is CH₃CH(CH₃)CH₂SCH₂ and R⁴ is 3-phenethynyl, R³ is not n-propyl.

15. A conjugate comprising a compound of formula (I) 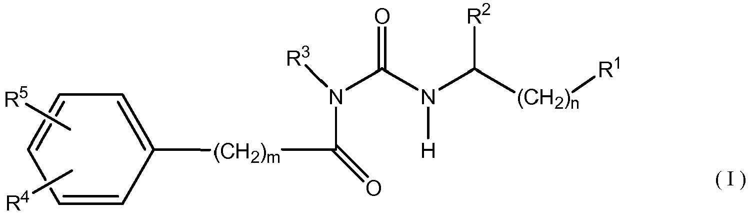 wherein
R¹ is selected from CO₂H or a carboxylic acid or carboxylate bioisostere selected from tetrazole, tetrazolate, oxadiazole, aryl or alkyl acylsulfonamide, phosphate and sulfonic acid;
R² is selected from an amino acid side chain selected from the group consisting of -CH₃, -(CH₂)₃NHC(=NH)NH₂, -CH₂CONH₂, -CH₂CO₂H, -CH₂SH, -(CH₂)₂CONH₂, -(CH₂)₂CO₂H, -CH₂(4-imidazole), -CH(CH₃)CH₂CH₃, -CH₂CH(CH₃)₂, -(CH₂)₄NH₂, -(CH₂)₂SCH₃, -CH₂Ph, -CH₂OH, -CH(CH₃)OH, -CH₂(3-indolyl), -CH₂(4-hydroxyphenyl), -CH(CH₃)₂, -(CH₂)₄NHC(=NH)NH₂, -(CH₂)₂OH, -(CH₂)₂SH, -CH₂CH₂CH₃, -(CH₂)₃CH₃, and (CH₂)ᵥC(=NH)NH₂ where v is an integer from 1 to 4, optionally in which carboxy, hydroxy, thiol or amino groups are protected with suitable carboxy, hydroxy, thiol or amino protecting groups; and cycloalkyl, cycloalkenyl, aryl, heterocyclyl, heteroaryl and a group
R^{a}-(CHR')ₓ-A-(CH₂)_{y}-
wherein A is a covalent bond or is selected from O, S, SO, SO₂ and NR⁶, R^{a} is H, cycloalkyl, cycloalkenyl, aryl, heterocyclyl, heteroaryl or R^{b} where R^{b} is 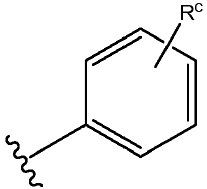 and R^{c} is selected from heteroaryl, aryl, aryl(C₂₋₆alkenyl), aryl(C₂₋₆alkynyl), heteroaryl(C₂₋₆alkenyl) and heteroaryl(C₂₋₆alkynyl), R' is H or C₁₋₆alkyl, x and y are independently 0 or an integer from 1 to 6 provided that the sum of x and y is 1 to 6;
R³ is selected from C₁₋₆alkyl, C₂₋₆alkenyl, C₂₋₆alkynyl, cycloalkyl, cycloalkenyl, aryl, heterocyclyl, heteroaryl and a group
R^{d}-(CH₂)ₚ-W-(CH₂)_{q}-
wherein W is selected from a covalent bond, O, S and NR⁶, R^{d} is selected from H, cycloalkyl, cycloalkenyl, aryl, heterocyclyl and heteroaryl; p is an integer from 1 to 6, q is 0 or an integer from 1 to 5 provided that the sum of p and q is 1 to 6;
R⁴ is selected from C₁₋₆alkyl, C₂₋₆alkenyl, C₂₋₆alkynyl, cycloalkyl, C₁₋₆alkyloxy, C₂₋₆alkenyloxy, C₂₋₆alkynyloxy, cycloalkoxy, C₁₋₆alkylthio, C₂₋₆alkenylthio, C₂₋₆alkynylthio, cycloalkylthio, halogen, aryl, aryl(C₁₋₆alkyl)-, aryl(C₂₋₆alkenyl), aryl(C₂₋₆alkynyl), heterocyclyl, heterocyclyl(C₁₋₆alkyl)-, heterocyclyl(C₂₋₆alkenyl), heterocyclyl(C₂₋₆alkynyl), heteroaryl, heteroaryl(C₁₋₆alkyl)-, heteroaryl(C₂₋₆alkenyl) and heteroaryl(C₂₋₆alkynyl);
R⁵ is selected from H, halogen, C₁₋₆alkyl, C₂₋₆alkenyl, C₂₋₆alkynyl, C₁₋₆alkyloxy, C₂₋₆alkenyloxy, C₂₋₆alkynyloxy, C₁₋₆alkythio, C₂₋₆alkenylthio, C₂₋₆alkynylthio, CN and C(R⁷)₃ or when R⁵ is in the 2- or 5-position, R⁵ and R³ taken together may form a 5 to 10 membered ring;
R⁶ is selected from H, C₁₋₆alkyl, C₂₋₆alkenyl and C₂₋₆alkynyl;
Each R⁷ is independently selected from H and halogen;
m is 0 or an integer from 1 to 6; and
n is 0 or an integer from 1 to 3;
wherein each alkyl, alkenyl, alkynyl, cycloalkyl, aryl, heterocyclyl and heteroaryl is optionally substituted with one or more optional substituents selected from C₁₋₆alkyl, C₂₋₆alkenyl, C₂₋₆alkynyl, C₃₋₆cycloalkyl, C₁₋₆alkyloxy(CH₂)ₚ-, C₂₋₆alkenyloxy(CH₂)ₚ-, C₂₋₆alkynyloxy(CH₂)ₚ-, C₃₋₆cycloalkoxy(CH₂)ₚ-, C₁₋₆alkylthio(CH₂)ₚ-, C₂₋₆alkenylthio(CH₂)ₚ-, C₂₋₆alkynylthio(CH₂)ₚ-, C₃₋₆cycloalkylthio(CH₂)ₚ-, hydroxy(CH₂)ₚ-, -(CH₂)ₚSH, -(CH₂)ₚCO₂H, -(CH₂)ₚCO₂C₁₋₆alkyl, C₂₋₆acyl(CH₂)ₚ-, C₂₋₆acyloxy(CH₂)ₚ-, C₂₋₆alkylSO₂(CH₂)ₚ-, C₂₋₆alkenylSO₂(CH₂)ₚ-, C₂₋₆alkynylSO₂(CH₂)ₚ-, aryl-SO₂(CH₂)ₚ-, heteroarylSO₂(CH₂)ₚ-, heterocyclylSO₂(CH₂)ₚ-, -(CH₂)ₚNH₂, -(CH₂)ₚNH(C₁₋₆alkyl), -(CH₂)pN(C₁₋₆alkyl)₂, -(CH₂)ₚNH(phenyl), -(CH₂)ₚN(phenyl)₂, -(CH₂)ₚNH(acyl), -(CH₂)ₚN(acyl)(phenyl), -(CH₂)ₚNH-(CH₂)ₚ-S-aryl, -(CH₂)ₚN=NHC(O)NH₂, -(CH₂)ₚC(R⁷)₃, -(CH₂)ₚOC(R⁷)₃, -(CH₂)ₚSC(R⁷)₃, -(CH₂)ₚCN, -(CH₂)ₚNO₂, -(CH₂)ₚhalogen, -(CH₂)ₚheterocyclyl, heterocyclyloxy(CH₂)ₚ-, -(CH₂)ₚheteroaryl, heteroaryloxy(CH₂)ₚ-, -(CH₂)ₚaryl, -(CH₂)ₚC(O)aryl and aryloxy(CH₂)ₚ- wherein p is 0 or an integer from 1 to 6 and each R⁷ is independently selected from hydrogen and halogen;
and pharmaceutically acceptable salts and prodrugs selected from N-α-acyloxy amides, N-(acyloxyalkoxycarbonyl) amines and α-acyloxyalkyl esters of phenols and alcohols; with the proviso that (i) when R¹ is COOH, R² is C₆H₅-CH₂S-CH₂-, R⁴ is 3-C₆H₅ and R⁵ is H, R³ is not CH₃CH₂- and (ii) when R² is CH₃CH(CH₃)CH₂SCH₂, R⁴ is 3-phenethynyl, R¹ is CO₂H, m and n are 0 and R⁵ is H, R³ is not n-propyl,
and a cell targeting moiety,
wherein the cell targeting moiety is selected from a hormone, a cytokine or an antigen binding molecule, where said antigen binding molecule is selected from an immunoglobulin or immunoglobulin fragment, such as a polyclonal antibody or a monoclonal antibody.

16. A conjugate according to claim 15, wherein the hormone is luteinising hormone-releasing hormone.

17. A conjugate according to claim 15, wherein the cytokine is selected from VEGF and EGF.

18. A conjugate according to claim 15, wherein the antibody is selected from CD19, CD20, CD22, CD79a, CD2, CD3, CD7, CD5, CD13, CD33, CD138 and antibodies targeting Erb1, Erb2, Erb3 or Erb4 receptors.

19. A conjugate according to claim 18, wherein the antibody is selected from CD19, CD20, CD22 and CD79a.

20. A pharmaceutical composition comprising a compound of formula (I) as defined in claim 15 or a conjugate according to claim 15, together with one or more pharmaceutically acceptable carriers and optionally, other therapeutic and/or prophylactic ingredients.

21. A method of regulating the death of a cell, wherein the cell is located *ex vivo,* comprising contacting the cell with an effective amount of a compound of formula (I) as defined in claim 15, or a conjugate according to claim 15.

22. A method of inducing apoptosis in unwanted or damaged cells, wherein the unwanted or damaged cells are located *ex vivo,* comprising contacting the damaged or unwanted cells with an effective amount of a compound of formula (I) as defined in claim 15, or a conjugate according to claim 15.

23. Use of a compound of formula (I) as defined in claim 15, or a conjugate of claim 15, in the manufacture of a medicament for the treatment and/or prophylaxis of a pro-survival Bcl-2 family member mediated disease or condition., wherein the disease or condition is an inflammatory condition, a cancer, an autoimmune disorder or a tissue hypertrophy.

24. Use of a compound of formula (I) as defined in claim 15, or a conjugate of claim 15, in the manufacture of a medicament for the treatment and/or prophylaxis of a disease or condition **characterised by** the inappropriate persistence or proliferation of unwanted or damaged cells.

25. Use according to claim 24, wherein the disease or condition is B cell non-Hodgkins lymphoma, B cell acute lymphoblastic leukemia, rheumatoid arthritis, systemic Lupus erythematosis and related arthropathies, T cell acute lymphoblastic leukemia, T cell non-Hodgkins lymphoma, Graft vs Host disease, acute myelogenous leukemia, chronic myelogenous leukemia, chronic myelomonocytic leukemia, multiple myeloma and cancer.

26. Use according to claim 25, wherein the cancer is ovarian cancer, breast cancer or prostate cancer.

27. Use of a compound of formula (I) as defined in claim 15, or a conjugate according to claim 15, in the manufacture of a medicament for regulating the death of a cell, or for inducing apoptosis in unwanted or damaged cells.

## Patentansprüche

1. Verbindung der Formel (I): 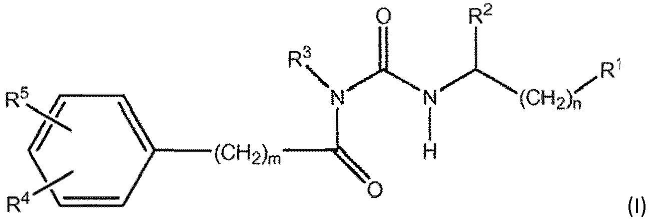 wobei
R¹ aus CO₂H oder einer Carbonsäure- oder Carboxylat-Bioisosterie ausgewählt ist, ausgewählt aus Tetrazol, Tetrazolat, Oxadiazol, Aryl oder Alkylsulfonamid, Phosphat und Sulfonsäure;
R² aus einer Aminosäureseitenkette ausgewählt ist, ausgewählt aus der Gruppe bestehend aus -CH₃, -(CH₂)₃NHC(=NH)NH₂, -CH₂CONH₂, -CH₂CO₂H, -CH₂SH, -(CH₂)₂CONH₂, -(CH₂)₂CO₂H, -CH₂(4-Imidazol), -CH(CH₃)CH₂CH₃, -CH₂CH(CH₃)₂, -(CH₂)₄NH₂, -(CH₂)₂SCH₃,-CH₂Ph, -CH₂0H, -CH(CH₃)OH, -CH₂(3-Indolyl), -CH₂(4-Hydroxyphenyl), -CH(CH₃)₂,-(CH₂)₄NHC(=NH)NH₂, -(CH₂)₂OH, -(CH₂)₂SH, -CH₂CH₂CH₃, -(CH₂)₃CH₃ und (CH₂)ᵥC(=NH)NH₂ wobei v eine ganze Zahl von 1 bis 4 ist, wobei gegebenenfalls Carboxy-, Hydroxy-, Thiol- oder Aminogruppen mit geeigneten Carboxy-, Hydroxy-, Thiol- oder Aminoschutzgruppen geschützt sind; und eine Gruppe
R^{a}-(CHR')ₓ-A-(CH₂)_{y}-
wobei A eine kovalente Bindung oder aus O, S, SO, SO₂ und NR⁶ ausgewählt ist, R^{a} H, Cycloalkyl, Cycloalkenyl, Aryl, Heterocyclyl, Heteroaryl oder R^{b} ist, wobei R^{b} 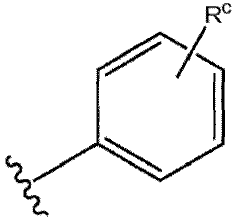 ist
und R^{c} aus Heteroaryl, Aryl, Aryl(C₂₋₆-Alkenyl), Aryl(C₂₋₆-Alkinyl), Heteroaryl(C₂₋₆-Alkenyl) und Heteroaryl(C₂₋₆-Alkinyl) ausgewählt ist, R' H oder C₁₋₆ -Alkyl ist, x und y unabhängig 0 oder eine ganze Zahl von 1 bis 6 sind, vorausgesetzt, dass die Summe von x und y 1 bis 6 ist; R³ aus C₁₋₆-Alkyl, C₂₋₆-Alkenyl, C₂₋₆-Alkinyl, Cycloalkyl, Cycloalkenyl, Aryl, Heterocyclyl, Heteroaryl und einer Gruppe
R^{d}-(CH₂)ₚ-W-(CH₂)_{q}-
ausgewählt ist,
wobei W aus einer kovalenten Bindung, O, S und NR⁶ ausgewählt ist, R^{d} aus H, Cycloalkyl, Cycloalkenyl, Aryl, Heterocyclyl und Heteroaryl ausgewählt ist; p eine ganze Zahl von 1 bis 6 ist, q 0 oder eine ganze Zahl von 1 bis 5 ist, vorausgesetzt, dass die Summe von p und q 1 bis 6 ist;
R⁴ aus C₁₋₆-Alkyl, C₂₋₆-Alkenyl, C₂₋₆-Alkinyl, Cycloalkyl, C₁₋₆-Alkyloxy, C₂₋₆-Alkenyloxy, C₂₋₆-Alkinyloxy, Cycloalkoxy, C₁₋₆-Alkylthio, C₂₋₆-Alkenylthio, C₂₋₆-Alkinylthio, Cycloalkylthio, Halogen, Aryl, Aryl(C₁₋₆-Alkyl)-, Aryl(C₂₋₆-Alkenyl), Aryl(C₂₋₆-Alkinyl), Heterocyclyl, Heterocyclyl (C₁₋₆-Alkyl), Heterocyclyl (C₂₋₆-Alkenyl), Heterocyclyl(C₂₋₆-Alkinyl), Heteroaryl, Heteroaryl(C₁₋₆-Alkyl), Heteroaryl(C₂₋₆-Alkenyl) und Heteroaryl(C₂₋₆-Alkinyl) ausgewählt ist;
R⁵ aus H, Halogen, C₁₋₆-Alkyl, C₂₋₆-Alkenyl, C₂₋₆-Alkinyl, C₁₋₆-Alkyloxy, C₂₋₆-Alkenyloxy, C₂₋₆-Alkinyloxy, C₁₋₆-Alkylthio, C₂₋₆-Alkenylthio, C₂₋₆-Alkylthio, CN und C(R⁷)₃ ausgewählt ist oder wenn R⁵ in der 2- oder 5-Position ist, R⁵ und R³ zusammen einen 5-bis 10-gliedrigen Ring bilden können;
R⁶ aus H, C₁₋₆-Alkyl, C₂₋₆-Alkenyl und C₂₋₆-Alkinyl ausgewählt ist;
Jedes R⁷ unabhängig aus H und Halogen ausgewählt ist;
m 0 oder eine ganze Zahl von 1 bis 6 ist; und
n 0 oder eine ganze Zahl von 1 bis 3 ist;
wobei jedes Alkyl, Alkenyl, Alkinyl, Cycloalkyl, Aryl, Heterocyclyl und Heteroaryl gegebenenfalls mit einem oder mehreren optionalen Substituenten substituiert ist, ausgewählt aus C₁₋₆-Alkyl, C₂₋₆-Alkenyl, C₂₋₆-Alkinyl, C₃₋₆-Cycloalkyl, C₁₋₆-Alkyloxy(CH₂)ₚ-, C₂₋₆-Alkenyloxy(CH₂)ₚ-, C₂₋₆-Alkinyloxy(CH₂)ₚ-, C₃₋₆-Cycloalkoxy (CH₂)ₚ-, C₁₋₆-Alkylthio(CH₂)ₚ-, C₂₋₆-Alkenylthio(CH₂)ₚ-, C₂₋₆-Alkinylthio(CH₂)ₚ-, C₃₋₆-Cycloalkylthio(CH₂)ₚ-, Hydroxy(CH₂)ₚ-,-(CH₂)ₚSH, -(CH₂)ₚCO₂H, -(CH₂)ₚCO₂C₁₋₆-Alkyl, C₂₋₆-Acyl(CH₂)ₚ-, C₂₋₆-Acyloxy(CH₂)ₚ-, C₂₋₆-Alkyl-SO₂(CH₂)ₚ-, C₂₋₆-Alkenyl-SO₂(CH₂)ₚ-, C₂₋₆-Alkinyl-SO₂(CH₂)ₚ-, Aryl-SO₂(CH₂)ₚ-, Heteroaryl-SO₂(CH₂)ₚ-, Heterocyclyl-SO₂(CH₂)ₚ-, -(CH₂)ₚNH₂, -(CH₂)ₚNH(C₁₋₆-Alkyl),-(CH₂)ₚN(C₁₋₆ -Alkyl)₂, -(CH₂)ₚNH(Phenyl), -(CH₂)ₚN(Phenyl)₂, -(CH₂)ₚNH(Acyl),-(CH₂)ₚN(Acyl)(Phenyl), -(CH₂)ₚNH-(CH₂)ₚ-S-Aryl, -(CH₂)ₚN=NHC(O)NH₂, -(CH₂)ₚC(R⁷)₃,-(CH₂)ₚOC(R⁷)₃, -(CH₂)ₚSC(R⁷)₃, -(CH₂)ₚCN, -(CH₂)ₚNO₂, -(CH₂)ₚHalogen, -(CH₂)ₚHeterocyclyl, Heterocyclyloxy(CH₂)ₚ-, -(CH₂)ₚHeteroaryl, Heteroaryloxy(CH₂)ₚ-, -(CH₂)ₚAryl,-(CH₂)ₚC(O)Aryl und Aryloxy (CH₂)ₚ-, wobei p 0 oder eine ganze Zahl von 1 bis 6 ist und jedes R⁷ unabhängig aus Wasserstoff und Halogen ausgewählt ist;
und pharmazeutisch verträgliche Salze und Prodrugs, aus N-α-Acyloxyamiden, N-(Acyloxyalkoxycarbonyl)-Aminen und α-Acyloxyalkylestern von Alkoholen oder Phenolen ausgewählt ist; mit der Maßgabe, dass (i) wenn R¹ COOH ist, R² C₆H₅-CH₂S-CH₂- ist, R⁴ 3-C₆H₅ ist und R⁵ H ist, R³ nicht CH₃CH₂- ist und (ii) wenn R² CH₃CH (CH₃)CH₂SCH₂ ist, R⁴ 3-Phenethynyl ist, R¹ CO₂H ist, m und n 0 sind und R⁵ H ist, R³ nicht n-Propyl ist.

2. Verbindung nach Anspruch 1, wobei R¹ CO₂H, Tetrazol, Tetrazolat oder ein Benzolacylsulfonamid ist.

3. Verbindung nach Anspruch 2, wobei R¹ CONH ist.

4. Verbindung nach Anspruch 1, wobei R² R^{a}-(CHR')ₓ-A-(CH₂)_{y}- ist, wobei R^{a} H, gegebenenfalls substituiertes Cycloalkyl oder gegebenenfalls substituiertes Aryl ist, x 0 oder 1 bis 4 ist, R' H oder C₁₋₃-Alkyl ist, A O, S oder SO ist und y 1 bis 3 ist.

5. Verbindung nach Anspruch 1, wobei R² R^{a}-(CHR')ₓ-A-(CH₂)_{y}- ist, wobei R^{a} gegebenenfalls substituiertes Aryl oder gegebenenfalls substituiertes Heteroaryl ist, R' H ist, die Summe von x und y 1 bis 4 ist und A eine kovalente Bindung ist.

6. Verbindung nach Anspruch 1, wobei R³ C₁₋₆-Alkyl, gegebenenfalls substituiertes Cycloalkyl oder eine Gruppe R^{d}-(CH₂)ₚ-W-(CH₂)_{q}- ist, wobei R^{d} gegebenenfalls substituiertes Cycloalkyl, gegebenenfalls substituiertes Aryl, gegebenenfalls substituiertes Heterocyclyl oder gegebenenfalls substituiertes Heteroaryl ist, W eine kovalente Bindung ist und die Summe von p und q 1 bis 3 oder R^{d} H ist, W O oder S ist und die Summe von p und q 2 bis 4 ist.

7. Verbindung nach Anspruch 1, wobei R⁴ C₁₋₆-Alkyl, C₂₋₆-Alkenyl, C₂₋₆-Alkinyl, 3- oder 4-Aryl, Aryl(C₁₋₃-Alkyl)-, Aryl(C₂₋₃-Alkenyl)- oder Aryl(C₂₋₃-Alkinyl)- ist, wobei Aryl gegebenenfalls mit einem oder mehreren Halogenen, C₁₋₆-Alkyl, C₁₋₆-Alkoxy, Trifluormethyl, Hydroxy (C₁₋₆-Alkyl), CN oder C₁₋₆-Acyl substituiert ist.

8. Verbindung nach Anspruch 7, wobei R⁴ 3- oder 4-Phenyl, Naphthyl oder Phenyl (Ethinyl) ist, wobei die Phenyl- oder Naphthylgruppen gegebenenfalls substituiert sind.

9. Verbindung nach Anspruch 1, wobei R⁵ Wasserstoff, Halogen, Methyl oder Methoxy ist.

10. Verbindung nach Anspruch 9, wobei R⁵ Wasserstoff ist.

11. Verbindung nach Anspruch 1, wobei m 0 ist.

12. Verbindung nach Anspruch 1, wobei n 0 ist.

13. Verbindung nach Anspruch 1, wobei die Verbindung eine Verbindung der Formel (II) ist: 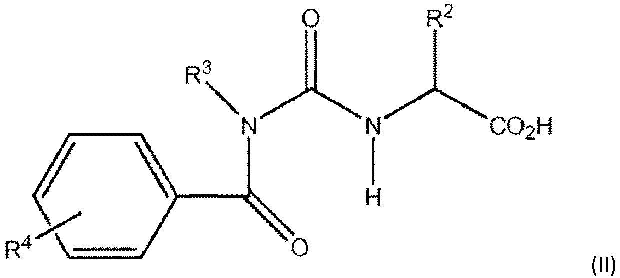 wobei R² und R³ wie für Formel (I) in Anspruch 1 definiert sind und R⁴ C₁₋₆-Alkyl, C₂₋₆-Alkenyl, C₂₋₆-Alkinyl, 3-Phenyl, 3-(2-Naphthyl), 3-(1-Naphthyl), 3-Benzyl, 4-Phenyl, 4-Benzyl, 3- (Phenylethinyl) oder 4- (Phenylethynyl) ist, wobei jede Phenyl-, Naphthyl- oder Benzylgruppe gegebenenfalls mit einem oder mehreren Substituenten ausgewählt aus C₁₋₆-Alkyl, C₂₋₆-Alkenyl, C₂₋₆-Alkinyl, C₁₋₆-Alkoxy, C₂₋₆-Alkenyloxy und Halogen und pharmazeutisch verträgliche Salze oder Prodrugs, ausgewählt aus N-α-Acyloxyamiden, N-(Acyloxyalkoxycarbonyl)-Aminen und α-Acyloxyalkylestern von Alkoholen oder Phenolen substituiert ist; mit der Maßgabe, dass (i) wenn R² C₆H₅-CH₂S-CH₂- und R⁴ 3-Phenyl ist, R³ nicht Ethyl ist und (ii) wenn R² CH₃CH(CH₃)CH₂SCH₂ und R⁴ 3-Phenethynyl ist, R³ nicht n-Propyl ist.

14. Verbindung nach Anspruch 1, wobei die Verbindung eine Verbindung der Formel (IIa) ist: 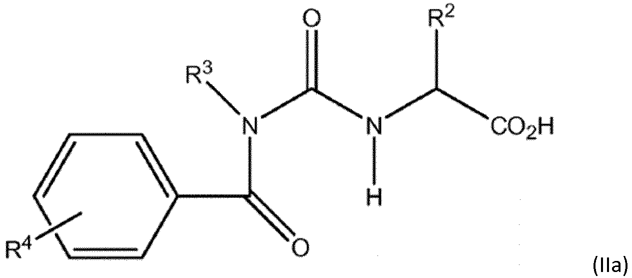
wobei R² wie oben für Formel (I) in Anspruch 1 definiert ist,
R³ aus C₃₋₆-Alkyl, C₂₋₆-Alkenyl, C₂₋₆-Alkinyl, Cycloalkyl, Aryl, Heterocyclyl, Heteroaryl und einer Gruppe
R^{d}-(CH₂)ₚ-W-(CH₂)_{q}-
ausgewählt ist,
wobei W aus einer kovalenten Bindung, O, S oder NR⁶ ausgewählt ist, R^{d} aus H, Cycloalkyl, Cycloalkenyl, Aryl, Heterocyclyl und Heteroaryl ausgewählt ist; p eine ganze Zahl von 1 bis 6 ist, q 0 oder eine ganze Zahl von 1 bis 5 ist, vorausgesetzt, dass die Summe von p und q 1 bis 6 ist;
R⁴ ist C₁₋₆-Alkyl, C₂₋₆-Alkenyl, C₂₋₆-Alkinyl, 3-Phenyl, 3- (2-Naphthyl), 3- (1-Naphthyl), 3-Benzyl, 4-Phenyl, 4-Benzyl, 3-(Phenylethinyl) oder 4-(Phenylethinyl), wobei jede Phenyl-, Naphthyl- oder Benzylgruppe gegebenenfalls substituiert ist mit einem oder mehreren Substituenten, ausgewählt aus C₁₋₆Alkyl, C₂₋₆Alkenyl, C₂₋₆Alkynyl, C₁₋₆Alkoxy, C₂₋₆Alkenyloxy und Halogen, und pharmazeutisch annehmbaren Salzen oder Prodrugs, ausgewählt aus N-α-Acyloxyamiden, N-(Acyloxyalkoxycarbonyl)aminen und α-Acyloxyalkylestern von Alkoholen oder Phenolen; mit der Maßgabe, dass (ii) wenn R² CH₃CH(CH₃)CH₂SCH₂ und R⁴ 3-Phenethinyl ist, R³ nicht n-Propyl ist.

15. Konjugat, umfassend eine Verbindung der Formel (I) 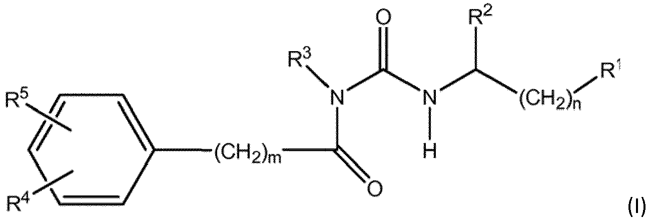
wobei R¹ aus CO₂H oder einer Carbonsäure- oder Carboxylat-Bioisosterie ausgewählt ist, ausgewählt aus Tetrazol, Tetrazolat, Oxadiazol, Aryl oder Alkylacylsulfonamid, Phosphat und Sulfonsäure;
R² aus einer Aminosäureseitenkette ausgewählt ist, ausgewählt aus der Gruppe bestehend aus -CH₃, -(CH₂)₃NHC(=NH)NH₂, -CH₂ CONH₂, -CH₂ CO₂H, -CH₂SH,-(CH₂)₂CONH₂, -(CH₂)₂CO₂H, -CH₂(4-Imidazol), -CH(CH₃)CH₂CH₃, -CH₂CH(CH₃)₂, -(CH₂)₄NH₂,-(CH₂)₂SCH₃, -CH₂Ph, -CH₂0H, -CH(CH₃)OH, -CH₂(3-Indolyl), -CH₂(4-Hydroxyphenyl),-CH(CH₃)₂, -(CH₂)₄NHC(=NH)NH₂, -(CH₂)₂OH, -(CH₂)₂SH, -CH₂CH₂CH₃, -(CH₂)₃CH₃ und (CH2)ᵥC(=NH)NH₂ wobei v eine ganze Zahl von 1 bis 4 ist, wobei gegebenenfalls Carboxy-, Hydroxy-, Thiol- oder Aminogruppen mit geeigneten Carboxy-, Hydroxy-, Thiol- oder Aminoschutzgruppen geschützt sind; und eine Gruppe
R^{a}-(CHR')ₓ-A-(CH₂)_{y}-
wobei A eine kovalente Bindung oder aus O, S, SO, SO2 und NR⁶ ausgewählt ist, R^{a} H, Cycloalkyl, Cycloalkenyl, Aryl, Heterocyclyl, Heteroaryl oder R^{b} ist, wobei R^{b} 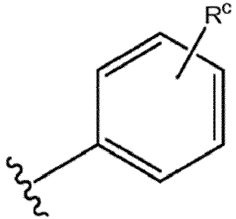 ist und R^{c} aus Heteroaryl, Aryl, Aryl(C₂₋₆-Alkenyl), Aryl(C₂₋₆-Alkinyl), Heteroaryl(C₂₋₆-Alkenyl) und Heteroaryl(C₂₋₆-Alkinyl) ausgewählt ist, R' H oder C₁₋₆ -Alkyl ist, x und y unabhängig 0 oder eine ganze Zahl von 1 bis 6 sind, vorausgesetzt, dass die Summe von x und y 1 bis 6 ist;
R³ aus C₁₋₆-Alkyl, C₂₋₆-Alkenyl, C₂₋₆-Alkinyl, Cycloalkyl, Cycloalkenyl, Aryl, Heterocyclyl, Heteroaryl und einer Gruppe
R^{d}-(CH₂)ₚ-W-(CH₂)_{q}-
ausgewählt ist,
wobei W aus einer kovalenten Bindung, O, S und NR⁶ ausgewählt ist, R^{d} aus H, Cycloalkyl, Cycloalkenyl, Aryl, Heterocyclyl und Heteroaryl ausgewählt ist; p eine ganze Zahl von 1 bis 6 ist, q 0 oder eine ganze Zahl von 1 bis 5 ist, vorausgesetzt, dass die Summe von p und q 1 bis 6 ist;
R⁴ aus C₁₋₆-Alkyl, C₂₋₆-Alkenyl, C₂₋₆-Alkinyl, Cycloalkyl, C₁₋₆-Alkyloxy, C₂₋₆-Alkenyloxy, C₂₋₆-Alkinyloxy, Cycloalkoxy, C₁₋₆-Alkylthio, C₂₋₆-Alkenylthio, C₂₋₆-Alkinylthio, Cycloalkylthio, Halogen, Aryl, Aryl(C₁₋₆-Alkyl)-, Aryl(C₂₋₆-Alkenyl), Aryl(C₂₋₆-Alkinyl), Heterocyclyl, Heterocyclyl (C₁₋₆-Alkyl), Heterocyclyl (C₂₋₆-Alkenyl), Heterocyclyl(C₂₋₆-Alkinyl), Heteroaryl, Heteroaryl(C₁₋₆-Alkyl), Heteroaryl(C₂₋₆-Alkenyl) und Heteroaryl(C₂₋₆-Alkinyl) ausgewählt ist;
R⁵ aus H, Halogen, C₁₋₆-Alkyl, C₂₋₆-Alkenyl, C₂₋₆-Alkinyl, C₁₋₆-Alkyloxy, C₂₋₆-Alkenyloxy, C₂₋₆-Alkinyloxy, C₁₋₆-Alkylthio, C₂₋₆-Alkenylthio, C₂₋₆-Alkylthio, CN und C(R⁷)₃ ausgewählt ist oder wenn R⁵ in der 2- oder 5-Position ist, R⁵ und R³ zusammen einen 5-bis 10-gliedrigen Ring bilden können;
R⁶ aus H, C₁₋₆-Alkyl, C₂₋₆-Alkenyl und C₂₋₆-Alkinyl ausgewählt ist;
Jedes R⁷ unabhängig aus H und Halogen ausgewählt ist;
m 0 oder eine ganze Zahl von 1 bis 6 ist; und
n 0 oder eine ganze Zahl von 1 bis 3 ist;
wobei jedes Alkyl, Alkenyl, Alkinyl, Cycloalkyl, Aryl, Heterocyclyl und Heteroaryl gegebenenfalls mit einem oder mehreren optionalen Substituenten substituiert ist, ausgewählt aus C₁₋₆-Alkyl, C₂₋₆-Alkenyl, C₂₋₆-Alkinyl, C₃₋₆-Cycloalkyl, C₁₋₆-Alkyloxy(CH₂)ₚ-, C₂₋₆-Alkenyloxy(CH₂)ₚ-, C₂₋₆-Alkinyloxy(CH₂)ₚ-, C₃₋₆-Cycloalkoxy (CH₂)ₚ-, C₁₋₆-Alkylthio(CH₂)ₚ-, C₂₋₆-Alkenylthio(CH₂)ₚ-, C₂₋₆-Alkinylthio(CH₂)ₚ-, C₃₋₆-Cycloalkylthio(CH₂)ₚ-, Hydroxy(CH₂)ₚ-,-(CH₂)ₚSH, -(CH₂)ₚCO₂H, -(CH₂)pCO₂C₁₋₆-Alkyl, C₂₋₆-Acyl(CH₂)ₚ-, C₂₋₆-Acyloxy(CH2)ₚ-, C₂₋₆-Alkyl-SO₂(CH₂)ₚ-, C₂₋₆-Alkenyl-SO₂(CH₂)ₚ-, C₂₋₆-Alkinyl-SO₂(CH₂)ₚ-, Aryl-SO₂(CH₂)ₚ-, Heteroaryl-SO₂(CH₂)ₚ-, Heterocyclyl-SO₂(CH₂)ₚ-, -(CH₂)ₚNH₂, -(CH₂)ₚNH(C₁₋₆-Alkyl), (CH₂)ₚN(C₁₋₆ -Alkyl)₂, -(CH₂)ₚNH(Phenyl), -(CH₂)ₚN(Phenyl)₂, -(CH₂)ₚNH(Acyl),-(CH₂)ₚN(Acyl)(Phenyl), -(CH₂)ₚNH-(CH₂)ₚ-S-Aryl, -(CH₂)ₚN=NHC(O)NH₂, -(CH₂)ₚC(R⁷)₃,-(CH₂)ₚOC(R⁷)₃, -(CH₂)ₚSC(R⁷)₃, -(CH₂)ₚCN, -(CH₂)pNO₂, -(CH₂)ₚHalogen, -(CH₂)ₚHeterocyclyl, Heterocyclyloxy(CH₂)ₚ-, -(CH₂)ₚHeteroaryl, Heteroaryloxy(CH 2)ₚ-, -(CH₂)ₚAryl,-(CH₂)ₚC(O)Aryl und Aryloxy (CH₂)ₚ-, wobei p 0 oder eine ganze Zahl von 1 bis 6 ist und jedes R⁷ unabhängig aus Wasserstoff und Halogen ausgewählt ist;
und pharmazeutisch verträgliche Salze und Prodrugs, aus N-α-Acyloxyamiden, N-(Acyloxyalkoxycarbonyl)-Aminen und α-Acyloxyalkylestern von Alkoholen oder Phenolen ausgewählt ist; mit der Maßgabe, dass (i) wenn R¹ COOH ist, R² C₆H₅-CH₂S-CH₂- ist, R⁴ 3-C₆H₅ ist und R⁵ H ist, R³ nicht CH₃CH₂- ist und (ii) wenn R² CH₃CH (CH₃)CH₂SCH₂ ist, R⁴ 3-Phenethynyl ist, R¹ CO₂H ist, m und n 0 sind und R⁵ H ist, R³ nicht n-Propyl ist,
und eine zell-orientierte Hälfte
wobei die zell-orientierte Hälfte aus einem Hormon, einem Zytokin oder einem antigenbindenden Molekül ausgewählt ist, wobei das antigenbindende Molekül ausgewählt ist aus einem Immunglobulin oder Immunglobulinfragment, wie einem polyklonalen Antikörper oder einem monoklonalen Antikörper.

16. Konjugat nach Anspruch 15, wobei das Hormon luteinisierendes Hormon freisetzendes Hormon ist.

17. Konjugat nach Anspruch 15, wobei das Zytokin aus VEGF und EGF ausgewählt ist.

18. Konjugat nach Anspruch 15, wobei der Antikörper ausgewählt ist aus CD19, CD20, CD22, CD79a, CD2, CD3, CD7, CDS, CD13, CD33, CD138 und Antikörpern, die gegen Erb1-, Erb2-, Erb3- oder Erb4-Rezeptoren orientiert sind.

19. Konjugat nach Anspruch 18, wobei der Antikörper ausgewählt ist aus CD19, CD20, CD22 und CD79a.

20. Pharmazeutische Zusammensetzung, umfassend eine Verbindung der Formel (I) nach Anspruch 15 oder ein Konjugat nach Anspruch 15 zusammen mit einem oder mehreren pharmazeutisch verträglichen Trägern und gegebenenfalls anderen therapeutischen und/oder prophylaktischen Bestandteilen.

21. Verfahren zur Regulierung des Absterbens einer Zelle, wobei die Zelle ex vivo lokalisiert ist, umfassend das Kontaktieren der Zelle mit einer wirksamen Menge einer Verbindung der Formel (I), wie in Anspruch 15 definiert, oder eines Konjugats nach Anspruch 15.

22. Verfahren zum Induzieren von Apoptose in unerwünschten oder beschädigten Zellen, wobei die unerwünschten oder beschädigten Zellen ex vivo lokalisiert sind, umfassend das Kontaktieren der beschädigten oder unerwünschten Zellen mit einer wirksamen Menge einer Verbindung der Formel (I) wie in Anspruch 15 definiert, oder eines Konjugats nach Anspruch 15.

23. Verwendung einer Verbindung der Formel (I), wie in Anspruch 15 definiert, oder eines Konjugats von Anspruch 15, bei der Herstellung eines Medikaments für die Behandlung und/oder Prophylaxe einer Pro-Überlebens-Bcl-2-Familienmitglied-vermittelten Krankheit oder Zustand, wobei die Krankheit oder der Zustand ein entzündlicher Zustand, ein Krebs, eine Autoimmunerkrankung oder eine Gewebehypertrophie ist.

24. Verwendung einer Verbindung der Formel (I), wie in Anspruch 15 definiert, oder eines Konjugats von Anspruch 15, bei der Herstellung eines Medikaments zur Behandlung und/oder Prophylaxe einer Krankheit oder eines Zustands, die durch die unangemessene Persistenz oder Proliferation von unerwünschten oder beschädigten Zellen gekennzeichnet sind.

25. Verwendung nach Anspruch 24, wobei die Krankheit oder der Zustand B-Zell-Non-Hodgkin-Lymphom, B-Zell-akute lymphoblastische Leukämie, rheumatoide Arthritis, systemischer Lupus erythematodes und verwandte Arthropathien, T-Zell-akute lymphoblastische Leukämie, T-Zell-Non-Hodgkin-Lymphom, Graft vs Host-Krankheit, akute myeloische Leukämie, chronische myeloische Leukämie, chronische myelomonozytäre Leukämie, multiples Myelom und Krebs ist.

26. Verwendung nach Anspruch 25, wobei der Krebs Eierstockkrebs, Brustkrebs oder Prostatakrebs ist.

27. Verwendung einer Verbindung der Formel (I) nach Anspruch 15 oder eines Konjugats nach Anspruch 15 zur Herstellung eines Medikaments zur Regulierung des Absterbens einer Zelle oder zur Induktion von Apoptose in unerwünschten oder beschädigten Zellen.

## Revendications

1. Composé de formule (I): 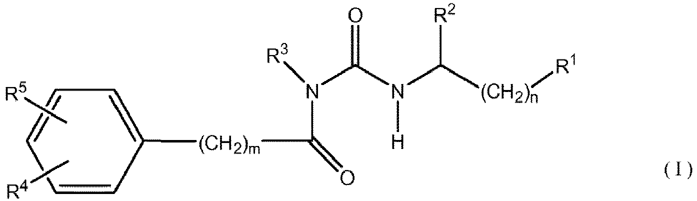 dans lequel
R¹ est sélectionné parmi CO₂H ou un bioisostère d'acide carboxylique ou de carboxylate, sélectionné parmi un tétrazole, un tétrazolate, un oxadiazole, un aryle ou alkyle acylsulfonamide, un phosphate et un acide sulfonique ;
R² est sélectionné parmi une chaîne latérale d'acides aminés sélectionnée dans le groupe constitué de -CH₃, -(CH₂)₃NHC(=NH)NH₂, -CH₂CONH₂, -CH₂CO₂H, CH₂SH, -(CH₂)₂CONH₂, -(CH₂)₂CO₂H, -CH₂(4-imidazole), -CH(CH₃)CH₂CH₃,-CH₂CH(CH₃)₂, -(CH₂)₄NH₂, -(CH₂)₂SCH₃, -CH₂Ph, -CH₂OH, -CH(CH₃)OH, -CH₂(3-indolyle),-CH₂(4-hydroxyphényle), -CH(CH₃)₂, -(CH₂)₄NHC(=NH)NH₂, -(CH₂)₂OH, -(CH₂)₂SH,-CH₂CH₂CH₃, -(CH₂)₃CH₃, et (CH₂)ᵥC(=NH)NH₂ dans lequel v est un nombre entier de 1 à 4, facultativement dans lequel les groupes carboxy, hydroxy, thiol ou amino sont protégés par des groupes protecteurs carboxy, hydroxy, thiol ou amino appropriés ; et un groupe
R^{a}-(CHR')ₓ-A-(CH₂)_{y}-
dans lequel A est une liaison covalente ou est sélectionné parmi O, S, SO, SO₂ et NR⁶, R^{a} est H, un cycloalkyle, un cycloalcényle, un aryle, un hétérocyclyle, un hétéroaryle ou R^{b} dans lequel R^{b} est 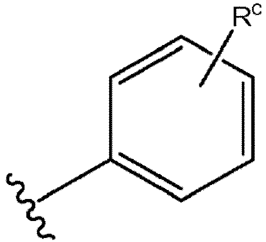 et R^{c} est sélectionné parmi un hétéroaryle, un aryle, un aryl(alcényle en C₂₋₆), un aryl(alcynyle en C₂₋₆), un hétéroaryl(alcényle C₂₋₆) et un hétéroaryl(alcynyle en C₂₋₆), R' est H ou un alkyle en C₁₋₆, x et y sont indépendamment 0 ou un nombre entier de 1 à 6 à condition que la somme de x et y soit de 1 à 6; R³ est sélectionné parmi un alkyle en C₁₋₆, un alcényle en C₂₋₆, un alcynyle en C₂₋₆, un cycloalkyle, un cycloalcényle, un aryle, un hétérocyclyle, un hétéroaryle et un groupe
R^{d}-(CH₂)ₚ-W-(CH₂)_{q}-
dans lequel W est sélectionné parmi une liaison covalente, O, S et NR⁶, R^{d} est sélectionné parmi H, un cycloalkyle, un cycloalcényle, un aryle, un hétérocyclyle et un hétéroaryle; p est un nombre entier de 1 à 6, q est 0 ou un nombre entier de 1 à 5 à condition que la somme de p et q soit de 1 à 6;
R⁴ est sélectionné parmi un alkyle en C₁₋₆, un alcényle en C₂₋₆, un alcynyle en C₂₋₆, un cycloalkyle, un alkyloxy en C₁₋₆, un alcényloxy en C₂₋₆, un alcynyloxy en C₂₋₆, un cycloalcoxy, un alkylthio en C₁₋₆, un alcénylthio en C₂₋₆, un alcynylthio en C₂₋₆, un cycloalkylthio, un halogène, un aryle, un aryl(alkyle en C₁₋₆)-, un aryl(alcényle en C₂₋₆), un aryl(alcynyle en C₂₋₆), un hétérocyclyle, un hétérocyclyl(alkyle en C₁₋₆)-, un hétérocyclyl(alcényle en C₂₋₆), un hétérocyclyl(alcynyle en C₂₋₆), un hétéroaryle, un hétéroaryl(alkyle en C₁₋₆)-, un hétéroaryl(alcényle en C₂₋₆) et un hétéroaryl(alcynyle en C₂₋₆) ;
R⁵ est sélectionné parmi H, un halogène, un alkyle en C₁₋₆, un alcényle en C₂₋₆, un alcynyle en C₂₋₆, un alkyloxy en C₁₋₆, un alcényloxy en C₂₋₆, un alcynyloxy en C₂₋₆, un alkylethio en C₁₋₆, un alcénylthio en C₂₋₆, un alcynylthio en C₂₋₆, CN et C(R⁷)₃ ou lorsque R⁵ est en position 2- ou 5-, R⁵ et R³ pris conjointement peuvent former un cycle à 5 à 10 chaînons ;
R⁶ est sélectionné parmi H, un alkyle en C₁₋₆, un alcényle en C₂₋₆ et un alcynyle en C₂₋₆;
chaque R⁷ est indépendamment sélectionné parmi H et un halogène;
m est 0 ou un nombre entier de 1 à 6 ; et
n est 0 ou un nombre entier de 1 à 3 ;
dans lequel chaque alkyle, alcényle, alcynyle, cycloalkyle, aryle, hétérocyclyle et hétéroaryle est facultativement substitué par un ou plusieurs substituants facultatifs sélectionnés parmi un alkyle en C₁₋₆, un alcényle en C₂₋₆, un alcynyle en C₂₋₆, un cycloalkyle en C₃₋₆, un alkyloxy en C₁₋₆ (CH₂)ₚ-, un alcényloxy en C₂₋₆ (CH₂)ₚ-, un alcynyloxy en C₂₋₆ (CH₂)ₚ-, un cycloalcoxy en C₃₋₆ (CH₂)ₚ-, un alkylthio en C₁₋₆ (CH₂)ₚ-, un alcénylthio en C₂₋₆ (CH₂)ₚ-, un alcynylthio en C₂₋₆ (CH₂)ₚ-, un cycloalkylthio en C₃₋₆ (CH₂)ₚ-, un hydroxy(CH₂)ₚ-, un -(CH₂)ₚSH, un -(CH₂)_{P}CO₂H, un -(CH₂)ₚCO₂(alkyle en C₁₋₆), un acyle en C₂₋₆ (CH₂)ₚ-, un acyloxy en C₁₋₆ (CH₂)ₚ-, un alkyle en C₂₋₆ SO₂(CH₂)ₚ-, un alcényle en C₂₋₆ SO₂(CH₂)ₚ-, un alcynyle en C₂₋₆ SO₂(CH₂)ₚ-, un aryl-SO₂(CH₂)ₚ-, un hétéroarylSO₂(CH₂)ₚ-, un hétérocyclylSO₂(CH₂)ₚ-, un -(CH₂)ₚNH₂, un -(CH₂)ₚNH(alkyle en C₁₋₆), un -(CH₂)ₚN(alkyle en C₁₋₆)₂, un -(CH₂)ₚNH(phényle), un -(CH₂)ₚN(phényle)₂, un -(CH₂)ₚNH(acyle), un -(CH₂)ₚN(acyl)(phényle), un -(CH₂)ₚNH-(CH₂)ₚ-S-aryle, un -(CH₂)ₚN=NHC(O)NH₂, un -(CH₂)_{P}C(R⁷)₃, un -(CH₂)ₚOC(R⁷)₃, un -(CH₂)ₚSC(R⁷)₃, un -(CH₂)ₚCN, -(CH₂)ₚNO₂, un -(CH₂)ₚhalogène, un -(CH₂)ₚhétérocyclyle, un hétérocyclyloxy(CH₂)ₚ-, un -(CH₂)ₚhétéroaryle, un hétéroaryloxy(CH₂)ₚ-, un -(CH₂)ₚaryle, un -(CH₂)ₚC(O)aryle et un aryloxy(CH₂)ₚ-, dans lesquels p est 0 ou un nombre entier de 1 à 6 et chaque R⁷ est indépendamment sélectionné parmi un hydrogène et un halogène;
et des sels et promédicaments pharmaceutiquement acceptables sélectionnés parmi les N-α-acyloxy amides, les N-(acyloxyalcoxycarbonyl)amines et les α-acyloxyalkyle esters d'alcools ou de phénols ; à condition que (i) lorsque R¹ est COOH, R² est C₆H₅-CH₂S-CH₂-, R⁴ est 3-C₆H₅ et R⁵ est H, R³ ne soit pas CH₃CH₂- et (ii) lorsque R² est CH₃CH(CH₃)CH₂SCH₂, R⁴ est un 3-phénéthynyle, R¹ est CO₂H m et n sont 0 et R⁵ est H, R³ ne soit pas un n-propyle.

2. Composé selon la revendication 1, dans lequel R¹ est CO₂H un tétrazole, un tétrazolate ou un benzène acylsulfonamide.

3. Composé selon la revendication 2, dans lequel R¹ est CO₂H.

4. Composé selon la revendication 1, dans lequel R² est R^{a}-(CHR')ₓ-A-(CH₂)_{y}- dans lequel R^{a} est H, un cycloalkyle facultativement substitué ou un aryle facultativement substitué, x est 0 ou 1 à 4, R' est H ou un alkyle en C₁₋₃, A est O, S ou SO et y est 1 à 3.

5. Composé selon la revendication 1, dans lequel R² est R^{a}-(CHR')ₓ-A-(CH₂)_{y}- dans lequel R^{a} est un aryle facultativement substitué ou un hétéroaryle facultativement substitué, R' est H, la somme de x et y est de 1 à 4 et A est une liaison covalente.

6. Composé selon la revendication 1, dans lequel R³ est un alkyle en C₁₋₆, un cycloalkyle facultativement substitué ou un groupe R^{d}-(CH₂)ₚ-W-(CH₂)_{q}- dans lequel R^{d} est un cycloalkyle facultativement substitué, un aryle facultativement substitué, un hétérocyclyle facultativement substitué ou un hétéroaryle facultativement substitué, W est une liaison covalente et la somme de p et q est de 1 à 3, ou R^{d} est H, W est O ou S et la somme de p et q est de 2 à 4.

7. Composé selon la revendication 1, dans lequel R⁴ est un alkyle en C₁₋₆, un alcényle en C₁₋₆, un alcynyle en C₂₋₆, un 3- ou 4-aryle, un aryl(alkyle en C₁₋₃)-, un aryl(alcényle en C₂₋₃)- ou un aryl(alcynyle en C₂₋₃)- dans lequel l'aryle est facultativement substitué par un ou plusieurs halogène, alkyle en C₁₋₆, alcoxy en C₁₋₆, trifluorométhyle, hydroxy(alkyle en C₁₋₆), CN ou acyle en C₁₋₆.

8. Composé selon la revendication 7, dans lequel R⁴ est un 3- ou 4-phényle, un naphtyle ou un phényl(éthynyle) dans lesquels les groupes phényle ou naphtyle sont facultativement substitués.

9. Composé selon la revendication 1, dans lequel R⁵ est un hydrogène, un halogène, un méthyle ou un méthoxy.

10. Composé selon la revendication 9, dans lequel R⁵ est un hydrogène.

11. Composé selon la revendication 1, dans lequel m est 0.

12. Composé selon la revendication 1, dans lequel n est 0.

13. Composé selon la revendication 1, dans lequel ledit composé est un composé de formule (II) 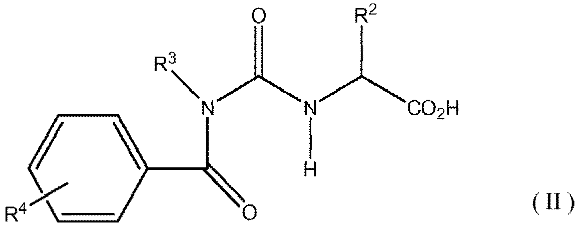 dans lequel R² et R³ sont tels que définis pour la formule (I) dans la revendication 1 et R⁴ est un alkyle en C₁₋₆, un alcényle en C₂₋₆, un alcynyle en C₂₋₆, un 3-phényle, un 3-(2-naphtyle), un 3-(1-naphtyle), un 3-benzyle, un 4-phényle, un 4-benzyle, un 3-(phényléthynyle) ou un 4-(phényléthynyle), dans lesquels chaque groupe phényle, naphtyle ou benzyle est facultativement substitué par un ou plusieurs substituants sélectionnés parmi un alkyle en C₁₋₆, un alcényle en C₂₋₆, un alcynyle en C₂₋₆, un alcoxy en C₁₋₆, un alcényloxy en C₂₋₆ et un halogène, et des sels ou promédicaments pharmaceutiquement acceptables sélectionnés parmi les N-α-acyloxy amides, les N-(acyloxyalcoxycarbonyl)amines et les α-acyloxyalkyl esters d'alcools ou de phénols; à condition que (i) lorsque R² est C₆H₅-CH₂S-CH₂- et R⁴ est un 3-phényle, R³ ne soit pas un éthyle et (ii) lorsque R² est CH₃CH(CH₃)CH₂SCH₂ et R⁴ est un 3-phénéthynyle, R³ ne soit pas un n-propyle.

14. Composé selon la revendication 1, dans lequel ledit composé est un composé de formule (IIa) 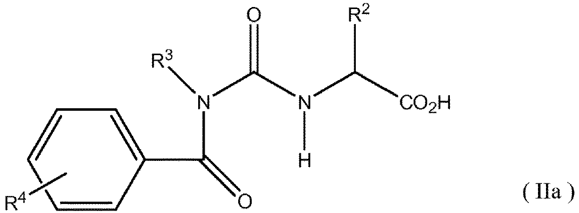
dans lequel R² est tel que défini ci-dessus pour la formule (I) dans la revendication 1,
R³ est sélectionné parmi un alkyle en C₃₋₆, un alcényle en C₂₋₆, un alcynyle en C₂₋₆, un cycloalkyle, un aryle, un hétérocyclyle, un hétéroaryle et un groupe
R^{d}-(CH₂)ₚ-W-(CH₂)_{q}-
dans lequel W est sélectionné parmi une liaison covalente, O, S ou NR⁶, R^{d} est sélectionné parmi H, un cycloalkyle, un cycloalcényle, un aryle, un hétérocyclyle et un hétéroaryle; p est un nombre entier de 1 à 6, q est 0 ou un nombre entier de 1 à 5 à condition que la somme de p et q soit de 1 à 6;
R⁴ est un alkyle en C₁₋₆, un alcényle en C₂₋₆, un alcynyle en C₂₋₆, un 3-phényle, un 3-(2-naphtyle), un 3-(1-naphtyle), un 3-benzyle, un 4-phényle, un 4-benzyle, un 3-(phényléthynyle) ou un 4-(phényléthynyle), dans lesquels chaque groupe phényle, naphtyle ou benzyle est facultativement substitué par un ou plusieurs substituants sélectionnés parmi un alkyle en C₁₋₆, un alcényle en C₂₋₆, un alcynyle en C₂₋₆, un alcoxy en C₁₋₆, un alcényloxy en C₂₋₆ et un halogène et des sels ou promédicaments pharmaceutiquement acceptables sélectionnés parmi les N-α-acyloxy amides, les N-(acyloxyalcoxycarbonyl)amines et les α-acyloxyalkyl esters d'alcools ou de phénols; à condition que (ii) lorsque R² est CH₃CH(CH₃)CH₂SCH₂ et R⁴ est un 3-phénéthynyle, R³ ne soit pas un n-propyle.

15. Conjugué comprenant un composé de formule (I) 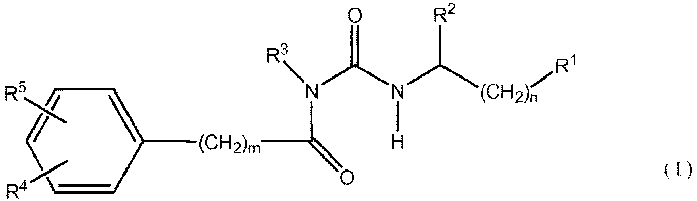 dans lequel
R¹ est sélectionné parmi CO₂H ou un bioisostère d'acide carboxylique ou de carboxylate, sélectionné parmi un tétrazole, un tétrazolate, un oxadiazole, un aryle ou un alkyle acylsulfonamide, un phosphate et un acide sulfonique;
R² est sélectionné parmi une chaîne latérale d'acides aminés sélectionnée dans le groupe constitué de -CH₃, -(CH₂)₃NHC(=NH)NH₂, -CH₂CONH₂, -CH₂CO₂H, - CH₂SH, -(CH₂)₂CONH₂, -(CH₂)₂CO₂H, -CH₂(4-imidazole), -CH(CH₃)CH₂CH₃, - CH₂CH(CH₃)₂, -(CH₂)₄NH₂, -(CH₂)₂SCH₃, -CH₂Ph, -CH₂OH, -CH(CH₃)OH, -CH₂(3-indolyle), - CH₂(4-hydroxyphényle), -CH(CH₃)₂, -(CH₂)₄NHC(=NH)NH₂, -(CH₂)₂OH, -(CH₂)₂SH, - CH₂CH₂CH₃, -(CH₂)₃CH₃, et (CH₂)ᵥC(=NH)NH₂ ; dans lesquels v est un nombre entier de 1 à 4, facultativement dans lesquels les groupes carboxy, hydroxy, thiol ou amino sont protégés par des groupes protecteurs carboxy, hydroxy, thiol ou amino appropriés; et un cycloalkyle, un cycloalcényle, un aryle, un hétérocyclyle, un hétéroaryle et un groupe
R^{a}-(CHR')ₓ-A-(CH₂)_{y}-
dans lequel A est une liaison covalente ou est sélectionné parmi O, S, SO, SO₂ et NR⁶, R^{a} est H, un cycloalkyle, un cycloalcényle, un aryle, un hétérocyclyle, un hétéroaryle ou R^{b} dans lequel R^{b} est 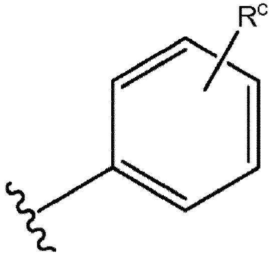 et R^{c} est sélectionné parmi un hétéroaryle, un aryle, un aryl(alcényle en C₂₋₆), un aryl(alcynyle en C₂₋₆), un hétéroaryl(alcényle C₂₋₆) et un hétéroaryl(alcynyle en C₂₋₆), R' est H ou un alkyle en C₁₋₆, x et y sont indépendamment 0 ou un nombre entier de 1 à 6 à condition que la somme de x et y soit de 1 à 6;
R³ est sélectionné parmi un alkyle en C₁₋₆, un alcényle en C₂₋₆, un alcynyle en C₂₋₆, un cycloalkyle, un cycloalcényle, un aryle, un hétérocyclyle, un hétéroaryle et un groupe
R^{d}-(CH₂)ₚ-W-(CH₂)_{q}-
dans lequel W est sélectionné parmi une liaison covalente, O, S et NR⁶, R^{d} est sélectionné parmi H, un cycloalkyle, un cycloalcényle, un aryle, un hétérocyclyle et un hétéroaryle; p est un nombre entier de 1 à 6, q est 0 ou un nombre entier de 1 à 5 à condition que la somme de p et q soit de 1 à 6;
R⁴ est sélectionné parmi un alkyle en C₁₋₆, un alcényle en C₂₋₆, un alcynyle en C₂₋₆, un cycloalkyle, un alkyloxy en C₁₋₆, un alcényloxy en C₂₋₆, un alcynyloxy en C₂₋₆, un cycloalcoxy, un alkylthio en C₁₋₆, un alcénylthio en C₂₋₆, un alcynylthio en C₂₋₆, un cycloalkylthio, un halogène, un aryle, un aryl(alkyle en C₁₋₆)-, un aryl(alcényle en C₂₋₆), un aryl(alcynyle en C₂₋₆), un hétérocyclyle, un hétérocyclyl(alkyle en C₁₋₆)-, un hétérocyclyl(alcényle en C₂₋₆), un hétérocyclyl(alcynyle en C₂₋₆), un hétéroaryle, un hétéroaryl(alkyle en C₁₋₆)-, un hétéroaryl(alcényle en C₂₋₆) et un hétéroaryl(alcynyle en C₂₋₆);
R⁵ est sélectionné parmi H, un halogène, un alkyle en C₁₋₆, un alcényle en C₂₋₆, un alcynyle en C₂₋₆, un alkyloxy en C₁₋₆, un alcényloxy en C₂₋₆, un alcynyloxy en C₂₋₆, un alkylthio en C₁₋₆, un alcénylthio en C₂₋₆, un alcynylthio en C₂₋₆, CN et C(R⁷)₃ ou lorsque R⁵ est en position 2- ou 5-, R⁵ et R³ pris conjointement peuvent former un cycle à 5 à 10 chaînons ;
R⁶ est sélectionné parmi H, un alkyle en C₁₋₆, un alcényle en C₂₋₆ et un alcynyle en C₂₋₆ ;
chaque R⁷ est indépendamment sélectionné parmi H et un halogène;
m est 0 ou un nombre entier de 1 à 6; et
n est 0 ou un nombre entier de 1 à 3 ;
dans lequel chaque alkyle, alcényle, alcynyle, cycloalkyle, aryle, hétérocyclyle et hétéroaryle est facultativement substitué par un ou plusieurs substituants facultatifs sélectionnés parmi un alkyle en C₁₋₆, un alcényle en C₂₋₆, un alcynyle en C₂₋₆, un cycloalkyle en C₃₋₆, un alkyloxy en C₁₋₆ (CH₂)ₚ-, un alcényloxy en C₂₋₆ (CH₂)ₚ-, un alcynyloxy en C₂₋₆ (CH₂)ₚ-, un cycloalcoxy en C₃₋₆ (CH₂)ₚ-, un alkylthio en C₁₋₆ (CH₂)ₚ-, un alcénylthio en C₂₋₆ (CH₂)ₚ-, un alcynylthio en C₂₋₆ (CH₂)ₚ-, un cycloalkylthio en C₃₋₆ (CH₂)ₚ-, un hydroxy(CH₂)ₚ-, un -(CH₂)ₚSH, un -(CH₂)ₚCO₂H, un -(CH₂)ₚCO₂(alkyle en C₁₋₆), un acyle en C₂₋₆ (CH₂)ₚ-, un acyloxy en C₁₋₆ (CH₂)ₚ-, un alkyle en C₂₋₆ SO₂(CH₂)ₚ-, un alcényle en C₂₋₆ SO₂(CH₂)ₚ-, un alcynyle en C₂₋₆ SO₂(CH₂)ₚ-, un aryl-SO₂(CH₂)ₚ-, un hétéroarylSO₂(CH₂)ₚ-, un hétérocyclylSO₂(CH₂)ₚ-, un -(CH₂)ₚNH₂, un -(CH₂)ₚNH(alkyle en C₁₋₆), un -(CH₂)ₚN(alkyle en C₁₋₆)₂, un -(CH₂)ₚNH(phényle), un -(CH₂)ₚN(phényl)₂, un -(CH₂)ₚNH(acyle), un -(CH₂)ₚN(acyl)(phényle), un -(CH₂)ₚNH-(CH₂)ₚ-S-aryle, un -(CH₂)ₚN=NHC(O)NH₂, un -(CH₂)_{P}C(R⁷)₃, un -(CH₂)_{P}OC(R⁷)₃, un -(CH₂)ₚSC(R⁷)₃, un -(CH₂)ₚCN, un -(CH₂)ₚNO₂, un -(CH₂)ₚhalogène, un -(CH₂)ₚhétérocyclyle, un hétérocyclyloxy(CH₂)ₚ-, un -(CH₂)ₚhétéroaryle, un hétéroaryloxy(CH₂)ₚ-, un -(CH₂)ₚaryle, un -(CH₂)ₚC(O)aryle et un aryloxy(CH₂)ₚ-, dans lesquels p est 0 ou un nombre entier de 1 à 6 et chaque R⁷ est indépendamment sélectionné parmi un hydrogène et un halogène;
et des sels et promédicaments pharmaceutiquement acceptables sélectionnés parmi les N-α-acyloxy amides, les N-(acyloxyalcoxycarbonyl)amines et les α-acyloxyalkyl esters de phénols et d'alcools ; à condition que (i) lorsque R¹ est COOH, R² est C₆H₅-CH₂S-CH₂-, R⁴ est 3-C₆H₅ et R⁵ est H, R³ ne soit pas CH₃CH₂- et (ii) lorsque R² est CH₃CH(CH₃)CH₂SCH₂, R⁴ est un 3-phénéthynyle, R¹ est CO₂H m et n sont 0 et R⁵ est H, R³ ne soit pas un n-propyle,
et une fraction de ciblage cellulaire,
dans lequel la fraction de ciblage cellulaire est sélectionnée parmi une hormone, une cytokine ou une molécule de liaison à un antigène, dans lequel ladite molécule de liaison à un antigène est sélectionnée parmi une immunoglobuline ou un fragment d'immunoglobuline, par exemple un anticorps polyclonal ou un anticorps monoclonal.

16. Conjugué selon la revendication 15, dans lequel l'hormone est l'hormone de libération des gonadotrophines.

17. Conjugué selon la revendication 15, dans lequel la cytokine est sélectionnée parmi le VEGF et l'EGF.

18. Conjugué selon la revendication 15, dans lequel l'anticorps est sélectionné parmi CD19, CD20, CD22, CD79a, CD2, CD3, CD7, CD5, CD13, CD33, CD138 et les anticorps ciblant les récepteurs Erb1, Erb2, Erb3 ou Erb4.

19. Conjugué selon la revendication 18, dans lequel l'anticorps est sélectionné parmi CD19, CD20, CD22 et CD79a.

20. Composition pharmaceutique comprenant un composé de formule (I) tel que défini dans la revendication 15, ou un conjugué selon la revendication 15, conjointement à un ou plusieurs vecteurs pharmaceutiquement acceptables et facultativement, d'autres ingrédients thérapeutiques et/ou prophylactiques.

21. Procédé de régulation de la mort d'une cellule, dans lequel la cellule se trouve ex *vivo,* comprenant la mise en contact de la cellule avec une quantité thérapeutiquement efficace d'un composé de formule (I) selon la revendication 15, ou d'un conjugué selon la revendication 15.

22. Procédé d'induction de l'apoptose dans des cellules indésirables ou endommagées, dans lequel les cellules indésirables ou endommagées se trouvent ex *vivo,* comprenant la mise en contact des cellules endommagées ou indésirables avec une quantité efficace d'un composé de formule (I) tel que défini dans la revendication 15, ou d'un conjugué selon la revendication 15.

23. Utilisation d'un composé de formule (I) tel que défini dans la revendication 15, ou d'un conjugué selon la revendication 15, dans la fabrication d'un médicament pour le traitement et/ou la prophylaxie d'une maladie ou affection induite par médiation d'un membre de la famille Bcl-2 pro-survie, dans laquelle la maladie ou l'affection est une affection inflammatoire, un cancer, un trouble auto-immun ou une hypertrophie tissulaire.

24. Utilisation d'un composé de formule (I) tel que défini dans la revendication 15, ou d'un conjugué selon la revendication 15, dans la fabrication d'un médicament pour le traitement et/ou la prophylaxie d'une maladie ou d'une affection **caractérisée par** la persistance ou la prolifération inappropriée des cellules indésirables ou endommagées.

25. Utilisation selon la revendication 24, dans lequel la maladie ou l'affection est un lymphome non hodgkinien à lymphocytes B, une leucémie lymphoblastique aiguë à lymphocytes B, la polyarthrite rhumatoïde, le lupus érythémateux systémique et les arthropathies liées, une leucémie lymphoblastique aiguë à lymphocytes T, un lymphome non hodgkinien à lymphocytes T, la maladie du greffon contre l'hôte, la leucémie myéloïde aiguë, la leucémie myéloïde chronique, la leucémie myélomonocytaire chronique, le myélome multiple et le cancer.

26. Utilisation selon la revendication 25, dans laquelle le cancer est le cancer des ovaires, le cancer du sein ou le cancer de la prostate.

27. Utilisation d'un composé de formule (I) tel que défini dans la revendication 15, ou d'un conjugué selon la revendication 15, dans la fabrication d'un médicament destiné à réguler la mort d'une cellule, ou à induire l'apoptose dans des cellules indésirables ou endommagées.
